(19) Europäisches Patentamt
European Patent Office
Office européen des brevets

(11) EP 2 652 129 B1

(12) **EUROPÄISCHE PATENTSCHRIFT**

(45) Veröffentlichungstag und Bekanntmachung des Hinweises auf die Patenterteilung:
**21.11.2018 Patentblatt 2018/47**

(21) Anmeldenummer: **11799094.5**

(22) Anmeldetag: **16.12.2011**

(51) Int Cl.:
***C12N 9/04*** *(2006.01)* ***C12P 33/02*** *(2006.01)*
***C12P 33/06*** *(2006.01)*

(86) Internationale Anmeldenummer:
**PCT/EP2011/073141**

(87) Internationale Veröffentlichungsnummer:
**WO 2012/080504 (21.06.2012 Gazette 2012/25)**

(54) **NEUARTIGE 7 BETA-HYDROXYSTEROID DEHYDROGENASE-MUTANTEN UND VERFAHREN ZUR HERSTELLUNG VON URSODESOXYCHOLSÄURE**

NOVEL 7 BETA-HYDROXYSTEROID DEHYDROGENASE MUTANTS AND PROCESS FOR THE PREPARATION OF URSODEOXYCHOLIC ACID

NOUVEAUX MUTANTS DE 7 BÊTA-HYDROXYSTÉROÏDE DÉSHYDROGÉNASE ET PROCÉDÉ DE PRODUCTION D’ACIDE URSODÉSOXYCHOLIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.12.2010 EP 10015726**

(43) Veröffentlichungstag der Anmeldung:
**23.10.2013 Patentblatt 2013/43**

(73) Patentinhaber: **PharmaZell GmbH**
**83064 Raubling (DE)**

(72) Erfinder:
- **WEUSTER-BOTZ, Dirk**
  **80634 München (DE)**
- **BRAUN, Michael**
  **97980 Bad Mergentheim-Edelfingen (DE)**
- **AIGNER, Arno**
  **83104 Tuntenhausen (DE)**
- **SUN, Boqiao**
  **80336 München (DE)**
- **KANTZOW, Christina**
  **81545 München (DE)**
- **BRESCH, Sven**
  **80807 München (DE)**
- **BAKONYI, Daniel**
  **50969 Köln (DE)**
- **HUMMEL, Werner**
  **52445 Titz (DE)**

(74) Vertreter: **Reitstötter Kinzebach**
**Patentanwälte**
**Sternwartstrasse 4**
**81679 München (DE)**

(56) Entgegenhaltungen:
- **DATABASE UniProt [Online] 18. Mai 2010 (2010-05-18), "SubName: Full=Short-chain dehydrogenases of various substrate specificities;", XP002671823, gefunden im EBI accession no. UNIPROT:D4M4P2 Database accession no. D4M4P2**
- **CARREA GIACOMO ET AL: "Enzymatic synthesis of 12-ketoursodeoxycholic acid from dehydrocholic acid in a membrane reactor", BIOTECHNOLOGY LETTERS, KEW, SURREY, GB, Bd. 14, Nr. 12, 1. Dezember 1992 (1992-12-01), Seiten 1131-1135, XP002579160, ISSN: 0141-5492**
- **MACDONALD I A ET AL: "Epimerization versus dehydroxylation of the 7alpha-hydroxyl- group of primary bile acids: Competitive studies with Clostridium absonum and 7alpha-dehydroxylating bacteria (Eubacterium SP.)", JOURNAL OF STEROID BIOCHEMISTRY, PERGAMON PRESS PLC, GB, Bd. 17, Nr. 3, 1. September 1982 (1982-09-01), Seiten 287-293, XP025877762, ISSN: 0022-4731, DOI: 10.1016/0022-4731(82)90203-5 [gefunden am 1982-09-01]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 652 129 B1

SEQUENCE LISTING

<110> PharmaZell GmbH

<120> Neuartige 7beta-Hydroxysteroid Dehydrogenase-Mutanten und Verfahren zur Herstellung von Ursodesoxycholsäure

<130> M/51187-PCT

<160> 69

<170> PatentIn version 3.5

<210> 1
<211> 792
<212> DNA
<213> Collinsella aerofaciens

<220>
<221> CDS
<222> (1)..(792)

<400> 1

```
atg aac ctg agg gag aag tac ggt gag tgg ggc ctg atc ctg ggc gcg      48
Met Asn Leu Arg Glu Lys Tyr Gly Glu Trp Gly Leu Ile Leu Gly Ala
1               5                   10                  15

acc gag ggc gtc ggc aag gcg ttc tgc gag aag atc gcc gcc ggc ggc      96
Thr Glu Gly Val Gly Lys Ala Phe Cys Glu Lys Ile Ala Ala Gly Gly
            20                  25                  30

atg aac gtc gtc atg gtc ggc cgt cgc gag gag aag ctg aac gtg ctc     144
Met Asn Val Val Met Val Gly Arg Arg Glu Glu Lys Leu Asn Val Leu
        35                  40                  45

gca ggc gag atc cgc gag acc tac ggc gtg gag acc aag gtc gtg cgc     192
Ala Gly Glu Ile Arg Glu Thr Tyr Gly Val Glu Thr Lys Val Val Arg
    50                  55                  60

gcc gac ttt agc cag ccc ggc gct gcc gag acc gtc ttc gcc gcg acc     240
Ala Asp Phe Ser Gln Pro Gly Ala Ala Glu Thr Val Phe Ala Ala Thr
65                  70                  75                  80

gag ggc ctg gac atg ggc ttc atg agc tac gtg gcc tgc ctg cac agc     288
Glu Gly Leu Asp Met Gly Phe Met Ser Tyr Val Ala Cys Leu His Ser
                85                  90                  95

ttc ggt aag atc cag gac acc ccc tgg gag aag cac gag gcc atg atc     336
Phe Gly Lys Ile Gln Asp Thr Pro Trp Glu Lys His Glu Ala Met Ile
            100                 105                 110

aac gtc aac gtc gtg acc ttc ctc aag tgc ttc cac cac tac atg cgg     384
Asn Val Asn Val Val Thr Phe Leu Lys Cys Phe His His Tyr Met Arg
        115                 120                 125

atc ttt gcc gcc cag gac cgc ggc gcc gtg atc aac gtc tcg tcg atg     432
Ile Phe Ala Ala Gln Asp Arg Gly Ala Val Ile Asn Val Ser Ser Met
    130                 135                 140

acc ggc atc agc tcc agc ccc tgg aac ggc cag tac ggc gcg ggc aag     480
Thr Gly Ile Ser Ser Ser Pro Trp Asn Gly Gln Tyr Gly Ala Gly Lys
145                 150                 155                 160
```

```
gcc ttc atc ctc aag atg acc gag gcc gtg gcc tgc gag tgc gag ggc        528
Ala Phe Ile Leu Lys Met Thr Glu Ala Val Ala Cys Glu Cys Glu Gly
                165                 170                 175

acc ggc gtc gac gtc gag gtc atc acc ctc ggc acc acc cta acc ccc        576
Thr Gly Val Asp Val Glu Val Ile Thr Leu Gly Thr Thr Leu Thr Pro
            180                 185                 190

agc ctg ctg tcc aac ctc ccc ggc ggc ccg cag ggc gag gcc gtc atg        624
Ser Leu Leu Ser Asn Leu Pro Gly Gly Pro Gln Gly Glu Ala Val Met
        195                 200                 205

aag atc gcc ctc acc ccc gag gag tgc gtt gac gag gcc ttt gag aag        672
Lys Ile Ala Leu Thr Pro Glu Glu Cys Val Asp Glu Ala Phe Glu Lys
    210                 215                 220

ctg ggt aag gag ctc tcc gtc atc gcc ggc cag cgc aac aag gac tcc        720
Leu Gly Lys Glu Leu Ser Val Ile Ala Gly Gln Arg Asn Lys Asp Ser
225                 230                 235                 240

gtc cac gac tgg aag gca aac cac acc gag gac gag tac atc cgc tac        768
Val His Asp Trp Lys Ala Asn His Thr Glu Asp Glu Tyr Ile Arg Tyr
                245                 250                 255

atg ggg tcg ttc tac cgc gac tag                                        792
Met Gly Ser Phe Tyr Arg Asp
            260
```

<210> 2
<211> 263
<212> PRT
<213> Collinsella aerofaciens

<400> 2

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Gly Arg Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110
```

```
Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 3
<211> 31
<212> DNA
<213> artificial

<220>
<223> PCR Primer

<400> 3
gggaattcca tatgaacctg agggagaagt a 31

<210> 4
<211> 27
<212> DNA
<213> Artificial

<220>
<223> PCR Primer

<400> 4
cccaagcttc tagtcgcggt agaacga 27

<210> 5
<211> 774
<212> DNA
<213> Comamonas testosteroni

<220>
<221> CDS
<222> (1)..(774)

<400> 5

```
atg tcc atc atc gtg ata agc ggc tgc gcc acc ggc att ggt gcc gct      48
Met Ser Ile Ile Val Ile Ser Gly Cys Ala Thr Gly Ile Gly Ala Ala
1               5                   10                  15

acg cgc aag gtc ctg gag gcg gcc ggt cac cag atc gta ggc atc gat      96
Thr Arg Lys Val Leu Glu Ala Ala Gly His Gln Ile Val Gly Ile Asp
            20                  25                  30

ata cgc gat gcg gaa gtg att gcc gat ctc tcg acg gcc gaa ggt cga     144
Ile Arg Asp Ala Glu Val Ile Ala Asp Leu Ser Thr Ala Glu Gly Arg
        35                  40                  45

aag cag gcg att gcc gat gta ctg gcg aag tgc agc aag ggc atg gac     192
Lys Gln Ala Ile Ala Asp Val Leu Ala Lys Cys Ser Lys Gly Met Asp
    50                  55                  60

ggc ctg gtg ctg tgc gcc ggc ctg gga ccg cag acc aag gtg ctt ggc     240
Gly Leu Val Leu Cys Ala Gly Leu Gly Pro Gln Thr Lys Val Leu Gly
65                  70                  75                  80

aat gtg gtt tcg gtc aat tat ttt ggc gcg acc gag ctg atg gat gcc     288
Asn Val Val Ser Val Asn Tyr Phe Gly Ala Thr Glu Leu Met Asp Ala
                85                  90                  95

ttt ttg cca gcg ctg aaa aaa ggc cat cag ccc gca gcc gtc gtc atc     336
Phe Leu Pro Ala Leu Lys Lys Gly His Gln Pro Ala Ala Val Val Ile
            100                 105                 110

tcg tcc gtg gct tcc gcg cat ctg gct ttt gac aag aac cca ctg gcg     384
Ser Ser Val Ala Ser Ala His Leu Ala Phe Asp Lys Asn Pro Leu Ala
        115                 120                 125

ctg gca ctg gaa gcc ggc gag gaa gcc aag gcc cgc gcc att gtc gaa     432
Leu Ala Leu Glu Ala Gly Glu Glu Ala Lys Ala Arg Ala Ile Val Glu
    130                 135                 140

cat gcg gga gag cag ggc gga aat ctg gcc tat gcg ggc agc aag aat     480
His Ala Gly Glu Gln Gly Gly Asn Leu Ala Tyr Ala Gly Ser Lys Asn
145                 150                 155                 160

gct ttg acg gtg gct gtg cgc aaa cgc gcc gcc gcc tgg ggc gag gct     528
Ala Leu Thr Val Ala Val Arg Lys Arg Ala Ala Ala Trp Gly Glu Ala
                165                 170                 175

ggc gtg cgc ctg aac acc atc gcc ccc ggt gca acc gag act ccc ttg     576
Gly Val Arg Leu Asn Thr Ile Ala Pro Gly Ala Thr Glu Thr Pro Leu
            180                 185                 190

ctg cag gcg ggc ctg cag gac ccg cgc tat ggc gaa tcc att gcc aag     624
```

```
Leu Gln Ala Gly Leu Gln Asp Pro Arg Tyr Gly Glu Ser Ile Ala Lys
        195                 200                 205

ttc gtt cct ccc atg ggc cgc cgt gcc gag ccg tcc gag atg gcg tcg        672
Phe Val Pro Pro Met Gly Arg Arg Ala Glu Pro Ser Glu Met Ala Ser
    210                 215                 220

gtc atc gcc ttt ttg atg agc ccg gcc gca agc tat gtg cat ggc gcg        720
Val Ile Ala Phe Leu Met Ser Pro Ala Ala Ser Tyr Val His Gly Ala
225                 230                 235                 240

cag atc gtc att gat ggc ggc att gat gcg gtg atg cgc ccg aca cag        768
Gln Ile Val Ile Asp Gly Gly Ile Asp Ala Val Met Arg Pro Thr Gln
                245                 250                 255

ttc tga                                                                774
Phe
```

<210> 6
<211> 257
<212> PRT
<213> Comamonas testosteroni

<400> 6

```
Met Ser Ile Ile Val Ile Ser Gly Cys Ala Thr Gly Ile Gly Ala Ala
1               5                   10                  15

Thr Arg Lys Val Leu Glu Ala Ala Gly His Gln Ile Val Gly Ile Asp
            20                  25                  30

Ile Arg Asp Ala Glu Val Ile Ala Asp Leu Ser Thr Ala Glu Gly Arg
        35                  40                  45

Lys Gln Ala Ile Ala Asp Val Leu Ala Lys Cys Ser Lys Gly Met Asp
    50                  55                  60

Gly Leu Val Leu Cys Ala Gly Leu Gly Pro Gln Thr Lys Val Leu Gly
65                  70                  75                  80

Asn Val Val Ser Val Asn Tyr Phe Gly Ala Thr Glu Leu Met Asp Ala
                85                  90                  95

Phe Leu Pro Ala Leu Lys Lys Gly His Gln Pro Ala Ala Val Val Ile
            100                 105                 110

Ser Ser Val Ala Ser Ala His Leu Ala Phe Asp Lys Asn Pro Leu Ala
        115                 120                 125

Leu Ala Leu Glu Ala Gly Glu Glu Ala Lys Ala Arg Ala Ile Val Glu
    130                 135                 140
```

```
His Ala Gly Glu Gln Gly Gly Asn Leu Ala Tyr Ala Gly Ser Lys Asn
145                 150                 155                 160

Ala Leu Thr Val Ala Val Arg Lys Arg Ala Ala Ala Trp Gly Glu Ala
                165                 170                 175

Gly Val Arg Leu Asn Thr Ile Ala Pro Gly Ala Thr Glu Thr Pro Leu
            180                 185                 190

Leu Gln Ala Gly Leu Gln Asp Pro Arg Tyr Gly Glu Ser Ile Ala Lys
        195                 200                 205

Phe Val Pro Pro Met Gly Arg Arg Ala Glu Pro Ser Glu Met Ala Ser
    210                 215                 220

Val Ile Ala Phe Leu Met Ser Pro Ala Ala Ser Tyr Val His Gly Ala
225                 230                 235                 240

Gln Ile Val Ile Asp Gly Gly Ile Asp Ala Val Met Arg Pro Thr Gln
                245                 250                 255

Phe
```

<210> 7
<211> 969
<212> DNA
<213> Rattus norvegicus

<220>
<221> CDS
<222> (1)..(969)

<400> 7

```
atg gat tcc ata tct ctg cgt gta gca cta aat gat ggt aac ttc att      48
Met Asp Ser Ile Ser Leu Arg Val Ala Leu Asn Asp Gly Asn Phe Ile
1               5                   10                  15

cct gta ctg ggg ttt gga acc act gtg cct gag aag gtt gct aag gat      96
Pro Val Leu Gly Phe Gly Thr Thr Val Pro Glu Lys Val Ala Lys Asp
            20                  25                  30

gaa gtt atc aag gct act aaa ata gct ata gat aat gga ttc cgc cat     144
Glu Val Ile Lys Ala Thr Lys Ile Ala Ile Asp Asn Gly Phe Arg His
        35                  40                  45

ttt gac tct gct tat ttg tac gaa gta gaa gag gaa gtg ggc caa gcc     192
Phe Asp Ser Ala Tyr Leu Tyr Glu Val Glu Glu Glu Val Gly Gln Ala
    50                  55                  60

att aga agc aag att gaa gac ggc act gtg aag aga gaa gat ata ttc     240
Ile Arg Ser Lys Ile Glu Asp Gly Thr Val Lys Arg Glu Asp Ile Phe
65                  70                  75                  80
```

```
tat act tca aag ctt tgg agc act ttc cat aga cca gag ctg gtc cga      288
Tyr Thr Ser Lys Leu Trp Ser Thr Phe His Arg Pro Glu Leu Val Arg
                85                  90                  95

act tgc ttg gaa aag aca ctg aaa agc act caa ctg gac tat gtg gat      336
Thr Cys Leu Glu Lys Thr Leu Lys Ser Thr Gln Leu Asp Tyr Val Asp
            100                 105                 110

ctt tat att att cat ttc cca atg gct ttg cag cct gga gat ata ttt      384
Leu Tyr Ile Ile His Phe Pro Met Ala Leu Gln Pro Gly Asp Ile Phe
        115                 120                 125

ttc cca cga gat gag cat gga aaa cta ttg ttt gaa aca gtg gat atc      432
Phe Pro Arg Asp Glu His Gly Lys Leu Leu Phe Glu Thr Val Asp Ile
    130                 135                 140

tgt gac aca tgg gag gcc atg gaa aag tgt aag gat gca gga ttg gcc      480
Cys Asp Thr Trp Glu Ala Met Glu Lys Cys Lys Asp Ala Gly Leu Ala
145                 150                 155                 160

aag tct att ggg gtg tcc aac ttt aac tgc agg cag ctg gag agg att      528
Lys Ser Ile Gly Val Ser Asn Phe Asn Cys Arg Gln Leu Glu Arg Ile
                165                 170                 175

ctg aat aag cca ggg ctc aaa tac aag cct gtg tgc aac cag gtg gaa      576
Leu Asn Lys Pro Gly Leu Lys Tyr Lys Pro Val Cys Asn Gln Val Glu
            180                 185                 190

tgt cac ctt tat ctc aac cag agc aaa atg ctg gac tat tgt aag tca      624
Cys His Leu Tyr Leu Asn Gln Ser Lys Met Leu Asp Tyr Cys Lys Ser
        195                 200                 205

aaa gac atc att ctg gtt tcc tac tgc acg ctg gga agt tca cga gac      672
Lys Asp Ile Ile Leu Val Ser Tyr Cys Thr Leu Gly Ser Ser Arg Asp
    210                 215                 220

aaa aca tgg gtg gat cag aaa agt cca gtt ctc cta gat gat cca gtt      720
Lys Thr Trp Val Asp Gln Lys Ser Pro Val Leu Leu Asp Asp Pro Val
225                 230                 235                 240

ctt tgt gcc ata gca aag aag tac aag caa acc cca gcc cta gtt gcc      768
Leu Cys Ala Ile Ala Lys Lys Tyr Lys Gln Thr Pro Ala Leu Val Ala
                245                 250                 255

ctt cgc tac cag ctg cag cgt ggg gtt gtg ccc ctg atc agg agt ttc      816
Leu Arg Tyr Gln Leu Gln Arg Gly Val Val Pro Leu Ile Arg Ser Phe
            260                 265                 270

aac gcg aag cgg atc aaa gag cta aca cag gtt ttt gaa ttc cag ttg      864
Asn Ala Lys Arg Ile Lys Glu Leu Thr Gln Val Phe Glu Phe Gln Leu
        275                 280                 285

gct tca gag gac atg aaa gcc ctg gat ggc ttg aac aga aat ttc aga      912
Ala Ser Glu Asp Met Lys Ala Leu Asp Gly Leu Asn Arg Asn Phe Arg
    290                 295                 300

tac aac aat gca aaa tat ttt gat gac cat ccc aat cat cca ttt act      960
Tyr Asn Asn Ala Lys Tyr Phe Asp Asp His Pro Asn His Pro Phe Thr
305                 310                 315                 320

gat gaa tag                                                          969
Asp Glu
```

<210> 8
<211> 322
<212> PRT
<213> Rattus norvegicus

<400> 8

```
Met Asp Ser Ile Ser Leu Arg Val Ala Leu Asn Asp Gly Asn Phe Ile
1               5                   10                  15

Pro Val Leu Gly Phe Gly Thr Thr Val Pro Glu Lys Val Ala Lys Asp
            20                  25                  30

Glu Val Ile Lys Ala Thr Lys Ile Ala Ile Asp Asn Gly Phe Arg His
        35                  40                  45

Phe Asp Ser Ala Tyr Leu Tyr Glu Val Glu Glu Glu Val Gly Gln Ala
    50                  55                  60

Ile Arg Ser Lys Ile Glu Asp Gly Thr Val Lys Arg Glu Asp Ile Phe
65                  70                  75                  80

Tyr Thr Ser Lys Leu Trp Ser Thr Phe His Arg Pro Glu Leu Val Arg
                85                  90                  95

Thr Cys Leu Glu Lys Thr Leu Lys Ser Thr Gln Leu Asp Tyr Val Asp
            100                 105                 110

Leu Tyr Ile Ile His Phe Pro Met Ala Leu Gln Pro Gly Asp Ile Phe
        115                 120                 125

Phe Pro Arg Asp Glu His Gly Lys Leu Leu Phe Glu Thr Val Asp Ile
    130                 135                 140

Cys Asp Thr Trp Glu Ala Met Glu Lys Cys Lys Asp Ala Gly Leu Ala
145                 150                 155                 160

Lys Ser Ile Gly Val Ser Asn Phe Asn Cys Arg Gln Leu Glu Arg Ile
                165                 170                 175

Leu Asn Lys Pro Gly Leu Lys Tyr Lys Pro Val Cys Asn Gln Val Glu
            180                 185                 190

Cys His Leu Tyr Leu Asn Gln Ser Lys Met Leu Asp Tyr Cys Lys Ser
        195                 200                 205

Lys Asp Ile Ile Leu Val Ser Tyr Cys Thr Leu Gly Ser Ser Arg Asp
    210                 215                 220
```

```
Lys Thr Trp Val Asp Gln Lys Ser Pro Val Leu Leu Asp Asp Pro Val
225                 230                 235                 240

Leu Cys Ala Ile Ala Lys Lys Tyr Lys Gln Thr Pro Ala Leu Val Ala
                245                 250                 255

Leu Arg Tyr Gln Leu Gln Arg Gly Val Val Pro Leu Ile Arg Ser Phe
            260                 265                 270

Asn Ala Lys Arg Ile Lys Glu Leu Thr Gln Val Phe Glu Phe Gln Leu
        275                 280                 285

Ala Ser Glu Asp Met Lys Ala Leu Asp Gly Leu Asn Arg Asn Phe Arg
    290                 295                 300

Tyr Asn Asn Ala Lys Tyr Phe Asp Asp His Pro Asn His Pro Phe Thr
305                 310                 315                 320

Asp Glu
```

<210> 9
<211> 26
<212> DNA
<213> Artificial

<220>
<223> PCR Primer

<400> 9
cgtcgtcatg gtcgcccgtc gcgagg 26

<210> 10
<211> 26
<212> DNA
<213> Artificial

<220>
<223> PCR Primer

<400> 10
cctcgcgacg ggcgaccatg acgacg 26

<210> 11
<211> 26
<212> DNA
<213> Artificial

<220>
<223> PCR Primer

<400> 11
cgtcgtcatg gtcagccgtc gcgagg 26

<210> 12
<211> 26
<212> DNA
<213> Artificial

<220>
<223> PCR Primer

<400> 12
cctcgcgacg gctgaccatg acgacg 26

<210> 13
<211> 47
<212> DNA
<213> Artificial

<220>
<223> PCR Primer

<400> 13
gcgaattcgt gaaaggagat atacatgaac ctgagggaga agtacgg 47

<210> 14
<211> 1206
<212> DNA
<213> Artificial

<220>
<223> Formiate Deydrogenase Mutant

<220>
<221> CDS
<222> (1)..(1206)

<400> 14

```
atg gca aag gtc ctg tgc gtt ctt tac gat gat ccg gtc gac ggc tac        48
Met Ala Lys Val Leu Cys Val Leu Tyr Asp Asp Pro Val Asp Gly Tyr
1               5                   10                  15

ccg aag acc tat gcc cgc gac gat ctt ccg aag atc gac cac tat ccg        96
Pro Lys Thr Tyr Ala Arg Asp Asp Leu Pro Lys Ile Asp His Tyr Pro
            20                  25                  30

ggc ggc cag atc ttg ccg acg ccg aag gcc atc gac ttc acg ccc ggg       144
Gly Gly Gln Ile Leu Pro Thr Pro Lys Ala Ile Asp Phe Thr Pro Gly
        35                  40                  45

cag ttg ctc ggc tcc gtc tcc ggc gag ctc ggc ctg cgc gaa tat ctc       192
Gln Leu Leu Gly Ser Val Ser Gly Glu Leu Gly Leu Arg Glu Tyr Leu
    50                  55                  60

gaa tcc aac ggc cac acc ctg gtc gtg acc tcc gac aag gac ggc ccc       240
Glu Ser Asn Gly His Thr Leu Val Val Thr Ser Asp Lys Asp Gly Pro
65                  70                  75                  80

gac tcg gtg ttc gag cgc gag ctg gtc gat gcg gat gtc gtc atc tcc       288
Asp Ser Val Phe Glu Arg Glu Leu Val Asp Ala Asp Val Val Ile Ser
```

```
                85                  90                  95

cag ccc ttc tgg ccg gcc tat ctg acg ccc gag cgc atc gcc aag gcc        336
Gln Pro Phe Trp Pro Ala Tyr Leu Thr Pro Glu Arg Ile Ala Lys Ala
            100                 105                 110

aag aac ctg aag ctc gcg ctc acc gcc ggc atc ggt tcc gac cac gtc        384
Lys Asn Leu Lys Leu Ala Leu Thr Ala Gly Ile Gly Ser Asp His Val
        115                 120                 125

gat ctt cag tcg gct atc gac cgc aac gtc acc gtg gcg gaa gtc acc        432
Asp Leu Gln Ser Ala Ile Asp Arg Asn Val Thr Val Ala Glu Val Thr
    130                 135                 140

tac tgc aac tcg atc agc gtc gcc gag cat gtg gtg atg atg atc ctg        480
Tyr Cys Asn Ser Ile Ser Val Ala Glu His Val Val Met Met Ile Leu
145                 150                 155                 160

tcg ctg gtg cgc aac tat ctg ccc tcg cac gaa tgg gcg cgg aag ggc        528
Ser Leu Val Arg Asn Tyr Leu Pro Ser His Glu Trp Ala Arg Lys Gly
                165                 170                 175

ggc tgg aac atc gcc gac tgc gtc tcc cac gcc tac gac ctc gag gcg        576
Gly Trp Asn Ile Ala Asp Cys Val Ser His Ala Tyr Asp Leu Glu Ala
            180                 185                 190

atg cat gtc ggc acc gtg gcc gcc ggc cgc atc ggt ctc gcg gtg ctg        624
Met His Val Gly Thr Val Ala Ala Gly Arg Ile Gly Leu Ala Val Leu
        195                 200                 205

cgc cgt ctg gcg ccg ttc gac gtg cac ctg cac tac acc ggc cgt cac        672
Arg Arg Leu Ala Pro Phe Asp Val His Leu His Tyr Thr Gly Arg His
    210                 215                 220

cgc ctg ccg gaa tcg gtc gag aag gag ctc aac ctc acc tgg cac gcg        720
Arg Leu Pro Glu Ser Val Glu Lys Glu Leu Asn Leu Thr Trp His Ala
225                 230                 235                 240

acc cgc gag gac atg tat ccg gtt tgc gac gtg gtg acg ctg aac tgc        768
Thr Arg Glu Asp Met Tyr Pro Val Cys Asp Val Val Thr Leu Asn Cys
                245                 250                 255

ccg ctg cac ccc gaa acc gag cac atg atc aat gac gag acg ctg aag        816
Pro Leu His Pro Glu Thr Glu His Met Ile Asn Asp Glu Thr Leu Lys
            260                 265                 270

ctg ttc aag cgt ggc gcc tac atc gtc aac acc gcc cgc ggc aag ctg        864
Leu Phe Lys Arg Gly Ala Tyr Ile Val Asn Thr Ala Arg Gly Lys Leu
        275                 280                 285

tgc gac cgc gat gcc gtg gca cgt gcg ctc gaa tcc ggc cgg ctg gcc        912
Cys Asp Arg Asp Ala Val Ala Arg Ala Leu Glu Ser Gly Arg Leu Ala
    290                 295                 300

ggc tat gcc ggc gac gtg tgg ttc ccg cag ccg gcg ccg aag gac cac        960
Gly Tyr Ala Gly Asp Val Trp Phe Pro Gln Pro Ala Pro Lys Asp His
305                 310                 315                 320

ccc tgg cgg acg atg ccc tat aac ggc atg acc ccg cac atc tcc ggc       1008
Pro Trp Arg Thr Met Pro Tyr Asn Gly Met Thr Pro His Ile Ser Gly
                325                 330                 335

acc acg ctg acc gcg cag gcg cgt tat gcg gcg ggc acc cgc gag atc       1056
```

```
Thr Thr Leu Thr Ala Gln Ala Arg Tyr Ala Ala Gly Thr Arg Glu Ile
            340                 345                 350

ctg gag tgc ttc ttc gag ggc cgt ccg atc cgc gac gaa tac ctc atc       1104
Leu Glu Cys Phe Phe Glu Gly Arg Pro Ile Arg Asp Glu Tyr Leu Ile
        355                 360                 365

gtg cag ggc ggc gct ctt gcc ggc acc ggc gcg cat tcc tac tcg aag       1152
Val Gln Gly Gly Ala Leu Ala Gly Thr Gly Ala His Ser Tyr Ser Lys
    370                 375                 380

ggc aat gcc acc ggc ggt tcg gaa gag gcc gcc aag ttc aag aag gcg       1200
Gly Asn Ala Thr Gly Gly Ser Glu Glu Ala Ala Lys Phe Lys Lys Ala
385                 390                 395                 400

gtc tga                                                               1206
Val
```

<210> 15
<211> 401
<212> PRT
<213> Artificial

<220>
<223> Synthetic Construct

<400> 15

```
Met Ala Lys Val Leu Cys Val Leu Tyr Asp Asp Pro Val Asp Gly Tyr
1               5                   10                  15

Pro Lys Thr Tyr Ala Arg Asp Asp Leu Pro Lys Ile Asp His Tyr Pro
            20                  25                  30

Gly Gly Gln Ile Leu Pro Thr Pro Lys Ala Ile Asp Phe Thr Pro Gly
        35                  40                  45

Gln Leu Leu Gly Ser Val Ser Gly Glu Leu Gly Leu Arg Glu Tyr Leu
    50                  55                  60

Glu Ser Asn Gly His Thr Leu Val Val Thr Ser Asp Lys Asp Gly Pro
65                  70                  75                  80

Asp Ser Val Phe Glu Arg Glu Leu Val Asp Ala Asp Val Val Ile Ser
                85                  90                  95

Gln Pro Phe Trp Pro Ala Tyr Leu Thr Pro Glu Arg Ile Ala Lys Ala
            100                 105                 110

Lys Asn Leu Lys Leu Ala Leu Thr Ala Gly Ile Gly Ser Asp His Val
        115                 120                 125

Asp Leu Gln Ser Ala Ile Asp Arg Asn Val Thr Val Ala Glu Val Thr
```

```
    130                 135                 140

Tyr Cys Asn Ser Ile Ser Val Ala Glu His Val Val Met Met Ile Leu
145                 150                 155                 160

Ser Leu Val Arg Asn Tyr Leu Pro Ser His Glu Trp Ala Arg Lys Gly
                165                 170                 175

Gly Trp Asn Ile Ala Asp Cys Val Ser His Ala Tyr Asp Leu Glu Ala
            180                 185                 190

Met His Val Gly Thr Val Ala Ala Gly Arg Ile Gly Leu Ala Val Leu
        195                 200                 205

Arg Arg Leu Ala Pro Phe Asp Val His Leu His Tyr Thr Gly Arg His
    210                 215                 220

Arg Leu Pro Glu Ser Val Glu Lys Glu Leu Asn Leu Thr Trp His Ala
225                 230                 235                 240

Thr Arg Glu Asp Met Tyr Pro Val Cys Asp Val Val Thr Leu Asn Cys
                245                 250                 255

Pro Leu His Pro Glu Thr Glu His Met Ile Asn Asp Glu Thr Leu Lys
            260                 265                 270

Leu Phe Lys Arg Gly Ala Tyr Ile Val Asn Thr Ala Arg Gly Lys Leu
        275                 280                 285

Cys Asp Arg Asp Ala Val Ala Arg Ala Leu Glu Ser Gly Arg Leu Ala
    290                 295                 300

Gly Tyr Ala Gly Asp Val Trp Phe Pro Gln Pro Ala Pro Lys Asp His
305                 310                 315                 320

Pro Trp Arg Thr Met Pro Tyr Asn Gly Met Thr Pro His Ile Ser Gly
                325                 330                 335

Thr Thr Leu Thr Ala Gln Ala Arg Tyr Ala Ala Gly Thr Arg Glu Ile
            340                 345                 350

Leu Glu Cys Phe Phe Glu Gly Arg Pro Ile Arg Asp Glu Tyr Leu Ile
        355                 360                 365

Val Gln Gly Gly Ala Leu Ala Gly Thr Gly Ala His Ser Tyr Ser Lys
    370                 375                 380
```

Gly Asn Ala Thr Gly Gly Ser Glu Glu Ala Ala Lys Phe Lys Lys Ala
385 390 395 400

Val

<210> 16
<211> 43
<212> DNA
<213> Artificial

<220>
<223> PCR Primer

<400> 16
cccaagctta aggagatata catgtccatc atcgtgataa gcg 43

<210> 17
<211> 34
<212> DNA
<213> Artificial

<220>
<223> PCR Primer

<400> 17
ataagaatgc ggccgctcag aactgtgtcg ggcg 34

<210> 18
<211> 7404
<212> DNA
<213> Artificial

<220>
<223> Plasmid pET21a FDH D221G 7beta-HSDH

<220>
<221> CDS
<222> (5208) .. (6422)
<223> FDH D221G

<220>
<221> CDS
<222> (6435) .. (7226)
<223> 7beta-HSDH

<400> 18

```
tggcgaatgg gacgcgccct gtagcggcgc attaagcgcg gcgggtgtgg tggttacgcg      60
cagcgtgacc gctacacttg ccagcgccct agcgcccgct cctttcgctt tcttcccttc     120
ctttctcgcc acgttcgccg gctttccccg tcaagctcta aatcgggggc tccctttagg     180
gttccgattt agtgctttac ggcacctcga ccccaaaaaa cttgattagg gtgatggttc     240
acgtagtggg ccatcgccct gatagacggt ttttcgccct ttgacgttgg agtccacgtt     300
ctttaatagt ggactcttgt tccaaactgg aacaacactc aaccctatct cggtctattc     360
```

```
ttttgattta taagggattt tgccgatttc ggcctattgg ttaaaaaatg agctgattta      420
acaaaaattt aacgcgaatt ttaacaaaat attaacgttt acaatttcag gtggcacttt      480
tcggggaaat gtgcgcggaa cccctatttg tttatttttc taaatacatt caaatatgta      540
tccgctcatg agacaataac cctgataaat gcttcaataa tattgaaaaa ggaagagtat      600
gagtattcaa catttccgtg tcgcccttat tccctttttt gcggcatttt gccttcctgt      660
ttttgctcac ccagaaacgc tggtgaaagt aaaagatgct gaagatcagt tgggtgcacg      720
agtgggttac atcgaactgg atctcaacag cggtaagatc cttgagagtt ttcgccccga      780
agaacgtttt ccaatgatga gcacttttaa agttctgcta tgtggcgcgg tattatcccg      840
tattgacgcc gggcaagagc aactcggtcg ccgcatacac tattctcaga atgacttggt      900
tgagtactca ccagtcacag aaaagcatct tacggatggc atgacagtaa gagaattatg      960
cagtgctgcc ataaccatga gtgataacac tgcggccaac ttacttctga caacgatcgg     1020
aggaccgaag gagctaaccg cttttttgca caacatgggg gatcatgtaa ctcgccttga     1080
tcgttgggaa ccggagctga atgaagccat accaaacgac gagcgtgaca ccacgatgcc     1140
tgcagcaatg gcaacaacgt tgcgcaaact attaactggc gaactactta ctctagcttc     1200
ccggcaacaa ttaatagact ggatggaggc ggataaagtt gcaggaccac ttctgcgctc     1260
ggcccttccg gctggctggt ttattgctga taaatctgga gccggtgagc gtgggtctcg     1320
cggtatcatt gcagcactgg ggccagatgg taagccctcc cgtatcgtag ttatctacac     1380
gacggggagt caggcaacta tggatgaacg aaatagacag atcgctgaga taggtgcctc     1440
actgattaag cattggtaac tgtcagacca agtttactca tatatacttt agattgattt     1500
aaaacttcat ttttaattta aaaggatcta ggtgaagatc ctttttgata atctcatgac     1560
caaaatccct taacgtgagt tttcgttcca ctgagcgtca gaccccgtag aaaagatcaa     1620
aggatcttct tgagatcctt tttttctgcg cgtaatctgc tgcttgcaaa caaaaaaacc     1680
accgctacca gcggtggttt gtttgccgga tcaagagcta ccaactcttt ttccgaaggt     1740
aactggcttc agcagagcgc agataccaaa tactgtcctt ctagtgtagc cgtagttagg     1800
ccaccacttc aagaactctg tagcaccgcc tacatacctc gctctgctaa tcctgttacc     1860
agtggctgct gccagtggcg ataagtcgtg tcttaccggg ttggactcaa gacgatagtt     1920
accggataag gcgcagcggt cgggctgaac ggggggttcg tgcacacagc ccagcttgga     1980
gcgaacgacc tacaccgaac tgagatacct acagcgtgag ctatgagaaa gcgccacgct     2040
tcccgaaggg agaaaggcgg acaggtatcc ggtaagcggc agggtcggaa caggagagcg     2100
cacgagggag cttccagggg gaaacgcctg gtatctttat agtcctgtcg ggtttcgcca     2160
cctctgactt gagcgtcgat ttttgtgatg ctcgtcaggg gggcggagcc tatggaaaaa     2220
```

cgccagcaac gcggcctttt tacggttcct ggccttttgc tggccttttg ctcacatgtt 2280
ctttcctgcg ttatcccctg attctgtgga taaccgtatt accgcctttg agtgagctga 2340
taccgctcgc cgcagccgaa cgaccgagcg cagcgagtca gtgagcgagg aagcggaaga 2400
gcgcctgatg cggtattttc tccttacgca tctgtgcggt atttcacacc gcatatatgg 2460
tgcactctca gtacaatctg ctctgatgcc gcatagttaa gccagtatac actccgctat 2520
cgctacgtga ctgggtcatg gctgcgcccc gacacccgcc aacacccgct gacgcgccct 2580
gacgggcttg tctgctcccg gcatccgctt acagacaagc tgtgaccgtc tccgggagct 2640
gcatgtgtca gaggttttca ccgtcatcac cgaaacgcgc gaggcagctg cggtaaagct 2700
catcagcgtg gtcgtgaagc gattcacaga tgtctgcctg ttcatccgcg tccagctcgt 2760
tgagtttctc cagaagcgtt aatgtctggc ttctgataaa gcgggccatg ttaagggcgg 2820
tttttttcctg tttggtcact gatgcctccg tgtaaggggg atttctgttc atgggggtaa 2880
tgataccgat gaaacgagag aggatgctca cgatacgggt tactgatgat gaacatgccc 2940
ggttactgga acgttgtgag ggtaaacaac tggcggtatg gatgcggcgg gaccagagaa 3000
aaatcactca gggtcaatgc cagcgcttcg ttaatacaga tgtaggtgtt ccacagggta 3060
gccagcagca tcctgcgatg cagatccgga acataatggt gcagggcgct gacttccgcg 3120
tttccagact ttacgaaaca cggaaaccga agaccattca tgttgttgct caggtcgcag 3180
acgttttgca gcagcagtcg cttcacgttc gctcgcgtat cggtgattca ttctgctaac 3240
cagtaaggca accccgccag cctagccggg tcctcaacga caggagcacg atcatgcgca 3300
cccgtggggc cgccatgccg gcgataatgg cctgcttctc gccgaaacgt ttggtggcgg 3360
gaccagtgac gaaggcttga gcgagggcgt gcaagattcc gaataccgca agcgacaggc 3420
cgatcatcgt cgcgctccag cgaaagcggt cctcgccgaa aatgacccag agcgctgccg 3480
gcacctgtcc tacgagttgc atgataaaga agacagtcat aagtgcggcg acgatagtca 3540
tgccccgcgc ccaccggaag gagctgactg ggttgaaggc tctcaagggc atcggtcgag 3600
atcccggtgc ctaatgagtg agctaactta cattaattgc gttgcgctca ctgcccgctt 3660
tccagtcggg aaacctgtcg tgccagctgc attaatgaat cggccaacgc gcggggagag 3720
gcggtttgcg tattgggcgc cagggtggtt tttcttttca ccagtgagac gggcaacagc 3780
tgattgccct tcaccgcctg gccctgagag agttgcagca agcggtccac gctggtttgc 3840
cccagcaggc gaaaatcctg tttgatggtg gttaacggcg ggatataaca tgagctgtct 3900
tcggtatcgt cgtatcccac taccgagata tccgcaccaa cgcgcagccc ggactcggta 3960
atggcgcgca ttgcgcccag cgccatctga tcgttggcaa ccagcatcgc agtgggaacg 4020
atgccctcat tcagcatttg catggtttgt tgaaaaccgg acatggcact ccagtcgcct 4080
tcccgttccg ctatcggctg aatttgattg cgagtgagat atttatgcca gccagccaga 4140

```
cgcagacgcg ccgagacaga acttaatggg cccgctaaca gcgcgatttg ctggtgaccc      4200

aatgcgacca gatgctccac gcccagtcgc gtaccgtctt catgggagaa aataatactg      4260

ttgatgggtg tctggtcaga gacatcaaga aataacgccg gaacattagt gcaggcagct      4320

tccacagcaa tggcatcctg gtcatccagc ggatagttaa tgatcagccc actgacgcgt      4380

tgcgcgagaa gattgtgcac cgccgcttta caggcttcga cgccgcttcg ttctaccatc      4440

gacaccacca cgctggcacc cagttgatcg gcgcgagatt taatcgccgc gacaatttgc      4500

gacggcgcgt gcagggccag actggaggtg gcaacgccaa tcagcaacga ctgtttgccc      4560

gccagttgtt gtgccacgcg gttgggaatg taattcagct ccgccatcgc cgcttccact      4620

ttttcccgcg ttttcgcaga aacgtggctg gcctggttca ccacgcggga aacggtctga      4680

taagagacac cggcatactc tgcgacatcg tataacgtta ctggtttcac attcaccacc      4740

ctgaattgac tctcttccgg gcgctatcat gccataccgc gaaaggtttt gcgccattcg      4800

atggtgtccg ggatctcgac gctctccctt atgcgactcc tgcattagga agcagcccag      4860

tagtaggttg aggccgttga gcaccgccgc cgcaaggaat ggtgcatgca aggagatggc      4920

gcccaacagt cccccggcca cggggcctgc caccataccc acgccgaaac aagcgctcat      4980

gagcccgaag tggcgagccc gatcttcccc atcggtgatg tcggcgatat aggcgccagc      5040

aaccgcacct gtggcgccgg tgatgccggc cacgatgcgt ccggcgtaga ggatcgagat      5100

ctcgatcccg cgaaattaat acgactcact ataggggaat tgtgagcgga taacaattcc      5160

cctctagaaa taattttgtt taactttaag aaggagatat acatatg atg gca aag        5216
                                                    Met Ala Lys
                                                    1

gtc ctg tgc gtt ctt tac gat gat ccg gtc gac ggc tac ccg aag acc        5264
Val Leu Cys Val Leu Tyr Asp Asp Pro Val Asp Gly Tyr Pro Lys Thr
    5                   10                  15

tat gcc cgc gac gat ctt ccg aag atc gac cac tat ccg ggc ggc cag        5312
Tyr Ala Arg Asp Asp Leu Pro Lys Ile Asp His Tyr Pro Gly Gly Gln
20                  25                  30                  35

atc ttg ccg acg ccg aag gcc atc gac ttc acg ccc ggg cag ttg ctc        5360
Ile Leu Pro Thr Pro Lys Ala Ile Asp Phe Thr Pro Gly Gln Leu Leu
                40                  45                  50

ggc tcc gtc tcc ggc gag ctc ggc ctg cgc gaa tat ctc gaa tcc aac        5408
Gly Ser Val Ser Gly Glu Leu Gly Leu Arg Glu Tyr Leu Glu Ser Asn
            55                  60                  65

ggc cac acc ctg gtc gtg acc tcc gac aag gac ggc ccc gac tcg gtg        5456
Gly His Thr Leu Val Val Thr Ser Asp Lys Asp Gly Pro Asp Ser Val
        70                  75                  80

ttc gag cgc gag ctg gtc gat gcg gat gtc gtc atc tcc cag ccc ttc        5504
Phe Glu Arg Glu Leu Val Asp Ala Asp Val Val Ile Ser Gln Pro Phe
    85                  90                  95
```

```
tgg ccg gcc tat ctg acg ccc gag cgc atc gcc aag gcc aag aac ctg      5552
Trp Pro Ala Tyr Leu Thr Pro Glu Arg Ile Ala Lys Ala Lys Asn Leu
100                 105                 110                 115

aag ctc gcg ctc acc gcc ggc atc ggt tcc gac cac gtc gat ctt cag      5600
Lys Leu Ala Leu Thr Ala Gly Ile Gly Ser Asp His Val Asp Leu Gln
                120                 125                 130

tcg gct atc gac cgc aac gtc acc gtg gcg gaa gtc acc tac tgc aac      5648
Ser Ala Ile Asp Arg Asn Val Thr Val Ala Glu Val Thr Tyr Cys Asn
            135                 140                 145

tcg atc agc gtc gcc gag cat gtg gtg atg atg atc ctg tcg ctg gtg      5696
Ser Ile Ser Val Ala Glu His Val Val Met Met Ile Leu Ser Leu Val
        150                 155                 160

cgc aac tat ctg ccc tcg cac gaa tgg gcg cgg aag ggc ggc tgg aac      5744
Arg Asn Tyr Leu Pro Ser His Glu Trp Ala Arg Lys Gly Gly Trp Asn
    165                 170                 175

atc gcc gac tgc gtc tcc cac gcc tac gac ctc gag gcg atg cat gtc      5792
Ile Ala Asp Cys Val Ser His Ala Tyr Asp Leu Glu Ala Met His Val
180                 185                 190                 195

ggc acc gtg gcc gcc ggc cgc atc ggt ctc gcg gtg ctg cgc cgt ctg      5840
Gly Thr Val Ala Ala Gly Arg Ile Gly Leu Ala Val Leu Arg Arg Leu
                200                 205                 210

gcg ccg ttc gac gtg cac ctg cac tac acc ggc cgt cac cgc ctg ccg      5888
Ala Pro Phe Asp Val His Leu His Tyr Thr Gly Arg His Arg Leu Pro
            215                 220                 225

gaa tcg gtc gag aag gag ctc aac ctc acc tgg cac gcg acc cgc gag      5936
Glu Ser Val Glu Lys Glu Leu Asn Leu Thr Trp His Ala Thr Arg Glu
        230                 235                 240

gac atg tat ccg gtt tgc gac gtg gtg acg ctg aac tgc ccg ctg cac      5984
Asp Met Tyr Pro Val Cys Asp Val Val Thr Leu Asn Cys Pro Leu His
    245                 250                 255

ccc gaa acc gag cac atg atc aat gac gag acg ctg aag ctg ttc aag      6032
Pro Glu Thr Glu His Met Ile Asn Asp Glu Thr Leu Lys Leu Phe Lys
260                 265                 270                 275

cgt ggc gcc tac atc gtc aac acc gcc cgc ggc aag ctg tgc gac cgc      6080
Arg Gly Ala Tyr Ile Val Asn Thr Ala Arg Gly Lys Leu Cys Asp Arg
                280                 285                 290

gat gcc gtg gca cgt gcg ctc gaa tcc ggc cgg ctg gcc ggc tat gcc      6128
Asp Ala Val Ala Arg Ala Leu Glu Ser Gly Arg Leu Ala Gly Tyr Ala
            295                 300                 305

ggc gac gtg tgg ttc ccg cag ccg gcg ccg aag gac cac ccc tgg cgg      6176
Gly Asp Val Trp Phe Pro Gln Pro Ala Pro Lys Asp His Pro Trp Arg
        310                 315                 320

acg atg ccc tat aac ggc atg acc ccg cac atc tcc ggc acc acg ctg      6224
Thr Met Pro Tyr Asn Gly Met Thr Pro His Ile Ser Gly Thr Thr Leu
    325                 330                 335

acc gcg cag gcg cgt tat gcg gcg ggc acc cgc gag atc ctg gag tgc      6272
Thr Ala Gln Ala Arg Tyr Ala Ala Gly Thr Arg Glu Ile Leu Glu Cys
340                 345                 350                 355
```

```
ttc ttc gag ggc cgt ccg atc cgc gac gaa tac ctc atc gtg cag ggc        6320
Phe Phe Glu Gly Arg Pro Ile Arg Asp Glu Tyr Leu Ile Val Gln Gly
                360                 365                 370

ggc gct ctt gcc ggc acc ggc gcg cat tcc tac tcg aag ggc aat gcc        6368
Gly Ala Leu Ala Gly Thr Gly Ala His Ser Tyr Ser Lys Gly Asn Ala
            375                 380                 385

acc ggc ggt tcg gaa gag gcc gcc aag ttc aag aag gcg gct gag aat        6416
Thr Gly Gly Ser Glu Glu Ala Ala Lys Phe Lys Lys Ala Ala Glu Asn
        390                 395                 400

tcg tga aaggagatat ac atg aac ctg agg gag aag tac ggt gag tgg ggc      6467
Ser                       Met Asn Leu Arg Glu Lys Tyr Gly Glu Trp Gly
                          405                 410                 415

ctg atc ctg ggc gcg acc gag ggc gtc ggc aag gcg ttc tgc gag aag        6515
Leu Ile Leu Gly Ala Thr Glu Gly Val Gly Lys Ala Phe Cys Glu Lys
                420                 425                 430

atc gcc gcc ggc ggc atg aac gtc gtc atg gtc ggc cgt cgc gag gag        6563
Ile Ala Ala Gly Gly Met Asn Val Val Met Val Gly Arg Arg Glu Glu
            435                 440                 445

aag ctg aac gtg ctc gca ggc gag atc cgc gag acc tac ggc gtg gag        6611
Lys Leu Asn Val Leu Ala Gly Glu Ile Arg Glu Thr Tyr Gly Val Glu
        450                 455                 460

acc aag gtc gtg cgc gcc gac ttt agc cag ccc ggc gct gcc gag acc        6659
Thr Lys Val Val Arg Ala Asp Phe Ser Gln Pro Gly Ala Ala Glu Thr
    465                 470                 475

gtc ttc gcc gcg acc gag ggc ctg gac atg ggc ttc atg agc tac gtg        6707
Val Phe Ala Ala Thr Glu Gly Leu Asp Met Gly Phe Met Ser Tyr Val
480                 485                 490                 495

gcc tgc ctg cac agc ttc ggt aag atc cag gac acc ccc tgg gag aag        6755
Ala Cys Leu His Ser Phe Gly Lys Ile Gln Asp Thr Pro Trp Glu Lys
                500                 505                 510

cac gag gcc atg atc aac gtc aac gtc gtg acc ttc ctc aag tgc ttc        6803
His Glu Ala Met Ile Asn Val Asn Val Val Thr Phe Leu Lys Cys Phe
            515                 520                 525

cac cac tac atg cgg atc ttt gcc gcc cag gac cgc ggc gcc gtg atc        6851
His His Tyr Met Arg Ile Phe Ala Ala Gln Asp Arg Gly Ala Val Ile
        530                 535                 540

aac gtc tcg tcg atg acc ggc atc agc tcc agc ccc tgg aac ggc cag        6899
Asn Val Ser Ser Met Thr Gly Ile Ser Ser Ser Pro Trp Asn Gly Gln
    545                 550                 555

tac ggc gcg ggc aag gcc ttc atc ctc aag atg acc gag gcc gtg gcc        6947
Tyr Gly Ala Gly Lys Ala Phe Ile Leu Lys Met Thr Glu Ala Val Ala
560                 565                 570                 575

tgc gag tgc gag ggc acc ggc gtc gac gtc gag gtc atc acc ctc ggc        6995
Cys Glu Cys Glu Gly Thr Gly Val Asp Val Glu Val Ile Thr Leu Gly
                580                 585                 590

acc acc cta acc ccc agc ctg ctg tcc aac ctc ccc ggc ggc ccg cag        7043
Thr Thr Leu Thr Pro Ser Leu Leu Ser Asn Leu Pro Gly Gly Pro Gln
```

```
            595                 600                 605

ggc gag gcc gtc atg aag atc gcc ctc acc ccc gag gag tgc gtt gac       7091
Gly Glu Ala Val Met Lys Ile Ala Leu Thr Pro Glu Glu Cys Val Asp
        610                 615                 620

gag gcc ttt gag aag ctg ggt aag gag ctc tcc gtc atc gcc ggc cag       7139
Glu Ala Phe Glu Lys Leu Gly Lys Glu Leu Ser Val Ile Ala Gly Gln
    625                 630                 635

cgc aac aag gac tcc gtc cac gac tgg aag gca aac cac acc gag gac       7187
Arg Asn Lys Asp Ser Val His Asp Trp Lys Ala Asn His Thr Glu Asp
640                 645                 650                 655

gag tac atc cgc tac atg ggg tcg ttc tac cgc gac tag aagcttgcgg        7236
Glu Tyr Ile Arg Tyr Met Gly Ser Phe Tyr Arg Asp
                660                 665

ccgcactcga gcaccaccac caccaccact gagatccggc tgctaacaaa gcccgaaagg     7296

aagctgagtt ggctgctgcc accgctgagc aataactagc ataacccctt ggggcctcta     7356

aacgggtctt gaggggtttt ttgctgaaag gaggaactat atccggat                  7404
```

<210> 19
<211> 404
<212> PRT
<213> Artificial

<220>
<223> Synthetic Construct

<400> 19

```
Met Ala Lys Val Leu Cys Val Leu Tyr Asp Asp Pro Val Asp Gly Tyr
1               5                   10                  15

Pro Lys Thr Tyr Ala Arg Asp Asp Leu Pro Lys Ile Asp His Tyr Pro
            20                  25                  30

Gly Gly Gln Ile Leu Pro Thr Pro Lys Ala Ile Asp Phe Thr Pro Gly
        35                  40                  45

Gln Leu Leu Gly Ser Val Ser Gly Glu Leu Gly Leu Arg Glu Tyr Leu
    50                  55                  60

Glu Ser Asn Gly His Thr Leu Val Val Thr Ser Asp Lys Asp Gly Pro
65                  70                  75                  80

Asp Ser Val Phe Glu Arg Glu Leu Val Asp Ala Asp Val Val Ile Ser
                85                  90                  95

Gln Pro Phe Trp Pro Ala Tyr Leu Thr Pro Glu Arg Ile Ala Lys Ala
            100                 105                 110
```

```
Lys Asn Leu Lys Leu Ala Leu Thr Ala Gly Ile Gly Ser Asp His Val
        115                 120                 125

Asp Leu Gln Ser Ala Ile Asp Arg Asn Val Thr Val Ala Glu Val Thr
    130                 135                 140

Tyr Cys Asn Ser Ile Ser Val Ala Glu His Val Val Met Met Ile Leu
145                 150                 155                 160

Ser Leu Val Arg Asn Tyr Leu Pro Ser His Glu Trp Ala Arg Lys Gly
                165                 170                 175

Gly Trp Asn Ile Ala Asp Cys Val Ser His Ala Tyr Asp Leu Glu Ala
            180                 185                 190

Met His Val Gly Thr Val Ala Ala Gly Arg Ile Gly Leu Ala Val Leu
        195                 200                 205

Arg Arg Leu Ala Pro Phe Asp Val His Leu His Tyr Thr Gly Arg His
    210                 215                 220

Arg Leu Pro Glu Ser Val Glu Lys Glu Leu Asn Leu Thr Trp His Ala
225                 230                 235                 240

Thr Arg Glu Asp Met Tyr Pro Val Cys Asp Val Val Thr Leu Asn Cys
                245                 250                 255

Pro Leu His Pro Glu Thr Glu His Met Ile Asn Asp Glu Thr Leu Lys
            260                 265                 270

Leu Phe Lys Arg Gly Ala Tyr Ile Val Asn Thr Ala Arg Gly Lys Leu
        275                 280                 285

Cys Asp Arg Asp Ala Val Ala Arg Ala Leu Glu Ser Gly Arg Leu Ala
    290                 295                 300

Gly Tyr Ala Gly Asp Val Trp Phe Pro Gln Pro Ala Pro Lys Asp His
305                 310                 315                 320

Pro Trp Arg Thr Met Pro Tyr Asn Gly Met Thr Pro His Ile Ser Gly
                325                 330                 335

Thr Thr Leu Thr Ala Gln Ala Arg Tyr Ala Ala Gly Thr Arg Glu Ile
            340                 345                 350

Leu Glu Cys Phe Phe Glu Gly Arg Pro Ile Arg Asp Glu Tyr Leu Ile
        355                 360                 365
```

Val Gln Gly Gly Ala Leu Ala Gly Thr Gly Ala His Ser Tyr Ser Lys
370 375 380

Gly Asn Ala Thr Gly Gly Ser Glu Glu Ala Ala Lys Phe Lys Lys Ala
385 390 395 400

Ala Glu Asn Ser

<210> 20
<211> 263
<212> PRT
<213> Artificial

<220>
<223> Synthetic Construct

<400> 20

Met Asn Leu Arg Glu Lys Tyr Gly Glu Trp Gly Leu Ile Leu Gly Ala
1 5 10 15

Thr Glu Gly Val Gly Lys Ala Phe Cys Glu Lys Ile Ala Ala Gly Gly
20 25 30

Met Asn Val Val Met Val Gly Arg Arg Glu Glu Lys Leu Asn Val Leu
35 40 45

Ala Gly Glu Ile Arg Glu Thr Tyr Gly Val Glu Thr Lys Val Val Arg
50 55 60

Ala Asp Phe Ser Gln Pro Gly Ala Ala Glu Thr Val Phe Ala Ala Thr
65 70 75 80

Glu Gly Leu Asp Met Gly Phe Met Ser Tyr Val Ala Cys Leu His Ser
85 90 95

Phe Gly Lys Ile Gln Asp Thr Pro Trp Glu Lys His Glu Ala Met Ile
100 105 110

Asn Val Asn Val Val Thr Phe Leu Lys Cys Phe His His Tyr Met Arg
115 120 125

Ile Phe Ala Ala Gln Asp Arg Gly Ala Val Ile Asn Val Ser Ser Met
130 135 140

Thr Gly Ile Ser Ser Ser Pro Trp Asn Gly Gln Tyr Gly Ala Gly Lys
145 150 155 160

```
Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 21
<211> 4302
<212> DNA
<213> Artificial

<220>
<223> Plasmid pCOLA(mod) 3alpha-HSDH

<220>
<221> CDS
<222> (303) .. (1076)
<223> 3alpha-HSDH

<400> 21

```
ggggaattgt gagcggataa caattcccct gtagaaataa ttttgtttaa ctttaataag      60

gagatatacc atgggcagca gccatcacca tcatcaccac agccaggatc cgaattcgag     120

ctcggcgcgc ctgcaggtcg acaagcttgc ggccgcataa tgcttaagtc gaacagaaag     180

taatcgtatt gtacacggcc gcataatcga aattaatacg actcactata ggggaattgt     240

gagcggataa caattcccca tcttagtata ttagttaagt ataagaagga gatatacata     300

tg atg tcc atc atc gtg ata agc ggc tgc gcc acc ggc att ggt gcc        347
   Met Ser Ile Ile Val Ile Ser Gly Cys Ala Thr Gly Ile Gly Ala
   1               5                   10                  15

gct acg cgc aag gtc ctg gag gcg gcc ggt cac cag atc gta ggc atc       395
Ala Thr Arg Lys Val Leu Glu Ala Ala Gly His Gln Ile Val Gly Ile
                20                  25                  30

gat ata cgc gat gcg gaa gtg att gcc gat ctc tcg acg gcc gaa ggt       443
Asp Ile Arg Asp Ala Glu Val Ile Ala Asp Leu Ser Thr Ala Glu Gly
```

```
                35                  40                  45

cga aag cag gcg att gcc gat gta ctg gcg aag tgc agc aag ggc atg        491
Arg Lys Gln Ala Ile Ala Asp Val Leu Ala Lys Cys Ser Lys Gly Met
        50                  55                  60

gac ggc ctg gtg ctg tgc gcc ggc ctg gga ccg cag acc aag gtg ctt        539
Asp Gly Leu Val Leu Cys Ala Gly Leu Gly Pro Gln Thr Lys Val Leu
    65                  70                  75

ggc aat gtg gtt tcg gtc aat tat ttt ggc gcg acc gag ctg atg gat        587
Gly Asn Val Val Ser Val Asn Tyr Phe Gly Ala Thr Glu Leu Met Asp
80                  85                  90                  95

gcc ttt ttg cca gcg ctg aaa aaa ggc cat cag ccc gca gcc gtc gtc        635
Ala Phe Leu Pro Ala Leu Lys Lys Gly His Gln Pro Ala Ala Val Val
                100                 105                 110

atc tcg tcc gtg gct tcc gcg cat ctg gct ttt gac aag aac cca ctg        683
Ile Ser Ser Val Ala Ser Ala His Leu Ala Phe Asp Lys Asn Pro Leu
            115                 120                 125

gcg ctg gca ctg gaa gcc ggc gag gaa gcc aag gcc cgc gcc att gtc        731
Ala Leu Ala Leu Glu Ala Gly Glu Glu Ala Lys Ala Arg Ala Ile Val
        130                 135                 140

gaa cat gcg gga gag cag ggc gga aat ctg gcc tat gcg ggc agc aag        779
Glu His Ala Gly Glu Gln Gly Gly Asn Leu Ala Tyr Ala Gly Ser Lys
    145                 150                 155

aat gct ttg acg gtg gct gtg cgc aaa cgc gcc gcc gcc tgg ggc gag        827
Asn Ala Leu Thr Val Ala Val Arg Lys Arg Ala Ala Ala Trp Gly Glu
160                 165                 170                 175

gct ggc gtg cgc ctg aac acc atc gcc ccc ggt gca acc gag act ccc        875
Ala Gly Val Arg Leu Asn Thr Ile Ala Pro Gly Ala Thr Glu Thr Pro
                180                 185                 190

ttg ctg cag gcg ggc ctg cag gac ccg cgc tat ggc gaa tcc att gcc        923
Leu Leu Gln Ala Gly Leu Gln Asp Pro Arg Tyr Gly Glu Ser Ile Ala
            195                 200                 205

aag ttc gtt cct ccc atg ggc cgc cgt gcc gag ccg tcc gag atg gcg        971
Lys Phe Val Pro Pro Met Gly Arg Arg Ala Glu Pro Ser Glu Met Ala
        210                 215                 220

tcg gtc atc gcc ttt ttg atg agc ccg gcc gca agc tat gtg cat ggc       1019
Ser Val Ile Ala Phe Leu Met Ser Pro Ala Ala Ser Tyr Val His Gly
    225                 230                 235

gcg cag atc gtc att gat ggc ggc att gat gcg gtg atg cgc ccg aca       1067
Ala Gln Ile Val Ile Asp Gly Gly Ile Asp Ala Val Met Arg Pro Thr
240                 245                 250                 255

cag ttc tga gaattcgagc tccgtcgaca agcttgcggc cgcactcgag               1116
Gln Phe

caccaccacc accaccactg agatccggct gctaacaaag cccgaaagga agctgagttg    1176

gctgctgcca ccgctgagca ataactagca taaccccttg gggcctctaa acgggtcttg    1236

aggggttttt tgctgaaacc tcaggcattt gagaagcaca cggtcacact gcttccggta    1296
```

gtcaataaac cggtaaacca gcaatagaca taagcggcta tttaacgacc ctgccctgaa 1356
ccgacgacaa gctgacgacc gggtctccgc aagtggcact tttcggggaa atgtgcgcgg 1416
aacccctatt tgtttatttt tctaaataca ttcaaatatg tatccgctca tgaattaatt 1476
cttacgcccc gccctgccac tcatcgcagt actgttgtaa ttcattaagc attctgccga 1536
catggaagcc atcacagacg gcatgatgaa cctgaatcgc cagcggcatc agcaccttgt 1596
cgccttgcgt ataatatttg cctatggtga aaacgggggc gaagaagttg tccatattgg 1656
ccacgtttaa atcaaaactg gtgaaactca cccagggatt ggctgagacg aaaaacatat 1716
tctcaataaa ccctttaggg aaataggcca ggttttcacc gtaacacgcc acatcttgcg 1776
aatatatgtg tagaaactgc cggaaatcgt cgtggtattc actccagagc gatgaaaacg 1836
tttcagtttg ctcatggaaa acggtgtaac aagggtgaac actatcccat atcaccagct 1896
caccgtcttt cattgccata cggaattccg gatgagcatt catcaggcgg gcaagaatgt 1956
gaataaaggc cggataaaac ttgtgcttat ttttctttac ggtctttaaa aaggccgtaa 2016
tatccagctg aacggtctgg ttataggtac attgagcaac tgactgaaat gcctcaaaat 2076
gttctttacg atgccattgg gatatatcaa cggtggtata tccagtgatt tttttctcca 2136
tactcttcct ttttcaatat tattgaagca tttatcaggg ttattgtctc atgagcggat 2196
acatatttga atgtatttag aaaaataaac aaataggcat gctagcgcag aaacgtccta 2256
gaagatgcca ggaggatact tagcagagag acaataaggc cggagcgaag ccgtttttcc 2316
ataggctccg cccccctgac gaacatcacg aaatctgacg ctcaaatcag tggtggcgaa 2376
acccgacagg actataaaga taccaggcgt ttccccctga tggctccctc ttgcgctctc 2436
ctgttcccgt cctgcggcgt ccgtgttgtg gtggaggctt tacccaaatc accacgtccc 2496
gttccgtgta gacagttcgc tccaagctgg gctgtgtgca agaacccccc gttcagcccg 2556
actgctgcgc cttatccggt aactatcatc ttgagtccaa cccggaaaga cacgacaaaa 2616
cgccactggc agcagccatt ggtaactgag aattagtgga tttagatatc gagagtcttg 2676
aagtggtggc ctaacagagg ctacactgaa aggacagtat ttggtatctg cgctccacta 2736
aagccagtta ccaggttaag cagttcccca actgacttaa ccttcgatca aaccgcctcc 2796
ccaggcggtt ttttcgttta cagagcagga gattacgacg atcgtaaaag gatctcaaga 2856
agatccttta cggattcccg acaccatcac tctagatttc agtgcaattt atctcttcaa 2916
atgtagcacc tgaagtcagc cccatacgat ataagttgta attctcatgt tagtcatgcc 2976
ccgcgcccac cggaaggagc tgactgggtt gaaggctctc aagggcatcg gtcgagatcc 3036
cggtgcctaa tgagtgagct aacttacatt aattgcgttg cgctcactgc ccgctttcca 3096
gtcgggaaac ctgtcgtgcc agctgcatta atgaatcggc caacgcgcgg ggagaggcgg 3156

```
tttgcgtatt gggcgccagg gtggtttttc ttttcaccag tgagacgggc aacagctgat     3216
tgcccttcac cgcctggccc tgagagagtt gcagcaagcg gtccacgctg gtttgcccca     3276
gcaggcgaaa atcctgtttg atggtggtta acggcgggat ataacatgag ctgtcttcgg     3336
tatcgtcgta tcccactacc gagatgtccg caccaacgcg cagcccggac tcggtaatgg     3396
cgcgcattgc gcccagcgcc atctgatcgt tggcaaccag catcgcagtg ggaacgatgc     3456
cctcattcag catttgcatg gtttgttgaa aaccggacat ggcactccag tcgccttccc     3516
gttccgctat cggctgaatt tgattgcgag tgagatattt atgccagcca gccagacgca     3576
gacgcgccga gacagaactt aatgggcccg ctaacagcgc gatttgctgg tgacccaatg     3636
cgaccagatg ctccacgccc agtcgcgtac cgtcttcatg ggagaaaata atactgttga     3696
tgggtgtctg gtcagagaca tcaagaaata acgccggaac attagtgcag gcagcttcca     3756
cagcaatggc atcctggtca tccagcggat agttaatgat cagcccactg acgcgttgcg     3816
cgagaagatt gtgcaccgcc gctttacagg cttcgacgcc gcttcgttct accatcgaca     3876
ccaccacgct ggcacccagt tgatcggcgc gagatttaat cgccgcgaca atttgcgacg     3936
gcgcgtgcag ggccagactg gaggtggcaa cgccaatcag caacgactgt ttgcccgcca     3996
gttgttgtgc cacgcggttg ggaatgtaat tcagctccgc catcgccgct tccacttttt     4056
cccgcgtttt cgcagaaacg tggctggcct ggttcaccac gcgggaaacg gtctgataag     4116
agacaccggc atactctgcg acatcgtata acgttactgg tttcacattc accaccctga     4176
attgactctc ttccgggcgc tatcatgcca taccgcgaaa ggttttgcgc cattcgatgg     4236
tgtccgggat ctcgacgctc tcccttatgc gactcctgca ttaggaaatt aatacgactc     4296
actata                                                                4302
```

<210> 22
<211> 257
<212> PRT
<213> Artificial

<220>
<223> Synthetic Construct

<400> 22

```
Met Ser Ile Ile Val Ile Ser Gly Cys Ala Thr Gly Ile Gly Ala Ala
1               5                   10                  15

Thr Arg Lys Val Leu Glu Ala Ala Gly His Gln Ile Val Gly Ile Asp
            20                  25                  30

Ile Arg Asp Ala Glu Val Ile Ala Asp Leu Ser Thr Ala Glu Gly Arg
        35                  40                  45
```

```
Lys Gln Ala Ile Ala Asp Val Leu Ala Lys Cys Ser Lys Gly Met Asp
    50                  55                  60

Gly Leu Val Leu Cys Ala Gly Leu Gly Pro Gln Thr Lys Val Leu Gly
65                  70                  75                  80

Asn Val Val Ser Val Asn Tyr Phe Gly Ala Thr Glu Leu Met Asp Ala
                85                  90                  95

Phe Leu Pro Ala Leu Lys Lys Gly His Gln Pro Ala Ala Val Val Ile
            100                 105                 110

Ser Ser Val Ala Ser Ala His Leu Ala Phe Asp Lys Asn Pro Leu Ala
        115                 120                 125

Leu Ala Leu Glu Ala Gly Glu Glu Ala Lys Ala Arg Ala Ile Val Glu
    130                 135                 140

His Ala Gly Glu Gln Gly Gly Asn Leu Ala Tyr Ala Gly Ser Lys Asn
145                 150                 155                 160

Ala Leu Thr Val Ala Val Arg Lys Arg Ala Ala Ala Trp Gly Glu Ala
                165                 170                 175

Gly Val Arg Leu Asn Thr Ile Ala Pro Gly Ala Thr Glu Thr Pro Leu
            180                 185                 190

Leu Gln Ala Gly Leu Gln Asp Pro Arg Tyr Gly Glu Ser Ile Ala Lys
        195                 200                 205

Phe Val Pro Pro Met Gly Arg Arg Ala Glu Pro Ser Glu Met Ala Ser
    210                 215                 220

Val Ile Ala Phe Leu Met Ser Pro Ala Ala Ser Tyr Val His Gly Ala
225                 230                 235                 240

Gln Ile Val Ile Asp Gly Gly Ile Asp Ala Val Met Arg Pro Thr Gln
                245                 250                 255

Phe
```

<210> 23
<211> 29
<212> DNA
<213> Artificial

<220>
<223> PCR Primer

<400> 23
cgatcatatg gcaaaggtcc tgtgcgttc 29

<210> 24
<211> 29
<212> DNA
<213> Artificial

<220>
<223> PCR Primer

<400> 24
gctagaattc tcagccgcct tcttgaact 29

<210> 25
<211> 768
<212> DNA
<213> Escherichia coli

<220>
<221> CDS
<222> (1)..(768)

<400> 25

```
gtg ttt aat tct gac aac ctg aga ctc gac gga aaa tgc gcc atc atc          48
Val Phe Asn Ser Asp Asn Leu Arg Leu Asp Gly Lys Cys Ala Ile Ile
1               5                   10                  15

aca ggt gcg ggt gca ggt att ggt aaa gaa atc gcc att aca ttc gcg          96
Thr Gly Ala Gly Ala Gly Ile Gly Lys Glu Ile Ala Ile Thr Phe Ala
            20                  25                  30

aca gct ggc gca tct gtg gtg gtc agt gat att aac gcc gac gca gct         144
Thr Ala Gly Ala Ser Val Val Val Ser Asp Ile Asn Ala Asp Ala Ala
        35                  40                  45

aac cat gtt gta gac gaa att caa caa ctg ggt ggt cag gca ttt gcc         192
Asn His Val Val Asp Glu Ile Gln Gln Leu Gly Gly Gln Ala Phe Ala
    50                  55                  60

tgc cgt tgt gat att act tcc gaa cag gaa ctc tct gca ctg gca gac         240
Cys Arg Cys Asp Ile Thr Ser Glu Gln Glu Leu Ser Ala Leu Ala Asp
65                  70                  75                  80

ttt gct atc agt aag ctg ggt aaa gtt gat att ctg gtt aac aac gcc         288
Phe Ala Ile Ser Lys Leu Gly Lys Val Asp Ile Leu Val Asn Asn Ala
                85                  90                  95

ggt ggc ggt gga cct aaa ccg ttt gat atg cca atg gcg gat ttt cgc         336
Gly Gly Gly Gly Pro Lys Pro Phe Asp Met Pro Met Ala Asp Phe Arg
            100                 105                 110

cgt gct tat gaa ctg aat gtg ttt tct ttt ttc cat ctg tca caa ctt         384
Arg Ala Tyr Glu Leu Asn Val Phe Ser Phe Phe His Leu Ser Gln Leu
        115                 120                 125

gtt gcg cca gaa atg gaa aaa aat ggc ggt ggc gtt att ctg acc atc         432
Val Ala Pro Glu Met Glu Lys Asn Gly Gly Gly Val Ile Leu Thr Ile
    130                 135                 140
```

```
act tct atg gcg gca gaa aat aaa aat ata aac atg act tcc tat gca          480
Thr Ser Met Ala Ala Glu Asn Lys Asn Ile Asn Met Thr Ser Tyr Ala
145                 150                 155                 160

tca tct aaa gct gcg gcc agt cat ctg gtc aga aat atg gcg ttt gac          528
Ser Ser Lys Ala Ala Ala Ser His Leu Val Arg Asn Met Ala Phe Asp
                165                 170                 175

cta ggt gaa aaa aat att cgg gta aat ggc att gcg ccg ggg gca ata          576
Leu Gly Glu Lys Asn Ile Arg Val Asn Gly Ile Ala Pro Gly Ala Ile
            180                 185                 190

tta acc gat gcc ctg aaa tcc gtt att aca cca gaa att gaa caa aaa          624
Leu Thr Asp Ala Leu Lys Ser Val Ile Thr Pro Glu Ile Glu Gln Lys
        195                 200                 205

atg tta cag cac acg ccg atc aga cgt ctg ggc caa ccg caa gat att          672
Met Leu Gln His Thr Pro Ile Arg Arg Leu Gly Gln Pro Gln Asp Ile
    210                 215                 220

gct aac gca gcg ctg ttc ctt tgc tcg cct gct gcg agc tgg gta agc          720
Ala Asn Ala Ala Leu Phe Leu Cys Ser Pro Ala Ala Ser Trp Val Ser
225                 230                 235                 240

gga caa att ctc acc gtc tcc ggt ggt ggg gta cag gag ctc aat taa          768
Gly Gln Ile Leu Thr Val Ser Gly Gly Gly Val Gln Glu Leu Asn
                245                 250                 255
```

<210> 26
<211> 255
<212> PRT
<213> Escherichia coli

<400> 26

```
Val Phe Asn Ser Asp Asn Leu Arg Leu Asp Gly Lys Cys Ala Ile Ile
1               5                   10                  15

Thr Gly Ala Gly Ala Gly Ile Gly Lys Glu Ile Ala Ile Thr Phe Ala
            20                  25                  30

Thr Ala Gly Ala Ser Val Val Val Ser Asp Ile Asn Ala Asp Ala Ala
        35                  40                  45

Asn His Val Val Asp Glu Ile Gln Gln Leu Gly Gly Gln Ala Phe Ala
    50                  55                  60

Cys Arg Cys Asp Ile Thr Ser Glu Gln Glu Leu Ser Ala Leu Ala Asp
65                  70                  75                  80

Phe Ala Ile Ser Lys Leu Gly Lys Val Asp Ile Leu Val Asn Asn Ala
                85                  90                  95

Gly Gly Gly Gly Pro Lys Pro Phe Asp Met Pro Met Ala Asp Phe Arg
            100                 105                 110

Arg Ala Tyr Glu Leu Asn Val Phe Ser Phe Phe His Leu Ser Gln Leu
        115                 120                 125

Val Ala Pro Glu Met Glu Lys Asn Gly Gly Gly Val Ile Leu Thr Ile
    130                 135                 140

Thr Ser Met Ala Ala Glu Asn Lys Asn Ile Asn Met Thr Ser Tyr Ala
145                 150                 155                 160

Ser Ser Lys Ala Ala Ala Ser His Leu Val Arg Asn Met Ala Phe Asp
                165                 170                 175

Leu Gly Glu Lys Asn Ile Arg Val Asn Gly Ile Ala Pro Gly Ala Ile
            180                 185                 190

Leu Thr Asp Ala Leu Lys Ser Val Ile Thr Pro Glu Ile Glu Gln Lys
        195                 200                 205

Met Leu Gln His Thr Pro Ile Arg Arg Leu Gly Gln Pro Gln Asp Ile
    210                 215                 220

Ala Asn Ala Ala Leu Phe Leu Cys Ser Pro Ala Ala Ser Trp Val Ser
225                 230                 235                 240

Gly Gln Ile Leu Thr Val Ser Gly Gly Gly Val Gln Glu Leu Asn
                245                 250                 255
```

<210> 27
<211> 53
<212> DNA
<213> Artificial

<220>
<223> PCR Primer

<400> 27
aaaaaagctt ataattatcc ttataggacg tcatggtgcg cccagatagg gtg 53

<210> 28
<211> 60
<212> DNA
<213> Artificial

<220>
<223> PCR Primer

<400> 28
cagattgtac aaatgtggtg ataacagata agtcgtcatg tttaacttac ctttctttgt 60

<210> 29
<211> 49
<212> DNA
<213> Artificial

<220>
<223> PCR Primer

<400> 29
tgaacgcaag tttctaattt cggtttccta tcgatagagg aaagtgtct 49

<210> 30
<211> 29
<212> DNA
<213> Artificial

<220>
<223> PCR Primer

<400> 30
cctgcactac accggccgtc accgcctgc 29

<210> 31
<211> 23
<212> DNA
<213> Artificial

<220>
<223> PCR Primer

<400> 31
gctcgaattc tcagaccgcc ttc 23

<210> 32
<211> 27
<212> DNA

<213> Artificial

<220>
<223> PCR Primer

<400> 32
ttaattgagc tcctgtaccc caccacc 27

<210> 33
<211> 30
<212> DNA
<213> Artificial

<220>
<223> PCR Primer

<400> 33
gtgtttaatt ctgacaacct gagactcgac 30

<210> 34
<211> 1266
<212> DNA
<213> Artificial

<220>
<223> FDH D221G Mutante mit His-Tag

<220>
<221> CDS
<222> (1) .. (1266)

<400> 34

```
atg gca aag gtc ctg tgc gtt ctt tac gat gat ccg gtc gac ggc tac        48
Met Ala Lys Val Leu Cys Val Leu Tyr Asp Asp Pro Val Asp Gly Tyr
1               5                   10                  15

ccg aag acc tat gcc cgc gac gat ctt ccg aag atc gac cac tat ccg        96
Pro Lys Thr Tyr Ala Arg Asp Asp Leu Pro Lys Ile Asp His Tyr Pro
            20                  25                  30

ggc ggc cag atc ttg ccg acg ccg aag gcc atc gac ttc acg ccc ggg       144
Gly Gly Gln Ile Leu Pro Thr Pro Lys Ala Ile Asp Phe Thr Pro Gly
        35                  40                  45

cag ttg ctc ggc tcc gtc tcc ggc gag ctc ggc ctg cgc gaa tat ctc       192
Gln Leu Leu Gly Ser Val Ser Gly Glu Leu Gly Leu Arg Glu Tyr Leu
    50                  55                  60

gaa tcc aac ggc cac acc ctg gtc gtg acc tcc gac aag gac ggc ccc       240
Glu Ser Asn Gly His Thr Leu Val Val Thr Ser Asp Lys Asp Gly Pro
65                  70                  75                  80

gac tcg gtg ttc gag cgc gag ctg gtc gat gcg gat gtc gtc atc tcc       288
Asp Ser Val Phe Glu Arg Glu Leu Val Asp Ala Asp Val Val Ile Ser
                85                  90                  95

cag ccc ttc tgg ccg gcc tat ctg acg ccc gag cgc atc gcc aag gcc       336
Gln Pro Phe Trp Pro Ala Tyr Leu Thr Pro Glu Arg Ile Ala Lys Ala
            100                 105                 110

aag aac ctg aag ctc gcg ctc acc gcc ggc atc ggt tcc gac cac gtc       384
Lys Asn Leu Lys Leu Ala Leu Thr Ala Gly Ile Gly Ser Asp His Val
        115                 120                 125

gat ctt cag tcg gct atc gac cgc aac gtc acc gtg gcg gaa gtc acc       432
Asp Leu Gln Ser Ala Ile Asp Arg Asn Val Thr Val Ala Glu Val Thr
    130                 135                 140

tac tgc aac tcg atc agc gtc gcc gag cat gtg gtg atg atg atc ctg       480
Tyr Cys Asn Ser Ile Ser Val Ala Glu His Val Val Met Met Ile Leu
145                 150                 155                 160

tcg ctg gtg cgc aac tat ctg ccc tcg cac gaa tgg gcg cgg aag ggc       528
Ser Leu Val Arg Asn Tyr Leu Pro Ser His Glu Trp Ala Arg Lys Gly
                165                 170                 175

ggc tgg aac atc gcc gac tgc gtc tcc cac gcc tac gac ctc gag gcg       576
Gly Trp Asn Ile Ala Asp Cys Val Ser His Ala Tyr Asp Leu Glu Ala
            180                 185                 190

atg cat gtc ggc acc gtg gcc gcc ggc cgc atc ggt ctc gcg gtg ctg       624
Met His Val Gly Thr Val Ala Ala Gly Arg Ile Gly Leu Ala Val Leu
        195                 200                 205

cgc cgt ctg gcg ccg ttc gac gtg cac ctg cac tac acc ggc cgt cac       672
```

```
Arg Arg Leu Ala Pro Phe Asp Val His Leu His Tyr Thr Gly Arg His
    210                 215                 220

cgc ctg ccg gaa tcg gtc gag aag gag ctc aac ctc acc tgg cac gcg       720
Arg Leu Pro Glu Ser Val Glu Lys Glu Leu Asn Leu Thr Trp His Ala
225                 230                 235                 240

acc cgc gag gac atg tat ccg gtt tgc gac gtg gtg acg ctg aac tgc       768
Thr Arg Glu Asp Met Tyr Pro Val Cys Asp Val Val Thr Leu Asn Cys
                245                 250                 255

ccg ctg cac ccc gaa acc gag cac atg atc aat gac gag acg ctg aag       816
Pro Leu His Pro Glu Thr Glu His Met Ile Asn Asp Glu Thr Leu Lys
            260                 265                 270

ctg ttc aag cgt ggc gcc tac atc gtc aac acc gcc cgc ggc aag ctg       864
Leu Phe Lys Arg Gly Ala Tyr Ile Val Asn Thr Ala Arg Gly Lys Leu
        275                 280                 285

tgc gac cgc gat gcc gtg gca cgt gcg ctc gaa tcc ggc cgg ctg gcc       912
Cys Asp Arg Asp Ala Val Ala Arg Ala Leu Glu Ser Gly Arg Leu Ala
    290                 295                 300

ggc tat gcc ggc gac gtg tgg ttc ccg cag ccg gcg ccg aag gac cac       960
Gly Tyr Ala Gly Asp Val Trp Phe Pro Gln Pro Ala Pro Lys Asp His
305                 310                 315                 320

ccc tgg cgg acg atg ccc tat aac ggc atg acc ccg cac atc tcc ggc      1008
Pro Trp Arg Thr Met Pro Tyr Asn Gly Met Thr Pro His Ile Ser Gly
                325                 330                 335

acc acg ctg acc gcg cag gcg cgt tat gcg gcg ggc acc cgc gag atc      1056
Thr Thr Leu Thr Ala Gln Ala Arg Tyr Ala Ala Gly Thr Arg Glu Ile
            340                 345                 350

ctg gag tgc ttc ttc gag ggc cgt ccg atc cgc gac gaa tac ctc atc      1104
Leu Glu Cys Phe Phe Glu Gly Arg Pro Ile Arg Asp Glu Tyr Leu Ile
        355                 360                 365

gtg cag ggc ggc gct ctt gcc ggc acc ggc gcg cat tcc tac tcg aag      1152
Val Gln Gly Gly Ala Leu Ala Gly Thr Gly Ala His Ser Tyr Ser Lys
    370                 375                 380

ggc aat gcc acc ggc ggt tcg gaa gag gcc gcc aag ttc aag aag gcg      1200
Gly Asn Ala Thr Gly Gly Ser Glu Glu Ala Ala Lys Phe Lys Lys Ala
385                 390                 395                 400

gct gag aat tcg agc tcc gtc gac aag ctt gcg gcc gca ctc gag cac      1248
Ala Glu Asn Ser Ser Ser Val Asp Lys Leu Ala Ala Ala Leu Glu His
                405                 410                 415

cac cac cac cac cac tga                                              1266
His His His His His
            420
```

<210> 35
<211> 421
<212> PRT
<213> Artificial

<220>

<223> Synthetic Construct

<400> 35

```
Met Ala Lys Val Leu Cys Val Leu Tyr Asp Asp Pro Val Asp Gly Tyr
1               5                   10                  15

Pro Lys Thr Tyr Ala Arg Asp Asp Leu Pro Lys Ile Asp His Tyr Pro
            20                  25                  30

Gly Gly Gln Ile Leu Pro Thr Pro Lys Ala Ile Asp Phe Thr Pro Gly
        35                  40                  45

Gln Leu Leu Gly Ser Val Ser Gly Glu Leu Gly Leu Arg Glu Tyr Leu
    50                  55                  60

Glu Ser Asn Gly His Thr Leu Val Val Thr Ser Asp Lys Asp Gly Pro
65                  70                  75                  80

Asp Ser Val Phe Glu Arg Glu Leu Val Asp Ala Asp Val Val Ile Ser
                85                  90                  95

Gln Pro Phe Trp Pro Ala Tyr Leu Thr Pro Glu Arg Ile Ala Lys Ala
            100                 105                 110

Lys Asn Leu Lys Leu Ala Leu Thr Ala Gly Ile Gly Ser Asp His Val
        115                 120                 125

Asp Leu Gln Ser Ala Ile Asp Arg Asn Val Thr Val Ala Glu Val Thr
    130                 135                 140

Tyr Cys Asn Ser Ile Ser Val Ala Glu His Val Val Met Met Ile Leu
145                 150                 155                 160

Ser Leu Val Arg Asn Tyr Leu Pro Ser His Glu Trp Ala Arg Lys Gly
                165                 170                 175

Gly Trp Asn Ile Ala Asp Cys Val Ser His Ala Tyr Asp Leu Glu Ala
            180                 185                 190

Met His Val Gly Thr Val Ala Ala Gly Arg Ile Gly Leu Ala Val Leu
        195                 200                 205

Arg Arg Leu Ala Pro Phe Asp Val His Leu His Tyr Thr Gly Arg His
    210                 215                 220

Arg Leu Pro Glu Ser Val Glu Lys Glu Leu Asn Leu Thr Trp His Ala
225                 230                 235                 240
```

```
Thr Arg Glu Asp Met Tyr Pro Val Cys Asp Val Val Thr Leu Asn Cys
                245                 250                 255

Pro Leu His Pro Glu Thr Glu His Met Ile Asn Asp Glu Thr Leu Lys
            260                 265                 270

Leu Phe Lys Arg Gly Ala Tyr Ile Val Asn Thr Ala Arg Gly Lys Leu
        275                 280                 285

Cys Asp Arg Asp Ala Val Ala Arg Ala Leu Glu Ser Gly Arg Leu Ala
    290                 295                 300

Gly Tyr Ala Gly Asp Val Trp Phe Pro Gln Pro Ala Pro Lys Asp His
305                 310                 315                 320

Pro Trp Arg Thr Met Pro Tyr Asn Gly Met Thr Pro His Ile Ser Gly
                325                 330                 335

Thr Thr Leu Thr Ala Gln Ala Arg Tyr Ala Ala Gly Thr Arg Glu Ile
            340                 345                 350

Leu Glu Cys Phe Phe Glu Gly Arg Pro Ile Arg Asp Glu Tyr Leu Ile
        355                 360                 365

Val Gln Gly Gly Ala Leu Ala Gly Thr Gly Ala His Ser Tyr Ser Lys
    370                 375                 380

Gly Asn Ala Thr Gly Gly Ser Glu Glu Ala Ala Lys Phe Lys Lys Ala
385                 390                 395                 400

Ala Glu Asn Ser Ser Ser Val Asp Lys Leu Ala Ala Ala Leu Glu His
                405                 410                 415

His His His His His
            420
```

<210> 36
<211> 401
<212> PRT
<213> Mycobacterium vaccae

<400> 36

```
Met Ala Lys Val Leu Cys Val Leu Tyr Asp Asp Pro Val Asp Gly Tyr
1               5                   10                  15

Pro Lys Thr Tyr Ala Arg Asp Asp Leu Pro Lys Ile Asp His Tyr Pro
            20                  25                  30
```

Gly Gly Gln Ile Leu Pro Thr Pro Lys Ala Ile Asp Phe Thr Pro Gly
35 40 45

Gln Leu Leu Gly Ser Val Ser Gly Glu Leu Gly Leu Arg Glu Tyr Leu
50 55 60

Glu Ser Asn Gly His Thr Leu Val Val Thr Ser Asp Lys Asp Gly Pro
65 70 75 80

Asp Ser Val Phe Glu Arg Glu Leu Val Asp Ala Asp Val Val Ile Ser
85 90 95

Gln Pro Phe Trp Pro Ala Tyr Leu Thr Pro Glu Arg Ile Ala Lys Ala
100 105 110

Lys Asn Leu Lys Leu Ala Leu Thr Ala Gly Ile Gly Ser Asp His Val
115 120 125

Asp Leu Gln Ser Ala Ile Asp Arg Asn Val Thr Val Ala Glu Val Thr
130 135 140

Tyr Cys Asn Ser Ile Ser Val Ala Glu His Val Val Met Met Ile Leu
145 150 155 160

Ser Leu Val Arg Asn Tyr Leu Pro Ser His Glu Trp Ala Arg Lys Gly
165 170 175

Gly Trp Asn Ile Ala Asp Cys Val Ser His Ala Tyr Asp Leu Glu Ala
180 185 190

Met His Val Gly Thr Val Ala Ala Gly Arg Ile Gly Leu Ala Val Leu
195 200 205

Arg Arg Leu Ala Pro Phe Asp Val His Leu His Tyr Thr Asp Arg His
210 215 220

Arg Leu Pro Glu Ser Val Glu Lys Glu Leu Asn Leu Thr Trp His Ala
225 230 235 240

Thr Arg Glu Asp Met Tyr Pro Val Cys Asp Val Val Thr Leu Asn Cys
245 250 255

Pro Leu His Pro Glu Thr Glu His Met Ile Asn Asp Glu Thr Leu Lys
260 265 270

Leu Phe Lys Arg Gly Ala Tyr Ile Val Asn Thr Ala Arg Gly Lys Leu
275 280 285

Cys Asp Arg Asp Ala Val Ala Arg Ala Leu Glu Ser Gly Arg Leu Ala
    290                 295                 300

Gly Tyr Ala Gly Asp Val Trp Phe Pro Gln Pro Ala Pro Lys Asp His
305                 310                 315                 320

Pro Trp Arg Thr Met Pro Tyr Asn Gly Met Thr Pro His Ile Ser Gly
                325                 330                 335

Thr Thr Leu Thr Ala Gln Ala Arg Tyr Ala Ala Gly Thr Arg Glu Ile
            340                 345                 350

Leu Glu Cys Phe Phe Glu Gly Arg Pro Ile Arg Asp Glu Tyr Leu Ile
        355                 360                 365

Val Gln Gly Gly Ala Leu Ala Gly Thr Gly Ala His Ser Tyr Ser Lys
    370                 375                 380

Gly Asn Ala Thr Gly Gly Ser Glu Glu Ala Ala Lys Phe Lys Lys Ala
385                 390                 395                 400

Val

<210> 37
<211> 263
<212> PRT
<213> artificial

<220>
<223> abgeleitet aus Collinsella aerofaciens (G39D)

<400> 37

Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Asp Arg Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

```
Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 38
<211> 263
<212> PRT
<213> artificial

<220>
<223> abgeleitet aus Collinsella aerofaciens (G39D R40L)

<400> 38

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
```

```
                20                  25                  30

Met Asn Val Val Met Val Asp Leu Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 39
<211> 263
<212> PRT
<213> artificial

<220>
<223> abegleitet aus Collinsella aerofaciens (G39D R40I)

<400> 39

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Asp Ile Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
```

210 215 220

Leu Gly Lys Glu Leu Ser Val Ile Ala Gly Gln Arg Asn Lys Asp Ser
225 230 235 240

Val His Asp Trp Lys Ala Asn His Thr Glu Asp Glu Tyr Ile Arg Tyr
245 250 255

Met Gly Ser Phe Tyr Arg Asp
260

<210> 40
<211> 263
<212> PRT
<213> artificial

<220>
<223> abgeleitet aus Collinsella aerofaciens

<400> 40

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Asp Val Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160
```

```
Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 41
<211> 26
<212> DNA
<213> artificial

<220>
<223> PCR Primer

<400> 41
cgtcgtcatg gtcgaccgtc gcgagg 26

<210> 42
<211> 26
<212> DNA
<213> artificial

<220>
<223> PCR Primer

<400> 42
cctcgcgacg gtcgaccatg acgacg 26

<210> 43
<211> 26
<212> DNA
<213> artificial

<220>
<223> PCR Primer

<400> 43
cgtcgtcatg gtcgacctgc gcgagg 26

<210> 44
<211> 31
<212> DNA
<213> artificial

<220>
<223> PCR Primer

<400> 44
ccgccgcatc cataccgcca gttgtttacc c 31

<210> 45
<211> 26
<212> DNA
<213> artificial

<220>
<223> PCR Primer

<400> 45
cgtcgtcatg gtcgacattc gcgagg 26

<210> 46
<211> 26
<212> DNA
<213> artificial

<220>
<223> PCR Primer

<400> 46
cgtcgtcatg gtcgacgttc gcgagg 26

<210> 47
<211> 786
<212> DNA
<213> Bacillus subtilis

<400> 47

```
atgtatccgg atttaaaagg aaaagtcgtc gctattacag gagctgcttc agggctcgga      60
aaggcgatgg ccattcgctt cggcaaggag caggcaaaag tggttatcaa ctattatagt     120
aataaacaag atccgaacga ggtaaaagaa gaggtcatca aggcgggcgg tgaagctgtt     180
gtcgtccaag gagatgtcac gaaagaggaa gatgtaaaaa atatcgtgca aacggcaatt     240
aaggagttcg gcacactcga tattatgatt aataatgccg gtcttgaaaa tcctgtgcca     300
tctcacgaaa tgccgctcaa ggattgggat aaagtcatcg gcacgaactt aacgggtgcc     360
tttttaggaa gccgtgaagc gattaaatat ttcgtagaaa acgatatcaa gggaaatgtc     420
attaacatgt ccagtgtgca cgaagtgatt ccttggccgt tatttgtcca ctatgcggca     480
agtaaaggcg ggataaagct gatgacagaa acattagcgt tggaatacgc gccgaagggc     540
```

attcgcgtca ataatattgg gccaggtgcg atcaacacgc caatcaatgc tgaaaaattc 600

gctgacccta aacagaaagc tgatgtagaa agcatgattc caatgggata tatcggcgaa 660

ccggaggaga tcgccgcagt agcagcctgg cttgcttcga aggaagccag ctacgtcaca 720

ggcatcacgt tattcgcgga cggcggtatg acacaatatc cttcattcca ggcaggccgc 780

ggttaa 786

<210> 48
<211> 261
<212> PRT
<213> Bacillus subtilis

<400> 48

```
Met Tyr Pro Asp Leu Lys Gly Lys Val Val Ala Ile Thr Gly Ala Ala
1               5                   10                  15

Ser Gly Leu Gly Lys Ala Met Ala Ile Arg Phe Gly Lys Glu Gln Ala
            20                  25                  30

Lys Val Val Ile Asn Tyr Tyr Ser Asn Lys Gln Asp Pro Asn Glu Val
        35                  40                  45

Lys Glu Glu Val Ile Lys Ala Gly Gly Glu Ala Val Val Val Gln Gly
    50                  55                  60

Asp Val Thr Lys Glu Glu Asp Val Lys Asn Ile Val Gln Thr Ala Ile
65                  70                  75                  80

Lys Glu Phe Gly Thr Leu Asp Ile Met Ile Asn Asn Ala Gly Leu Glu
                85                  90                  95

Asn Pro Val Pro Ser His Glu Met Pro Leu Lys Asp Trp Asp Lys Val
            100                 105                 110

Ile Gly Thr Asn Leu Thr Gly Ala Phe Leu Gly Ser Arg Glu Ala Ile
        115                 120                 125

Lys Tyr Phe Val Glu Asn Asp Ile Lys Gly Asn Val Ile Asn Met Ser
    130                 135                 140

Ser Val His Glu Val Ile Pro Trp Pro Leu Phe Val His Tyr Ala Ala
145                 150                 155                 160

Ser Lys Gly Gly Ile Lys Leu Met Thr Glu Thr Leu Ala Leu Glu Tyr
                165                 170                 175

Ala Pro Lys Gly Ile Arg Val Asn Asn Ile Gly Pro Gly Ala Ile Asn
```

```
                180                 185                 190

Thr Pro Ile Asn Ala Glu Lys Phe Ala Asp Pro Lys Gln Lys Ala Asp
        195                 200                 205

Val Glu Ser Met Ile Pro Met Gly Tyr Ile Gly Glu Pro Glu Glu Ile
    210                 215                 220

Ala Ala Val Ala Ala Trp Leu Ala Ser Lys Glu Ala Ser Tyr Val Thr
225                 230                 235                 240

Gly Ile Thr Leu Phe Ala Asp Gly Gly Met Thr Gln Tyr Pro Ser Phe
                245                 250                 255

Gln Ala Gly Arg Gly
            260
```

<210> 49
<211> 7404
<212> DNA
<213> artificial

<220>
<223> Vektor pFr7 (D)

<400> 49

```
atccggatat agttcctcct ttcagcaaaa aacccctcaa gacccgttta gaggccccaa      60
ggggttatgc tagttattgc tcagcggtgg cagcagccaa ctcagcttcc tttcgggctt     120
tgttagcagc cggatctcag tggtggtggt ggtggtgctc gagtgcggcc gcaagcttct     180
agtcgcggta gaacgacccc atgtagcgga tgtactcgtc ctcggtgtgg tttgccttcc     240
agtcgtggac ggagtccttg ttgcgctggc cggcgatgac ggagagctcc ttacccagct     300
tctcaaaggc ctcgtcaacg cactcctcgg gggtgagggc gatcttcatg acggcctcgc     360
cctgcgggcc gccggggagg ttggacagca ggctgggggt tagggtggtg ccgagggtga     420
tgacctcgac gtcgacgccg gtgccctcgc actcgcaggc cacggcctcg gtcatcttga     480
ggatgaaggc cttgcccgcg ccgtactggc cgttccaggg gctggagctg atgccggtca     540
tcgacgagac gttgatcacg gcgccgcggt cctgggcggc aaagatccgc atgtagtggt     600
ggaagcactt gaggaaggtc acgacgttga cgttgatcat ggcctcgtgc ttctcccagg     660
gggtgtcctg gatcttaccg aagctgtgca ggcaggccac gtagctcatg aagcccatgt     720
ccaggccctc ggtcgcggcg aagacggtct cggcagcgcc gggctggcta aagtcggcgc     780
gcacgacctt ggtctccacg ccgtaggtct cgcggatctc gcctgcgagc acgttcagct     840
tctcctcgcg acggtcgacc atgacgacgt tcatgccgcc ggcggcgatc ttctcgcaga     900
acgccttgcc gacgccctcg gtcgcgccca ggatcaggcc ccactcaccg tacttctccc     960
```

tcaggttcat gtatatctcc tttcacgaat tctcagccgc cttcttgaac ttggcggcct 1020
cttccgaacc gccggtggca ttgcccttcg agtaggaatg cgcgccggtg ccggcaagag 1080
cgccgccctg cacgatgagg tattcgtcgc ggatcggacg gccctcgaag aagcactcca 1140
ggatctcgcg ggtgcccgcc gcataacgcg cctgcgcggt cagcgtggtg ccggagatgt 1200
gcggggtcat gccgttatag ggcatcgtcc gccaggggtg gtccttcggc gccggctgcg 1260
ggaaccacac gtcgccggca tagccggcca gccggccgga ttcgagcgca cgtgccacgg 1320
catcgcggtc gcacagcttg ccgcgggcgg tgttgacgat gtaggcgcca cgcttgaaca 1380
gcttcagcgt ctcgtcattg atcatgtgct cggtttcggg gtgcagcggg cagttcagcg 1440
tcaccacgtc gcaaaccgga tacatgtcct cgcgggtcgc gtgccaggtg aggttgagct 1500
ccttctcgac cgattccggc aggcggtgac ggccggtgta gtgcaggtgc acgtcgaacg 1560
gcgccagacg gcgcagcacc gcgagaccga tgcggccggc ggccacggtg ccgacatgca 1620
tcgcctcgag gtcgtaggcg tgggagacgc agtcggcgat gttccagccg cccttccgcg 1680
cccattcgtg cgagggcaga tagttgcgca ccagcgacag gatcatcatc accacatgct 1740
cggcgacgct gatcgagttg cagtaggtga cttccgccac ggtgacgttg cggtcgatag 1800
ccgactgaag atcgacgtgg tcggaaccga tgccggcggt gagcgcgagc ttcaggttct 1860
tggccttggc gatgcgctcg ggcgtcagat aggccggcca gaagggctgg gagatgacga 1920
catccgcatc gaccagctcg cgctcgaaca ccgagtcggg gccgtccttg tcggaggtca 1980
cgaccagggt gtggccgttg gattcgagat attcgcgcag gccgagctcg ccggagacgg 2040
agccgagcaa ctgcccgggc gtgaagtcga tggccttcgg cgtcggcaag atctggccgc 2100
ccggatagtg gtcgatcttc ggaagatcgt cgcgggcata ggtcttcggg tagccgtcga 2160
ccggatcatc gtaaagaacg cacaggacct ttgccatcat atgtatatct ccttcttaaa 2220
gttaaacaaa attatttcta gaggggaatt gttatccgct cacaattccc ctatagtgag 2280
tcgtattaat ttcgcgggat cgagatctcg atcctctacg ccggacgcat cgtggccggc 2340
atcaccggcg ccacaggtgc ggttgctggc gcctatatcg ccgacatcac cgatggggaa 2400
gatcgggctc gccacttcgg gctcatgagc gcttgtttcg gcgtgggtat ggtggcaggc 2460
cccgtggccg ggggactgtt gggcgccatc tccttgcatg caccattcct tgcggcggcg 2520
gtgctcaacg gcctcaacct actactgggc tgcttcctaa tgcaggagtc gcataaggga 2580
gagcgtcgag atcccggaca ccatcgaatg gcgcaaaacc tttcgcggta tggcatgata 2640
gcgcccggaa gagagtcaat tcagggtggt gaatgtgaaa ccagtaacgt tatacgatgt 2700
cgcagagtat gccggtgtct cttatcagac cgtttcccgc gtggtgaacc aggccagcca 2760
cgtttctgcg aaaacgcggg aaaaagtgga agcggcgatg gcggagctga attacattcc 2820

caaccgcgtg gcacaacaac tggcgggcaa acagtcgttg ctgattggcg ttgccacctc 2880
cagtctggcc ctgcacgcgc cgtcgcaaat tgtcgcggcg attaaatctc gcgccgatca 2940
actgggtgcc agcgtggtgg tgtcgatggt agaacgaagc ggcgtcgaag cctgtaaagc 3000
ggcggtgcac aatcttctcg cgcaacgcgt cagtgggctg atcattaact atccgctgga 3060
tgaccaggat gccattgctg tggaagctgc ctgcactaat gttccggcgt tatttcttga 3120
tgtctctgac cagacaccca tcaacagtat tattttctcc catgaagacg gtacgcgact 3180
gggcgtggag catctggtcg cattgggtca ccagcaaatc gcgctgttag cgggcccatt 3240
aagttctgtc tcggcgcgtc tgcgtctggc tggctggcat aaatatctca ctcgcaatca 3300
aattcagccg atagcggaac gggaaggcga ctggagtgcc atgtccggtt ttcaacaaac 3360
catgcaaatg ctgaatgagg gcatcgttcc cactgcgatg ctggttgcca acgatcagat 3420
ggcgctgggc gcaatgcgcg ccattaccga gtccgggctg cgcgttggtg cggatatctc 3480
ggtagtggga tacgacgata ccgaagacag ctcatgttat atcccgccgt taaccaccat 3540
caaacaggat tttcgcctgc tggggcaaac cagcgtggac cgcttgctgc aactctctca 3600
gggccaggcg gtgaagggca atcagctgtt gcccgtctca ctggtgaaaa gaaaaaccac 3660
cctggcgccc aatacgcaaa ccgcctctcc ccgcgcgttg gccgattcat taatgcagct 3720
ggcacgacag gtttcccgac tggaaagcgg gcagtgagcg caacgcaatt aatgtaagtt 3780
agctcactca ttaggcaccg ggatctcgac cgatgccctt gagagccttc aacccagtca 3840
gctccttccg gtgggcgcgg ggcatgacta tcgtcgccgc acttatgact gtcttcttta 3900
tcatgcaact cgtaggacag gtgccggcag cgctctgggt cattttcggc gaggaccgct 3960
ttcgctggag cgcgacgatg atcggcctgt cgcttgcggt attcggaatc ttgcacgccc 4020
tcgctcaagc cttcgtcact ggtcccgcca ccaaacgttt cggcgagaag caggccatta 4080
tcgccggcat ggcggcccca cgggtgcgca tgatcgtgct cctgtcgttg aggacccggc 4140
taggctggcg gggttgcctt actggttagc agaatgaatc accgatacgc gagcgaacgt 4200
gaagcgactg ctgctgcaaa acgtctgcga cctgagcaac aacatgaatg gtcttcggtt 4260
tccgtgtttc gtaaagtctg gaaacgcgga agtcagcgcc ctgcaccatt atgttccgga 4320
tctgcatcgc aggatgctgc tggctaccct gtggaacacc tacatctgta ttaacgaagc 4380
gctggcattg accctgagtg atttttctct ggtcccgccg catccatacc gccagttgtt 4440
taccctcaca acgttccagt aaccgggcat gttcatcatc agtaacccgt atcgtgagca 4500
tcctctctcg tttcatcggt atcattaccc ccatgaacag aaatccccct tacacggagg 4560
catcagtgac caaacaggaa aaaaccgccc ttaacatggc ccgctttatc agaagccaga 4620
cattaacgct tctggagaaa ctcaacgagc tggacgcgga tgaacaggca gacatctgtg 4680
aatcgcttca cgaccacgct gatgagcttt accgcagctg cctcgcgcgt ttcggtgatg 4740

acggtgaaaa cctctgacac atgcagctcc cggagacggt cacagcttgt ctgtaagcgg 4800

atgccgggag cagacaagcc cgtcagggcg cgtcagcggg tgttggcggg tgtcggggcg 4860

cagccatgac ccagtcacgt agcgatagcg gagtgtatac tggcttaact atgcggcatc 4920

agagcagatt gtactgagag tgcaccatat atgcggtgtg aaataccgca cagatgcgta 4980

aggagaaaat accgcatcag gcgctcttcc gcttcctcgc tcactgactc gctgcgctcg 5040

gtcgttcggc tgcggcgagc ggtatcagct cactcaaagg cggtaatacg gttatccaca 5100

gaatcagggg ataacgcagg aaagaacatg tgagcaaaag gccagcaaaa ggccaggaac 5160

cgtaaaaagg ccgcgttgct ggcgtttttc cataggctcc gcccccctga cgagcatcac 5220

aaaaatcgac gctcaagtca gaggtggcga aacccgacag gactataaag ataccaggcg 5280

tttccccctg gaagctccct cgtgcgctct cctgttccga ccctgccgct taccggatac 5340

ctgtccgcct ttctcccttc gggaagcgtg gcgctttctc atagctcacg ctgtaggtat 5400

ctcagttcgg tgtaggtcgt tcgctccaag ctgggctgtg tgcacgaacc ccccgttcag 5460

cccgaccgct gcgccttatc cggtaactat cgtcttgagt ccaacccggt aagacacgac 5520

ttatcgccac tggcagcagc cactggtaac aggattagca gagcgaggta tgtaggcggt 5580

gctacagagt tcttgaagtg gtggcctaac tacggctaca ctagaaggac agtatttggt 5640

atctgcgctc tgctgaagcc agttaccttc ggaaaaagag ttggtagctc ttgatccggc 5700

aaacaaacca ccgctggtag cggtggtttt tttgtttgca agcagcagat tacgcgcaga 5760

aaaaaaggat ctcaagaaga tcctttgatc ttttctacgg ggtctgacgc tcagtggaac 5820

gaaaactcac gttaagggat tttggtcatg agattatcaa aaaggatctt cacctagatc 5880

cttttaaatt aaaaatgaag ttttaaatca atctaaagta tatatgagta aacttggtct 5940

gacagttacc aatgcttaat cagtgaggca cctatctcag cgatctgtct atttcgttca 6000

tccatagttg cctgactccc cgtcgtgtag ataactacga tacgggaggg cttaccatct 6060

ggccccagtg ctgcaatgat accgcgagac ccacgctcac cggctccaga tttatcagca 6120

ataaaccagc cagccggaag ggccgagcgc agaagtggtc ctgcaacttt atccgcctcc 6180

atccagtcta ttaattgttg ccgggaagct agagtaagta gttcgccagt taatagtttg 6240

cgcaacgttg ttgccattgc tgcaggcatc gtggtgtcac gctcgtcgtt tggtatggct 6300

tcattcagct ccggttccca acgatcaagg cgagttacat gatcccccat gttgtgcaaa 6360

aaagcggtta gctccttcgg tcctccgatc gttgtcagaa gtaagttggc cgcagtgtta 6420

tcactcatgg ttatggcagc actgcataat tctcttactg tcatgccatc cgtaagatgc 6480

ttttctgtga ctggtgagta ctcaaccaag tcattctgag aatagtgtat gcggcgaccg 6540

agttgctctt gcccggcgtc aatacgggat aataccgcgc cacatagcag aactttaaaa 6600

gtgctcatca ttggaaaacg ttcttcgggg cgaaaactct caaggatctt accgctgttg 6660

agatccagtt cgatgtaacc cactcgtgca cccaactgat cttcagcatc ttttactttc 6720

accagcgttt ctgggtgagc aaaaacagga aggcaaaatg ccgcaaaaaa gggaataagg 6780

gcgacacgga aatgttgaat actcatactc ttcctttttc aatattattg aagcatttat 6840

cagggttatt gtctcatgag cggatacata tttgaatgta tttagaaaaa taaacaaata 6900

ggggttccgc gcacatttcc ccgaaaagtg ccacctgaaa ttgtaaacgt taatattttg 6960

ttaaaattcg cgttaaattt ttgttaaatc agctcatttt ttaaccaata ggccgaaatc 7020

ggcaaaatcc cttataaatc aaaagaatag accgagatag ggttgagtgt tgttccagtt 7080

tggaacaaga gtccactatt aaagaacgtg gactccaacg tcaaagggcg aaaaaccgtc 7140

tatcagggcg atggcccact acgtgaacca tcaccctaat caagtttttt ggggtcgagg 7200

tgccgtaaag cactaaatcg gaaccctaaa gggagccccc gatttagagc ttgacgggga 7260

aagccggcga acgtggcgag aaaggaaggg aagaaagcga aaggagcggg cgctagggcg 7320

ctggcaagtg tagcggtcac gctgcgcgta accaccacac ccgccgcgct taatgcgccg 7380

ctacagggcg cgtcccattc gcca 7404

<210> 50
<211> 7404
<212> DNA
<213> artificial

<220>
<223> Vektor pFr7 (DL)

<400> 50

```
atccggatat agttcctcct ttcagcaaaa aacccctcaa gacccgttta gaggccccaa       60
ggggttatgc tagttattgc tcagcggtgg cagcagccaa ctcagcttcc tttcgggctt      120
tgttagcagc cggatctcag tggtggtggt ggtggtgctc gagtgcggcc gcaagcttct      180
agtcgcggta gaacgacccc atgtagcgga tgtactcgtc ctcggtgtgg tttgccttcc      240
agtcgtggac ggagtccttg ttgcgctggc cggcgatgac ggagagctcc ttacccagct      300
tctcaaaggc ctcgtcaacg cactcctcgg gggtgagggc gatcttcatg acggcctcgc      360
cctgcgggcc gccggggagg ttggacagca ggctgggggt tagggtggtg ccgagggtga      420
tgacctcgac gtcgacgccg gtgccctcgc actcgcaggc cacggcctcg gtcatcttga      480
ggatgaaggc cttgcccgcg ccgtactggc cgttccaggg gctggagctg atgccggtca      540
tcgacgagac gttgatcacg gcgccgcggt cctgggcggc aaagatccgc atgtagtggt      600
ggaagcactt gaggaaggtc acgacgttga cgttgatcat ggcctcgtgc ttctcccagg      660
gggtgtcctg gatcttaccg aagctgtgca ggcaggccac gtagctcatg aagcccatgt      720
ccaggccctc ggtcgcggcg aagacggtct cggcagcgcc gggctggcta aagtcggcgc      780
```

gcacgacctt ggtctccacg ccgtaggtct cgcggatctc gcctgcgagc acgttcagct 840
tctcctcgcg caggtcgacc atgacgacgt tcatgccgcc ggcggcgatc ttctcgcaga 900
acgccttgcc gacgccctcg gtcgcgccca ggatcaggcc ccactcaccg tacttctccc 960
tcaggttcat gtatatctcc tttcacgaat tctcagccgc cttcttgaac ttggcggcct 1020
cttccgaacc gccggtggca ttgcccttcg agtaggaatg cgcgccggtg ccggcaagag 1080
cgccgccctg cacgatgagg tattcgtcgc ggatcggacg gccctcgaag aagcactcca 1140
ggatctcgcg ggtgcccgcc gcataacgcg cctgcgcggt cagcgtggtg ccggagatgt 1200
gcggggtcat gccgttatag ggcatcgtcc gccaggggtg gtccttcggc gccggctgcg 1260
ggaaccacac gtcgccggca tagccggcca gccggccgga ttcgagcgca cgtgccacgg 1320
catcgcggtc gcacagcttg ccgcgggcgg tgttgacgat gtaggcgcca cgcttgaaca 1380
gcttcagcgt ctcgtcattg atcatgtgct cggtttcggg gtgcagcggg cagttcagcg 1440
tcaccacgtc gcaaaccgga tacatgtcct cgcgggtcgc gtgccaggtg aggttgagct 1500
ccttctcgac cgattccggc aggcggtgac ggccggtgta gtgcaggtgc acgtcgaacg 1560
gcgccagacg gcgcagcacc gcgagaccga tgcggccggc ggccacggtg ccgacatgca 1620
tcgcctcgag gtcgtaggcg tgggagacgc agtcggcgat gttccagccg cccttccgcg 1680
cccattcgtg cgagggcaga tagttgcgca ccagcgacag gatcatcatc accacatgct 1740
cggcgacgct gatcgagttg cagtaggtga cttccgccac ggtgacgttg cggtcgatag 1800
ccgactgaag atcgacgtgg tcggaaccga tgccggcggt gagcgcgagc ttcaggttct 1860
tggccttggc gatgcgctcg ggcgtcagat aggccggcca gaagggctgg gagatgacga 1920
catccgcatc gaccagctcg cgctcgaaca ccgagtcggg gccgtccttg tcggaggtca 1980
cgaccagggt gtggccgttg gattcgagat attcgcgcag gccgagctcg ccggagacgg 2040
agccgagcaa ctgcccgggc gtgaagtcga tggccttcgg cgtcggcaag atctggccgc 2100
ccggatagtg gtcgatcttc ggaagatcgt cgcgggcata ggtcttcggg tagccgtcga 2160
ccggatcatc gtaaagaacg cacaggacct ttgccatcat atgtatatct ccttcttaaa 2220
gttaaacaaa attatttcta gaggggaatt gttatccgct cacaattccc ctatagtgag 2280
tcgtattaat ttcgcgggat cgagatctcg atcctctacg ccggacgcat cgtggccggc 2340
atcaccggcg ccacaggtgc ggttgctggc gcctatatcg ccgacatcac cgatggggaa 2400
gatcgggctc gccacttcgg gctcatgagc gcttgtttcg gcgtgggtat ggtggcaggc 2460
cccgtggccg ggggactgtt gggcgccatc tccttgcatg caccattcct tgcggcggcg 2520
gtgctcaacg gcctcaacct actactgggc tgcttcctaa tgcaggagtc gcataaggga 2580
gagcgtcgag atcccggaca ccatcgaatg gcgcaaaacc tttcgcggta tggcatgata 2640

gcgcccggaa gagagtcaat tcagggtggt gaatgtgaaa ccagtaacgt tatacgatgt 2700

cgcagagtat gccggtgtct cttatcagac cgtttcccgc gtggtgaacc aggccagcca 2760

cgtttctgcg aaaacgcggg aaaaagtgga agcggcgatg gcggagctga attacattcc 2820

caaccgcgtg gcacaacaac tggcgggcaa acagtcgttg ctgattggcg ttgccacctc 2880

cagtctggcc ctgcacgcgc cgtcgcaaat tgtcgcggcg attaaatctc gcgccgatca 2940

actgggtgcc agcgtggtgg tgtcgatggt agaacgaagc ggcgtcgaag cctgtaaagc 3000

ggcggtgcac aatcttctcg cgcaacgcgt cagtgggctg atcattaact atccgctgga 3060

tgaccaggat gccattgctg tggaagctgc ctgcactaat gttccggcgt tatttcttga 3120

tgtctctgac cagacaccca tcaacagtat tattttctcc catgaagacg gtacgcgact 3180

gggcgtggag catctggtcg cattgggtca ccagcaaatc gcgctgttag cgggcccatt 3240

aagttctgtc tcggcgcgtc tgcgtctggc tggctggcat aaatatctca ctcgcaatca 3300

aattcagccg atagcggaac gggaaggcga ctggagtgcc atgtccggtt ttcaacaaac 3360

catgcaaatg ctgaatgagg gcatcgttcc cactgcgatg ctggttgcca acgatcagat 3420

ggcgctgggc gcaatgcgcg ccattaccga gtccgggctg cgcgttggtg cggatatctc 3480

ggtagtggga tacgacgata ccgaagacag ctcatgttat atcccgccgt taaccaccat 3540

caaacaggat tttcgcctgc tggggcaaac cagcgtggac cgcttgctgc aactctctca 3600

gggccaggcg gtgaagggca atcagctgtt gcccgtctca ctggtgaaaa gaaaaaccac 3660

cctggcgccc aatacgcaaa ccgcctctcc ccgcgcgttg gccgattcat taatgcagct 3720

ggcacgacag gtttcccgac tggaaagcgg gcagtgagcg caacgcaatt aatgtaagtt 3780

agctcactca ttaggcaccg ggatctcgac cgatgccctt gagagccttc aacccagtca 3840

gctccttccg gtgggcgcgg ggcatgacta tcgtcgccgc acttatgact gtcttcttta 3900

tcatgcaact cgtaggacag gtgccggcag cgctctgggt cattttcggc gaggaccgct 3960

ttcgctggag cgcgacgatg atcggcctgt cgcttgcggt attcggaatc ttgcacgccc 4020

tcgctcaagc cttcgtcact ggtcccgcca ccaaacgttt cggcgagaag caggccatta 4080

tcgccggcat ggcggcccca cgggtgcgca tgatcgtgct cctgtcgttg aggacccggc 4140

taggctggcg gggttgcctt actggttagc agaatgaatc accgatacgc gagcgaacgt 4200

gaagcgactg ctgctgcaaa acgtctgcga cctgagcaac aacatgaatg gtcttcggtt 4260

tccgtgtttc gtaaagtctg gaaacgcgga agtcagcgcc ctgcaccatt atgttccgga 4320

tctgcatcgc aggatgctgc tggctaccct gtggaacacc tacatctgta ttaacgaagc 4380

gctggcattg accctgagtg atttttctct ggtcccgccg catccatacc gccagttgtt 4440

taccctcaca acgttccagt aaccgggcat gttcatcatc agtaacccgt atcgtgagca 4500

tcctctctcg tttcatcggt atcattaccc ccatgaacag aaatccccct tacacggagg 4560

catcagtgac caaacaggaa aaaaccgccc ttaacatggc ccgctttatc agaagccaga 4620
cattaacgct tctggagaaa ctcaacgagc tggacgcgga tgaacaggca gacatctgtg 4680
aatcgcttca cgaccacgct gatgagcttt accgcagctg cctcgcgcgt ttcggtgatg 4740
acggtgaaaa cctctgacac atgcagctcc cggagacggt cacagcttgt ctgtaagcgg 4800
atgccgggag cagacaagcc cgtcagggcg cgtcagcggg tgttggcggg tgtcggggcg 4860
cagccatgac ccagtcacgt agcgatagcg gagtgtatac tggcttaact atgcggcatc 4920
agagcagatt gtactgagag tgcaccatat atgcggtgtg aaataccgca cagatgcgta 4980
aggagaaaat accgcatcag gcgctcttcc gcttcctcgc tcactgactc gctgcgctcg 5040
gtcgttcggc tgcggcgagc ggtatcagct cactcaaagg cggtaatacg gttatccaca 5100
gaatcagggg ataacgcagg aaagaacatg tgagcaaaag gccagcaaaa ggccaggaac 5160
cgtaaaaagg ccgcgttgct ggcgtttttc cataggctcc gcccccctga cgagcatcac 5220
aaaaatcgac gctcaagtca gaggtggcga aacccgacag gactataaag ataccaggcg 5280
tttccccctg gaagctccct cgtgcgctct cctgttccga ccctgccgct taccggatac 5340
ctgtccgcct ttctcccttc gggaagcgtg gcgctttctc atagctcacg ctgtaggtat 5400
ctcagttcgg tgtaggtcgt tcgctccaag ctgggctgtg tgcacgaacc ccccgttcag 5460
cccgaccgct gcgccttatc cggtaactat cgtcttgagt ccaacccggt aagacacgac 5520
ttatcgccac tggcagcagc cactggtaac aggattagca gagcgaggta tgtaggcggt 5580
gctacagagt tcttgaagtg gtggcctaac tacggctaca ctagaaggac agtatttggt 5640
atctgcgctc tgctgaagcc agttaccttc ggaaaaagag ttggtagctc ttgatccggc 5700
aaacaaacca ccgctggtag cggtggtttt tttgtttgca agcagcagat tacgcgcaga 5760
aaaaaaggat ctcaagaaga tcctttgatc ttttctacgg ggtctgacgc tcagtggaac 5820
gaaaactcac gttaagggat tttggtcatg agattatcaa aaaggatctt cacctagatc 5880
cttttaaatt aaaaatgaag ttttaaatca atctaaagta tatatgagta aacttggtct 5940
gacagttacc aatgcttaat cagtgaggca cctatctcag cgatctgtct atttcgttca 6000
tccatagttg cctgactccc cgtcgtgtag ataactacga tacgggaggg cttaccatct 6060
ggccccagtg ctgcaatgat accgcgagac ccacgctcac cggctccaga tttatcagca 6120
ataaaccagc cagccggaag ggccgagcgc agaagtggtc ctgcaacttt atccgcctcc 6180
atccagtcta ttaattgttg ccgggaagct agagtaagta gttcgccagt taatagtttg 6240
cgcaacgttg ttgccattgc tgcaggcatc gtggtgtcac gctcgtcgtt tggtatggct 6300
tcattcagct ccggttccca acgatcaagg cgagttacat gatcccccat gttgtgcaaa 6360
aaagcggtta gctccttcgg tcctccgatc gttgtcagaa gtaagttggc cgcagtgtta 6420

tcactcatgg ttatggcagc actgcataat tctcttactg tcatgccatc cgtaagatgc 6480
ttttctgtga ctggtgagta ctcaaccaag tcattctgag aatagtgtat gcggcgaccg 6540
agttgctctt gcccggcgtc aatacgggat aataccgcgc cacatagcag aactttaaaa 6600
gtgctcatca ttggaaaacg ttcttcgggg cgaaaactct caaggatctt accgctgttg 6660
agatccagtt cgatgtaacc cactcgtgca cccaactgat cttcagcatc ttttactttc 6720
accagcgttt ctgggtgagc aaaaacagga aggcaaaatg ccgcaaaaaa gggaataagg 6780
gcgacacgga aatgttgaat actcatactc ttcctttttc aatattattg aagcatttat 6840
cagggttatt gtctcatgag cggatacata tttgaatgta tttagaaaaa taaacaaata 6900
ggggttccgc gcacatttcc ccgaaaagtg ccacctgaaa ttgtaaacgt taatattttg 6960
ttaaaattcg cgttaaattt ttgttaaatc agctcatttt ttaaccaata ggccgaaatc 7020
ggcaaaatcc cttataaatc aaaagaatag accgagatag ggttgagtgt tgttccagtt 7080
tggaacaaga gtccactatt aaagaacgtg gactccaacg tcaaagggcg aaaaaccgtc 7140
tatcagggcg atggcccact acgtgaacca tcaccctaat caagtttttt ggggtcgagg 7200
tgccgtaaag cactaaatcg gaaccctaaa gggagccccc gatttagagc ttgacgggga 7260
aagccggcga acgtggcgag aaaggaaggg aagaaagcga aaggagcggg cgctagggcg 7320
ctggcaagtg tagcggtcac gctgcgcgta accaccacac ccgccgcgct taatgcgccg 7380
ctacagggcg cgtcccattc gcca 7404

<210> 51
<211> 7404
<212> DNA
<213> artificial

<220>
<223> Vektor pFr7 (DI)

<400> 51

atccggatat agttcctcct ttcagcaaaa aacccctcaa gacccgttta gaggccccaa 60

ggggttatgc tagttattgc tcagcggtgg cagcagccaa ctcagcttcc tttcgggctt 120

tgttagcagc cggatctcag tggtggtggt ggtggtgctc gagtgcggcc gcaagcttct 180

agtcgcggta gaacgacccc atgtagcgga tgtactcgtc ctcggtgtgg tttgccttcc 240

agtcgtggac ggagtccttg ttgcgctggc cggcgatgac ggagagctcc ttacccagct 300

tctcaaaggc ctcgtcaacg cactcctcgg gggtgagggc gatcttcatg acggcctcgc 360

cctgcgggcc gccggggagg ttggacagca ggctgggggt tagggtggtg ccgagggtga 420

tgacctcgac gtcgacgccg gtgccctcgc actcgcaggc cacggcctcg gtcatcttga 480

ggatgaaggc cttgcccgcg ccgtactggc cgttccaggg gctggagctg atgccggtca 540

tcgacgagac gttgatcacg gcgccgcggt cctgggcggc aaagatccgc atgtagtggt 600

ggaagcactt gaggaaggtc acgacgttga cgttgatcat ggcctcgtgc ttctcccagg 660
gggtgtcctg gatcttaccg aagctgtgca ggcaggccac gtagctcatg aagcccatgt 720
ccaggccctc ggtcgcggcg aagacggtct cggcagcgcc gggctggcta aagtcggcgc 780
gcacgacctt ggtctccacg ccgtaggtct cgcggatctc gcctgcgagc acgttcagct 840
tctcctcgcg aatgtcgacc atgacgacgt tcatgccgcc ggcggcgatc ttctcgcaga 900
acgccttgcc gacgccctcg gtcgcgccca ggatcaggcc ccactcaccg tacttctccc 960
tcaggttcat gtatatctcc tttcacgaat tctcagccgc cttcttgaac ttggcggcct 1020
cttccgaacc gccggtggca ttgcccttcg agtaggaatg cgcgccggtg ccggcaagag 1080
cgccgccctg cacgatgagg tattcgtcgc ggatcggacg gccctcgaag aagcactcca 1140
ggatctcgcg ggtgcccgcc gcataacgcg cctgcgcggt cagcgtggtg ccggagatgt 1200
gcggggtcat gccgttatag ggcatcgtcc gccaggggtg gtccttcggc gccggctgcg 1260
ggaaccacac gtcgccggca tagccggcca gccggccgga ttcgagcgca cgtgccacgg 1320
catcgcggtc gcacagcttg ccgcgggcgg tgttgacgat gtaggcgcca cgcttgaaca 1380
gcttcagcgt ctcgtcattg atcatgtgct cggtttcggg gtgcagcggg cagttcagcg 1440
tcaccacgtc gcaaaccgga tacatgtcct cgcgggtcgc gtgccaggtg aggttgagct 1500
ccttctcgac cgattccggc aggcggtgac ggccggtgta gtgcaggtgc acgtcgaacg 1560
gcgccagacg gcgcagcacc gcgagaccga tgcggccggc ggccacggtg ccgacatgca 1620
tcgcctcgag gtcgtaggcg tgggagacgc agtcggcgat gttccagccg cccttccgcg 1680
cccattcgtg cgagggcaga tagttgcgca ccagcgacag gatcatcatc accacatgct 1740
cggcgacgct gatcgagttg cagtaggtga cttccgccac ggtgacgttg cggtcgatag 1800
ccgactgaag atcgacgtgg tcggaaccga tgccggcggt gagcgcgagc ttcaggttct 1860
tggccttggc gatgcgctcg ggcgtcagat aggccggcca gaagggctgg gagatgacga 1920
catccgcatc gaccagctcg cgctcgaaca ccgagtcggg gccgtccttg tcggaggtca 1980
cgaccagggt gtggccgttg gattcgagat attcgcgcag gccgagctcg ccggagacgg 2040
agccgagcaa ctgcccgggc gtgaagtcga tggccttcgg cgtcggcaag atctggccgc 2100
ccggatagtg gtcgatcttc ggaagatcgt cgcgggcata ggtcttcggg tagccgtcga 2160
ccggatcatc gtaaagaacg cacaggacct ttgccatcat atgtatatct ccttcttaaa 2220
gttaaacaaa attatttcta gaggggaatt gttatccgct cacaattccc ctatagtgag 2280
tcgtattaat ttcgcgggat cgagatctcg atcctctacg ccggacgcat cgtggccggc 2340
atcaccggcg ccacaggtgc ggttgctggc gcctatatcg ccgacatcac cgatggggaa 2400
gatcgggctc gccacttcgg gctcatgagc gcttgtttcg gcgtgggtat ggtggcaggc 2460

cccgtggccg ggggactgtt gggcgccatc tccttgcatg caccattcct tgcggcggcg 2520
gtgctcaacg gcctcaacct actactgggc tgcttcctaa tgcaggagtc gcataaggga 2580
gagcgtcgag atcccggaca ccatcgaatg gcgcaaaacc tttcgcggta tggcatgata 2640
gcgcccggaa gagagtcaat tcagggtggt gaatgtgaaa ccagtaacgt tatacgatgt 2700
cgcagagtat gccggtgtct cttatcagac cgtttcccgc gtggtgaacc aggccagcca 2760
cgtttctgcg aaaacgcggg aaaaagtgga agcggcgatg gcggagctga attacattcc 2820
caaccgcgtg gcacaacaac tggcgggcaa acagtcgttg ctgattggcg ttgccacctc 2880
cagtctggcc ctgcacgcgc cgtcgcaaat tgtcgcggcg attaaatctc gcgccgatca 2940
actgggtgcc agcgtggtgg tgtcgatggt agaacgaagc ggcgtcgaag cctgtaaagc 3000
ggcggtgcac aatcttctcg cgcaacgcgt cagtgggctg atcattaact atccgctgga 3060
tgaccaggat gccattgctg tggaagctgc ctgcactaat gttccggcgt tatttcttga 3120
tgtctctgac cagacaccca tcaacagtat tattttctcc catgaagacg gtacgcgact 3180
gggcgtggag catctggtcg cattgggtca ccagcaaatc gcgctgttag cgggcccatt 3240
aagttctgtc tcggcgcgtc tgcgtctggc tggctggcat aaatatctca ctcgcaatca 3300
aattcagccg atagcggaac gggaaggcga ctggagtgcc atgtccggtt ttcaacaaac 3360
catgcaaatg ctgaatgagg gcatcgttcc cactgcgatg ctggttgcca acgatcagat 3420
ggcgctgggc gcaatgcgcg ccattaccga gtccgggctg cgcgttggtg cggatatctc 3480
ggtagtggga tacgacgata ccgaagacag ctcatgttat atcccgccgt taaccaccat 3540
caaacaggat tttcgcctgc tggggcaaac cagcgtggac cgcttgctgc aactctctca 3600
gggccaggcg gtgaagggca atcagctgtt gcccgtctca ctggtgaaaa gaaaaaccac 3660
cctggcgccc aatacgcaaa ccgcctctcc ccgcgcgttg gccgattcat taatgcagct 3720
ggcacgacag gtttcccgac tggaaagcgg gcagtgagcg caacgcaatt aatgtaagtt 3780
agctcactca ttaggcaccg ggatctcgac cgatgccctt gagagccttc aacccagtca 3840
gctccttccg gtgggcgcgg ggcatgacta tcgtcgccgc acttatgact gtcttcttta 3900
tcatgcaact cgtaggacag gtgccggcag cgctctgggt cattttcggc gaggaccgct 3960
ttcgctggag cgcgacgatg atcggcctgt cgcttgcggt attcggaatc ttgcacgccc 4020
tcgctcaagc cttcgtcact ggtcccgcca ccaaacgttt cggcgagaag caggccatta 4080
tcgccggcat ggcggcccca cgggtgcgca tgatcgtgct cctgtcgttg aggacccggc 4140
taggctggcg gggttgcctt actggttagc agaatgaatc accgatacgc gagcgaacgt 4200
gaagcgactg ctgctgcaaa acgtctgcga cctgagcaac aacatgaatg gtcttcggtt 4260
tccgtgtttc gtaaagtctg gaaacgcgga agtcagcgcc ctgcaccatt atgttccgga 4320
tctgcatcgc aggatgctgc tggctaccct gtggaacacc tacatctgta ttaacgaagc 4380

gctggcattg accctgagtg atttttctct ggtcccgccg catccatacc gccagttgtt 4440
taccctcaca acgttccagt aaccgggcat gttcatcatc agtaacccgt atcgtgagca 4500
tcctctctcg tttcatcggt atcattaccc ccatgaacag aaatccccct tacacggagg 4560
catcagtgac caaacaggaa aaaaccgccc ttaacatggc ccgctttatc agaagccaga 4620
cattaacgct tctggagaaa ctcaacgagc tggacgcgga tgaacaggca gacatctgtg 4680
aatcgcttca cgaccacgct gatgagcttt accgcagctg cctcgcgcgt ttcggtgatg 4740
acggtgaaaa cctctgacac atgcagctcc cggagacggt cacagcttgt ctgtaagcgg 4800
atgccgggag cagacaagcc cgtcagggcg cgtcagcggg tgttggcggg tgtcggggcg 4860
cagccatgac ccagtcacgt agcgatagcg gagtgtatac tggcttaact atgcggcatc 4920
agagcagatt gtactgagag tgcaccatat atgcggtgtg aaataccgca cagatgcgta 4980
aggagaaaat accgcatcag gcgctcttcc gcttcctcgc tcactgactc gctgcgctcg 5040
gtcgttcggc tgcggcgagc ggtatcagct cactcaaagg cggtaatacg gttatccaca 5100
gaatcagggg ataacgcagg aaagaacatg tgagcaaaag gccagcaaaa ggccaggaac 5160
cgtaaaaagg ccgcgttgct ggcgtttttc cataggctcc gcccccctga cgagcatcac 5220
aaaaatcgac gctcaagtca gaggtggcga aacccgacag gactataaag ataccaggcg 5280
tttccccctg gaagctccct cgtgcgctct cctgttccga ccctgccgct taccggatac 5340
ctgtccgcct ttctcccttc gggaagcgtg gcgctttctc atagctcacg ctgtaggtat 5400
ctcagttcgg tgtaggtcgt tcgctccaag ctgggctgtg tgcacgaacc ccccgttcag 5460
cccgaccgct gcgccttatc cggtaactat cgtcttgagt ccaacccggt aagacacgac 5520
ttatcgccac tggcagcagc cactggtaac aggattagca gagcgaggta tgtaggcggt 5580
gctacagagt tcttgaagtg gtggcctaac tacggctaca ctagaaggac agtatttggt 5640
atctgcgctc tgctgaagcc agttaccttc ggaaaaagag ttggtagctc ttgatccggc 5700
aaacaaacca ccgctggtag cggtggtttt tttgtttgca agcagcagat tacgcgcaga 5760
aaaaaaggat ctcaagaaga tcctttgatc ttttctacgg ggtctgacgc tcagtggaac 5820
gaaaactcac gttaagggat tttggtcatg agattatcaa aaaggatctt cacctagatc 5880
cttttaaatt aaaaatgaag ttttaaatca atctaaagta tatatgagta aacttggtct 5940
gacagttacc aatgcttaat cagtgaggca cctatctcag cgatctgtct atttcgttca 6000
tccatagttg cctgactccc cgtcgtgtag ataactacga tacgggaggg cttaccatct 6060
ggccccagtg ctgcaatgat accgcgagac ccacgctcac cggctccaga tttatcagca 6120
ataaaccagc cagccggaag ggccgagcgc agaagtggtc ctgcaacttt atccgcctcc 6180
atccagtcta ttaattgttg ccgggaagct agagtaagta gttcgccagt taatagtttg 6240

cgcaacgttg ttgccattgc tgcaggcatc gtggtgtcac gctcgtcgtt tggtatggct 6300
tcattcagct ccggttccca acgatcaagg cgagttacat gatcccccat gttgtgcaaa 6360
aaagcggtta gctccttcgg tcctccgatc gttgtcagaa gtaagttggc cgcagtgtta 6420
tcactcatgg ttatggcagc actgcataat tctcttactg tcatgccatc cgtaagatgc 6480
ttttctgtga ctggtgagta ctcaaccaag tcattctgag aatagtgtat gcggcgaccg 6540
agttgctctt gcccggcgtc aatacgggat aataccgcgc cacatagcag aactttaaaa 6600
gtgctcatca ttggaaaacg ttcttcgggg cgaaaactct caaggatctt accgctgttg 6660
agatccagtt cgatgtaacc cactcgtgca cccaactgat cttcagcatc ttttactttc 6720
accagcgttt ctgggtgagc aaaaacagga aggcaaaatg ccgcaaaaaa gggaataagg 6780
gcgacacgga aatgttgaat actcatactc ttcctttttc aatattattg aagcatttat 6840
cagggttatt gtctcatgag cggatacata tttgaatgta tttagaaaaa taaacaaata 6900
ggggttccgc gcacatttcc ccgaaaagtg ccacctgaaa ttgtaaacgt taatattttg 6960
ttaaaattcg cgttaaattt ttgttaaatc agctcatttt ttaaccaata ggccgaaatc 7020
ggcaaaatcc cttataaatc aaaagaatag accgagatag ggttgagtgt tgttccagtt 7080
tggaacaaga gtccactatt aaagaacgtg gactccaacg tcaaagggcg aaaaaccgtc 7140
tatcagggcg atggcccact acgtgaacca tcaccctaat caagtttttt ggggtcgagg 7200
tgccgtaaag cactaaatcg gaaccctaaa gggagccccc gatttagagc ttgacgggga 7260
aagccggcga acgtggcgag aaaggaaggg aagaaagcga aaggagcggg cgctagggcg 7320
ctggcaagtg tagcggtcac gctgcgcgta accaccacac ccgccgcgct taatgcgccg 7380
ctacagggcg cgtcccattc gcca 7404

<210> 52
<211> 7404
<212> DNA
<213> artificial

<220>
<223> Vektor pFr7 (DV)

<400> 52

atccggatat agttcctcct ttcagcaaaa aacccctcaa gacccgttta gaggccccaa 60
ggggttatgc tagttattgc tcagcggtgg cagcagccaa ctcagcttcc tttcgggctt 120
tgttagcagc cggatctcag tggtggtggt ggtggtgctc gagtgcggcc gcaagcttct 180
agtcgcggta gaacgacccc atgtagcgga tgtactcgtc ctcggtgtgg tttgccttcc 240
agtcgtggac ggagtccttg ttgcgctggc cggcgatgac ggagagctcc ttacccagct 300
tctcaaaggc ctcgtcaacg cactcctcgg gggtgagggc gatcttcatg acggcctcgc 360
cctgcgggcc gccggggagg ttggacagca ggctggggt tagggtggtg ccgagggtga 420

tgacctcgac gtcgacgccg gtgccctcgc actcgcaggc cacggcctcg gtcatcttga 480
ggatgaaggc cttgcccgcg ccgtactggc cgttccaggg gctggagctg atgccggtca 540
tcgacgagac gttgatcacg gcgccgcggt cctgggcggc aaagatccgc atgtagtggt 600
ggaagcactt gaggaaggtc acgacgttga cgttgatcat ggcctcgtgc ttctcccagg 660
gggtgtcctg gatcttaccg aagctgtgca ggcaggccac gtagctcatg aagcccatgt 720
ccaggccctc ggtcgcggcg aagacggtct cggcagcgcc gggctggcta aagtcggcgc 780
gcacgacctt ggtctccacg ccgtaggtct cgcggatctc gcctgcgagc acgttcagct 840
tctcctcgcg aacgtcgacc atgacgacgt tcatgccgcc ggcggcgatc ttctcgcaga 900
acgccttgcc gacgccctcg gtcgcgccca ggatcaggcc ccactcaccg tacttctccc 960
tcaggttcat gtatatctcc tttcacgaat tctcagccgc cttcttgaac ttggcggcct 1020
cttccgaacc gccggtggca ttgcccttcg agtaggaatg cgcgccggtg ccggcaagag 1080
cgccgccctg cacgatgagg tattcgtcgc ggatcggacg gccctcgaag aagcactcca 1140
ggatctcgcg ggtgcccgcc gcataacgcg cctgcgcggt cagcgtggtg ccggagatgt 1200
gcggggtcat gccgttatag ggcatcgtcc gccaggggtg gtccttcggc gccggctgcg 1260
ggaaccacac gtcgccggca tagccggcca gccggccgga ttcgagcgca cgtgccacgg 1320
catcgcggtc gcacagcttg ccgcgggcgg tgttgacgat gtaggcgcca cgcttgaaca 1380
gcttcagcgt ctcgtcattg atcatgtgct cggtttcggg gtgcagcggg cagttcagcg 1440
tcaccacgtc gcaaaccgga tacatgtcct cgcgggtcgc gtgccaggtg aggttgagct 1500
ccttctcgac cgattccggc aggcggtgac ggccggtgta gtgcaggtgc acgtcgaacg 1560
gcgccagacg gcgcagcacc gcgagaccga tgcggccggc ggccacggtg ccgacatgca 1620
tcgcctcgag gtcgtaggcg tgggagacgc agtcggcgat gttccagccg cccttccgcg 1680
cccattcgtg cgagggcaga tagttgcgca ccagcgacag gatcatcatc accacatgct 1740
cggcgacgct gatcgagttg cagtaggtga cttccgccac ggtgacgttg cggtcgatag 1800
ccgactgaag atcgacgtgg tcggaaccga tgccggcggt gagcgcgagc ttcaggttct 1860
tggccttggc gatgcgctcg ggcgtcagat aggccggcca gaagggctgg gagatgacga 1920
catccgcatc gaccagctcg cgctcgaaca ccgagtcggg gccgtccttg tcggaggtca 1980
cgaccagggt gtggccgttg gattcgagat attcgcgcag gccgagctcg ccggagacgg 2040
agccgagcaa ctgcccgggc gtgaagtcga tggccttcgg cgtcggcaag atctggccgc 2100
ccggatagtg gtcgatcttc ggaagatcgt cgcgggcata ggtcttcggg tagccgtcga 2160
ccggatcatc gtaaagaacg cacaggacct ttgccatcat atgtatatct ccttcttaaa 2220
gttaaacaaa attatttcta gaggggaatt gttatccgct cacaattccc ctatagtgag 2280

tcgtattaat ttcgcgggat cgagatctcg atcctctacg ccggacgcat cgtggccggc 2340
atcaccggcg ccacaggtgc ggttgctggc gcctatatcg ccgacatcac cgatggggaa 2400
gatcgggctc gccacttcgg gctcatgagc gcttgtttcg gcgtgggtat ggtggcaggc 2460
cccgtggccg ggggactgtt gggcgccatc tccttgcatg caccattcct tgcggcggcg 2520
gtgctcaacg gcctcaacct actactgggc tgcttcctaa tgcaggagtc gcataaggga 2580
gagcgtcgag atcccggaca ccatcgaatg gcgcaaaacc tttcgcggta tggcatgata 2640
gcgcccggaa gagagtcaat tcagggtggt gaatgtgaaa ccagtaacgt tatacgatgt 2700
cgcagagtat gccggtgtct cttatcagac cgtttcccgc gtggtgaacc aggccagcca 2760
cgtttctgcg aaaacgcggg aaaaagtgga agcggcgatg gcggagctga attacattcc 2820
caaccgcgtg gcacaacaac tggcgggcaa acagtcgttg ctgattggcg ttgccacctc 2880
cagtctggcc ctgcacgcgc cgtcgcaaat tgtcgcggcg attaaatctc gcgccgatca 2940
actgggtgcc agcgtggtgg tgtcgatggt agaacgaagc ggcgtcgaag cctgtaaagc 3000
ggcggtgcac aatcttctcg cgcaacgcgt cagtgggctg atcattaact atccgctgga 3060
tgaccaggat gccattgctg tggaagctgc ctgcactaat gttccggcgt tatttcttga 3120
tgtctctgac cagacaccca tcaacagtat tattttctcc catgaagacg gtacgcgact 3180
gggcgtggag catctggtcg cattgggtca ccagcaaatc gcgctgttag cgggcccatt 3240
aagttctgtc tcggcgcgtc tgcgtctggc tggctggcat aaatatctca ctcgcaatca 3300
aattcagccg atagcggaac gggaaggcga ctggagtgcc atgtccggtt ttcaacaaac 3360
catgcaaatg ctgaatgagg gcatcgttcc cactgcgatg ctggttgcca acgatcagat 3420
ggcgctgggc gcaatgcgcg ccattaccga gtccgggctg cgcgttggtg cggatatctc 3480
ggtagtggga tacgacgata ccgaagacag ctcatgttat atcccgccgt taaccaccat 3540
caaacaggat tttcgcctgc tggggcaaac cagcgtggac cgcttgctgc aactctctca 3600
gggccaggcg gtgaagggca atcagctgtt gcccgtctca ctggtgaaaa gaaaaaccac 3660
cctggcgccc aatacgcaaa ccgcctctcc ccgcgcgttg gccgattcat taatgcagct 3720
ggcacgacag gtttcccgac tggaaagcgg gcagtgagcg caacgcaatt aatgtaagtt 3780
agctcactca ttaggcaccg ggatctcgac cgatgccctt gagagccttc aacccagtca 3840
gctccttccg gtgggcgcgg ggcatgacta tcgtcgccgc acttatgact gtcttcttta 3900
tcatgcaact cgtaggacag gtgccggcag cgctctgggt cattttcggc gaggaccgct 3960
ttcgctggag cgcgacgatg atcggcctgt cgcttgcggt attcggaatc ttgcacgccc 4020
tcgctcaagc cttcgtcact ggtcccgcca ccaaacgttt cggcgagaag caggccatta 4080
tcgccggcat ggcggcccca cgggtgcgca tgatcgtgct cctgtcgttg aggacccggc 4140
taggctggcg gggttgcctt actggttagc agaatgaatc accgatacgc gagcgaacgt 4200

gaagcgactg ctgctgcaaa acgtctgcga cctgagcaac aacatgaatg gtcttcggtt 4260
tccgtgtttc gtaaagtctg gaaacgcgga agtcagcgcc ctgcaccatt atgttccgga 4320
tctgcatcgc aggatgctgc tggctaccct gtggaacacc tacatctgta ttaacgaagc 4380
gctggcattg accctgagtg atttttctct ggtcccgccg catccatacc gccagttgtt 4440
taccctcaca acgttccagt aaccgggcat gttcatcatc agtaacccgt atcgtgagca 4500
tcctctctcg tttcatcggt atcattaccc ccatgaacag aaatccccct tacacggagg 4560
catcagtgac caaacaggaa aaaaccgccc ttaacatggc ccgctttatc agaagccaga 4620
cattaacgct tctggagaaa ctcaacgagc tggacgcgga tgaacaggca gacatctgtg 4680
aatcgcttca cgaccacgct gatgagcttt accgcagctg cctcgcgcgt ttcggtgatg 4740
acggtgaaaa cctctgacac atgcagctcc cggagacggt cacagcttgt ctgtaagcgg 4800
atgccgggag cagacaagcc cgtcagggcg cgtcagcggg tgttggcggg tgtcggggcg 4860
cagccatgac ccagtcacgt agcgatagcg gagtgtatac tggcttaact atgcggcatc 4920
agagcagatt gtactgagag tgcaccatat atgcggtgtg aaataccgca cagatgcgta 4980
aggagaaaat accgcatcag gcgctcttcc gcttcctcgc tcactgactc gctgcgctcg 5040
gtcgttcggc tgcggcgagc ggtatcagct cactcaaagg cggtaatacg gttatccaca 5100
gaatcagggg ataacgcagg aaagaacatg tgagcaaaag gccagcaaaa ggccaggaac 5160
cgtaaaaagg ccgcgttgct ggcgtttttc cataggctcc gcccccctga cgagcatcac 5220
aaaaatcgac gctcaagtca gaggtggcga aacccgacag gactataaag ataccaggcg 5280
tttccccctg gaagctccct cgtgcgctct cctgttccga ccctgccgct taccggatac 5340
ctgtccgcct ttctcccttc gggaagcgtg gcgctttctc atagctcacg ctgtaggtat 5400
ctcagttcgg tgtaggtcgt tcgctccaag ctgggctgtg tgcacgaacc ccccgttcag 5460
cccgaccgct gcgccttatc cggtaactat cgtcttgagt ccaacccggt aagacacgac 5520
ttatcgccac tggcagcagc cactggtaac aggattagca gagcgaggta tgtaggcggt 5580
gctacagagt tcttgaagtg gtggcctaac tacggctaca ctagaaggac agtatttggt 5640
atctgcgctc tgctgaagcc agttaccttc ggaaaaagag ttggtagctc ttgatccggc 5700
aaacaaacca ccgctggtag cggtggtttt tttgtttgca agcagcagat tacgcgcaga 5760
aaaaaaggat ctcaagaaga tcctttgatc ttttctacgg ggtctgacgc tcagtggaac 5820
gaaaactcac gttaagggat tttggtcatg agattatcaa aaaggatctt cacctagatc 5880
cttttaaatt aaaaatgaag ttttaaatca atctaaagta tatatgagta aacttggtct 5940
gacagttacc aatgcttaat cagtgaggca cctatctcag cgatctgtct atttcgttca 6000
tccatagttg cctgactccc cgtcgtgtag ataactacga tacgggaggg cttaccatct 6060

ggccccagtg ctgcaatgat accgcgagac ccacgctcac cggctccaga tttatcagca 6120

ataaaccagc cagccggaag ggccgagcgc agaagtggtc ctgcaacttt atccgcctcc 6180

atccagtcta ttaattgttg ccgggaagct agagtaagta gttcgccagt taatagtttg 6240

cgcaacgttg ttgccattgc tgcaggcatc gtggtgtcac gctcgtcgtt tggtatggct 6300

tcattcagct ccggttccca acgatcaagg cgagttacat gatcccccat gttgtgcaaa 6360

aaagcggtta gctccttcgg tcctccgatc gttgtcagaa gtaagttggc cgcagtgtta 6420

tcactcatgg ttatggcagc actgcataat tctcttactg tcatgccatc cgtaagatgc 6480

ttttctgtga ctggtgagta ctcaaccaag tcattctgag aatagtgtat gcggcgaccg 6540

agttgctctt gcccggcgtc aatacgggat aataccgcgc cacatagcag aactttaaaa 6600

gtgctcatca ttggaaaacg ttcttcgggg cgaaaactct caaggatctt accgctgttg 6660

agatccagtt cgatgtaacc cactcgtgca cccaactgat cttcagcatc ttttactttc 6720

accagcgttt ctgggtgagc aaaaacagga aggcaaaatg ccgcaaaaaa gggaataagg 6780

gcgacacgga aatgttgaat actcatactc ttcctttttc aatattattg aagcatttat 6840

cagggttatt gtctcatgag cggatacata tttgaatgta tttagaaaaa taaacaaata 6900

ggggttccgc gcacatttcc ccgaaaagtg ccacctgaaa ttgtaaacgt taatattttg 6960

ttaaaattcg cgttaaattt ttgttaaatc agctcatttt ttaaccaata ggccgaaatc 7020

ggcaaaatcc cttataaatc aaaagaatag accgagatag ggttgagtgt tgttccagtt 7080

tggaacaaga gtccactatt aaagaacgtg gactccaacg tcaaagggcg aaaaaccgtc 7140

tatcagggcg atggcccact acgtgaacca tcaccctaat caagtttttt ggggtcgagg 7200

tgccgtaaag cactaaatcg gaaccctaaa gggagccccc gatttagagc ttgacgggga 7260

aagccggcga acgtggcgag aaaggaaggg aagaaagcga aaggagcggg cgctagggcg 7320

ctggcaagtg tagcggtcac gctgcgcgta accaccacac ccgccgcgct taatgcgccg 7380

ctacagggcg cgtcccattc gcca 7404

<210> 53
<211> 27
<212> DNA
<213> artificial

<220>
<223> PCR Primer "G39D for"

<400> 53
gtcgtcatgg tcgaccgtcg cgaggag 27

<210> 54
<211> 27
<212> DNA

<213> artificial

<220>
<223> PCR Primer "G39D_rev"

<400> 54
ctcctcgcga cggtcgacca tgacgac 27

<210> 55
<211> 24
<212> DNA
<213> artificial

<220>
<223> PCR Primer "G39D/R40I_for"

<400> 55
gtcatggtcg acattcgcga ggag 24

<210> 56
<211> 24
<212> DNA
<213> artificial

<220>
<223> PCR Primer "G39D/R40I_rev"

<400> 56
ctcctcgcga atgtcgacca tgac 24

<210> 57
<211> 27
<212> DNA
<213> artificial

<220>
<223> PCR Primer "R40D_for"

<400> 57
gtcatggtcg gcgatcgcga ggagaag 27

<210> 58
<211> 27
<212> DNA
<213> artificial

<220>
<223> PCR Primer "R40D_rev"

<400> 58
cttctcctcg cgatcgccga ccatgac 27

<210> 59
<211> 30
<212> DNA
<213> artificial

<220>

<223> PCR Primer "R40D/R41I_for"

<400> 59
gtcatggtcg gcgatatcga ggagaagctg 30

<210> 60
<211> 30
<212> DNA
<213> artificial

<220>
<223> PCR Primer "R40D/R41I_rev"

<400> 60
cagcttctcc tcgatatcgc cgaccatgac 30

<210> 61
<211> 27
<212> DNA
<213> artificial

<220>
<223> PCR Primer "DIN_for"

<400> 61
atggtcgaca ttaacgagga gaagctg 27

<210> 62
<211> 27
<212> DNA
<213> artificial

<220>
<223> PCR Primer "DIN_rev"

<400> 62
cagcttctcc tcgttaatgt cgaccat 27

<210> 63
<211> 8321
<212> DNA
<213> artificial

<220>
<223> Plasmid pFr3T7 (D)

<400> 63

atccggatat agttcctcct ttcagcaaaa aacccctcaa gacccgttta gaggccccaa 60
ggggttatgc tagttattgc tcagcggtgg cagcagccaa ctcagcttcc tttcgggctt 120
tgttagcagc cggatctcag tggtggtggt ggtggtgctc gagtgcggcc gcctagtcgc 180
ggtagaacga ccccatgtag cggatgtact cgtcctcggt gtggtttgcc ttccagtcgt 240
ggacggagtc cttgttgcgc tggccggcga tgacggagag ctccttaccc agcttctcaa 300
aggcctcgtc aacgcactcc tcgggggtga gggcgatctt catgacggcc tcgccctgcg 360
ggccgccggg gaggttggac agcaggctgg gggttagggt ggtgccgagg gtgatgacct 420

cgacgtcgac gccggtgccc tcgcactcgc aggccacggc ctcggtcatc ttgaggatga 480
aggccttgcc cgcgccgtac tggccgttcc aggggctgga gctgatgccg gtcatcgacg 540
agacgttgat cacggcgccg cggtcctggg cggcaaagat ccgcatgtag tggtggaagc 600
acttgaggaa ggtcacgacg ttgacgttga tcatggcctc gtgcttctcc caggggggtgt 660
cctggatctt accgaagctg tgcaggcagg ccacgtagct catgaagccc atgtccaggc 720
cctcggtcgc ggcgaagacg gtctcggcag cgccgggctg gctaaagtcg gcgcgcacga 780
ccttggtctc cacgccgtag gtctcgcgga tctcgcctgc gagcacgttc agcttctcct 840
cgcgacggtc gaccatgacg acgttcatgc cgccggcggc gatcttctcg cagaacgcct 900
tgccgacgcc ctcggtcgcg cccaggatca ggccccactc accgtacttc tccctcaggt 960
tcatatgtat atctccttct tatacttaac taatatacta agatggggaa ttgttatccg 1020
ctcacaattc ccctatagtg agtcgtatta atttcgatta tgcggccgtg tacaatacga 1080
ttactttctg ttcgacttaa gcattataag ctttcagaac tgtgtcgggc gcatcaccgc 1140
atcaatgccg ccatcaatga cgatctgcgc gccatgcaca tagcttgcgg ccgggctcat 1200
caaaaaggcg atgaccgacg ccatctcgga cggctcggca cggcggccca tgggaggaac 1260
gaacttggca atggattcgc catagcgcgg gtcctgcagg cccgcctgca gcaagggagt 1320
ctcggttgca ccgggggcga tggtgttcag gcgcacgcca gcctcgcccc aggcggcggc 1380
gcgtttgcgc acagccaccg tcaaagcatt cttgctgccc gcataggcca gatttccgcc 1440
ctgctctccc gcatgttcga caatggcgcg ggccttggct tcctcgccgg cttccagtgc 1500
cagcgccagt gggttcttgt caaaagccag atgcgcggaa gccacggacg agatgacgac 1560
ggctgcgggc tgatggcctt ttttcagcgc tggcaaaaag gcatccatca gctcggtcgc 1620
gccaaaataa ttgaccgaaa ccacattgcc aagcaccttg gtctgcggtc ccaggccggc 1680
gcacagcacc aggccgtcca tgcccttgct gcacttcgcc agtacatcgg caatcgcctg 1740
ctttcgacct tcggccgtcg agagatcggc aatcacttcc gcatcgcgta tatcgatgcc 1800
tacgatctgg tgaccggccg cctccaggac cttgcgcgta gccgcaccaa tgccggtggc 1860
gcagccgctt atcacgatga tggacatgta tatctccttt cacgaattct cagccgcctt 1920
cttgaacttg gcggcctctt ccgaaccgcc ggtggcattg cccttcgagt aggaatgcgc 1980
gccggtgccg gcaagagcgc cgccctgcac gatgaggtat tcgtcgcgga tcggacggcc 2040
ctcgaagaag cactccagga tctcgcgggt gcccgccgca taacgcgcct gcgcggtcag 2100
cgtggtgccg gagatgtgcg gggtcatgcc gttatagggc atcgtccgcc aggggtggtc 2160
cttcggcgcc ggctgcggga accacacgtc gccggcatag ccggccagcc ggccggattc 2220
gagcgcacgt gccacggcat cgcggtcgca cagcttgccg cgggcggtgt tgacgatgta 2280

```
ggcgccacgc ttgaacagct tcagcgtctc gtcattgatc atgtgctcgg tttcggggtg     2340
cagcgggcag ttcagcgtca ccacgtcgca aaccggatac atgtcctcgc gggtcgcgtg     2400
ccaggtgagg ttgagctcct tctcgaccga ttccggcagg cggtgacggt cggtgtagtg     2460
caggtgcacg tcgaacggcg ccagacggcg cagcaccgcg agaccgatgc ggccggcggc     2520
cacggtgccg acatgcatcg cctcgaggtc gtaggcgtgg gagacgcagt cggcgatgtt     2580
ccagccgccc ttccgcgccc attcgtgcga gggcagatag ttgcgcacca gcgacaggat     2640
catcatcacc acatgctcgg cgacgctgat cgagttgcag taggtgactt ccgccacggt     2700
gacgttgcgg tcgatagccg actgaagatc gacgtggtcg gaaccgatgc cggcggtgag     2760
cgcgagcttc aggttcttgg ccttggcgat gcgctcgggc gtcagatagg ccggccagaa     2820
gggctgggag atgacgacat ccgcatcgac cagctcgcgc tcgaacaccg agtcggggcc     2880
gtccttgtcg gaggtcacga ccagggtgtg gccgttggat tcgagatatt cgcgcaggcc     2940
gagctcgccg gagacggagc cgagcaactg cccgggcgtg aagtcgatgg ccttcggcgt     3000
cggcaagatc tggccgcccg gatagtggtc gatcttcgga agatcgtcgc gggcataggt     3060
cttcgggtag ccgtcgaccg gatcatcgta aagaacgcac aggacctttg ccatcatatg     3120
tatatctcct tcttaaagtt aaacaaaatt atttctagag gggaattgtt atccgctcac     3180
aattccccta tagtgagtcg tattaatttc gcgggatcga gatctcgatc ctctacgccg     3240
gacgcatcgt ggccggcatc accggcgcca caggtgcggt tgctggcgcc tatatcgccg     3300
acatcaccga tggggaagat cgggctcgcc acttcgggct catgagcgct tgtttcggcg     3360
tgggtatggt ggcaggcccc gtggccgggg gactgttggg cgccatctcc ttgcatgcac     3420
cattccttgc ggcggcggtg ctcaacggcc tcaacctact actgggctgc ttcctaatgc     3480
aggagtcgca taagggagag cgtcgagatc ccggacacca tcgaatggcg caaaaccttt     3540
cgcggtatgg catgatagcg cccggaagag agtcaattca gggtggtgaa tgtgaaacca     3600
gtaacgttat acgatgtcgc agagtatgcc ggtgtctctt atcagaccgt ttcccgcgtg     3660
gtgaaccagg ccagccacgt ttctgcgaaa acgcgggaaa aagtggaagc ggcgatggcg     3720
gagctgaatt acattcccaa ccgcgtggca caacaactgg cgggcaaaca gtcgttgctg     3780
attggcgttg ccacctccag tctggccctg cacgcgccgt cgcaaattgt cgcggcgatt     3840
aaatctcgcg ccgatcaact gggtgccagc gtggtggtgt cgatggtaga acgaagcggc     3900
gtcgaagcct gtaaagcggc ggtgcacaat cttctcgcgc aacgcgtcag tgggctgatc     3960
attaactatc cgctggatga ccaggatgcc attgctgtgg aagctgcctg cactaatgtt     4020
ccggcgttat ttcttgatgt ctctgaccag acacccatca acagtattat tttctcccat     4080
gaagacggta cgcgactggg cgtggagcat ctggtcgcat tgggtcacca gcaaatcgcg     4140
ctgttagcgg gcccattaag ttctgtctcg gcgcgtctgc gtctggctgg ctggcataaa     4200
```

tatctcactc gcaatcaaat tcagccgata gcggaacggg aaggcgactg gagtgccatg 4260
tccggttttc aacaaaccat gcaaatgctg aatgagggca tcgttcccac tgcgatgctg 4320
gttgccaacg atcagatggc gctgggcgca atgcgcgcca ttaccgagtc cgggctgcgc 4380
gttggtgcgg atatctcggt agtgggatac gacgataccg aagacagctc atgttatatc 4440
ccgccgttaa ccaccatcaa acaggatttt cgcctgctgg ggcaaaccag cgtggaccgc 4500
ttgctgcaac tctctcaggg ccaggcggtg aagggcaatc agctgttgcc cgtctcactg 4560
gtgaaaagaa aaaccaccct ggcgcccaat acgcaaaccg cctctccccg cgcgttggcc 4620
gattcattaa tgcagctggc acgacaggtt tcccgactgg aaagcgggca gtgagcgcaa 4680
cgcaattaat gtaagttagc tcactcatta ggcaccggga tctcgaccga tgcccttgag 4740
agccttcaac ccagtcagct ccttccggtg ggcgcggggc atgactatcg tcgccgcact 4800
tatgactgtc ttctttatca tgcaactcgt aggacaggtg ccggcagcgc tctgggtcat 4860
tttcggcgag gaccgctttc gctggagcgc gacgatgatc ggcctgtcgc ttgcggtatt 4920
cggaatcttg cacgccctcg ctcaagcctt cgtcactggt cccgccacca aacgtttcgg 4980
cgagaagcag gccattatcg ccggcatggc ggccccacgg gtgcgcatga tcgtgctcct 5040
gtcgttgagg acccggctag gctggcgggg ttgccttact ggttagcaga atgaatcacc 5100
gatacgcgag cgaacgtgaa gcgactgctg ctgcaaaacg tctgcgacct gagcaacaac 5160
atgaatggtc ttcggtttcc gtgtttcgta aagtctggaa acgcggaagt cagcgccctg 5220
caccattatg ttccggatct gcatcgcagg atgctgctgg ctaccctgtg gaacacctac 5280
atctgtatta acgaagcgct ggcattgacc ctgagtgatt tttctctggt cccgccgcat 5340
ccataccgcc agttgtttac cctcacaacg ttccagtaac cgggcatgtt catcatcagt 5400
aacccgtatc gtgagcatcc tctctcgttt catcggtatc attaccccca tgaacagaaa 5460
tcccccttac acggaggcat cagtgaccaa acaggaaaaa accgccctta acatggcccg 5520
ctttatcaga agccagacat taacgcttct ggagaaactc aacgagctgg acgcggatga 5580
acaggcagac atctgtgaat cgcttcacga ccacgctgat gagctttacc gcagctgcct 5640
cgcgcgtttc ggtgatgacg gtgaaaacct ctgacacatg cagctcccgg agacggtcac 5700
agcttgtctg taagcggatg ccgggagcag acaagcccgt cagggcgcgt cagcgggtgt 5760
tggcgggtgt cggggcgcag ccatgaccca gtcacgtagc gatagcggag tgtatactgg 5820
cttaactatg cggcatcaga gcagattgta ctgagagtgc accatatatg cggtgtgaaa 5880
taccgcacag atgcgtaagg agaaaatacc gcatcaggcg ctcttccgct tcctcgctca 5940
ctgactcgct gcgctcggtc gttcggctgc ggcgagcggt atcagctcac tcaaaggcgg 6000
taatacggtt atccacagaa tcaggggata acgcaggaaa gaacatgtga gcaaaaggcc 6060

agcaaaaggc caggaaccgt aaaaaggccg cgttgctggc gtttttccat aggctccgcc 6120
cccctgacga gcatcacaaa aatcgacgct caagtcagag gtggcgaaac ccgacaggac 6180
tataaagata ccaggcgttt ccccctggaa gctccctcgt gcgctctcct gttccgaccc 6240
tgccgcttac cggatacctg tccgcctttc tcccttcggg aagcgtggcg ctttctcata 6300
gctcacgctg taggtatctc agttcggtgt aggtcgttcg ctccaagctg ggctgtgtgc 6360
acgaaccccc cgttcagccc gaccgctgcg ccttatccgg taactatcgt cttgagtcca 6420
acccggtaag acacgactta tcgccactgg cagcagccac tggtaacagg attagcagag 6480
cgaggtatgt aggcggtgct acagagttct tgaagtggtg gcctaactac ggctacacta 6540
gaaggacagt atttggtatc tgcgctctgc tgaagccagt taccttcgga aaaagagttg 6600
gtagctcttg atccggcaaa caaaccaccg ctggtagcgg tggttttttt gtttgcaagc 6660
agcagattac gcgcagaaaa aaaggatctc aagaagatcc tttgatcttt tctacggggt 6720
ctgacgctca gtggaacgaa aactcacgtt aagggatttt ggtcatgaga ttatcaaaaa 6780
ggatcttcac ctagatcctt ttaaattaaa aatgaagttt taaatcaatc taaagtatat 6840
atgagtaaac ttggtctgac agttaccaat gcttaatcag tgaggcacct atctcagcga 6900
tctgtctatt tcgttcatcc atagttgcct gactccccgt cgtgtagata actacgatac 6960
gggagggctt accatctggc cccagtgctg caatgatacc gcgagaccca cgctcaccgg 7020
ctccagattt atcagcaata aaccagccag ccggaagggc cgagcgcaga agtggtcctg 7080
caactttatc cgcctccatc cagtctatta attgttgccg ggaagctaga gtaagtagtt 7140
cgccagttaa tagtttgcgc aacgttgttg ccattgctgc aggcatcgtg gtgtcacgct 7200
cgtcgtttgg tatggcttca ttcagctccg gttcccaacg atcaaggcga gttacatgat 7260
cccccatgtt gtgcaaaaaa gcggttagct ccttcggtcc tccgatcgtt gtcagaagta 7320
agttggccgc agtgttatca ctcatggtta tggcagcact gcataattct cttactgtca 7380
tgccatccgt aagatgcttt tctgtgactg gtgagtactc aaccaagtca ttctgagaat 7440
agtgtatgcg gcgaccgagt tgctcttgcc cggcgtcaat acgggataat accgcgccac 7500
atagcagaac tttaaaagtg ctcatcattg gaaaacgttc ttcggggcga aaactctcaa 7560
ggatcttacc gctgttgaga tccagttcga tgtaacccac tcgtgcaccc aactgatctt 7620
cagcatcttt tactttcacc agcgtttctg ggtgagcaaa aacaggaagg caaaatgccg 7680
caaaaaaggg aataagggcg acacggaaat gttgaatact catactcttc ctttttcaat 7740
attattgaag catttatcag ggttattgtc tcatgagcgg atacatattt gaatgtattt 7800
agaaaaataa acaaataggg gttccgcgca catttccccg aaaagtgcca cctgaaattg 7860
taaacgttaa tattttgtta aaattcgcgt taaatttttg ttaaatcagc tcatttttta 7920
accaataggc cgaaatcggc aaaatccctt ataaatcaaa agaatagacc gagatagggt 7980

tgagtgttgt tccagtttgg aacaagagtc cactattaaa gaacgtggac tccaacgtca 8040

aagggcgaaa aaccgtctat cagggcgatg gcccactacg tgaaccatca ccctaatcaa 8100

gtttttttggg gtcgaggtgc cgtaaagcac taaatcggaa ccctaaaggg agcccccgat 8160

ttagagcttg acggggaaag ccggcgaacg tggcgagaaa ggaagggaag aaagcgaaag 8220

gagcgggcgc tagggcgctg gcaagtgtag cggtcacgct gcgcgtaacc accacacccg 8280

ccgcgcttaa tgcgccgcta cagggcgcgt cccattcgcc a 8321

<210> 64
<211> 8190
<212> DNA
<213> artificial

<220>
<223> pF(G)r7(A)r3

<400> 64

```
atccggatat agttcctcct ttcagcaaaa aacccctcaa gacccgttta gaggccccaa      60
ggggttatgc tagttattgc tcagcggtgg cagcagccaa ctcagcttcc tttcgggctt     120
tgttagcagc cggatctcag tggtggtggt ggtggtgctc gagtgcggcc gctcagaact     180
gtgtcgggcg catcaccgca tcaatgccgc catcaatgac gatctgcgcg ccatgcacat     240
agcttgcggc cgggctcatc aaaaaggcga tgaccgacgc catctcggac ggctcggcac     300
ggcggcccat gggaggaacg aacttggcaa tggattcgcc atagcgcggg tcctgcaggc     360
ccgcctgcag caagggagtc tcggttgcac cgggggcgat ggtgttcagg cgcacgccag     420
cctcgcccca ggcggcggcg cgtttgcgca cagccaccgt caaagcattc ttgctgcccg     480
cataggccag atttccgccc tgctctcccg catgttcgac aatggcgcgg gccttggctt     540
cctcgccggc ttccagtgcc agcgccagtg ggttcttgtc aaaagccaga tgcgcggaag     600
ccacggacga gatgacgacg gctgcgggct gatggccttt tttcagcgct ggcaaaaagg     660
catccatcag ctcggtcgcg ccaaaataat tgaccgaaac cacattgcca agcaccttgg     720
tctgcggtcc caggccggcg cacagcacca ggccgtccat gcccttgctg cacttcgcca     780
gtacatcggc aatcgcctgc tttcgacctt cggccgtcga gagatcggca atcacttccg     840
catcgcgtat atcgatgcct acgatctggt gaccggccgc ctccaggacc ttgcgcgtag     900
cggcaccaat gccggtggcg cagccgctta tcacgatgat ggacatgtat atctccttaa     960
gcttctagtc gcggtagaac gaccccatgt agcggatgta ctcgtcctcg gtgtggtttg    1020
ccttccagtc gtggacggag tccttgttgc gctggccggc gatgacggag agctccttac    1080
ccagcttctc aaaggcctcg tcaacgcact cctcgggggt gagggcgatc ttcatgacgg    1140
cctcgccctg cgggccgccg gggaggttgg acagcaggct gggggttagg gtggtgccga    1200
```

gggtgatgac ctcgacgtcg acgccggtgc cctcgcactc gcaggccacg gcctcggtca 1260
tcttgaggat gaaggccttg cccgcgccgt actggccgtt ccaggggctg gagctgatgc 1320
cggtcatcga cgagacgttg atcacggcgc cgcggtcctg ggcggcaaag atccgcatgt 1380
agtggtggaa gcacttgagg aaggtcacga cgttgacgtt gatcatggcc tcgtgcttct 1440
cccagggggt gtcctggatc ttaccgaagc tgtgcaggca ggccacgtag ctcatgaagc 1500
ccatgtccag gccctcggtc gcggcgaaga cggtctcggc agcgccgggc tggctaaagt 1560
cggcgcgcac gaccttggtc tccacgccgt aggtctcgcg gatctcgcct gcgagcacgt 1620
tcagcttctc ctcgcgacgg gcgaccatga cgacgttcat gccgccggcg gcgatcttct 1680
cgcagaacgc cttgccgacg ccctcggtcg cgcccaggat caggccccac tcaccgtact 1740
tctccctcag gttcatgtat atctcctttc acgaattctc agccgccttc ttgaacttgg 1800
cggcctcttc cgaaccgccg gtggcattgc ccttcgagta ggaatgcgcg ccggtgccgg 1860
caagagcgcc gccctgcacg atgaggtatt cgtcgcggat cggacggccc tcgaagaagc 1920
actccaggat ctcgcgggtg cccgccgcat aacgcgcctg cgcggtcagc gtggtgccgg 1980
agatgtgcgg ggtcatgccg ttatagggca tcgtccgcca ggggtggtcc ttcggcgccg 2040
gctgcgggaa ccacacgtcg ccggcatagc cggccagccg gccggattcg agcgcacgtg 2100
ccacggcatc gcggtcgcac agcttgccgc gggcggtgtt gacgatgtag gcgccacgct 2160
tgaacagctt cagcgtctcg tcattgatca tgtgctcggt ttcggggtgc agcgggcagt 2220
tcagcgtcac cacgtcgcaa accggataca tgtcctcgcg ggtcgcgtgc caggtgaggt 2280
tgagctcctt ctcgaccgat tccggcaggc ggtgacggcc ggtgtagtgc aggtgcacgt 2340
cgaacggcgc cagacggcgc agcaccgcga gaccgatgcg gccggcggcc acggtgccga 2400
catgcatcgc ctcgaggtcg taggcgtggg agacgcagtc ggcgatgttc cagccgccct 2460
tccgcgccca ttcgtgcgag ggcagatagt tgcgcaccag cgacaggatc atcatcacca 2520
catgctcggc gacgctgatc gagttgcagt aggtgacttc cgccacggtg acgttgcggt 2580
cgatagccga ctgaagatcg acgtggtcgg aaccgatgcc ggcggtgagc gcgagcttca 2640
ggttcttggc cttggcgatg cgctcgggcg tcagataggc cggccagaag ggctgggaga 2700
tgacgacatc cgcatcgacc agctcgcgct cgaacaccga gtcggggccg tccttgtcgg 2760
aggtcacgac cagggtgtgg ccgttggatt cgagatattc gcgcaggccg agctcgccgg 2820
agacggagcc gagcaactgc ccgggcgtga agtcgatggc cttcggcgtc ggcaagatct 2880
ggccgcccgg atagtggtcg atcttcggaa gatcgtcgcg ggcataggtc ttcgggtagc 2940
cgtcgaccgg atcatcgtaa agaacgcaca ggacctttgc catcatatgt atatctcctt 3000
cttaaagtta aacaaaatta tttctagagg ggaattgtta tccgctcaca attcccctat 3060
agtgagtcgt attaatttcg cgggatcgag atctcgatcc tctacgccgg acgcatcgtg 3120

gccggcatca ccggcgccac aggtgcggtt gctggcgcct atatcgccga catcaccgat 3180
ggggaagatc gggctcgcca cttcgggctc atgagcgctt gtttcggcgt gggtatggtg 3240
gcaggccccg tggccggggg actgttgggc gccatctcct tgcatgcacc attccttgcg 3300
gcggcggtgc tcaacggcct caacctacta ctgggctgct tcctaatgca ggagtcgcat 3360
aagggagagc gtcgagatcc cggacaccat cgaatggcgc aaaacctttc gcggtatggc 3420
atgatagcgc ccggaagaga gtcaattcag ggtggtgaat gtgaaaccag taacgttata 3480
cgatgtcgca gagtatgccg gtgtctctta tcagaccgtt tcccgcgtgg tgaaccaggc 3540
cagccacgtt tctgcgaaaa cgcgggaaaa agtggaagcg gcgatggcgg agctgaatta 3600
cattcccaac cgcgtggcac aacaactggc gggcaaacag tcgttgctga ttggcgttgc 3660
cacctccagt ctggccctgc acgcgccgtc gcaaattgtc gcggcgatta aatctcgcgc 3720
cgatcaactg ggtgccagcg tggtggtgtc gatggtagaa cgaagcggcg tcgaagcctg 3780
taaagcggcg gtgcacaatc ttctcgcgca acgcgtcagt gggctgatca ttaactatcc 3840
gctggatgac caggatgcca ttgctgtgga agctgcctgc actaatgttc cggcgttatt 3900
tcttgatgtc tctgaccaga cacccatcaa cagtattatt ttctcccatg aagacggtac 3960
gcgactgggc gtggagcatc tggtcgcatt gggtcaccag caaatcgcgc tgttagcggg 4020
cccattaagt tctgtctcgg cgcgtctgcg tctggctggc tggcataaat atctcactcg 4080
caatcaaatt cagccgatag cggaacggga aggcgactgg agtgccatgt ccggttttca 4140
acaaaccatg caaatgctga atgagggcat cgttcccact gcgatgctgg ttgccaacga 4200
tcagatggcg ctgggcgcaa tgcgcgccat taccgagtcc gggctgcgcg ttggtgcgga 4260
tatctcggta gtgggatacg acgataccga agacagctca tgttatatcc cgccgttaac 4320
caccatcaaa caggattttc gcctgctggg gcaaaccagc gtggaccgct tgctgcaact 4380
ctctcagggc caggcggtga agggcaatca gctgttgccc gtctcactgg tgaaaagaaa 4440
aaccaccctg gcgcccaata cgcaaaccgc ctctccccgc gcgttggccg attcattaat 4500
gcagctggca cgacaggttt cccgactgga aagcgggcag tgagcgcaac gcaattaatg 4560
taagttagct cactcattag gcaccgggat ctcgaccgat gcccttgaga gccttcaacc 4620
cagtcagctc cttccggtgg gcgcggggca tgactatcgt cgccgcactt atgactgtct 4680
tctttatcat gcaactcgta ggacaggtgc cggcagcgct ctgggtcatt ttcggcgagg 4740
accgctttcg ctggagcgcg acgatgatcg gcctgtcgct tgcggtattc ggaatcttgc 4800
acgccctcgc tcaagccttc gtcactggtc ccgccaccaa acgtttcggc gagaagcagg 4860
ccattatcgc cggcatggcg gccccacggg tgcgcatgat cgtgctcctg tcgttgagga 4920
cccggctagg ctggcggggt tgccttactg gttagcagaa tgaatcaccg atacgcgagc 4980

gaacgtgaag cgactgctgc tgcaaaacgt ctgcgacctg agcaacaaca tgaatggtct 5040
tcggtttccg tgtttcgtaa agtctggaaa cgcggaagtc agcgccctgc accattatgt 5100
tccggatctg catcgcagga tgctgctggc taccctgtgg aacacctaca tctgtattaa 5160
cgaagcgctg gcattgaccc tgagtgattt ttctctggtc ccgccgcatc cataccgcca 5220
gttgtttacc ctcacaacgt tccagtaacc gggcatgttc atcatcagta acccgtatcg 5280
tgagcatcct ctctcgtttc atcggtatca ttacccccat gaacagaaat cccccttaca 5340
cggaggcatc agtgaccaaa caggaaaaaa ccgcccttaa catggcccgc tttatcagaa 5400
gccagacatt aacgcttctg gagaaactca acgagctgga cgcggatgaa caggcagaca 5460
tctgtgaatc gcttcacgac cacgctgatg agctttaccg cagctgcctc gcgcgtttcg 5520
gtgatgacgg tgaaaacctc tgacacatgc agctcccgga gacggtcaca gcttgtctgt 5580
aagcggatgc cgggagcaga caagcccgtc agggcgcgtc agcgggtgtt ggcgggtgtc 5640
ggggcgcagc catgacccag tcacgtagcg atagcggagt gtatactggc ttaactatgc 5700
ggcatcagag cagattgtac tgagagtgca ccatatatgc ggtgtgaaat accgcacaga 5760
tgcgtaagga gaaaataccg catcaggcgc tcttccgctt cctcgctcac tgactcgctg 5820
cgctcggtcg ttcggctgcg gcgagcggta tcagctcact caaaggcggt aatacggtta 5880
tccacagaat caggggataa cgcaggaaag aacatgtgag caaaaggcca gcaaaaggcc 5940
aggaaccgta aaaaggccgc gttgctggcg tttttccata ggctccgccc ccctgacgag 6000
catcacaaaa atcgacgctc aagtcagagg tggcgaaacc cgacaggact ataaagatac 6060
caggcgtttc cccctggaag ctccctcgtg cgctctcctg ttccgaccct gccgcttacc 6120
ggatacctgt ccgcctttct cccttcggga agcgtggcgc tttctcatag ctcacgctgt 6180
aggtatctca gttcggtgta ggtcgttcgc tccaagctgg gctgtgtgca cgaacccccc 6240
gttcagcccg accgctgcgc cttatccggt aactatcgtc ttgagtccaa cccggtaaga 6300
cacgacttat cgccactggc agcagccact ggtaacagga ttagcagagc gaggtatgta 6360
ggcggtgcta cagagttctt gaagtggtgg cctaactacg gctacactag aaggacagta 6420
tttggtatct gcgctctgct gaagccagtt accttcggaa aaagagttgg tagctcttga 6480
tccggcaaac aaaccaccgc tggtagcggt ggtttttttg tttgcaagca gcagattacg 6540
cgcagaaaaa aaggatctca agaagatcct ttgatctttt ctacggggtc tgacgctcag 6600
tggaacgaaa actcacgtta agggattttg gtcatgagat tatcaaaaag gatcttcacc 6660
tagatccttt taaattaaaa atgaagtttt aaatcaatct aaagtatata tgagtaaact 6720
tggtctgaca gttaccaatg cttaatcagt gaggcaccta tctcagcgat ctgtctattt 6780
cgttcatcca tagttgcctg actccccgtc gtgtagataa ctacgatacg ggagggctta 6840
ccatctggcc ccagtgctgc aatgataccg cgagacccac gctcaccggc tccagattta 6900

tcagcaataa accagccagc cggaagggcc gagcgcagaa gtggtcctgc aactttatcc 6960

gcctccatcc agtctattaa ttgttgccgg gaagctagag taagtagttc gccagttaat 7020

agtttgcgca acgttgttgc cattgctgca ggcatcgtgg tgtcacgctc gtcgtttggt 7080

atggcttcat tcagctccgg ttcccaacga tcaaggcgag ttacatgatc ccccatgttg 7140

tgcaaaaaag cggttagctc cttcggtcct ccgatcgttg tcagaagtaa gttggccgca 7200

gtgttatcac tcatggttat ggcagcactg cataattctc ttactgtcat gccatccgta 7260

agatgctttt ctgtgactgg tgagtactca accaagtcat tctgagaata gtgtatgcgg 7320

cgaccgagtt gctcttgccc ggcgtcaata cgggataata ccgcgccaca tagcagaact 7380

ttaaaagtgc tcatcattgg aaaacgttct tcggggcgaa aactctcaag gatcttaccg 7440

ctgttgagat ccagttcgat gtaacccact cgtgcaccca actgatcttc agcatctttt 7500

actttcacca gcgtttctgg gtgagcaaaa acaggaaggc aaaatgccgc aaaaaaggga 7560

ataagggcga cacggaaatg ttgaatactc atactcttcc tttttcaata ttattgaagc 7620

atttatcagg gttattgtct catgagcgga tacatatttg aatgtattta gaaaaataaa 7680

caaatagggg ttccgcgcac atttccccga aaagtgccac ctgaaattgt aaacgttaat 7740

attttgttaa aattcgcgtt aaatttttgt taaatcagct cattttttaa ccaataggcc 7800

gaaatcggca aaatccctta taaatcaaaa gaatagaccg agatagggtt gagtgttgtt 7860

ccagtttgga acaagagtcc actattaaag aacgtggact ccaacgtcaa agggcgaaaa 7920

accgtctatc agggcgatgg cccactacgt gaaccatcac cctaatcaag ttttttgggg 7980

tcgaggtgcc gtaaagcact aaatcggaac cctaaaggga gcccccgatt tagagcttga 8040

cggggaaagc cggcgaacgt ggcgagaaag gaagggaaga aagcgaaagg agcgggcgct 8100

agggcgctgg caagtgtagc ggtcacgctg cgcgtaacca ccacacccgc cgcgcttaat 8160

gcgccgctac agggcgcgtc ccattcgcca 8190

<210> 65
<211> 8190
<212> DNA
<213> artificial

<220>
<223> pF(G)r7(S)r3

<400> 65

```
atccggatat agttcctcct ttcagcaaaa aacccctcaa gacccgttta gaggccccaa      60
ggggttatgc tagttattgc tcagcggtgg cagcagccaa ctcagcttcc tttcgggctt     120
tgttagcagc cggatctcag tggtggtggt ggtggtgctc gagtgcggcc gctcagaact     180
gtgtcgggcg catcaccgca tcaatgccgc catcaatgac gatctgcgcg ccatgcacat     240
```

agcttgcggc cgggctcatc aaaaaggcga tgaccgacgc catctcggac ggctcggcac 300
ggcggcccat gggaggaacg aacttggcaa tggattcgcc atagcgcggg tcctgcaggc 360
ccgcctgcag caagggagtc tcggttgcac cgggggcgat ggtgttcagg cgcacgccag 420
cctcgcccca ggcggcggcg cgtttgcgca cagccaccgt caaagcattc ttgctgcccg 480
cataggccag atttccgccc tgctctcccg catgttcgac aatggcgcgg gccttggctt 540
cctcgccggc ttccagtgcc agcgccagtg ggttcttgtc aaaagccaga tgcgcggaag 600
ccacggacga gatgacgacg gctgcgggct gatggccttt tttcagcgct ggcaaaaagg 660
catccatcag ctcggtcgcg ccaaaataat tgaccgaaac cacattgcca agcaccttgg 720
tctgcggtcc caggccggcg cacagcacca ggccgtccat gcccttgctg cacttcgcca 780
gtacatcggc aatcgcctgc tttcgacctt cggccgtcga gagatcggca atcacttccg 840
catcgcgtat atcgatgcct acgatctggt gaccggccgc ctccaggacc ttgcgcgtag 900
cggcaccaat gccggtggcg cagccgctta tcacgatgat ggacatgtat atctccttaa 960
gcttctagtc gcggtagaac gaccccatgt agcggatgta ctcgtcctcg gtgtggtttg 1020
ccttccagtc gtggacggag tccttgttgc gctggccggc gatgacggag agctccttac 1080
ccagcttctc aaaggcctcg tcaacgcact cctcgggggt gagggcgatc ttcatgacgg 1140
cctcgccctg cgggccgccg gggaggttgg acagcaggct gggggttagg gtggtgccga 1200
gggtgatgac ctcgacgtcg acgccggtgc cctcgcactc gcaggccacg gcctcggtca 1260
tcttgaggat gaaggccttg cccgcgccgt actggccgtt ccaggggctg gagctgatgc 1320
cggtcatcga cgagacgttg atcacggcgc cgcggtcctg ggcggcaaag atccgcatgt 1380
agtggtggaa gcacttgagg aaggtcacga cgttgacgtt gatcatggcc tcgtgcttct 1440
cccagggggt gtcctggatc ttaccgaagc tgtgcaggca ggccacgtag ctcatgaagc 1500
ccatgtccag gccctcggtc gcggcgaaga cggtctcggc agcgccgggc tggctaaagt 1560
cggcgcgcac gaccttggtc tccacgccgt aggtctcgcg gatctcgcct gcgagcacgt 1620
tcagcttctc ctcgcgacgg ctgaccatga cgacgttcat gccgccggcg gcgatcttct 1680
cgcagaacgc cttgccgacg ccctcggtcg cgcccaggat caggccccac tcaccgtact 1740
tctccctcag gttcatgtat atctcctttc acgaattctc agccgccttc ttgaacttgg 1800
cggcctcttc cgaaccgccg gtggcattgc ccttcgagta ggaatgcgcg ccggtgccgg 1860
caagagcgcc gccctgcacg atgaggtatt cgtcgcggat cggacggccc tcgaagaagc 1920
actccaggat ctcgcgggtg cccgccgcat aacgcgcctg cgcggtcagc gtggtgccgg 1980
agatgtgcgg ggtcatgccg ttatagggca tcgtccgcca ggggtggtcc ttcggcgccg 2040
gctgcgggaa ccacacgtcg ccggcatagc cggccagccg gccggattcg agcgcacgtg 2100
ccacggcatc gcggtcgcac agcttgccgc gggcggtgtt gacgatgtag gcgccacgct 2160

tgaacagctt cagcgtctcg tcattgatca tgtgctcggt ttcggggtgc agcgggcagt 2220
tcagcgtcac cacgtcgcaa accggataca tgtcctcgcg ggtcgcgtgc caggtgaggt 2280
tgagctcctt ctcgaccgat tccggcaggc ggtgacggcc ggtgtagtgc aggtgcacgt 2340
cgaacggcgc cagacggcgc agcaccgcga gaccgatgcg gccggcggcc acggtgccga 2400
catgcatcgc ctcgaggtcg taggcgtggg agacgcagtc ggcgatgttc cagccgccct 2460
tccgcgccca ttcgtgcgag ggcagatagt tgcgcaccag cgacaggatc atcatcacca 2520
catgctcggc gacgctgatc gagttgcagt aggtgacttc cgccacggtg acgttgcggt 2580
cgatagccga ctgaagatcg acgtggtcgg aaccgatgcc ggcggtgagc gcgagcttca 2640
ggttcttggc cttggcgatg cgctcgggcg tcagataggc cggccagaag ggctgggaga 2700
tgacgacatc cgcatcgacc agctcgcgct cgaacaccga gtcggggccg tccttgtcgg 2760
aggtcacgac cagggtgtgg ccgttggatt cgagatattc gcgcaggccg agctcgccgg 2820
agacggagcc gagcaactgc ccgggcgtga agtcgatggc cttcggcgtc ggcaagatct 2880
ggccgcccgg atagtggtcg atcttcggaa gatcgtcgcg ggcataggtc ttcgggtagc 2940
cgtcgaccgg atcatcgtaa agaacgcaca ggacctttgc catcatatgt atatctcctt 3000
cttaaagtta aacaaaatta tttctagagg ggaattgtta tccgctcaca attcccctat 3060
agtgagtcgt attaatttcg cgggatcgag atctcgatcc tctacgccgg acgcatcgtg 3120
gccggcatca ccggcgccac aggtgcggtt gctggcgcct atatcgccga catcaccgat 3180
ggggaagatc gggctcgcca cttcgggctc atgagcgctt gtttcggcgt gggtatggtg 3240
gcaggccccg tggccggggg actgttgggc gccatctcct tgcatgcacc attccttgcg 3300
gcggcggtgc tcaacggcct caacctacta ctgggctgct tcctaatgca ggagtcgcat 3360
aagggagagc gtcgagatcc cggacaccat cgaatggcgc aaaacctttc gcggtatggc 3420
atgatagcgc ccggaagaga gtcaattcag ggtggtgaat gtgaaaccag taacgttata 3480
cgatgtcgca gagtatgccg gtgtctctta tcagaccgtt tcccgcgtgg tgaaccaggc 3540
cagccacgtt tctgcgaaaa cgcgggaaaa agtggaagcg gcgatggcgg agctgaatta 3600
cattcccaac cgcgtggcac aacaactggc gggcaaacag tcgttgctga ttggcgttgc 3660
cacctccagt ctggccctgc acgcgccgtc gcaaattgtc gcggcgatta aatctcgcgc 3720
cgatcaactg ggtgccagcg tggtggtgtc gatggtagaa cgaagcggcg tcgaagcctg 3780
taaagcggcg gtgcacaatc ttctcgcgca acgcgtcagt gggctgatca ttaactatcc 3840
gctggatgac caggatgcca ttgctgtgga agctgcctgc actaatgttc cggcgttatt 3900
tcttgatgtc tctgaccaga cacccatcaa cagtattatt ttctcccatg aagacggtac 3960
gcgactgggc gtggagcatc tggtcgcatt gggtcaccag caaatcgcgc tgttagcggg 4020

cccattaagt tctgtctcgg cgcgtctgcg tctggctggc tggcataaat atctcactcg 4080
caatcaaatt cagccgatag cggaacggga aggcgactgg agtgccatgt ccggttttca 4140
acaaaccatg caaatgctga atgagggcat cgttcccact gcgatgctgg ttgccaacga 4200
tcagatggcg ctgggcgcaa tgcgcgccat taccgagtcc gggctgcgcg ttggtgcgga 4260
tatctcggta gtgggatacg acgataccga agacagctca tgttatatcc cgccgttaac 4320
caccatcaaa caggattttc gcctgctggg gcaaaccagc gtggaccgct tgctgcaact 4380
ctctcagggc caggcggtga agggcaatca gctgttgccc gtctcactgg tgaaaagaaa 4440
aaccaccctg gcgcccaata cgcaaaccgc ctctccccgc gcgttggccg attcattaat 4500
gcagctggca cgacaggttt cccgactgga aagcgggcag tgagcgcaac gcaattaatg 4560
taagttagct cactcattag gcaccgggat ctcgaccgat gcccttgaga gccttcaacc 4620
cagtcagctc cttccggtgg gcgcggggca tgactatcgt cgccgcactt atgactgtct 4680
tctttatcat gcaactcgta ggacaggtgc cggcagcgct ctgggtcatt ttcggcgagg 4740
accgctttcg ctggagcgcg acgatgatcg gcctgtcgct tgcggtattc ggaatcttgc 4800
acgccctcgc tcaagccttc gtcactggtc ccgccaccaa acgtttcggc gagaagcagg 4860
ccattatcgc cggcatggcg gccccacggg tgcgcatgat cgtgctcctg tcgttgagga 4920
cccggctagg ctggcggggt tgccttactg gttagcagaa tgaatcaccg atacgcgagc 4980
gaacgtgaag cgactgctgc tgcaaaacgt ctgcgacctg agcaacaaca tgaatggtct 5040
tcggtttccg tgtttcgtaa agtctggaaa cgcggaagtc agcgccctgc accattatgt 5100
tccggatctg catcgcagga tgctgctggc taccctgtgg aacacctaca tctgtattaa 5160
cgaagcgctg gcattgaccc tgagtgattt ttctctggtc ccgccgcatc cataccgcca 5220
gttgtttacc ctcacaacgt tccagtaacc gggcatgttc atcatcagta acccgtatcg 5280
tgagcatcct ctctcgtttc atcggtatca ttacccccat gaacagaaat cccccttaca 5340
cggaggcatc agtgaccaaa caggaaaaaa ccgcccttaa catggcccgc tttatcagaa 5400
gccagacatt aacgcttctg gagaaactca acgagctgga cgcggatgaa caggcagaca 5460
tctgtgaatc gcttcacgac cacgctgatg agctttaccg cagctgcctc gcgcgtttcg 5520
gtgatgacgg tgaaaacctc tgacacatgc agctcccgga gacggtcaca gcttgtctgt 5580
aagcggatgc cgggagcaga caagcccgtc agggcgcgtc agcgggtgtt ggcgggtgtc 5640
ggggcgcagc catgacccag tcacgtagcg atagcggagt gtatactggc ttaactatgc 5700
ggcatcagag cagattgtac tgagagtgca ccatatatgc ggtgtgaaat accgcacaga 5760
tgcgtaagga gaaaataccg catcaggcgc tcttccgctt cctcgctcac tgactcgctg 5820
cgctcggtcg ttcggctgcg gcgagcggta tcagctcact caaaggcggt aatacggtta 5880
tccacagaat caggggataa cgcaggaaag aacatgtgag caaaaggcca gcaaaaggcc 5940

aggaaccgta aaaaggccgc gttgctggcg tttttccata ggctccgccc ccctgacgag 6000
catcacaaaa atcgacgctc aagtcagagg tggcgaaacc cgacaggact ataaagatac 6060
caggcgtttc cccctggaag ctccctcgtg cgctctcctg ttccgaccct gccgcttacc 6120
ggatacctgt ccgcctttct cccttcggga agcgtggcgc tttctcatag ctcacgctgt 6180
aggtatctca gttcggtgta ggtcgttcgc tccaagctgg gctgtgtgca cgaacccccc 6240
gttcagcccg accgctgcgc cttatccggt aactatcgtc ttgagtccaa cccggtaaga 6300
cacgacttat cgccactggc agcagccact ggtaacagga ttagcagagc gaggtatgta 6360
ggcggtgcta cagagttctt gaagtggtgg cctaactacg gctacactag aaggacagta 6420
tttggtatct gcgctctgct gaagccagtt accttcggaa aaagagttgg tagctcttga 6480
tccggcaaac aaaccaccgc tggtagcggt ggtttttttg tttgcaagca gcagattacg 6540
cgcagaaaaa aaggatctca agaagatcct ttgatctttt ctacggggtc tgacgctcag 6600
tggaacgaaa actcacgtta agggattttg gtcatgagat tatcaaaaag gatcttcacc 6660
tagatccttt taaattaaaa atgaagtttt aaatcaatct aaagtatata tgagtaaact 6720
tggtctgaca gttaccaatg cttaatcagt gaggcaccta tctcagcgat ctgtctattt 6780
cgttcatcca tagttgcctg actccccgtc gtgtagataa ctacgatacg ggagggctta 6840
ccatctggcc ccagtgctgc aatgataccg cgagacccac gctcaccggc tccagattta 6900
tcagcaataa accagccagc cggaagggcc gagcgcagaa gtggtcctgc aactttatcc 6960
gcctccatcc agtctattaa ttgttgccgg gaagctagag taagtagttc gccagttaat 7020
agtttgcgca acgttgttgc cattgctgca ggcatcgtgg tgtcacgctc gtcgtttggt 7080
atggcttcat tcagctccgg ttcccaacga tcaaggcgag ttacatgatc ccccatgttg 7140
tgcaaaaaag cggttagctc cttcggtcct ccgatcgttg tcagaagtaa gttggccgca 7200
gtgttatcac tcatggttat ggcagcactg cataattctc ttactgtcat gccatccgta 7260
agatgctttt ctgtgactgg tgagtactca accaagtcat tctgagaata gtgtatgcgg 7320
cgaccgagtt gctcttgccc ggcgtcaata cgggataata ccgcgccaca tagcagaact 7380
ttaaaagtgc tcatcattgg aaaacgttct tcggggcgaa aactctcaag gatcttaccg 7440
ctgttgagat ccagttcgat gtaacccact cgtgcaccca actgatcttc agcatctttt 7500
actttcacca gcgtttctgg gtgagcaaaa acaggaaggc aaaatgccgc aaaaaaggga 7560
ataagggcga cacggaaatg ttgaatactc atactcttcc tttttcaata ttattgaagc 7620
atttatcagg gttattgtct catgagcgga tacatatttg aatgtattta gaaaaataaa 7680
caaatagggg ttccgcgcac atttccccga aaagtgccac ctgaaattgt aaacgttaat 7740
attttgttaa aattcgcgtt aaatttttgt taaatcagct cattttttaa ccaataggcc 7800

gaaatcggca aaatccctta taaatcaaaa gaatagaccg agatagggtt gagtgttgtt 7860

ccagtttgga acaagagtcc actattaaag aacgtggact ccaacgtcaa agggcgaaaa 7920

accgtctatc agggcgatgg cccactacgt gaaccatcac cctaatcaag ttttttgggg 7980

tcgaggtgcc gtaaagcact aaatcggaac cctaaaggga gcccccgatt tagagcttga 8040

cggggaaagc cggcgaacgt ggcgagaaag gaagggaaga aagcgaaagg agcgggcgct 8100

agggcgctgg caagtgtagc ggtcacgctg cgcgtaacca ccacacccgc cgcgcttaat 8160

gcgccgctac agggcgcgtc ccattcgcca 8190

<210> 66
<211> 7910
<212> DNA
<213> artificial

<220>
<223> p3T7 (A) rG

<400> 66

atccggatat agttcctcct ttcagcaaaa aaccoctcaa gacccgttta gaggccccaa 60
ggggttatgc tagttattgc tcagcggtgg cagcagccaa ctcagcttcc tttcgggctt 120
tgttagcagc cggatctcag tggtggtggt ggtggtgctc gagttaaccg cggcctgcct 180
ggaatgaagg atattgtgtc ataccgccgt ccgcgaataa cgtgatgcct gtgacgtagc 240
tggcttcctt cgaagcaagc caggctgcta ctgcggcgat ctcctccggt tcgccgatat 300
atcccattgg aatcatgctt tctacatcag ctttctgttt agggtcagcg aatttttcag 360
cattgattgg cgtgttgatc gcacctggcc caatattatt gacgcgaatg cccttcggcg 420
cgtattccaa cgctaatgtt tctgtcatca gctttatccc gcctttactt gccgcatagt 480
ggacaaataa cggccaagga atcacttcgt gcacactgga catgttaatg acatttccct 540
tgatatcgtt ttctacgaaa tatttaatcg cttcacggct tcctaaaaag gcacccgtta 600
agttcgtgcc gatgacttta tcccaatcct tgagcggcat ttcgtgagat ggcacaggat 660
tttcaagacc ggcattatta atcataatat cgagtgtgcc gaactcctta attgccgttt 720
gcacgatatt ttttacatct tcctctttcg tgacatctcc ttggacgaca acagcttcac 780
cgcccgcctt gatgacctct tcttttacct cgttcggatc ttgtttatta ctataatagt 840
tgataaccac ttttgcctgc tccttgccga agcgaatggc catcgccttt ccgagccctg 900
aagcagctcc tgtaatagcg acgacttttc cttttaaatc cggatacatg tatatctcct 960
tgcggccgcc tagtcgcggt agaacgaccc catgtagcgg atgtactcgt cctcggtgtg 1020
gtttgccttc cagtcgtgga cggagtcctt gttgcgctgg ccggcgatga cggagagctc 1080
cttacccagc ttctcaaagg cctcgtcaac gcactcctcg gggtgagggg cgatcttcat 1140
gacggcctcg ccctgcgggc cgccggggag gttggacagc aggctggggg ttagggtggt 1200

gccgagggtg atgacctcga cgtcgacgcc ggtgccctcg cactcgcagg ccacggcctc 1260
ggtcatcttg aggatgaagg ccttgcccgc gccgtactgg ccgttccagg ggctggagct 1320
gatgccggtc atcgacgaga cgttgatcac ggcgccgcgg tcctgggcgg caaagatccg 1380
catgtagtgg tggaagcact tgaggaaggt cacgacgttg acgttgatca tggcctcgtg 1440
cttctcccag gggtgtcct ggatcttacc gaagctgtgc aggcaggcca cgtagctcat 1500
gaagcccatg tccaggccct cggtcgcggc gaagacggtc tcggcagcgc cgggctggct 1560
aaagtcggcg cgcacgacct tggtctccac gccgtaggtc tcgcggatct cgcctgcgag 1620
cacgttcagc ttctcctcgc gacgggcgac catgacgacg ttcatgccgc cggcggcgat 1680
cttctcgcag aacgccttgc cgacgccctc ggtcgcgccc aggatcaggc cccactcacc 1740
gtacttctcc ctcaggttca tatgtatatc tccttcttat acttaactaa tatactaaga 1800
tggggaattg ttatccgctc acaattcccc tatagtgagt cgtattaatt tcgattatgc 1860
ggccgtgtac aatacgatta ctttctgttc gacttaagca ttataagctt gtcgacggag 1920
ctcgaattct cagaactgtg tcgggcgcat caccgcatca atgccgccat caatgacgat 1980
ctgcgcgcca tgcacatagc ttgcggccgg gctcatcaaa aaggcgatga ccgacgccat 2040
ctcggacggc tcggcacggc ggcccatggg aggaacgaac ttggcaatgg attcgccata 2100
gcgcgggtcc tgcaggcccg cctgcagcaa gggagtctcg gttgcaccgg gggcgatggt 2160
gttcaggcgc acgccagcct cgccccaggc ggcggcgcgt ttgcgcacag ccaccgtcaa 2220
agcattcttg ctgcccgcat aggccagatt tccgccctgc tctcccgcat gttcgacaat 2280
ggcgcgggcc ttggcttcct cgccggcttc cagtgccagc gccagtgggt tcttgtcaaa 2340
agccagatgc gcggaagcca cggacgagat gacgacggct gcgggctgat ggcctttttt 2400
cagcgctggc aaaaaggcat ccatcagctc ggtcgcgcca aaataattga ccgaaaccac 2460
attgccaagc accttggtct gcggtcccag gccggcgcac agcaccaggc cgtccatgcc 2520
cttgctgcac ttcgccagta catcggcaat cgcctgcttt cgaccttcgg ccgtcgagag 2580
atcggcaatc acttccgcat cgcgtatatc gatgcctacg atctggtgac cggccgcctc 2640
caggaccttg cgcgtagccg caccaatgcc ggtggcgcag ccgcttatca cgatgatgga 2700
catcatatgt atatctcctt cttaaagtta aacaaaatta tttctagagg ggaattgtta 2760
tccgctcaca attcccctat agtgagtcgt attaatttcg cgggatcgag atctcgatcc 2820
tctacgccgg acgcatcgtg gccggcatca ccggcgccac aggtgcggtt gctggcgcct 2880
atatcgccga catcaccgat ggggaagatc gggctcgcca cttcgggctc atgagcgctt 2940
gtttcggcgt gggtatggtg gcaggccccg tggccggggg actgttgggc gccatctcct 3000
tgcatgcacc attccttgcg gcggcggtgc tcaacggcct caacctacta ctgggctgct 3060

tcctaatgca ggagtcgcat aagggagagc gtcgagatcc cggacaccat cgaatggcgc 3120
aaaacctttc gcggtatggc atgatagcgc ccggaagaga gtcaattcag ggtggtgaat 3180
gtgaaaccag taacgttata cgatgtcgca gagtatgccg gtgtctctta tcagaccgtt 3240
tcccgcgtgg tgaaccaggc cagccacgtt tctgcgaaaa cgcgggaaaa agtggaagcg 3300
gcgatggcgg agctgaatta cattcccaac cgcgtggcac aacaactggc gggcaaacag 3360
tcgttgctga ttggcgttgc cacctccagt ctggccctgc acgcgccgtc gcaaattgtc 3420
gcggcgatta aatctcgcgc cgatcaactg ggtgccagcg tggtggtgtc gatggtagaa 3480
cgaagcggcg tcgaagcctg taaagcggcg gtgcacaatc ttctcgcgca acgcgtcagt 3540
gggctgatca ttaactatcc gctggatgac caggatgcca ttgctgtgga agctgcctgc 3600
actaatgttc cggcgttatt tcttgatgtc tctgaccaga cacccatcaa cagtattatt 3660
ttctcccatg aagacggtac gcgactgggc gtggagcatc tggtcgcatt gggtcaccag 3720
caaatcgcgc tgttagcggg cccattaagt tctgtctcgg cgcgtctgcg tctggctggc 3780
tggcataaat atctcactcg caatcaaatt cagccgatag cggaacggga aggcgactgg 3840
agtgccatgt ccggttttca acaaaccatg caaatgctga atgagggcat cgttcccact 3900
gcgatgctgg ttgccaacga tcagatggcg ctgggcgcaa tgcgcgccat taccgagtcc 3960
gggctgcgcg ttggtgcgga tatctcggta gtgggatacg acgataccga agacagctca 4020
tgttatatcc cgccgttaac caccatcaaa caggattttc gcctgctggg gcaaaccagc 4080
gtggaccgct tgctgcaact ctctcagggc caggcggtga agggcaatca gctgttgccc 4140
gtctcactgg tgaaaagaaa aaccaccctg gcgcccaata cgcaaaccgc ctctccccgc 4200
gcgttggccg attcattaat gcagctggca cgacaggttt cccgactgga aagcgggcag 4260
tgagcgcaac gcaattaatg taagttagct cactcattag gcaccgggat ctcgaccgat 4320
gcccttgaga gccttcaacc cagtcagctc cttccggtgg gcgcggggca tgactatcgt 4380
cgccgcactt atgactgtct tctttatcat gcaactcgta ggacaggtgc cggcagcgct 4440
ctgggtcatt ttcggcgagg accgctttcg ctggagcgcg acgatgatcg gcctgtcgct 4500
tgcggtattc ggaatcttgc acgccctcgc tcaagccttc gtcactggtc ccgccaccaa 4560
acgtttcggc gagaagcagg ccattatcgc cggcatggcg gccccacggg tgcgcatgat 4620
cgtgctcctg tcgttgagga cccggctagg ctggcggggt tgccttactg gttagcagaa 4680
tgaatcaccg atacgcgagc gaacgtgaag cgactgctgc tgcaaaacgt ctgcgacctg 4740
agcaacaaca tgaatggtct tcggtttccg tgtttcgtaa agtctggaaa cgcggaagtc 4800
agcgccctgc accattatgt tccggatctg catcgcagga tgctgctggc taccctgtgg 4860
aacacctaca tctgtattaa cgaagcgctg gcattgaccc tgagtgattt ttctctggtc 4920
ccgccgcatc cataccgcca gttgtttacc ctcacaacgt tccagtaacc gggcatgttc 4980

atcatcagta acccgtatcg tgagcatcct ctctcgtttc atcggtatca ttacccccat 5040
gaacagaaat cccccttaca cggaggcatc agtgaccaaa caggaaaaaa ccgcccttaa 5100
catggcccgc tttatcagaa gccagacatt aacgcttctg gagaaactca acgagctgga 5160
cgcggatgaa caggcagaca tctgtgaatc gcttcacgac cacgctgatg agctttaccg 5220
cagctgcctc gcgcgtttcg gtgatgacgg tgaaaacctc tgacacatgc agctcccgga 5280
gacggtcaca gcttgtctgt aagcggatgc cgggagcaga caagcccgtc agggcgcgtc 5340
agcgggtgtt ggcgggtgtc ggggcgcagc catgacccag tcacgtagcg atagcggagt 5400
gtatactggc ttaactatgc ggcatcagag cagattgtac tgagagtgca ccatatatgc 5460
ggtgtgaaat accgcacaga tgcgtaagga gaaaataccg catcaggcgc tcttccgctt 5520
cctcgctcac tgactcgctg cgctcggtcg ttcggctgcg gcgagcggta tcagctcact 5580
caaaggcggt aatacggtta tccacagaat caggggataa cgcaggaaag aacatgtgag 5640
caaaaggcca gcaaaaggcc aggaaccgta aaaaggccgc gttgctggcg tttttccata 5700
ggctccgccc ccctgacgag catcacaaaa atcgacgctc aagtcagagg tggcgaaacc 5760
cgacaggact ataaagatac caggcgtttc cccctggaag ctccctcgtg cgctctcctg 5820
ttccgaccct gccgcttacc ggatacctgt ccgcctttct cccttcggga agcgtggcgc 5880
tttctcatag ctcacgctgt aggtatctca gttcggtgta ggtcgttcgc tccaagctgg 5940
gctgtgtgca cgaacccccc gttcagcccg accgctgcgc cttatccggt aactatcgtc 6000
ttgagtccaa cccggtaaga cacgacttat cgccactggc agcagccact ggtaacagga 6060
ttagcagagc gaggtatgta ggcggtgcta cagagttctt gaagtggtgg cctaactacg 6120
gctacactag aaggacagta tttggtatct gcgctctgct gaagccagtt accttcggaa 6180
aaagagttgg tagctcttga tccggcaaac aaaccaccgc tggtagcggt ggtttttttg 6240
tttgcaagca gcagattacg cgcagaaaaa aaggatctca agaagatcct ttgatctttt 6300
ctacggggtc tgacgctcag tggaacgaaa actcacgtta agggattttg gtcatgagat 6360
tatcaaaaag gatcttcacc tagatccttt taaattaaaa atgaagtttt aaatcaatct 6420
aaagtatata tgagtaaact tggtctgaca gttaccaatg cttaatcagt gaggcaccta 6480
tctcagcgat ctgtctattt cgttcatcca tagttgcctg actccccgtc gtgtagataa 6540
ctacgatacg ggagggctta ccatctggcc ccagtgctgc aatgataccg cgagacccac 6600
gctcaccggc tccagattta tcagcaataa accagccagc cggaagggcc gagcgcagaa 6660
gtggtcctgc aactttatcc gcctccatcc agtctattaa ttgttgccgg gaagctagag 6720
taagtagttc gccagttaat agtttgcgca acgttgttgc cattgctgca ggcatcgtgg 6780
tgtcacgctc gtcgtttggt atggcttcat tcagctccgg ttcccaacga tcaaggcgag 6840

ttacatgatc ccccatgttg tgcaaaaaag cggttagctc cttcggtcct ccgatcgttg 6900
tcagaagtaa gttggccgca gtgttatcac tcatggttat ggcagcactg cataattctc 6960
ttactgtcat gccatccgta agatgctttt ctgtgactgg tgagtactca accaagtcat 7020
tctgagaata gtgtatgcgg cgaccgagtt gctcttgccc ggcgtcaata cgggataata 7080
ccgcgccaca tagcagaact ttaaaagtgc tcatcattgg aaaacgttct tcggggcgaa 7140
aactctcaag gatcttaccg ctgttgagat ccagttcgat gtaacccact cgtgcaccca 7200
actgatcttc agcatctttt actttcacca gcgtttctgg gtgagcaaaa acaggaaggc 7260
aaaatgccgc aaaaaaggga ataagggcga cacggaaatg ttgaatactc atactcttcc 7320
tttttcaata ttattgaagc atttatcagg gttattgtct catgagcgga tacatatttg 7380
aatgtattta gaaaaataaa caaatagggg ttccgcgcac atttccccga aaagtgccac 7440
ctgaaattgt aaacgttaat attttgttaa aattcgcgtt aaatttttgt taaatcagct 7500
cattttttaa ccaataggcc gaaatcggca aaatccctta taaatcaaaa gaatagaccg 7560
agatagggtt gagtgttgtt ccagtttgga acaagagtcc actattaaag aacgtggact 7620
ccaacgtcaa agggcgaaaa accgtctatc agggcgatgg cccactacgt gaaccatcac 7680
cctaatcaag ttttttgggg tcgaggtgcc gtaaagcact aaatcggaac cctaaaggga 7740
gcccccgatt tagagcttga cggggaaagc cggcgaacgt ggcgagaaag gaagggaaga 7800
aagcgaaagg agcgggcgct agggcgctgg caagtgtagc ggtcacgctg cgcgtaacca 7860
ccacacccgc cgcgcttaat gcgccgctac agggcgcgtc ccattcgcca 7910

<210> 67
<211> 7891
<212> DNA
<213> artificial

<220>
<223> p7 (A) T3rG

<400> 67

atccggatat agttcctcct ttcagcaaaa aacccctcaa gacccgttta gaggccccaa 60
ggggttatgc tagttattgc tcagcggtgg cagcagccaa ctcagcttcc tttcgggctt 120
tgttagcagc cggatctcag tggtggtggt ggtggtgctc gagttaaccg cggcctgcct 180
ggaatgaagg atattgtgtc ataccgccgt ccgcgaataa cgtgatgcct gtgacgtagc 240
tggcttcctt cgaagcaagc caggctgcta ctgcggcgat ctcctccggt tcgccgatat 300
atcccattgg aatcatgctt tctacatcag ctttctgttt agggtcagcg aatttttcag 360
cattgattgg cgtgttgatc gcacctggcc caatattatt gacgcgaatg cccttcggcg 420
cgtattccaa cgctaatgtt tctgtcatca gctttatccc gcctttactt gccgcatagt 480
ggacaaataa cggccaagga atcacttcgt gcacactgga catgttaatg acatttccct 540

```
tgatatcgtt ttctacgaaa tatttaatcg cttcacggct tcctaaaaag gcacccgtta      600
agttcgtgcc gatgacttta tcccaatcct tgagcggcat ttcgtgagat ggcacaggat      660
tttcaagacc ggcattatta atcataatat cgagtgtgcc gaactcctta attgccgttt      720
gcacgatatt ttttacatct tcctctttcg tgacatctcc ttggacgaca acagcttcac      780
cgcccgcctt gatgacctct tcttttacct cgttcggatc ttgtttatta ctataatagt      840
tgataaccac ttttgcctgc tccttgccga agcgaatggc catcgccttt ccgagccctg      900
aagcagctcc tgtaatagcg acgacttttc cttttaaatc cggatacatg tatatctcct      960
tgcggccgct cagaactgtg tcgggcgcat caccgcatca atgccgccat caatgacgat     1020
ctgcgcgcca tgcacatagc ttgcggccgg gctcatcaaa aaggcgatga ccgacgccat     1080
ctcggacggc tcggcacggc ggcccatggg aggaacgaac ttggcaatgg attcgccata     1140
gcgcgggtcc tgcaggcccg cctgcagcaa gggagtctcg gttgcaccgg gggcgatggt     1200
gttcaggcgc acgccagcct cgccccaggc ggcggcgcgt ttgcgcacag ccaccgtcaa     1260
agcattcttg ctgcccgcat aggccagatt tccgccctgc tctcccgcat gttcgacaat     1320
ggcgcgggcc ttggcttcct cgccggcttc cagtgccagc gccagtgggt tcttgtcaaa     1380
agccagatgc gcggaagcca cggacgagat gacgacggct gcgggctgat ggcctttttt     1440
cagcgctggc aaaaaggcat ccatcagctc ggtcgcgcca aaataattga ccgaaaccac     1500
attgccaagc accttggtct gcggtcccag gccggcgcac agcaccaggc cgtccatgcc     1560
cttgctgcac ttcgccagta catcggcaat cgcctgcttt cgaccttcgg ccgtcgagag     1620
atcggcaatc acttccgcat cgcgtatatc gatgcctacg atctggtgac cggccgcctc     1680
caggaccttg cgcgtagcgg caccaatgcc ggtggcgcag ccgcttatca cgatgatgga     1740
catcatatgt atatctcctt cttatactta actaatatac taagatgggg aattgttatc     1800
cgctcacaat tcccctatag tgagtcgtat taatttcgat tatgcggccg tgtacaatac     1860
gattactttc tgttcgactt aagcattata agcttctagt cgcggtagaa cgaccccatg     1920
tagcggatgt actcgtcctc ggtgtggttt gccttccagt cgtggacgga gtccttgttg     1980
cgctggccgg cgatgacgga gagctcctta cccagcttct caaaggcctc gtcaacgcac     2040
tcctcggggg tgagggcgat cttcatgacg gcctcgccct gcgggccgcc ggggaggttg     2100
gacagcaggc tgggggttag ggtggtgccg agggtgatga cctcgacgtc gacgccggtg     2160
ccctcgcact cgcaggccac ggcctcggtc atcttgagga tgaaggcctt gcccgcgccg     2220
tactggccgt tccaggggct ggagctgatg ccggtcatcg acgagacgtt gatcacggcg     2280
ccgcggtcct gggcggcaaa gatccgcatg tagtggtgga agcacttgag gaaggtcacg     2340
acgttgacgt tgatcatggc ctcgtgcttc tcccaggggg tgtcctggat cttaccgaag     2400
```

ctgtgcaggc aggccacgta gctcatgaag cccatgtcca ggccctcggt cgcggcgaag 2460
acggtctcgg cagcgccggg ctggctaaag tcggcgcgca cgaccttggt ctccacgccg 2520
taggtctcgc ggatctcgcc tgcgagcacg ttcagcttct cctcgcgacg ggcgaccatg 2580
acgacgttca tgccgccggc ggcgatcttc tcgcagaacg ccttgccgac gccctcggtc 2640
gcgcccagga tcaggcccca ctcaccgtac ttctccctca ggttcatatg tatatctcct 2700
tcttaaagtt aaacaaaatt atttctagag gggaattgtt atccgctcac aattccccta 2760
tagtgagtcg tattaatttc gcgggatcga gatctcgatc ctctacgccg gacgcatcgt 2820
ggccggcatc accggcgcca caggtgcggt tgctggcgcc tatatcgccg acatcaccga 2880
tggggaagat cgggctcgcc acttcgggct catgagcgct tgtttcggcg tgggtatggt 2940
ggcaggcccc gtggccgggg gactgttggg cgccatctcc ttgcatgcac cattccttgc 3000
ggcggcggtg ctcaacggcc tcaacctact actgggctgc ttcctaatgc aggagtcgca 3060
taagggagag cgtcgagatc ccggacacca tcgaatggcg caaaaccttt cgcggtatgg 3120
catgatagcg cccggaagag agtcaattca gggtggtgaa tgtgaaacca gtaacgttat 3180
acgatgtcgc agagtatgcc ggtgtctctt atcagaccgt ttcccgcgtg gtgaaccagg 3240
ccagccacgt ttctgcgaaa acgcgggaaa aagtggaagc ggcgatggcg gagctgaatt 3300
acattcccaa ccgcgtggca caacaactgg cgggcaaaca gtcgttgctg attggcgttg 3360
ccacctccag tctggccctg cacgcgccgt cgcaaattgt cgcggcgatt aaatctcgcg 3420
ccgatcaact gggtgccagc gtggtggtgt cgatggtaga acgaagcggc gtcgaagcct 3480
gtaaagcggc ggtgcacaat cttctcgcgc aacgcgtcag tgggctgatc attaactatc 3540
cgctggatga ccaggatgcc attgctgtgg aagctgcctg cactaatgtt ccggcgttat 3600
ttcttgatgt ctctgaccag acacccatca acagtattat tttctcccat gaagacggta 3660
cgcgactggg cgtggagcat ctggtcgcat tgggtcacca gcaaatcgcg ctgttagcgg 3720
gcccattaag ttctgtctcg gcgcgtctgc gtctggctgg ctggcataaa tatctcactc 3780
gcaatcaaat tcagccgata gcggaacggg aaggcgactg gagtgccatg tccggttttc 3840
aacaaaccat gcaaatgctg aatgagggca tcgttcccac tgcgatgctg gttgccaacg 3900
atcagatggc gctgggcgca atgcgcgcca ttaccgagtc cgggctgcgc gttggtgcgg 3960
atatctcggt agtgggatac gacgataccg aagacagctc atgttatatc ccgccgttaa 4020
ccaccatcaa acaggatttt cgcctgctgg ggcaaaccag cgtggaccgc ttgctgcaac 4080
tctctcaggg ccaggcggtg aagggcaatc agctgttgcc cgtctcactg gtgaaaagaa 4140
aaaccaccct ggcgcccaat acgcaaaccg cctctccccg cgcgttggcc gattcattaa 4200
tgcagctggc acgacaggtt tcccgactgg aaagcgggca gtgagcgcaa cgcaattaat 4260
gtaagttagc tcactcatta ggcaccggga tctcgaccga tgcccttgag agccttcaac 4320

ccagtcagct ccttccggtg ggcgcggggc atgactatcg tcgccgcact tatgactgtc 4380

ttctttatca tgcaactcgt aggacaggtg ccggcagcgc tctgggtcat tttcggcgag 4440

gaccgctttc gctggagcgc gacgatgatc ggcctgtcgc ttgcggtatt cggaatcttg 4500

cacgccctcg ctcaagcctt cgtcactggt cccgccacca aacgtttcgg cgagaagcag 4560

gccattatcg ccggcatggc ggccccacgg gtgcgcatga tcgtgctcct gtcgttgagg 4620

acccggctag gctggcgggg ttgccttact ggttagcaga atgaatcacc gatacgcgag 4680

cgaacgtgaa gcgactgctg ctgcaaaacg tctgcgacct gagcaacaac atgaatggtc 4740

ttcggtttcc gtgtttcgta aagtctggaa acgcggaagt cagcgccctg caccattatg 4800

ttccggatct gcatcgcagg atgctgctgg ctaccctgtg gaacacctac atctgtatta 4860

acgaagcgct ggcattgacc ctgagtgatt tttctctggt cccgccgcat ccataccgcc 4920

agttgtttac cctcacaacg ttccagtaac cgggcatgtt catcatcagt aacccgtatc 4980

gtgagcatcc tctctcgttt catcggtatc attaccccca tgaacagaaa tcccccttac 5040

acggaggcat cagtgaccaa acaggaaaaa accgccctta acatggcccg ctttatcaga 5100

agccagacat taacgcttct ggagaaactc aacgagctgg acgcggatga acaggcagac 5160

atctgtgaat cgcttcacga ccacgctgat gagctttacc gcagctgcct cgcgcgtttc 5220

ggtgatgacg gtgaaaacct ctgacacatg cagctcccgg agacggtcac agcttgtctg 5280

taagcggatg ccgggagcag acaagcccgt cagggcgcgt cagcgggtgt tggcgggtgt 5340

cggggcgcag ccatgaccca gtcacgtagc gatagcggag tgtatactgg cttaactatg 5400

cggcatcaga gcagattgta ctgagagtgc accatatatg cggtgtgaaa taccgcacag 5460

atgcgtaagg agaaaatacc gcatcaggcg ctcttccgct tcctcgctca ctgactcgct 5520

gcgctcggtc gttcggctgc ggcgagcggt atcagctcac tcaaaggcgg taatacggtt 5580

atccacagaa tcaggggata acgcaggaaa gaacatgtga gcaaaaggcc agcaaaaggc 5640

caggaaccgt aaaaaggccg cgttgctggc gtttttccat aggctccgcc cccctgacga 5700

gcatcacaaa aatcgacgct caagtcagag gtggcgaaac ccgacaggac tataaagata 5760

ccaggcgttt ccccctggaa gctccctcgt gcgctctcct gttccgaccc tgccgcttac 5820

cggatacctg tccgcctttc tcccttcggg aagcgtggcg ctttctcata gctcacgctg 5880

taggtatctc agttcggtgt aggtcgttcg ctccaagctg ggctgtgtgc acgaaccccc 5940

cgttcagccc gaccgctgcg ccttatccgg taactatcgt cttgagtcca acccggtaag 6000

acacgactta tcgccactgg cagcagccac tggtaacagg attagcagag cgaggtatgt 6060

aggcggtgct acagagttct tgaagtggtg gcctaactac ggctacacta gaaggacagt 6120

atttggtatc tgcgctctgc tgaagccagt taccttcgga aaaagagttg gtagctcttg 6180

atccggcaaa caaaccaccg ctggtagcgg tggttttttt gtttgcaagc agcagattac 6240
gcgcagaaaa aaaggatctc aagaagatcc tttgatcttt tctacggggt ctgacgctca 6300
gtggaacgaa aactcacgtt aagggatttt ggtcatgaga ttatcaaaaa ggatcttcac 6360
ctagatcctt ttaaattaaa aatgaagttt taaatcaatc taaagtatat atgagtaaac 6420
ttggtctgac agttaccaat gcttaatcag tgaggcacct atctcagcga tctgtctatt 6480
tcgttcatcc atagttgcct gactccccgt cgtgtagata actacgatac gggagggctt 6540
accatctggc cccagtgctg caatgatacc gcgagaccca cgctcaccgg ctccagattt 6600
atcagcaata aaccagccag ccggaagggc cgagcgcaga agtggtcctg caactttatc 6660
cgcctccatc cagtctatta attgttgccg ggaagctaga gtaagtagtt cgccagttaa 6720
tagtttgcgc aacgttgttg ccattgctgc aggcatcgtg gtgtcacgct cgtcgtttgg 6780
tatggcttca ttcagctccg gttcccaacg atcaaggcga gttacatgat cccccatgtt 6840
gtgcaaaaaa gcggttagct ccttcggtcc tccgatcgtt gtcagaagta agttggccgc 6900
agtgttatca ctcatggtta tggcagcact gcataattct cttactgtca tgccatccgt 6960
aagatgcttt tctgtgactg gtgagtactc aaccaagtca ttctgagaat agtgtatgcg 7020
gcgaccgagt tgctcttgcc cggcgtcaat acgggataat accgcgccac atagcagaac 7080
tttaaaagtg ctcatcattg gaaaacgttc ttcggggcga aaactctcaa ggatcttacc 7140
gctgttgaga tccagttcga tgtaacccac tcgtgcaccc aactgatctt cagcatcttt 7200
tactttcacc agcgtttctg ggtgagcaaa aacaggaagg caaaatgccg caaaaaaggg 7260
aataagggcg acacggaaat gttgaatact catactcttc ctttttcaat attattgaag 7320
catttatcag ggttattgtc tcatgagcgg atacatattt gaatgtattt agaaaaataa 7380
acaaataggg gttccgcgca catttccccg aaaagtgcca cctgaaattg taaacgttaa 7440
tattttgtta aaattcgcgt taaatttttg ttaaatcagc tcatttttta accaataggc 7500
cgaaatcggc aaaatccctt ataaatcaaa agaatagacc gagatagggt tgagtgttgt 7560
tccagtttgg aacaagagtc cactattaaa gaacgtggac tccaacgtca aagggcgaaa 7620
aaccgtctat cagggcgatg gcccactacg tgaaccatca ccctaatcaa gttttttggg 7680
gtcgaggtgc cgtaaagcac taaatcggaa ccctaaaggg agcccccgat ttagagcttg 7740
acggggaaag ccggcgaacg tggcgagaaa ggaagggaag aaagcgaaag gagcgggcgc 7800
tagggcgctg gcaagtgtag cggtcacgct gcgcgtaacc accacacccg ccgcgcttaa 7860
tgcgccgcta cagggcgcgt cccattcgcc a 7891

<210> 68
<211> 7404
<212> DNA

<213> artificial

<220>
<223> pF (G) r7 (A)

<400> 68

atccggatat agttcctcct ttcagcaaaa aacccctcaa gacccgttta gaggccccaa 60
ggggttatgc tagttattgc tcagcggtgg cagcagccaa ctcagcttcc tttcgggctt 120
tgttagcagc cggatctcag tggtggtggt ggtggtgctc gagtgcggcc gcaagcttct 180
agtcgcggta gaacgacccc atgtagcgga tgtactcgtc ctcggtgtgg tttgccttcc 240
agtcgtggac ggagtccttg ttgcgctggc cggcgatgac ggagagctcc ttacccagct 300
tctcaaaggc ctcgtcaacg cactcctcgg gggtgagggc gatcttcatg acggcctcgc 360
cctgcgggcc gccggggagg ttggacagca ggctgggggt tagggtggtg ccgagggtga 420
tgacctcgac gtcgacgccg gtgccctcgc actcgcaggc cacggcctcg gtcatcttga 480
ggatgaaggc cttgcccgcg ccgtactggc cgttccaggg gctggagctg atgccggtca 540
tcgacgagac gttgatcacg gcgccgcggt cctgggcggc aaagatccgc atgtagtggt 600
ggaagcactt gaggaaggtc acgacgttga cgttgatcat ggcctcgtgc ttctcccagg 660
gggtgtcctg gatcttaccg aagctgtgca ggcaggccac gtagctcatg aagcccatgt 720
ccaggccctc ggtcgcggcg aagacggtct cggcagcgcc gggctggcta aagtcggcgc 780
gcacgacctt ggtctccacg ccgtaggtct cgcggatctc gcctgcgagc acgttcagct 840
tctcctcgcg acgggcgacc atgacgacgt tcatgccgcc ggcggcgatc ttctcgcaga 900
acgccttgcc gacgccctcg gtcgcgccca ggatcaggcc ccactcaccg tacttctccc 960
tcaggttcat gtatatctcc tttcacgaat tctcagccgc cttcttgaac ttggcggcct 1020
cttccgaacc gccggtggca ttgcccttcg agtaggaatg cgcgccggtg ccggcaagag 1080
cgccgccctg cacgatgagg tattcgtcgc ggatcggacg gccctcgaag aagcactcca 1140
ggatctcgcg ggtgcccgcc gcataacgcg cctgcgcggt cagcgtggtg ccggagatgt 1200
gcggggtcat gccgttatag ggcatcgtcc gccaggggtg gtccttcggc gccggctgcg 1260
ggaaccacac gtcgccggca tagccggcca gccggccgga ttcgagcgca cgtgccacgg 1320
catcgcggtc gcacagcttg ccgcgggcgg tgttgacgat gtaggcgcca cgcttgaaca 1380
gcttcagcgt ctcgtcattg atcatgtgct cggtttcggg gtgcagcggg cagttcagcg 1440
tcaccacgtc gcaaaccgga tacatgtcct cgcgggtcgc gtgccaggtg aggttgagct 1500
ccttctcgac cgattccggc aggcggtgac ggccggtgta gtgcaggtgc acgtcgaacg 1560
gcgccagacg gcgcagcacc gcgagaccga tgcggccggc ggccacggtg ccgacatgca 1620
tcgcctcgag gtcgtaggcg tgggagacgc agtcggcgat gttccagccg cccttccgcg 1680
cccattcgtg cgagggcaga tagttgcgca ccagcgacag gatcatcatc accacatgct 1740

cggcgacgct gatcgagttg cagtaggtga cttccgccac ggtgacgttg cggtcgatag 1800
ccgactgaag atcgacgtgg tcggaaccga tgccggcggt gagcgcgagc ttcaggttct 1860
tggccttggc gatgcgctcg ggcgtcagat aggccggcca gaagggctgg gagatgacga 1920
catccgcatc gaccagctcg cgctcgaaca ccgagtcggg gccgtccttg tcggaggtca 1980
cgaccagggt gtggccgttg gattcgagat attcgcgcag gccgagctcg ccggagacgg 2040
agccgagcaa ctgcccgggc gtgaagtcga tggccttcgg cgtcggcaag atctggccgc 2100
ccggatagtg gtcgatcttc ggaagatcgt cgcgggcata ggtcttcggg tagccgtcga 2160
ccggatcatc gtaaagaacg cacaggacct ttgccatcat atgtatatct ccttcttaaa 2220
gttaaacaaa attatttcta gaggggaatt gttatccgct cacaattccc ctatagtgag 2280
tcgtattaat ttcgcgggat cgagatctcg atcctctacg ccggacgcat cgtggccggc 2340
atcaccggcg ccacaggtgc ggttgctggc gcctatatcg ccgacatcac cgatggggaa 2400
gatcgggctc gccacttcgg gctcatgagc gcttgtttcg gcgtgggtat ggtggcaggc 2460
cccgtggccg ggggactgtt gggcgccatc tccttgcatg caccattcct tgcggcggcg 2520
gtgctcaacg gcctcaacct actactgggc tgcttcctaa tgcaggagtc gcataaggga 2580
gagcgtcgag atcccggaca ccatcgaatg gcgcaaaacc tttcgcggta tggcatgata 2640
gcgcccggaa gagagtcaat tcagggtggt gaatgtgaaa ccagtaacgt tatacgatgt 2700
cgcagagtat gccggtgtct cttatcagac cgtttcccgc gtggtgaacc aggccagcca 2760
cgtttctgcg aaaacgcggg aaaaagtgga agcggcgatg gcggagctga attacattcc 2820
caaccgcgtg gcacaacaac tggcgggcaa acagtcgttg ctgattggcg ttgccacctc 2880
cagtctggcc ctgcacgcgc cgtcgcaaat tgtcgcggcg attaaatctc gcgccgatca 2940
actgggtgcc agcgtggtgg tgtcgatggt agaacgaagc ggcgtcgaag cctgtaaagc 3000
ggcggtgcac aatcttctcg cgcaacgcgt cagtgggctg atcattaact atccgctgga 3060
tgaccaggat gccattgctg tggaagctgc ctgcactaat gttccggcgt tatttcttga 3120
tgtctctgac cagacaccca tcaacagtat tattttctcc catgaagacg gtacgcgact 3180
gggcgtggag catctggtcg cattgggtca ccagcaaatc gcgctgttag cgggcccatt 3240
aagttctgtc tcggcgcgtc tgcgtctggc tggctggcat aaatatctca ctcgcaatca 3300
aattcagccg atagcggaac gggaaggcga ctggagtgcc atgtccggtt ttcaacaaac 3360
catgcaaatg ctgaatgagg gcatcgttcc cactgcgatg ctggttgcca acgatcagat 3420
ggcgctgggc gcaatgcgcg ccattaccga gtccgggctg cgcgttggtg cggatatctc 3480
ggtagtggga tacgacgata ccgaagacag ctcatgttat atcccgccgt taaccaccat 3540
caaacaggat tttcgcctgc tggggcaaac cagcgtggac cgcttgctgc aactctctca 3600
gggccaggcg gtgaagggca atcagctgtt gcccgtctca ctggtgaaaa gaaaaaccac 3660

cctggcgccc aatacgcaaa ccgcctctcc ccgcgcgttg gccgattcat taatgcagct 3720
ggcacgacag gtttcccgac tggaaagcgg gcagtgagcg caacgcaatt aatgtaagtt 3780
agctcactca ttaggcaccg ggatctcgac cgatgccctt gagagccttc aacccagtca 3840
gctccttccg gtgggcgcgg ggcatgacta tcgtcgccgc acttatgact gtcttcttta 3900
tcatgcaact cgtaggacag gtgccggcag cgctctgggt cattttcggc gaggaccgct 3960
ttcgctggag cgcgacgatg atcggcctgt cgcttgcggt attcggaatc ttgcacgccc 4020
tcgctcaagc cttcgtcact ggtcccgcca ccaaacgttt cggcgagaag caggccatta 4080
tcgccggcat ggcggcccca cgggtgcgca tgatcgtgct cctgtcgttg aggacccggc 4140
taggctggcg gggttgcctt actggttagc agaatgaatc accgatacgc gagcgaacgt 4200
gaagcgactg ctgctgcaaa acgtctgcga cctgagcaac aacatgaatg gtcttcggtt 4260
tccgtgtttc gtaaagtctg gaaacgcgga agtcagcgcc ctgcaccatt atgttccgga 4320
tctgcatcgc aggatgctgc tggctaccct gtggaacacc tacatctgta ttaacgaagc 4380
gctggcattg accctgagtg atttttctct ggtcccgccg catccatacc gccagttgtt 4440
taccctcaca acgttccagt aaccgggcat gttcatcatc agtaacccgt atcgtgagca 4500
tcctctctcg tttcatcggt atcattaccc ccatgaacag aaatccccct tacacggagg 4560
catcagtgac caaacaggaa aaaaccgccc ttaacatggc ccgctttatc agaagccaga 4620
cattaacgct tctggagaaa ctcaacgagc tggacgcgga tgaacaggca gacatctgtg 4680
aatcgcttca cgaccacgct gatgagcttt accgcagctg cctcgcgcgt ttcggtgatg 4740
acggtgaaaa cctctgacac atgcagctcc cggagacggt cacagcttgt ctgtaagcgg 4800
atgccgggag cagacaagcc cgtcagggcg cgtcagcggg tgttggcggg tgtcggggcg 4860
cagccatgac ccagtcacgt agcgatagcg gagtgtatac tggcttaact atgcggcatc 4920
agagcagatt gtactgagag tgcaccatat atgcggtgtg aaataccgca cagatgcgta 4980
aggagaaaat accgcatcag gcgctcttcc gcttcctcgc tcactgactc gctgcgctcg 5040
gtcgttcggc tgcggcgagc ggtatcagct cactcaaagg cggtaatacg gttatccaca 5100
gaatcagggg ataacgcagg aaagaacatg tgagcaaaag gccagcaaaa ggccaggaac 5160
cgtaaaaagg ccgcgttgct ggcgtttttc cataggctcc gcccccctga cgagcatcac 5220
aaaaatcgac gctcaagtca gaggtggcga aacccgacag gactataaag ataccaggcg 5280
tttccccctg gaagctccct cgtgcgctct cctgttccga ccctgccgct taccggatac 5340
ctgtccgcct ttctcccttc gggaagcgtg gcgctttctc atagctcacg ctgtaggtat 5400
ctcagttcgg tgtaggtcgt tcgctccaag ctgggctgtg tgcacgaacc ccccgttcag 5460
cccgaccgct gcgccttatc cggtaactat cgtcttgagt ccaacccggt aagacacgac 5520

ttatcgccac tggcagcagc cactggtaac aggattagca gagcgaggta tgtaggcggt 5580
gctacagagt tcttgaagtg gtggcctaac tacggctaca ctagaaggac agtatttggt 5640
atctgcgctc tgctgaagcc agttaccttc ggaaaaagag ttggtagctc ttgatccggc 5700
aaacaaacca ccgctggtag cggtggtttt tttgtttgca agcagcagat tacgcgcaga 5760
aaaaaaggat ctcaagaaga tcctttgatc ttttctacgg ggtctgacgc tcagtggaac 5820
gaaaactcac gttaagggat tttggtcatg agattatcaa aaaggatctt cacctagatc 5880
cttttaaatt aaaaatgaag ttttaaatca atctaaagta tatatgagta aacttggtct 5940
gacagttacc aatgcttaat cagtgaggca cctatctcag cgatctgtct atttcgttca 6000
tccatagttg cctgactccc cgtcgtgtag ataactacga tacgggaggg cttaccatct 6060
ggccccagtg ctgcaatgat accgcgagac ccacgctcac cggctccaga tttatcagca 6120
ataaaccagc cagccggaag ggccgagcgc agaagtggtc ctgcaacttt atccgcctcc 6180
atccagtcta ttaattgttg ccgggaagct agagtaagta gttcgccagt taatagtttg 6240
cgcaacgttg ttgccattgc tgcaggcatc gtggtgtcac gctcgtcgtt tggtatggct 6300
tcattcagct ccggttccca acgatcaagg cgagttacat gatcccccat gttgtgcaaa 6360
aaagcggtta gctccttcgg tcctccgatc gttgtcagaa gtaagttggc cgcagtgtta 6420
tcactcatgg ttatggcagc actgcataat tctcttactg tcatgccatc cgtaagatgc 6480
ttttctgtga ctggtgagta ctcaaccaag tcattctgag aatagtgtat gcggcgaccg 6540
agttgctctt gcccggcgtc aatacgggat aataccgcgc cacatagcag aactttaaaa 6600
gtgctcatca ttggaaaacg ttcttcgggg cgaaaactct caaggatctt accgctgttg 6660
agatccagtt cgatgtaacc cactcgtgca cccaactgat cttcagcatc ttttactttc 6720
accagcgttt ctgggtgagc aaaaacagga aggcaaaatg ccgcaaaaaa gggaataagg 6780
gcgacacgga aatgttgaat actcatactc ttcctttttc aatattattg aagcatttat 6840
cagggttatt gtctcatgag cggatacata tttgaatgta tttagaaaaa taaacaaata 6900
ggggttccgc gcacatttcc ccgaaaagtg ccacctgaaa ttgtaaacgt taatattttg 6960
ttaaaattcg cgttaaattt ttgttaaatc agctcatttt ttaaccaata ggccgaaatc 7020
ggcaaaatcc cttataaatc aaaagaatag accgagatag ggttgagtgt tgttccagtt 7080
tggaacaaga gtccactatt aaagaacgtg gactccaacg tcaaagggcg aaaaaccgtc 7140
tatcagggcg atggcccact acgtgaacca tcaccctaat caagtttttt ggggtcgagg 7200
tgccgtaaag cactaaatcg gaaccctaaa gggagccccc gatttagagc ttgacgggga 7260
aagccggcga acgtggcgag aaaggaaggg aagaaagcga aaggagcggg cgctagggcg 7320
ctggcaagtg tagcggtcac gctgcgcgta accaccacac ccgccgcgct taatgcgccg 7380
ctacagggcg cgtcccattc gcca 7404

<210> 69
<211> 7386
<212> DNA
<213> artificial

<220>
<223> pFr3

<400> 69

atccggatat agttcctcct ttcagcaaaa aacccctcaa gacccgttta gaggccccaa 60
ggggttatgc tagttattgc tcagcggtgg cagcagccaa ctcagcttcc tttcgggctt 120
tgttagcagc cggatctcag tggtggtggt ggtggtgctc gagtgcggcc gcaagctttc 180
agaactgtgt cgggcgcatc accgcatcaa tgccgccatc aatgacgatc tgcgcgccat 240
gcacatagct tgcggccggg ctcatcaaaa aggcgatgac cgacgccatc tcggacggct 300
cggcacggcg gcccatggga ggaacgaact tggcaatgga ttcgccatag cgcgggtcct 360
gcaggcccgc ctgcagcaag ggagtctcgg ttgcaccggg ggcgatggtg ttcaggcgca 420
cgccagcctc gccccaggcg gcggcgcgtt tgcgcacagc caccgtcaaa gcattcttgc 480
tgcccgcata ggccagattt ccgccctgct ctcccgcatg ttcgacaatg gcgcgggcct 540
tggcttcctc gccggcttcc agtgccagcg ccagtgggtt cttgtcaaaa gccagatgcg 600
cggaagccac ggacgagatg acgacggctg cgggctgatg gccttttttc agcgctggca 660
aaaaggcatc catcagctcg gtcgcgccaa aataattgac cgaaaccaca ttgccaagca 720
ccttggtctg cggtcccagg ccggcgcaca gcaccaggcc gtccatgccc ttgctgcact 780
tcgccagtac atcggcaatc gcctgctttc gaccttcggc cgtcgagaga tcggcaatca 840
cttccgcatc gcgtatatcg atgcctacga tctggtgacc ggccgcctcc aggaccttgc 900
gcgtagccgc accaatgccg gtggcgcagc cgcttatcac gatgatggac atgtatatct 960
cctttcacga attctcagcc gccttcttga acttggcggc ctcttccgaa ccgccggtgg 1020
cattgccctt cgagtaggaa tgcgcgccgg tgccggcaag agcgccgccc tgcacgatga 1080
ggtattcgtc gcggatcgga cggccctcga agaagcactc caggatctcg cgggtgcccg 1140
ccgcataacg cgcctgcgcg gtcagcgtgg tgccggagat gtgcggggtc atgccgttat 1200
agggcatcgt ccgccagggg tggtccttcg gcgccggctg cgggaaccac acgtcgccgg 1260
catagccggc cagccggccg gattcgagcg cacgtgccac ggcatcgcgg tcgcacagct 1320
tgccgcgggc ggtgttgacg atgtaggcgc cacgcttgaa cagcttcagc gtctcgtcat 1380
tgatcatgtg ctcggtttcg ggtgcagcg ggcagttcag cgtcaccacg tcgcaaaccg 1440
gatacatgtc ctcgcgggtc gcgtgccagg tgaggttgag ctccttctcg accgattccg 1500
gcaggcggtg acggtcggtg tagtgcaggt gcacgtcgaa cggcgccaga cggcgcagca 1560

ccgcgagacc gatgcggccg gcggccacgg tgccgacatg catcgcctcg aggtcgtagg 1620
cgtgggagac gcagtcggcg atgttccagc cgcccttccg cgcccattcg tgcgagggca 1680
gatagttgcg caccagcgac aggatcatca tcaccacatg ctcggcgacg ctgatcgagt 1740
tgcagtaggt gacttccgcc acggtgacgt tgcggtcgat agccgactga agatcgacgt 1800
ggtcggaacc gatgccggcg gtgagcgcga gcttcaggtt cttggccttg gcgatgcgct 1860
cgggcgtcag ataggccggc cagaagggct gggagatgac gacatccgca tcgaccagct 1920
cgcgctcgaa caccgagtcg gggccgtcct tgtcggaggt cacgaccagg gtgtggccgt 1980
tggattcgag atattcgcgc aggccgagct cgccggagac ggagccgagc aactgcccgg 2040
gcgtgaagtc gatggccttc ggcgtcggca agatctggcc gcccggatag tggtcgatct 2100
tcggaagatc gtcgcgggca taggtcttcg ggtagccgtc gaccggatca tcgtaaagaa 2160
cgcacaggac ctttgccatc atatgtatat ctccttctta aagttaaaca aaattatttc 2220
tagaggggaa ttgttatccg ctcacaattc ccctatagtg agtcgtatta atttcgcggg 2280
atcgagatct cgatcctcta cgccggacgc atcgtggccg gcatcaccgg cgccacaggt 2340
gcggttgctg gcgcctatat cgccgacatc accgatgggg aagatcgggc tcgccacttc 2400
gggctcatga gcgcttgttt cggcgtgggt atggtggcag gccccgtggc cgggggactg 2460
ttgggcgcca tctccttgca tgcaccattc cttgcggcgg cggtgctcaa cggcctcaac 2520
ctactactgg gctgcttcct aatgcaggag tcgcataagg gagagcgtcg agatcccgga 2580
caccatcgaa tggcgcaaaa cctttcgcgg tatggcatga tagcgcccgg aagagagtca 2640
attcagggtg gtgaatgtga aaccagtaac gttatacgat gtcgcagagt atgccggtgt 2700
ctcttatcag accgtttccc gcgtggtgaa ccaggccagc cacgtttctg cgaaaacgcg 2760
ggaaaaagtg gaagcggcga tggcggagct gaattacatt cccaaccgcg tggcacaaca 2820
actggcgggc aaacagtcgt tgctgattgg cgttgccacc tccagtctgg ccctgcacgc 2880
gccgtcgcaa attgtcgcgg cgattaaatc tcgcgccgat caactgggtg ccagcgtggt 2940
ggtgtcgatg gtagaacgaa gcggcgtcga agcctgtaaa gcggcggtgc acaatcttct 3000
cgcgcaacgc gtcagtgggc tgatcattaa ctatccgctg gatgaccagg atgccattgc 3060
tgtggaagct gcctgcacta atgttccggc gttatttctt gatgtctctg accagacacc 3120
catcaacagt attattttct cccatgaaga cggtacgcga ctgggcgtgg agcatctggt 3180
cgcattgggt caccagcaaa tcgcgctgtt agcgggccca ttaagttctg tctcggcgcg 3240
tctgcgtctg gctggctggc ataaatatct cactcgcaat caaattcagc cgatagcgga 3300
acgggaaggc gactggagtg ccatgtccgg ttttcaacaa accatgcaaa tgctgaatga 3360
gggcatcgtt cccactgcga tgctggttgc caacgatcag atggcgctgg gcgcaatgcg 3420
cgccattacc gagtccgggc tgcgcgttgg tgcggatatc tcggtagtgg gatacgacga 3480

taccgaagac agctcatgtt atatcccgcc gttaaccacc atcaaacagg attttcgcct 3540
gctggggcaa accagcgtgg accgcttgct gcaactctct cagggccagg cggtgaaggg 3600
caatcagctg ttgcccgtct cactggtgaa aagaaaaacc accctggcgc ccaatacgca 3660
aaccgcctct ccccgcgcgt tggccgattc attaatgcag ctggcacgac aggtttcccg 3720
actggaaagc gggcagtgag cgcaacgcaa ttaatgtaag ttagctcact cattaggcac 3780
cgggatctcg accgatgccc ttgagagcct tcaacccagt cagctccttc cggtgggcgc 3840
ggggcatgac tatcgtcgcc gcacttatga ctgtcttctt tatcatgcaa ctcgtaggac 3900
aggtgccggc agcgctctgg gtcattttcg gcgaggaccg ctttcgctgg agcgcgacga 3960
tgatcggcct gtcgcttgcg gtattcggaa tcttgcacgc cctcgctcaa gccttcgtca 4020
ctggtcccgc caccaaacgt ttcggcgaga agcaggccat tatcgccggc atggcggccc 4080
cacgggtgcg catgatcgtg ctcctgtcgt tgaggacccg gctaggctgg cggggttgcc 4140
ttactggtta gcagaatgaa tcaccgatac gcgagcgaac gtgaagcgac tgctgctgca 4200
aaacgtctgc gacctgagca acaacatgaa tggtcttcgg tttccgtgtt tcgtaaagtc 4260
tggaaacgcg gaagtcagcg ccctgcacca ttatgttccg gatctgcatc gcaggatgct 4320
gctggctacc ctgtggaaca cctacatctg tattaacgaa gcgctggcat tgaccctgag 4380
tgatttttct ctggtcccgc cgcatccata ccgccagttg tttaccctca caacgttcca 4440
gtaaccgggc atgttcatca tcagtaaccc gtatcgtgag catcctctct cgtttcatcg 4500
gtatcattac ccccatgaac agaaatcccc cttacacgga ggcatcagtg accaaacagg 4560
aaaaaaccgc ccttaacatg gcccgcttta tcagaagcca gacattaacg cttctggaga 4620
aactcaacga gctggacgcg gatgaacagg cagacatctg tgaatcgctt cacgaccacg 4680
ctgatgagct ttaccgcagc tgcctcgcgc gtttcggtga tgacggtgaa aacctctgac 4740
acatgcagct cccggagacg gtcacagctt gtctgtaagc ggatgccggg agcagacaag 4800
cccgtcaggg cgcgtcagcg ggtgttggcg ggtgtcgggg cgcagccatg acccagtcac 4860
gtagcgatag cggagtgtat actggcttaa ctatgcggca tcagagcaga ttgtactgag 4920
agtgcaccat atatgcggtg tgaaataccg cacagatgcg taaggagaaa ataccgcatc 4980
aggcgctctt ccgcttcctc gctcactgac tcgctgcgct cggtcgttcg gctgcggcga 5040
gcggtatcag ctcactcaaa ggcggtaata cggttatcca cagaatcagg ggataacgca 5100
ggaaagaaca tgtgagcaaa aggccagcaa aaggccagga accgtaaaaa ggccgcgttg 5160
ctggcgtttt tccataggct ccgcccccct gacgagcatc acaaaaatcg acgctcaagt 5220
cagaggtggc gaaacccgac aggactataa agataccagg cgtttccccc tggaagctcc 5280
ctcgtgcgct ctcctgttcc gaccctgccg cttaccggat acctgtccgc ctttctccct 5340

tcgggaagcg tggcgctttc tcatagctca cgctgtaggt atctcagttc ggtgtaggtc 5400

gttcgctcca agctgggctg tgtgcacgaa ccccccgttc agcccgaccg ctgcgcctta 5460

tccggtaact atcgtcttga gtccaacccg gtaagacacg acttatcgcc actggcagca 5520

gccactggta acaggattag cagagcgagg tatgtaggcg gtgctacaga gttcttgaag 5580

tggtggccta actacggcta cactagaagg acagtatttg gtatctgcgc tctgctgaag 5640

ccagttacct tcggaaaaag agttggtagc tcttgatccg gcaaacaaac caccgctggt 5700

agcggtggtt tttttgtttg caagcagcag attacgcgca gaaaaaaagg atctcaagaa 5760

gatcctttga tcttttctac ggggtctgac gctcagtgga acgaaaactc acgttaaggg 5820

attttggtca tgagattatc aaaaaggatc ttcacctaga tccttttaaa ttaaaaatga 5880

agttttaaat caatctaaag tatatatgag taaacttggt ctgacagtta ccaatgctta 5940

atcagtgagg cacctatctc agcgatctgt ctatttcgtt catccatagt tgcctgactc 6000

cccgtcgtgt agataactac gatacgggag ggcttaccat ctggccccag tgctgcaatg 6060

ataccgcgag acccacgctc accggctcca gatttatcag caataaacca gccagccgga 6120

agggccgagc gcagaagtgg tcctgcaact ttatccgcct ccatccagtc tattaattgt 6180

tgccgggaag ctagagtaag tagttcgcca gttaatagtt tgcgcaacgt tgttgccatt 6240

gctgcaggca tcgtggtgtc acgctcgtcg tttggtatgg cttcattcag ctccggttcc 6300

caacgatcaa ggcgagttac atgatccccc atgttgtgca aaaaagcggt tagctccttc 6360

ggtcctccga tcgttgtcag aagtaagttg gccgcagtgt tatcactcat ggttatggca 6420

gcactgcata attctcttac tgtcatgcca tccgtaagat gcttttctgt gactggtgag 6480

tactcaacca agtcattctg agaatagtgt atgcggcgac cgagttgctc ttgcccggcg 6540

tcaatacggg ataataccgc gccacatagc agaactttaa aagtgctcat cattggaaaa 6600

cgttcttcgg ggcgaaaact ctcaaggatc ttaccgctgt tgagatccag ttcgatgtaa 6660

cccactcgtg cacccaactg atcttcagca tcttttactt tcaccagcgt ttctgggtga 6720

gcaaaaacag gaaggcaaaa tgccgcaaaa aagggaataa gggcgacacg gaaatgttga 6780

atactcatac tcttcctttt tcaatattat tgaagcattt atcagggtta ttgtctcatg 6840

agcggataca tatttgaatg tatttagaaa aataaacaaa taggggttcc gcgcacattt 6900

ccccgaaaag tgccacctga aattgtaaac gttaatattt tgttaaaatt cgcgttaaat 6960

ttttgttaaa tcagctcatt ttttaaccaa taggccgaaa tcggcaaaat cccttataaa 7020

tcaaaagaat agaccgagat agggttgagt gttgttccag tttggaacaa gagtccacta 7080

ttaaagaacg tggactccaa cgtcaaaggg cgaaaaaccg tctatcaggg cgatggccca 7140

ctacgtgaac catcacccta atcaagtttt ttggggtcga ggtgccgtaa agcactaaat 7200

cggaacccta aagggagccc ccgatttaga gcttgacggg gaaagccggc gaacgtggcg 7260

```
agaaaggaag ggaagaaagc gaaaggagcg ggcgctaggg cgctggcaag tgtagcggtc      7320
acgctgcgcg taaccaccac acccgccgcg cttaatgcgc cgctacaggg cgcgtcccat      7380
tcgcca                                                                 7386
```

## Beschreibung

Die Erfindung betrifft neuartige 7ß-Hydroxysteroid Dehydrogenase-Mutanten, die für diese Enzym-Mutanten kodierenden Sequenzen, Verfahren zur Herstellung der Enzym-Mutanten und deren Verwendung bei enzymatischen Umsetzungen von Cholsäureverbindungen, und insbesondere bei der Herstellung von Ursodesoxycholsäure (UDCS); Gegenstand der Erfindung sind auch neuartige Verfahren zur Synthese von UDCS unter Verwendung der Enzym-Mutanten; sowie die Herstellung von UDCS unter Verwendung rekombinanter, mehrfach modifizierter Mikroorganismen.

## Hintergrund der Erfindung:

Zur medikamentösen Behandlung von Gallensteinleiden werden seit vielen Jahren u. a. die Wirkstoffe Ursodesoxycholsäure (UDCS oder UDCA) und das zugehörige Diastereomer Chenodesoxycholsäure (CDCS oder CDCA) eingesetzt. Beide Verbindungen unterscheiden sich lediglich durch die Konfiguration der Hydroxygruppe am C-Atom 7 (UDCS: β-Konfiguration, CDCS: α-Konfiguration). Zur Herstellung von UDCS sind im Stand der Technik verschiedene Verfahren beschrieben, welche rein chemisch durchgeführt werden oder aus einer Kombination chemischer und enzymatischer Verfahrensschritte bestehen. Ausgangspunkt ist jeweils Cholsäure (CS oder CA) oder die aus Cholsäure hergestellte CDCS.

So kann die klassisch chemische Methode zur UDCS Herstellung wie folgt schematisch dargestellt werden:

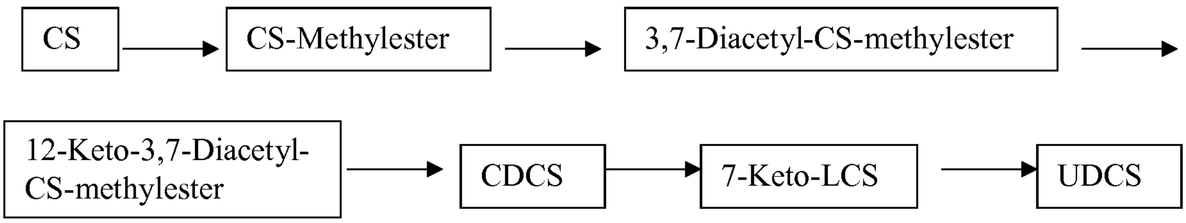


Ein gravierender Nachteil ist unter anderem folgender: da die chemische Oxidation nicht selektiv ist, müssen die Carboxy-Gruppe und die 3α- und 7α-Hydroxy-Gruppe durch Veresterung geschützt werden.

Ein alternatives chemisch/enzymatisches Verfahren basierend auf der Verwendung des Enzyms 12α-Hydroxysteroid Dehydrogenase (12α-HSDH) kann wie folgt dargestellt werden und ist z.B. beschrieben in der PCT/EP2009/002190 der vorliegenden Anmelderin.

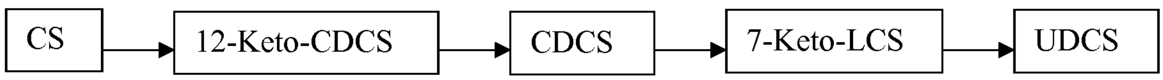


Die 12α-HSDH oxidiert dabei CS selektiv zu 12-Keto-CDCS. Die zwei nach der klassisch chemischen Methode erforderlichen Schützschritte entfallen dabei.

Weiterhin wird von Monti, D., et al., (One-Pot Multienzymatic Synthesis of 12-Ketoursodeoxycholic Acid: Subtle Cofactor Specificities Rule the Reaction Equilibria of Five Biocatalysts Working in a Row. Advanced Synthesis & Catalysis, 2009) ein alternatives enzymatisch-chemisches Verfahren beschrieben, das wie folgt schematisch darstellbar ist:

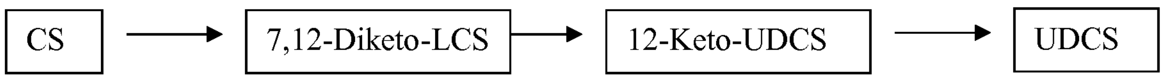


Die CS wird zuerst von 7α-HSDH aus *Bacteroides fragilis* ATCC 25285 (Zhu, D., et al., Enzymatic enantioselective reduction of -ketoesters by a thermostable 7 -hydroxysteroid dehydrogenase from Bacteroides fragilis. Tetrahedron, 2006. 62(18): p. 4535-4539) und 12α-HSDH zu 7,12-Diketo-LCS oxidiert. Diese beiden Enzyme sind jeweils NADH abhängig. Nach der Reduktion durch 7β-HSDH (NADPH abhängig) aus *Clostridium absonum* ATCC 27555 (DSM 599) (MacDonald, I.A. and P.D. Roach, Bile induction of 7 alpha- and 7 beta-hydroxysteroid dehydrogenases in Clostridium absonum. Biochim Biophys Acta, 1981. 665(2): p. 262-9) entsteht 12-Keto-UDCS. Durch Wolff-Kishner-Reduktion wird das Endprodukt erhalten. Nachteilig bei diesem Verfahren ist, dass wegen der Gleichgewichtslage der katalysieren Reaktion eine komplette Umsetzung nicht möglich ist, und dass für die erste Stufe der Umsetzung zwei unterschiedliche Enzyme eingesetzt werden müssen, was das Verfahren verteuert. Zur Kofaktor-Regenerierung werden Lactat Dehydrogenase (LDH; zur Regenerierung von $NAD^+$) und Glucose Dehydrogenase (GlcDH oder GDH, zur Regenerierung

von NADPH) eingesetzt. Nachteilig bei der dort verwendeten Kofaktor-Regenerierung ist, dass das entstehende Ko-Produkt nur sehr schwer aus dem Reaktionsgemisch zu entfernen ist, so dass das Reaktionsgleichgewicht nicht positiv beeinflusst werden kann, was eine unvollständige Umsetzung des Edukts bedingt.

Eine 7β-HSDH aus Stamm *Collinsella aerofaciens* ATCC 25986 (DSM 3979; ehemalige *Eubacterium aerofaciens*) wurde im Jahr 1982 von Hirano und Masuda beschrieben (Hirano, S. and N. Masuda, Characterization of NADP-dependent 7 beta-hydroxysteroid dehydrogenases from Peptostreptococcus productus and Eubacterium aerofaciens. Appl Environ Microbiol, 1982. 43(5): p. 1057-63). Sequenzinformationen wurden zu diesem Enzym nicht offenbart. Das durch Gelfiltration bestimmte Molekulargewicht betrug 45,000 Da (vgl. Hirano, Seite 1059, linke Spalte). Weiterhin konnte für das dortige Enzym die Reduktion der 7-Oxo-Gruppe zur 7ß-Hydroxygruppe nicht beobachtet werden (vgl. Hirano, Seite 1061, Diskussion 1. Absatz). Der Fachmann erkennt somit, dass das von Hirano et al. beschriebene Enzym nicht zur Katalyse der Reduktion von Dehydrocholsäure (DHCS oder DHCA) in 7-Position zu 3,12-Diketo-7ß-CS geeignet ist.

In der älteren internationalen Patentanmeldung PCT/EP2010/068576 der Anmelderin wird eine neuartige 7ß-HSDH aus *Collinsella aerofaciens* ATCC 25986 beschrieben, welche unter anderem ein Molekulargewicht (bei SDS-Gelelektrophorese) von etwa 28-32 kDa, ein Molekulargewicht (bei Gelfiltration, unter nicht denaturierenden Bedingungen, wie insbesondere ohne SDS) von etwa 53 bis 60 kDa, und die Befähigung zur stereoselektiven Reduktion der 7-Carbonyl-Gruppe von 7-Keto-LCS in eine 7β-Hydroxy-Gruppe aufweist.

Außerdem wird in der PCT/EP2010/068576 ein Verfahren zur UDCS Herstellung bereitgestellt, welches schematisch wie folgt darstellbar ist:

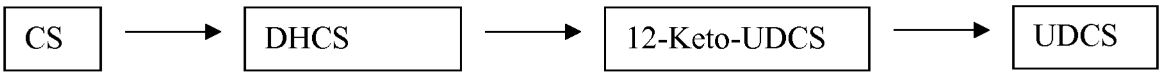


Dabei erfolgt die Oxidation von CS auf klassisch chemischem Weg in einfacher Weise. Die DHCS wird vom Enzympaar 7β-HSDH und 3α-HSDH einzeln nacheinander oder in einem Topf zu 12-Keto-UDCS reduziert. Kombiniert mit der Wolff-Kishner-Reduktion kann UDCS somit in nur drei Schritten aus CS synthetisiert werden. Während die 7β-HSDH vom Kofaktor NADPH abhängig ist, benötigt die 3α-HSDH den Kofaktor NADH. Die Verfügbarkeit von Enzympaaren mit Abhängigkeit vom gleichen Kofaktor oder erweiterter Anhängigkeit, (z.B. von den Kofaktoren NADH und NADPH) wäre von Vorteil, weil damit die Kofaktor-Regenerierung vereinfacht werden könnte.

Aufgabe der Erfindung ist die Bereitstellung weiter verbesserter 7ß-HSDHs. Insbesondere sollten Enzym-Mutanten bereitgestellt werden, welche noch vorteilhafter zur enzymatischen oder mikrobiellen Herstellung von UDCS über die stereospezifische Reduktion von DHCS in 7-Position zu 3,12-Diketo-7ß-CS eingesetzt werden können, und insbesondere eine geringere Substratinhibition aufweisen und/oder veränderte Kofaktor-Nutzung (erhöhte, veränderte Spezifität oder erweiterte Abhängigkeit) aufweisen.

Eine weitere Aufgabe besteht in der Bereitstellung neuartiger enzymatischer und mikrobieller Synthesewege, welche sich insbesondere auch durch eine vereinfachte Kofaktor-Regenerierung bei der reduktiven Herstellung von UDCS über DHCS auszeichnen.

## Kurzfassung der Erfindung:

Obige Aufgaben konnten überraschenderweise gelöst werden durch die Erzeugung und Charakterisierung von Mutanten einer neuartigen regio- und stereospezifischen 7ß-HSDH aus aeroben Bakterien der Gattung *Collinsella,* insbesondere des Stammes *Collinsella aerofaciens* und deren Einsatz bei der Umsetzung von Cholsäureverbindungen, insbesondere bei der Herstellung von UDCS.

Weiterhin wurde obige Aufgabe gelöst durch Bereitstellung eines biokatalytischen (mikrobiellen bzw. enzymatischen) Prozesses, umfassend die enzymatische Umsetzung von DHCS über zwei durch 7β-HSDH bzw. 3α-HSDH katalysierte reduktive Teilschritte, die gleichzeitig oder zeitlich versetzt in beliebiger Reihenfolge ablaufen können, zu 12-Keto-UDCS und Kofaktorregenerierung durch Verwendung von Dehydrogenasen, wie insbesondere Formiatdehydrogenase (FDH)-Enzymen oder Glucosedehydrogenase (GDH)-Enzymen, welche den verbrauchten Kofaktor aus beiden reduktiven Teilschritten regenerieren.

## Figurenbeschreibung:

Figur 1a zeigt die Aminosäuresequenz der 7ß-HSDH aus *Collinsella aerofaciens* und Figur 1b die kodierende Nukleinsäuresequenz zur Aminosäuresequenz von Figur 1a; Figur 1c zeigt die Aminosäuresequenz der 3α-HSDH aus *Comanomonas testosteroni* und Figur 1d die kodierende Nukleinsäuresequenz zur Aminosäuresequenz von

Figur 1c; Figur 1e zeigt die Aminosäuresequenz der 3α-HSDH aus *Rattus norvegicus* und Figur 1f die kodierende Nukleinsäuresequenz zur Aminosäuresequenz von Figur 1e; Figur 1g zeigt die kodierende Nukleinsäuresequenz der FDH-Mutante D221G und Figur 1h die Aminosäuresequenz zur Nukleinsäuresequenz von Figur 1g.

Figur 2 zeigt das SDS-Gel einer erfindungsgemäßen hergestellten, gereinigten 7ß-HSDH und zwar auf Bahn 1: Zellrohextrakt; Bahn 2: gereinigtes Protein; Bahn M: Page Rouler™, Molekulargewichtsmarker (Fermentas, Deutschland).

Figur 3 zeigt die Konstruktionsschema von (A) pET21a(+), (B) pET22b(+), (C) pCOLA (Mod) und (D) pET28a(+).

Figur 4 zeigt die Konstruktionsschemata von pET21a(+) FDH D221G (Fig. 4a) und pET21a(+) FDH 7β-HSDH (Fig. 4b).

Figur 5 zeigt das Konstruktionsschema von pCOLA(mod) 3α-HSDH.

Figur 6 zeigt einen Aktivitätsvergleich für /ß-HSDH Wildtyp und die 7ß-HSDH Mutanten G39A und G39S, und zwar in Reihe A: die Auftragung der spezifischen Enzymaktivität gegen unterschiedliche eingesetzte Substratkonzentrationen bei konstanter Kofaktorkonzentration von 100 μM; und in Reihe B die Auftragung der Enzymaktivität gegen unterschiedliche eingesetzt Kofaktorkonzentrationen bei konstanter Substratkonzentration von 0,3 mM.

Figur 7 zeigt das HPLC-Chromatogramm der Biotransformation von DHCS mit 7β-HSDH und 3α-HSDH im Ganzzellprozess. Gezeigt ist das HPLC-Chromatogramm der Ganzzellumsetzung des Stammes *E. coli* BL21 (DE3) hdhA$^-$ KanR$^+$ pET21a(+) FDH 7β-HSDH pCOLA(mod) 3α-HSDH. Als Startbedingungen wurden 50 mM Substrat, 400 mM Cosubstrat, pH 6,0 gewählt. Die Probenahme erfolgte nach 48 h. Zu sehen sind die Peaks von 12-Keto-UDCS (1), eines unbekannten Nebenprodukts (2), 3,12-Diketo-UDCS(3), 7,12-Diketo-UDCS (4) und DHCS (5).

Figur 8 zeigt das Konstruktionsschema des Triple-Vektors pET21a(+) FDH 7beta (G39A) der die kodierenden Sequenzen für FDH D221G, 7β-HSDH G39A und 3α-HSDH trägt.

Figur 9 zeigt den Verlauf der Ganzzell-Biotransformation von DHCS. Es sind die Peakflächenanteile (nach HPLC Analyse) von 12-keto-UDCS, 3,12-diketo-UDCS, 7,12-diketo-UDCS und DHCS im zeitlichen Verlauf angegeben.

Figur 10 zeigt einen Sequenzvergleich zwischen dem 7β-HSDH-Wildtyp und ausgewählten Mutanten. Die als "7beta-HSDH Wildtyp", "7beta-HSDH G39D", "7beta-HSDH G39D R40L", "7beta-HSDH G39D R40I" und "7beta-HSDH G39D R40V" bezeichneten Sequenzen entsprechen den SEQ ID NO:2, SEQ ID NO:37, SEQ ID NO:38, SEQ ID NO:39 und SEQ ID NO:40.

Figur 11 zeigt eine enzymkinetische Untersuchung der 7β-HSDH und deren Mutanten. Aufgetragen ist die spezifische Enzymaktivität gegen unterschiedliche eingesetzte Substratkonzentrationen (DHCA-Konzentration) bei einer konstanten Kofaktorkonzentration von 0,1 mM NADPH bzw. 0,5 mM NADH.

Figur 12 zeigt eine schematische Darstellung einer erfindungsgemäßen zweistufigen enzymatischen Reduktion von Dehydrocholsäure zu 12-keto-Ursodesoxycholsäure, wobei eine NADH-abhängige 7ß-HSDH eingesetzt wird und eine Formiatdehydrogenase (FDH) zur Kofaktorregenerierung verwendet wird.

Figur 13 zeigt eine schematische Darstellung des Konstruktionsprinzips der Vektoren pFr7(D), pFr7(DI), pFr7(DL) und pFr7(DV), die zur Expression verschiedener NADH-abhängigen Mutanten von 7ß-HSDH eingesetzt wurden.

Figur 14 zeigt Gallensalzanteile von Biotransformationsansätzen unter Verwendung von NADH-abhängigen 7β-HSDH-Mutanten. Gezeigt sind Ergebnisse nach 24 h Prozesszeit und Einsatz von 100 mM Substrat (DHCA).

Figur 15 zeigt eine schematische Darstellung des Vektors pFr3T7(D).

Figur 16 zeigt das Ergebnis einer Ganzzellbiotransformation mit dem Stamm *E. coli* BL49 pFr3T7(D), welcher die NADH-abhängige 7β-HSDH (G39D), eine NADH-abhängige 3α-HSDH und eine NADH-abhängige FDH beinhaltet. Die Biotransformationen wurden bei folgenden Bedingungen durchgeführt: 20 mL Reaktionsvolumen, 17,7 g/IBTM Zellen, 100 mM DHCA, 500 mM Ammoniumformiat, 26 % Glycerin, 50 mM MgCl2, 50 mM KPi-Puffer (pH 6,5). Der pH wurde während der ersten 5 Stunden stündlich manuell mit Ameisensäure auf den Ausgangswert eingestellt.

Figur 17 zeigt schematische Darstellungen verschiedener verwendeter Vektoren: a) pF(G)r7(A)r3 (wobei dieser dem in Figur 8 gezeigten Vektor entspricht) und b) pF(G)r7(S)r3.

Figur 18 zeigt eine schematische Darstellung der zweistufigen enzymatischen Reduktion von Dehydrocholsäure zu 12-keto-Ursodesoxycholsäure, wobei eine NADPH-abhängige 7ß-HSDH eingesetzt wird und eine Formiatdehydrogenase (FDH)-Mutante, die sowohl NADPH- als auch NADH regeneriert, zur Kofaktorregenerierung verwendet wird.

Figur 19 zeigt einen zeitlich aufgelösten Verlauf der Biotransformation mit dem Stamm *E. coli* BL49 pF(G)r7(A)r3 im Litermaßstab. Der Ansatz enthielt 70 mM DHCA, 17,7 g/l BTM des gelagerten Biokatalysators, 500 mM Natriumformiat, 26 % (v/v) Glycerin, 50 mM $MgCl_2$, in 50 mM KPi-Puffer (pH 6,5). Durch pH-Regelung wurde der pH während der gesamten Biotransformation auf pH 6,5 gehalten.

Figur 20 zeigt eine schematische Darstellung des Vektors p3T7(A)rG.

Figur 21 zeigt eine schematische Darstellung des Vektors p7(A)T3rG.

Figur 22 zeigt einen zeitlich aufgelösten Verlauf der Biotransformation mit dem Stamm *E. coli* BL49 p7(A)T3rG, welcher eine GDH zur Kofaktorregenerierung beinhaltet. Der Ansatz enthielt 100 mM DHCA, 17,7 g/l BTM des gelagerten Biokatalysators, 500 mM Glucose, 10 mM $MgCl_2$, in 50 mM KPi-Puffer (pH 7) ohne (obere Teilfigur) und

mit 0,1 mM NAD (untere Teilfigur). Der pH wurde manuell mit Kalilauge auf den Ausgangswert eingestellt.
Figur 23 zeigt schematische Darstellungen verschiedener Vektoren: a) Vektor pF(G)r7(A) und b) Vektor pFr3.
Figur 24 zeigt eine schematische Darstellung der zweistufigen enzymatischen Reduktion von Dehydrocholsäure zu 12-keto-Ursodesoxycholsäure unter Verwendung von zwei unterschiedlichen Ganzzellbiokatalysatoren. Die Reaktionen A und D werden hierbei durch einen 7β-HSDH enthaltenden Ganzzellbiokatalysator katalysiert, die Reaktionen B und C werden von einen 3α-HSDH enthaltenden Ganzzellbiokatalysator katalysiert. Im Verlauf der zweistufigen Umsetzung muss das Intermediat 3,12-diketo-Ursodesoxycholsäure aus dem 7β-HSDH enthaltenden Ganzzellbiokatalysator heraus und in den 3α-HSDH enthaltenden Ganzzellbiokatalysator hinein gelangen, das Intermediat 7,12-diketo-Ursodesoxycholsäure muss aus dem 3α-HSDH enthaltenden Ganzzellbiokatalysatoren heraus und in den 7β-HSDH enthaltenden Ganzzellbiokatalysator hinein gelangen.
Figur 25 zeigt die Gallensalzanteile von Biotransformationsansätzen nach 24 h bei Einsatz von 70 mM Substrat (DHCA). Gezeigt sind die Ansätze bei Einsatz unterschiedlicher Anteile an den beiden Biokatalysatorstämmen *E. coli* BL49 pF(G)r7(A) und *E. coli* BL49 pFr3.
Figur 26 zeigt einen zeitlich aufgelösten Verlauf der Biotransformation mit dem Biokatalysatoren *E. coli* BL49 pF(G)r7(A) und *E. coli* BL49 pFr3 Litermaßstab. Der Ansatz enthielt 90 mM DHCA, 8,85 g/l $_{BTM}$ *E. coli* BL49 pF(G)r7(A) und 8,85 g/l $_{BTM}$ *E. coli* BL49 pFr3, 500 mM Ammoniumformiat, 26 % (v/v) Glycerin, 50 mM MgCl2, in 50 mM KPi-Puffer (pH 6,5). Durch pH-Regelung wurde der pH während der gesamten Biotransformation mit Ameisensäure auf pH 6,5 gehalten.

## Spezielle Ausführungsformen der Erfindung

Die Erfindung betrifft insbesondere folgende spezielle Ausführungsformen:

1. Eine 7ß-Hydroxysteroiddehydrogenase (7ß-HSDH), welche wenigstens die stereospezifische enzymatische Reduktion eines 7-Ketosteroids zum korrespondierenden 7-Hydroxysteroid katalysiert, wobei das Enzym wenigstens eine Mutation im Sequenzmotiv VMVGRRE gemäß Position 36 bis 42 von SEQ ID NO:2 und wenigstens 80 % Sequenzidentität aufweist, wie z.B. wenigstens 85 oder 90, wie z.B. wenigstens 91, 92, 93, 94, 95, 96, 97, 98, 99 oder 99,5 % zu dieser Sequenz.
Weiterhin können die erfindungsgemäßen Mutanten eine verringerte Substratinhibition (insbesondere für das 7-Ketosteroid-Substrat) und/oder eine veränderte Kofaktor-Nutzung (z.B. erhöhte, veränderte Spezifität bezüglich eines Kofaktors (insbesondere NADH bzw. NADPH) oder eine erweiterte Abhängigkeit, das heißt Nutzung eines zusätzlichen, bisher nicht genutzten Kofaktors) aufweisen.

2. Erfindungsgemäße Mutanten können keine Substratinhibition zeigen oder einen $K_i$-Wert für das 7-Ketosteroid-Substrat, insbesondere für Dehydrocholsäure (DHCS), im Bereich von >10 mM, wie z.B. bei 11 bis 200 mM, 12 bis 150 mM, 15 bis 100 mM, aufweisen.

3. Bei erfindungsgemäßen Mutanten kann die spezifische Aktivität (U/mg) in Gegenwart des Kofaktors NADPH im Vergleich zum nichtmutierten Enzym um wenigsten 1, 5 oder 10 %, insbesondere aber wenigstens um das 1-fache, insbesondere um das 2- bis 10-fache erhöht oder erniedrigt sein; oder im Wesentlichen fehlen und durch die Nutzung von NADH ersetzt sein, oder eine erweiterte Nutzung von NADPH und HADH aufweisen.

4. Eine 7ß-HSDH, welche wenigstens die stereospezifische enzymatische Reduktion eines 7-Ketosteroids zum korrespondierenden 7-Hydroxysteroid katalysiert, wobei das Enzym wenigstens eine Mutation im Sequenzmotiv VMVGRRE gemäß Position 36 bis 42 von SEQ ID NO:2 und wenigstens 60 % Sequenzidentität, wie z.B. wenigstens 65, 70, 75, 80, 85 oder 90, wie z.B. wenigstens 91, 92, 93, 94, 95, 96, 97, 98, 99 oder 99,5% zu SEQ ID NO:2 aufweist, ausgewählt unter

a) den Einfachmutanten G39$X_1$ und R40$X_2$,
b) den Zweifach-Mutanten G39$X_1$ R40$X_2$, R40$X_2$ R41$X_3$ und G39$X_1$ R41$X_3$ oder
c) der Dreifach-Mutante G39$X_1$ R40$X_2$ R41$X_3$,

(jeweils bezogen auf SEQ ID NO:2)
wobei $X_1$, $X_2$ und $X_3$ jeweils unabhängig voneinander für eine beliebige, insbesondere eine beliebige, die Substratinhibition verringernde und/oder die Kofaktornutzung oder Kofaktor-Abhängigkeit modifizierende, von G bzw. R verschiedene, insbesondere natürliche Aminosäure stehen;
Beispiele geeigneter Einfachmutanten umfassen: G39A, G39S, G39D, G39V, G39T, G39P, G39N, G39E, G39Q, G39H, G39R, G39K und G39W, und R40D, R40E, R40I, R40V, R40L, R40G, R40A.

Beispiele geeigneter Doppelmutanten umfassen:

Zweierkombinationen obiger $G39X_1$ und $R40X_2$-Mutanten, wobei $X_1$ insbesondere für D oder E steht und/oder $X_2$ eine beliebige Aminosäure, insbesondere proteinogene Aminosäure, wie insbesondere eine Aminosäure mit aliphatischer Seitenkette sein kann, wie z.B. G39D R40I, G39D R40L, G39D R40V; sowie die analogen Doppelmutanten mit G39E anstelle von G39D; sowie
Zweierkombinationen obiger $G39X_1$-Mutanten oder $R40X_2$-Mutanten mit $R41X_3$-Mutanten, worin $X_3$ eine beliebige, insbesondere eine die Substratinhibition verringernde und/oder die Kofaktornutzung oder Kofaktorabhängigkeit modifizierende, von R verschiedene insbesondere natürliche Aminosäure ist, wie z.B. des Typs $G39X_1$ $R41X_3$) oder $R40X_2$ $R41X_3$,
z.B. mit $X_1$=D oder E; $X_2$=I, L oder V; $X_3$= N, I, L oder V,
wie z.B. R40D R41I; R40D R41L; R40D R41V; R40I R41I; R40V R41I, R40L R41I.

Beispiele geeigneter Dreifachmutanten umfassen beliebige Dreier-Kombinationen obiger Einfachmutanten $G39X_1$, $R40X_2$ und $R41X_3$; wobei $X_1$, $X_2$ und $X_3$ wie oben definiert sind; insbesondere aber wobei $X_1$ für D, oder E steht und/oder $X_2$ und $X_3$ unabhängig voneinander für eine beliebige Aminosäure, insbesondere eine proteinogene Aminosäure stehen, wie insbesondere Dreifachmutanten des Typs ($G39X_1$=D oder E; $R40X_2$=I, L oder V; $R41X_3$= N, I, L oder V), wie z.B. G39D R40I R41N.
Gegebenenfalls können die Mutanten der Ausführungsformen 1 bis 4 zusätzlich oder alternativ, insbesondere zusätzlich, wenigstens eine weitere Substitution, wie z.B. 1, 2, 3 oder 4 Substitutionen in den Positionen K44, R53, K61 und R64 aufweisen. Hierbei können diese Reste unabhängig voneinander durch eine beliebige Aminosäure, insbesondere eine proteinogene Aminosäure ersetzt sein, insbesondere eine Substitution derart, dass die so erzeugte Mutante eine verringerte Substratinhibition (insbesondere für das 7-Ketosteroid-Substrat) aufweist; und/oder eine veränderte Kofaktornutzung oder Kofaktorabhängigkeit (z.B. erhöhte, veränderte Spezifität bezüglich eines Kofaktors oder eine erweiterte Abhängigkeit, das heißt Nutzung eines zusätzlichen, bisher nicht genutzten Kofaktors) wie hierin definiert aufweist.

5. Nukleinsäuremolekül, umfassend eine für eine 7ß-HSDH nach einer der vorhergehenden Ausführungsformen kodierende Nukleinsäuresequenz.

6. Expressionskassette, umfassend eine Nukleinsäuresequenz gemäß Ausführungsform 5 unter der genetischen Kontrolle wenigstens einer regulativen Nukleinsäuresequenz, sowie ggf. kodierende Sequenzen für wenigstens ein (wie z.B. 1, 2 oder 3) weiteres Enzym, ausgewählt unter Hydroxysteroiddehydrogenasen, insbesondere 3$\alpha$-HSDH, und zur Kofaktorregenerierung geeignete Dehydrogenasen, wie z.B. FDH, GDH, ADH, G-6-PDH, PDH. Insbesondere können die in einer Expressionskassette enthaltenen Enzyme verschiedene, bevorzugt aber gleiche Kofaktorenpaare nutzen, wie z.B. das Kofaktorenpaar $NAD^+$/NADH oder $NADP^+$/NADPH.

7. Vektor, umfassend wenigstens eine Expressionskassette nach Ausführungsform 6.

8. Rekombinanter Mikroorganismus, der wenigstens eine Nukleinsäuresequenz gemäß Ausführungsform 5 oder wenigstens eine diese kodierende Nukleinsäuresequenz enthaltende Expressionskassette nach Ausführungsform 6 trägt oder wenigstens einen diese kodierende Nukleinsäuresequenz enthaltenden Vektor nach Ausführungsform 7 trägt und zusätzlich gegebenenfalls die kodierende Sequenz für ein weiteres Enzym, ausgewählt unter Hydroxysteroiddehydrogenasen, wie eine 3$\alpha$-Hydroxysteroiddehydrogenase (3$\alpha$-HSDH), und zur Kofaktorregenerierung geeigneten Dehydrogenasen, trägt.

9. Verfahren zur enzymatischen oder mikrobiellen Synthese von 7ß-Hydroxysteroiden, wobei man das korrespondierende 7-Ketosteroid in Gegenwart einer 7ß-HSDH gemäß der Definition in einer der Ausführungsformen 1 bis 4 oder in Gegenwart eines dieses Enzym exprimierenden rekombinanten Mikroorganismus umsetzt, und wenigstens ein gebildetes Reduktionsprodukt aus dem Reaktionsansatz gegebenenfalls isoliert.

10. Verfahren nach Ausführungsform 9, wobei das zu reduzierende Ketosteroid ausgewählt ist unter

a) Dehydrocholsäure (DHCS),
b) 7-Keto-lithocholsäure (7-Keto-LCS),
c) 7,12-Diketo-lithocholsäure (7,12-Diketo-LCS) und
d) den Derivaten davon, wie insbesondere einem Salz, Amid oder Alkylester der Säure.

11. Verfahren nach einer der Ausführungsformen 9 und 10, wobei die Reduktion in Gegenwart (und unter Verbrauch) von NADPH und/oder NADH erfolgt.

12. Ebenfalls hierin beschrieben ist ein Verfahren zur enzymatischen oder mikrobiellen Oxidation von 7ß-Hydroxysteroiden, wobei man das Hydroxysteroid in Gegenwart einer 7ß-HSDH-Mutante gemäß der Definition in einer der Ausführungsformen 1 bis 4 oder in Gegenwart eines diese Mutante exprimierenden Mikroorganismus nach Ausführungsform 8 umsetzt, und ein gebildetes Oxidationsprodukt aus dem Reaktionsansatz gegebenenfalls isoliert.

13. Ebenfalls hierin beschrieben ist ein Verfahren nach Ausführungsform 12, wobei das 7ß-Hydroxysteroid 3,12-Diketo-7ß-CS oder ein Derivat davon, wie insbesondere ein Salz, Amid oder Alkylester, ist.

14. Ebenfalls hierin beschrieben ist ein Verfahren nach einer der Ausführungsformen 12 und 13, wobei die Oxidation in Gegenwart (und unter Verbrauch) von $NADP^+$ und/oder $NAD^+$ erfolgt.

15. Verfahren nach einer der Ausführungsformen 11 und 14, wobei die verbrauchten Redoxäquivalente chemisch, elektrochemisch oder enzymatisch, insbesondere in situ, regeneriert werden.

16. Verfahren nach Ausführungsform 15, wobei verbrauchtes NADPH durch Kopplung mit einem NADPH-regenerierenden Enzym regeneriert wird, wobei dieses insbesondere ausgewählt ist unter NADPH Dehydrogenasen, Alkoholdehydrogenasen (ADH), und NADPH-regenerierenden Formiatdehydrogenasen (FDH), sowie Glucosedehydrogenase (GDH)-, Glucose-6-Phosphat-Dehydrogenase (G-6-PDH), oder Phosphit-Dehydrogenasen (PtDH), wobei das NADPH-regenerierende Enzym gegebenenfalls von einem rekombinanten Mikroorganismus exprimiert wird; und/oder
wobei verbrauchtes NADH durch Kopplung mit einem NADH-regenerierenden Enzym regeneriert wird, wobei dieses insbesondere ausgewählt ist unter NADH Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH), wobei das NADH-regenerierende Enzym ggf. in einem rekombinanten Mikroorganismus exprimiert wird.
Die Expression des Kofaktor-regenerierenden Enzyms in einem rekombinanten Mikroorganismus ist dabei bevorzugt.

17. Verfahren nach Ausführungsform 16, wobei das NADPH-regenerierende Enzym ausgewählt ist unter natürlichen oder rekombinanten, isolierten oder angereicherten

a) Alkoholdehydrogenasen (EC 1.1.1.2) und
b) davon abgeleiteten funktionalen Äquivalenten

18. Verfahren nach Ausführungsform 16, wobei das NADPH-regenerierende Enzym ausgewählt ist unter Mutanten einer $NAD^+$-abhängigen Formiatdehydrogenase (FDH), welche insbesondere wenigstens die enzymatische Oxidation von Ameisensäure zu $CO_2$ katalysiert, wobei die Mutante im Vergleich zum nicht-mutierten Enzym ausschließlich oder zusätzlich, insbesondere zusätzlich $NADP^+$ als Kofaktor akzeptiert; oder
oder wobei das NADH-regenerierende Enzym ausgewählt ist unter einer $NAD^+$-abhängigen FDH oder einer $NAD^+$-abhängigen GDH, wie insbesondere einer FDH aus *Mycobacterium vaccae* N10, gemäß SEQ ID NO:36 und einer GDH aus *Bacillus subtilis* gemäß SEQ ID NO:48 (welche NADPH und/oder NADH regeneriert) oder einem zu den beiden genannten Enzymen jeweils funktional äquivalenten (bezüglich Kofaktor-Regenerierung), abgewandelten Form davon.

19. Verfahren nach Ausführungsform 18 wobei die FDH-Mutante $NADP^+$ mit einer spezifischen Aktivität zu NADPH reduziert, die etwa 0,1 bis 1.000 %, wie etwa 1 bis 100 %, 5 bis 80 % oder 10 bis 50 %, der spezifischen Aktivität des nichtmutierten (Wildtyp-) Enzyms für die Reduktion von $NAD^+$ zu NADH entspricht.

20. Verfahren nach einer der Ausführungsformen 18 und 19, wobei die $NADP^+$-akzeptierende FDH-Mutante wenigstens eine Mutation in der Aminosäure-Sequenz einer FDH aus *Mycobacterium vaccae* N10 gemäß SEQ ID NO: 36 oder einer davon abgeleiteten Aminosäuresequenz mit wenigstens 60 % Sequenzidentität, wie z.B. wenigstens 65, 70, 75, 80, 85 oder 90, wie z.B. wenigstens 91, 92, 93, 94, 95, 96, 97, 98, 99 oder 99,5 % zu dieser Sequenz, aufweist.

21. Verfahren nach einer der Ausführungsformen 18 bis 20, wobei die $NADP^+$-akzeptierende Mutante wenigstens eine Mutation im Sequenzmotiv TDRHRL gemäß Position 221 bis 226 von SEQ ID NO:36 und wenigstens 60 % Sequenzidentität, wie z.B. wenigstens 65, 70, 75, 80, 85 oder 90, wie z.B. wenigstens 91, 92, 93, 94, 95, 96, 97, 98, 99 oder 99,5 % zu dieser Sequenz, aufweist.
Nichtlimitierende Beispiele möglicherweise geeigneter FDH-Mutanten umfassen Mutationen in den Positionen D222 und /oder R223 von SEQ ID NO:36. Als Beispiele können genannt werden D222X mit X = G, A, K oder N; und R223X mit X = H oder Y, sowie Kombinationen von Mutationen in Position 222 und 223.

22. Verfahren nach einer der Ausführungsformen 18 bis 21 wobei die $NADP^+$-akzeptierende Mutante ausgewählt ist unter der Einfachmutante D222G gemäß SEQ ID NO:36 (hierin häufiger auch als "D221G" Mutante bezeichnet (bei Zählung, ausgehend von Ala in Position 2 von SEQ ID NO:36 als erste Aminosäure) oder den korrespondierenden Einfachmutanten einer davon abgeleiteten Aminosäuresequenz mit wenigstens 60 % Sequenzidentität, wie z.B. wenigstens 65, 70, 75, 80, 85 oder 90, wie z.B. wenigstens 91, 92, 93, 94, 95, 96, 97, 98, 99 oder 99,5 % zu dieser Sequenz. Als konkrete Beispiele können genannt werde FDH Mutanten nach SEQ ID NO: 15, 19 und 35.
Weitere geeignete FDH Enzyme sind ausgehend von den z.B. aus *Candida boidinii* oder *Pseudomonas sp,* isolierbaren Wildtyp-Enzymen und Einführung wenigstens einer zu obigen Mutationen entsprechenden funktionale Mutation zur Veränderung der Kofaktor-Spezifität zugänglich. Zudem gibt es im Stand der Technik zahlreiche Untersuchungen zur Verbesserung verschiedener FDH Eigenschaften, wie chemische oder thermische Stabilität oder katalytische Aktivität. Diese sind z.B. in Tishkov et al., Biomolecular Engineering 23 (2006), 89-110 zusammengefasst. So können dort beschriebene einfache oder mehrfache Punktmutationen, wie z.B. zur Erhöhung der Enzymstabilität, mit den erfindungsgemäß beschriebenen Mutationen zur modifizierten Kofaktor-Nutzung kombiniert werden.

23. Nukleinsäuresequenz, ausgewählt unter Nukleinsäuresequenzen a) gleichzeitig kodierend für eine FDH nach einer der Ausführungsformen 20 bis 22 und eine 7ß-HSDH nach einer der Ausführungsformen 1 bis 4 und gegebenenfalls eine 3$\alpha$-HSDH; oder b) kodierend für ein Fusionsprotein umfassend eine FDH nach einer der Ausführungsformen 20 bis 22 und eine 7ß-HSDH nach einer der Ausführungsformen 1 bis 4 und gegebenenfalls eine 3$\alpha$-HSDH; oder c) gleichzeitig kodierend für FDH und eine 7$\beta$-HSDH nach einer der Ausführungsformen 1 bis 4 und ggf. eine 3$\alpha$-HSDH; oder d) kodierend für ein Fusionsprotein, umfassend die FDH, eine 7$\beta$-HSDH nach einer der Ausführungsformen 1 bis 4 und ggf. eine 3$\alpha$-HSDH; oder e) gleichzeitig kodierend für eine GDH, eine 7$\beta$-HSDH nach einer der Ausführungsformen 1 bis 4 und ggf. eine 3$\alpha$-HSDH; oder f) kodierend für ein Fusionsprotein, umfassend eine GDH, eine 7$\beta$-HSDH nach einer der Ausführungsformen 1 bis 4 und ggf. eine 3$\alpha$-HSDH;
wobei die kodierenden Sequenzen unabhängig voneinander ein oder mehrfach in dem Konstrukt, wie z.B. in 2, 3, 4, 5, oder 6 bis 10 Kopien, enthalten sein können. Durch Wahl der geeigneten Kopienzahl können so eventuell gegebene Aktivitätsunterschiede in den einzelnen Expressionsprodukten kompensiert werden.

24. Expressionskassette, umfassend eine Nukleinsäuresequenz gemäß Ausführungsform 23 unter der genetischen Kontrolle wenigstens einer regulativen Nukleinsäuresequenz, wobei die kodierenden Sequenzen unabhängig voneinander ein oder mehrfach in dem Konstrukt, wie z.B. in 2, 3, 4, 5, oder 6 bis 10 Kopien, enthalten sein können. Durch Wahl der geeigneten Kopienzahl können so eventuell gegebene Aktivitätsunterschiede in den einzelnen Expressionsprodukten kompensiert werden.

25. Vektor, umfassend wenigstens eine Expressionskassette nach Ausführungsform 24, wobei die kodierenden Sequenzen unabhängig voneinander ein oder mehrfach in dem Vektorkonstrukt, wie z.B. in 2, 3, 4, 5, oder 6 bis 10 Kopien, enthalten sein können. Durch Wahl der geeigneten Kopienzahl können so eventuell gegebene Aktivitätsunterschiede in den einzelnen Expressionsprodukten kompensiert werden.

26. Rekombinanter Mikroorganismus, der wenigstens eine Nukleinsäuresequenz gemäß Ausführungsform 23 oder wenigstens eine Expressionskassette nach Ausführungsform 25 trägt oder wenigstens einem Vektor nach Ausführungsform 26 trägt.

27. Rekombinanter Mikroorganismus, welcher zur gleichzeitigen Expression einer 7ß-HSDH nach einer der Ausführungsformen 1 bis 4, einer $NADP^+$-akzeptierenden FDH nach einer der Ausführungsformen 18 bis 22 und/oder die entsprechende $NAD^+$-abhängige FDH und gegebenenfalls von 3$\alpha$-HSDH befähigt ist; oder welcher zur gleichzeitigen Expression einer 7$\beta$-HSDH nach einer der Ausführungsformen 1 bis 4, einer hierin beschriebenen GDH und ggf. einer hierin beschriebenen 3$\alpha$-HSDH befähigt ist.

28. Rekombinanter Mikroorganismus nach Ausführungsform 27, wobei die FDH ein Enzym gemäß der Definition

in einer der Ausführungsformen 18 bis 22 ist; und wobei die $NAD^+$-abhängige FDH eine FDH aus *Mycobacterium vaccae* N10 gemäß SEQ ID NO: 36 oder eine davon abgeleitete FDH mit wenigstens 60 % Sequenzidentität, wie z.B. wenigstens 65, 70, 75, 80, 85 oder 90, wie z.B. wenigstens 91, 92, 93, 94, 95, 96, 97, 98, 99 oder 99,5 % zu dieser Sequenz, ist.

29. Rekombinanter Mikroorganismus nach Ausführungsform 27, wobei die 3α-HSDH ein Enzym, umfassend eine Aminosäuresequenz nach SEQ ID NO: 6 oder 8 oder 22 oder eine davon abgeleitete Aminosäuresequenz mit wenigstens 60 % Sequenzidentität, wie z.B. wenigstens 65, 70, 75, 80, 85 oder 90, wie z.B. wenigstens 91, 92, 93, 94, 95, 96, 97, 98, 99 oder 99,5 % zu dieser Sequenz, ist.

30. Rekombinanter Mikroorganismus nach einer der Ausführungsformen 27 bis 29, welcher die kodierenden Sequenzen für 7ß-HSDH, FDH und 3α-HSDH auf einem oder mehreren (verschiedenen) Expressionskonstrukten trägt. Gegenstand der Erfindung sind somit rekombinante Mikroorganismen, welche mit einem Einplasmidsystem modifiziert (wie z.B. transformiert) sind, das die kodierenden Sequenzen für 7ß-HSDH bzw. Mutanten davon, FDH bzw. Mutanten davon und 3α-HSDH bzw. Mutanten davon in einer oder mehreren Kopien, wie z.B. in 2, 3, 4, 5 oder 6 bis 10 Kopien, tragen. Gegenstand der Erfindung sind somit auch rekombinante Mikroorganismen, welche mit einem Einplasmidsystem modifiziert (wie z.B. transformiert) sind, das die kodierenden Sequenzen für 7β-HSDH bzw. Mutanten davon, GDH bzw. Mutanten davon und 3α-HSDH bzw. Mutanten davon in einer oder mehreren Kopien, wie z.B. in 2, 3, 4, 5 oder 6 bis 10 Kopien tragen. Die Enzyme (7ß-HSDH, FDH und 3α-HSDH bzw. deren Mutanten) können aber auch auf 2 oder 3 getrennten, miteinander kompatiblen Plasmiden in einer oder mehreren Kopien enthalten sein. Geeignete Basisvektoren zur Herstellung von Einplasmidsystemen und Multicopy-Plasmiden sind dem Fachmann bekannt. Als Beispiele können genannt werden für Einplasmidsystem, z.B. pET21aund für Multicopyplasmide z.B. die von der Firma Novagen vertriebenen Duet-Vektoren, wie pACYCDuet-1, pETDuet-1, pCDFDuet-1, pRSFDuet-1 und pCOLADuet-1. Derartige Vektoren, deren Kompatibilität mit anderen Vektoren und mikrobiellen Wirtsstämmen sind z.B. dem "User Protocol TB340 Rev. E0305 der Firma Novagen zu entnehmen.
Die optimale Kombination von Enzymen für die Generierung von Plasmidsystemen kann der Fachmann unter Berücksichtigung der Lehre der vorliegenden Erfindung ohne unzumutbaren Aufwand vornehmen. So kann der Fachmann beispielsweise in Abhängigkeit von der Kofaktorspezifität des jeweils verwendeten 7β-HSDH-Enzyms das zur Kofaktorregenerierung am besten geeignete Enzym, ausgewählt unter den oben genannten Dehydrogenasen, insbesondere FDH, GDH und den jeweiligen Mutanten davon, auswählen.
Weiterhin besteht die Möglichkeit, die zur Umsetzung gewählten Enzyme auf zwei oder mehrere Plasmide zu verteilen und mit den so hergestellten Plasmiden zwei oder mehrere verschiedene rekombinante Mikroorganismen herzustellen, die dann gemeinsam für die erfindungsgemäße biokatalytische Umsetzung eingesetzt werden. Die jeweilige zur Herstellung des Plasmids verwendete Enzymkombination kann dabei insbesondere auch unter der Vorgabe einer vergleichbaren Kofaktor-Nutzung erfolgen. So kann beispielsweise ein erster Mikroorganismus mit einem Plasmid modifiziert werden, welches die kodierende Sequenz für eine NADPH-abhängige 7β-HSDH-Mutante und eine diesen Kofaktor regenerierende FDH-Mutante gemäß vorliegender Erfindung trägt, oder welches die kodierende Sequenz für eine NADPH-abhängige 7β-HSDH-Mutante und NADPH-regenerierende GDH, oder die kodierende Sequenz für eine NADH-abhängige 7β-HSDH und eine NADH-regenerierende FDH und / oder GDH trägt. Ein zweiter Mikroorganismus kann dagegen mit einem Plasmid modifiziert sein, das die kodierende Sequenz für eine NADH-abhängige 3α-HSDH und die kodierende Sequenz für einen NADH-regenerierenden FDH-Wildtyp und/ oder für eine NADH-regenerierende GDH trägt. Beide Mikroorganismen können dann gleichzeitig zur erfindungsgemäßen biokatalytischen Umsetzung eingesetzt werden.
Die Verwendung von zwei getrennten Biokatalysatoren (rekombinanter Mikroorganismen) kann gegenüber der Verwendung nur eines Biokatalysators, in dem alle Syntheseenzyme exprimiert werden, zwei wesentliche Vorteile bieten:

a) Beide Biokatalysatoren können separat voneinander gentechnisch modifiziert und optimiert werden. Insbesondere ist der Einsatz von verschiedenen Kofaktorregenerierungsenzymen möglich, die entweder auf NADH- oder auf NADPH-Regenerierung optimiert sind.

b) Für die Biokatalyse können die Biokatalysatoren in unterschiedlichen Anteilen eingesetzt werden. Dies ermöglicht ein Eingreifen in die einzelnen Reaktionsgeschwindigkeiten des Multienzymprozesses während der Biokatalyse, selbst nachdem sämtliche Biokatalysatoren bereits hergestellt sind.

Überraschenderweise konnte im Rahmen der vorliegenden Erfindung auch gezeigt werden, dass die durch die Verwendung zweier Biokatalysatoren erforderten zusätzlichen Membrantransportschritte der umzusetzenden Substanzen sich nicht oder nur geringfügig auf die Reaktionsgeschwindigkeiten auswirken, so dass diese vermeintlichen negativen Aspekte von den Vorteilen des Zweizellsystems überwogen werden.

31. Verfahren zur Herstellung von Ursodesoxycholsäure (UDCS) der Formel (1)

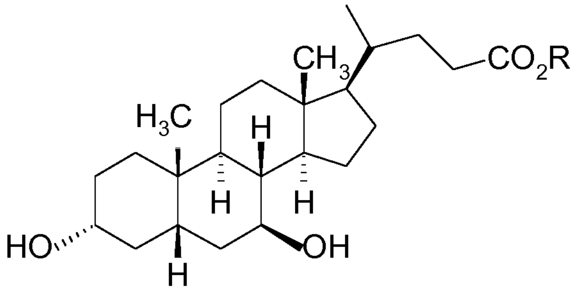

(1)

worin
R für Alkyl, H, ein Alkalimetallion oder $N(R^3)_4^+$steht, worin die Reste $R^3$ gleich oder verschieden sind und für H oder Alkyl stehen,
wobei man

a) gegebenenfalls eine Cholsäure (CS) der Formel (2)

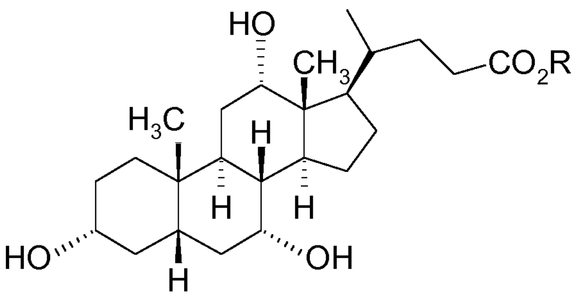

(2)

worin R die oben angegebenen Bedeutungen besitzt, zur Dehydrocholsäure (DHCS) der Formel (3)

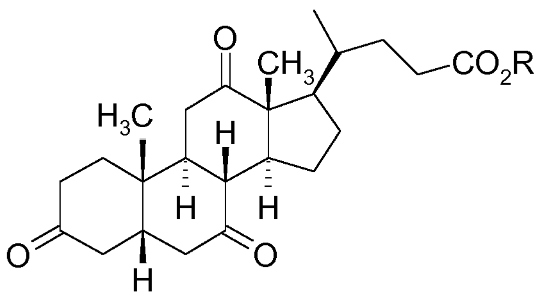

(3)

worin R die oben angegebenen Bedeutungen besitzt, chemisch oxidiert;

b) DHCS in Gegenwart wenigstens einer 7ß-HSDH (vorliegend als isoliertes Enzym oder exprimiert von einer entsprechenden rekombinanten Mikroorganismus) gemäß der Definition in einer der Ausführungsformen 1 bis 4 und in Gegenwart wenigstens einer 3α-Hydroxysteroiddehydrogenase (3α-HSDH) (vorliegend als isoliertes Enzym oder exprimiert von einem entsprechenden rekombinanten Mikroorganismus) zur korrespondierenden 12-Keto-ursodesoxycholsäure (12-Keto UDCS) der Formel (5)

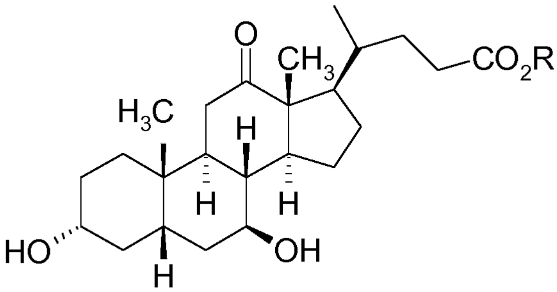

(5)

worin R die oben angegebenen Bedeutungen besitzt, (in Gegenwart und unter Verbrauch von NADH und/oder NADPH) reduziert und anschließend

d) 12-Keto-UDCS der Formel (5) chemisch zu UDCS reduziert; und

e) das Reaktionsprodukt gegebenenfalls weiter aufreinigt.

Der Verfahrensschritt b) kann dabei unterschiedlich ausgestaltet sein. Entweder können beide Enzyme (7ß-HSDH und 3α-HSDH) gleichzeitig zugegen sein (z.B. Ein-Topf-Reaktion mit beiden isolierten Enzymen oder ein oder mehrere entsprechende rekombinante Mikroorganismen sind zugegen, der beide Enzyme exprimiert), oder die Teilreaktionen können in beliebiger Reihenfolge (erst die 7ß-HSDH -katalysierte Reduktion und dann die 3α-HSDHkatalysierte Reduktion; oder erst die 3α-HSDH -katalysierte Reduktion und dann die 7ß-HSDH -katalysierte Reduktion) ablaufen.
Eine Verfahrensvariante zur Herstellung von UDCS der Formel (1) könnte daher z.B. sein, dass man

a) gegebenenfalls eine Cholsäure (CS) der Formel (2) chemisch oxidiert;

b) DHCS in Gegenwart wenigstens einer 7ß-HSDH (vorliegend als isoliertes Enzym oder exprimiert von einer entsprechenden rekombinanten Mikroorganismus) gemäß der Definition in einer der Ausführungsformen 1 bis 4 zur 3,12-Diketo-7ß-cholansäure (3,12-Diketo-7ß-CS) der Formel (4)

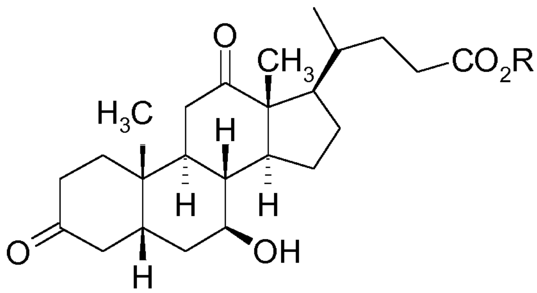

(4)

(in Gegenwart und unter Verbrauch von NADPH und/oder NADH) reduziert,

c) 3,12-Diketo-7ß-CS in Gegenwart wenigstens einer 3a-Hydroxysteroiddehydrogenase (3α-HSDH) (vorliegend als isoliertes Enzym oder exprimiert von einem entsprechenden rekombinanten Mikroorganismus) zur korrespondierenden 12-Keto-ursodesoxycholsäure (12-Keto UDCS) der Formel (5)

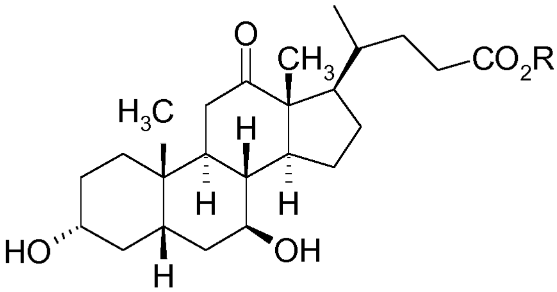

(5)

worin R die oben angegebenen Bedeutungen besitzt, (in Gegenwart und unter Verbrauch von NADH und /oder NADPH, je nach verwendeter 3α-HSDH) reduziert und anschließend

d) 12-Keto-UDCS der Formel (5) chemisch zu UDCS reduziert; und

e) das Reaktionsprodukt gegebenenfalls weiter aufreinigt.

32. Verfahren nach Ausführungsform 31, wobei die Schritte b) und c) in Gegenwart eines oder mehrerer hierin beschriebener rekombinanten Mikroorganismen, wie wenigstens einem Mikroorganismus nach Ausführungsform 8 durchgeführt werden.

33. Verfahren nach Ausführungsform 31 oder 32, wobei die Schritte b) und/oder c) mit gleichen oder verschiedenen Kofaktor-Regenerierungssystemen (vorliegend als isoliertes Enzym oder exprimiert von einem entsprechenden rekombinanten Mikroorganismus) gekoppelt sind.

34. Verfahren nach Ausführungsform 33, wobei Schritt b) mit einem Kofaktorregenerierungssystem gekoppelt ist, bei welchem NADPH durch eine $NADP^+$-akzeptierenden FDH gemäß der Definition in einer der Ausführungsformen 18 bis 22 unter Verbrauch von Ameisensäure oder eines Salzes davon regeneriert wird; oder mit einem Kofaktorregenerierungssystem gekoppelt ist, bei welchem NADPH durch eine ADH unter Verbrauch von Isopropanol regeneriert wird; oder mit einem Kofaktorregenerierungssystem gekoppelt ist, bei welchem NADPH durch eine GDH unter Verbrauch von Glucose regeneriert wird; oder mit einem Kofaktorregenerierungssystem gekoppelt ist, bei welchem verbrauchtes NADH durch eine NADH regenerierende GDH, ADH oder FDH regeneriert wird.

35. Verfahren nach Ausführungsform 33 oder 34 wobei Schritt c) mit einem Kofaktorregenerierungsschritt gekoppelt ist, bei welchem NADPH durch eine $NADP^+$-akzeptierenden FDH gemäß der Definition in einer der Ausführungsformen 18 bis 22 unter Verbrauch von Ameisensäure oder eines Salzes davon regeneriert wird; oder wobei Schritt c) mit einem Kofaktorregenerierungsschritt gekoppelt ist, bei welchem NADH durch eine $NAD^+$- und $NADP^+$-akzeptierende FDH gemäß der Definition in einer der Ausführungsformen 18 bis 22 oder durch die nichtmutierte FDH unter Verbrauch von Ameisensäure oder eines Salzes davon, oder durch eine $NAD^+$-akzeptierende GDH unter Verbrauch von Glucose regeneriert wird.

36. Verfahren zur mikrobiellen Herstellung von Ursodesoxycholsäure (UDCS) der Formel (1)

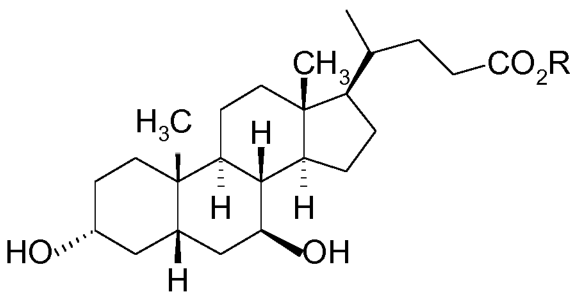

(1)

worin
R für Alkyl, H, ein Alkalimetallion oder $N(R^3)_4^+$ steht, worin die Reste $R^3$ gleich oder verschieden sind und für H oder Alkyl stehen,

wobei man

a) gegebenenfalls eine Cholsäure (CS) der Formel (2)

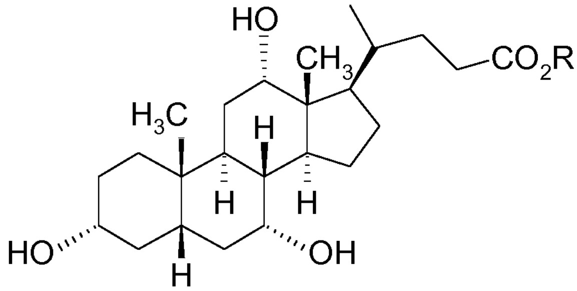

(2)

worin R die oben angegebenen Bedeutungen besitzt, zur Dehydrocholsäure (DHCS) der Formel (3)

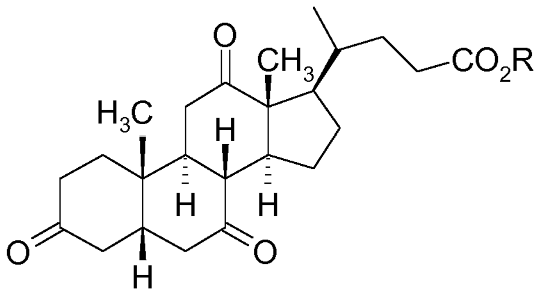

(3)

worin R die oben angegebenen Bedeutungen besitzt, chemisch oxidiert;

b) DHCS in Gegenwart wenigstens einer 7ß-HSDH und in Gegenwart wenigstens einer 3α-HSDH zur korrespondierenden 12-Keto-ursodesoxycholsäure (12-Keto UDCS) der Formel (5)

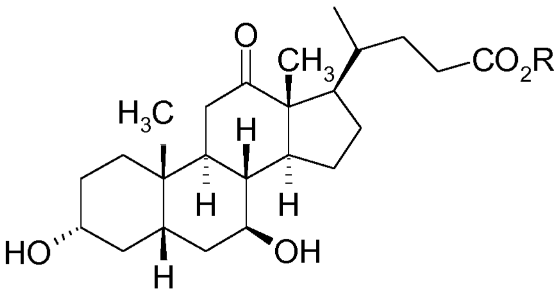

(5)

worin R die oben angegebenen Bedeutungen besitzt, (in Gegenwart und unter Verbrauch von NADH und/oder NADPH) reduziert und anschließend

c) 12-Keto-UDCS der Formel (5) chemisch zu UDCS reduziert; und

d) das Reaktionsprodukt gegebenenfalls weiter aufreinigt; wobei die Umsetzungen der Stufe b) mikrobiell, d.h. in Gegenwart ganzer Zellen eines oder mehrerer verschiedener rekombinanter Mikroorganismen nach einer der Ausführungsformen 2628 oder 27 bis 30 erfolgen, wobei der/die Mikroorganismen die zur Umsetzung und Kofaktorregnerierung erforderlichen Enzyme in einer hierin näher beschriebenen Weise tragen, oder aber unter Verwendung wenigsten einer Nukleinsäuresequenz nach Ausführungsform 23.

Beispielsweise kann dabei DHCS in Gegenwart wenigstens einer 7ß-HSDH oder Mutante davon zur 3,12-Diketo-7ß-cholansäure (3,12-Diketo-7ß-CS) der Formel (4)

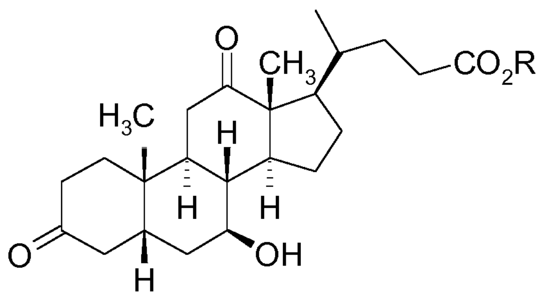

(4)

(in Gegenwart und unter Verbrauch von NADPH) reduziert werden, und
3,12-Diketo-7ß-CS in Gegenwart wenigstens einer 3α-Hydroxysteroiddehydrogenase (3α-HSDH) oder Mutante davon zur korrespondierenden 12-Keto-ursodesoxycholsäure (12-Keto UDCS) der Formel (5)

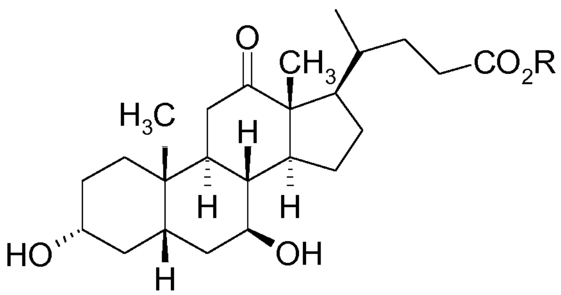

(5)

worin R die oben angegebenen Bedeutungen besitzt, (in Gegenwart und unter Verbrauch von NADH und/oder NADPH) reduziert werden.
Weiterhin ist aber auch eine Reaktionssequenz, umfassend die Reduktion von DHCS zunächst mit 3α-HSDH und die anschließende Reduktion des dabei gebildeten Reaktionsprodukts mit 7ß-HSDH denkbar, sowie ein gleichzeitiger Ablauf beider Reaktionssequenzen, aufgrund der gleichzeitigen Anwesenheit beider HSDHs.

37. Verfahren nach Ausführungsform 36, wobei man einen oder mehrere, insbesondere einen rekombinanten Mikroorganismus nach einer der Ausführungsformen 27 bis 30 einsetzt, der beispielsweise wenigstens eine 7ß-HSDH nach einer der Ausführungsformen 1 bis 4, wenigstens eine $NADP^+$-akzeptierende FDH nach einer der Ausführungsformen 18 bis 22 und wenigstens eine 3α-HSDH, wie insbesondere gemäß SEQ ID NO: 6, 8 oder 22 oder Mutanten davon im eine Sequenzidentität von mindestens etwa 60 %, gleichzeitig exprimiert; oder der wenigstens eine 7ß-HSDH nach einer der Ausführungsformen 1 bis 4, wenigstens eine GDH und wenigstens eine 3α-HSDH, wie insbesondere gemäß SEQ ID NO: 6, 8 oder 22 oder Mutanten davon im eine Sequenzidentität von mindestens etwa 60 %, gleichzeitig exprimiert.

38. Ebenfalls hierin beschrieben ist ein Bioreaktor zur Durchführung eines Verfahrens nach einer der Ausführungsformen 31 bis 37, insbesondere enthaltend wenigstens eines der Enzyme (7ß-HSDH, FDH, und/oder 3α-HSDH bzw. deren Mutanten; oder 7ß-HSDH, GDH und/oder 3α-HSDH bzw. deren Mutanten) oder einen wenigstens eines dieser Enzyme rekombinant exprimierenden rekombinanten Mikroorganismus, insbesondere in immobilisierter Form.

Vorliegende Erfindung ist nicht auf die hierin beschriebenen konkreten Ausführungsformen beschränkt. Der Fachmann wird durch die Lehre der vorliegenden Erfindung vielmehr in die Lage versetzt, ohne unzumutbaren Aufwand weitere Ausgestaltungen der Erfindung bereitzustellen. So kann er beispielsweise auch weitere Enzymmutanten gezielt generieren und diese auf das gewünschte Eigenschaftsprofil (verbesserte Kofaktor-Abhängigkeit und /der Stabilität, verringerte Substratinhibition) screenen und optimieren; oder weitere geeignete Wildtypenzyme (7ß- und 3α-HSDHs, FDHs, GDHs ADHs usw.) isolieren und erfindungsgemäß verwenden. Weiterhin kann er beispielsweise je nach Eigenschaftsprofil (insbesondere Kofaktor-Abhängigkeit) der verwendeten HSDHs, wie insbesondere 7ß-HSDH und 3α-HSDH oder Mutanten davon, geeignete zur Kofaktorregenerierung brauchbare Dehydrogenasen (GDH, FHD, ADH usw.) und Mutanten davon auswählen, und die ausgewählten Enzyme auf einen oder mehrere Expressionskonstrukte oder Vektoren verteilen und damit erforderlichenfalls einen oder mehrere rekombinante Mikroorganismen erzeugen, die dann ein optimiertes ganzzellbasiertes Herstellungsverfahren ermöglichen.

## Weitere Ausgestaltungen der Erfindung

1. Allgemeine Definitionen und verwendete Abkürzungen

Werden keine anderen Angaben gemacht, so bezeichnet der Begriff "7ß-HSDH" ein Dehydrogenase-Enzym, welches wenigstens die stereospezifische und/oder regiospezifische Reduktion von DHCS oder 7,12-Diketo-3α-CS (7,12-Diketo-LCS) zu 3,12-Diketo-7ß-CS oder 12-Keto-UDCS insbesondere unter stöchiometrischem Verbrauch von NADPH, und gegebenenfalls die entsprechende Umkehrreaktion katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen. Geeignete Nachweisverfahren sind z.B. im folgenden experimentellen Teil beschrieben oder aus der Literatur bekannt (z.B. Characterization of NADP-dependent 7 beta-hydroxysteroid dehydrogenases from Peptostreptococcus productus and Eubacterium aerofaciens. S Hirano and N Masuda. Appl Environ Microbiol. 1982). Enzyme dieser Aktivität sind unter der EC Nummer 1.1.1.201 klassifiziert.

Werden keine anderen Angaben gemacht, so bezeichnet der Begriff "3α-HSDH" ein Dehydrogenase-Enzym, welches wenigstens die stereospezifische und/oder regiospezifische Reduktion von 3,12-Diketo-7ß-CS oder DHCS zu 12-Keto-UDCS oder 7,12-Diketo-3a-CS (7,12-Diketo-LCS), insbesondere unter stöchiometrischem Verbrauch von NADH und/oder NADPH, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus *Comanomonas testosteroni* (z.B. ATCC11996) erhältlich. Eine NADPH abhängige 3α-HSDH ist z.B. aus der Nagern bekannt und ebenfalls einsetzbar (Cloning and sequencing of the cDNA for rat liver 3 alphahydroxysteroid/dihydrodiol dehydrogenase, J E Pawlowski, M Huizinga and T M Penning, May 15, 1991 The Journal of Biological Chemistry, 266, 8820-8825). Enzyme dieser Aktivität sind unter der EC Nummer 1.1.1.50 klassifiziert.

Werden keine anderen Angaben gemacht, so bezeichnet der Begriff "GDH" ein Dehydrogenase-Enzym, welches wenigstens die Oxidation von β-D-Glucose zu D-Glucono-1,5-Lacton unter stöchiometrischem Verbrauch von $NAD^+$ und/oder $NADP^+$ sowie ggf. die entsprechende Umkehrreaktion katalysiert. Geeignete Enzyme sind z.B. aus *Bacillus subtili* oder *Bacillus megaterium* erhältlich. Enzyme dieser Aktivität sind unter der EC Nummer 1.1.1.47 klassifiziert. Werden keine anderen Angaben gemacht, so bezeichnet der Begriff "FDH" ein Dehydrogenase-Enzym, welches wenigstens die Oxidation von Ameisensäure (oder korrespondierenden Formiat-Salzen) zu Kohlendioxid unter stöchiometrischem Verbrauch von $NAD^+$ und/oder $NADP^+$, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus *Candida boidinii, Pseudomonas sp,* oder *Mycobacterium vaccae* erhältlich. Enzyme dieser Aktivität sind unter der EC Nummer 1.2.1.2 klassifiziert.

Unter einer "Reinform" oder einem "reinen" oder "im wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie z.B. der Biuret-Methode oder dem Proteinnachweis nach Lowry.et al.(vgl Beschreibung in R.K. Scopes, Protein Purification, Springer Verlag, New York, Heidelberg, Berlin (1982)).

Unter einem "Redoxäquivalent" versteht man eine als Elektronendonor bzw. Elektronenakzeptor brauchbare, niedermolekulare organische Verbindung, wie beispielsweise Nikotinamidderivate wie $NAD^+$ und $NADH^+$ bzw. deren reduzierte Formen NADH bzw. NADPH. "Redoxäquivalent" und "Kofaktor" werden im Kontext der vorliegenden Erfindung als Synonym verwendet. So kann ein "Kofaktor" im Sinne der Erfindung auch als "redoxfähiger Kofaktor", d.h. als Kofaktor, der in reduzierter und einer oxidierter Form vorliegen kann, umschrieben werden.

Unter einem "verbrauchten" Kofaktor versteht man diejenige reduzierte bzw. oxidierte Form des Kofaktors, die im Verlauf einer vorgegebene Reduktions- bzw. Oxidationsreaktion eines Substrats in die korrespondierende oxidierte bzw. reduzierte Form überführt wird. Durch Regenerierung wird die bei der Reaktion gebildete oxidierte bzw. reduzierte Kofaktor-Form wieder in die reduzierte bzw. oxidierte Ausgangsform zurück überführt, so dass diese für die Umsetzung des Substrats wieder zur Verfügung steht.

Unter einer "veränderten Kofaktor-Nutzung" versteht man im Rahmen der vorliegenden Erfindung eine qualitative oder quantitative Veränderung im Vergleich zu einer Referenz. Insbesondere ist eine veränderte Kofaktor-Nutzung durch Vornahme von Aminosäuresequenzmutationen zu beobachten. Diese Veränderung ist dann im Vergleich zum nichtmutierten Ausgangsenzym feststellbar. Dabei kann die Aktivität in Bezug auf einen bestimmten Kofaktor durch Vornahme einer Mutation erhöht oder erniedrigt, oder vollständig unterbunden werden. Eine veränderte Kofaktor-Nutzung umfasst aber auch Änderungen dergestalt, dass anstelle einer Spezifität für einen einzelnen Kofaktor nunmehr wenigstens ein weiterer, vom ersten Kofaktor verschiedener, zweiter Kofaktor nutzbar ist. (d.h. es liegt eine erweiterte Kofaktor-Nutzung vor) Umgekehrt kann aber auch eine ursprünglich vorhandene Befähigung zur Nutzung zweier verschiedener Kofaktoren so abgeändert werden, dass Spezifität nur für einen dieser Kofaktoren erhöht bzw. für einen dieser Kofaktoren verringert oder vollständig aufgehoben wird. So kann beispielsweise ein Enzym, das vom Kofaktor NAD (NADH) abhängig ist, aufgrund einer Veränderung der Kofaktor-Nutzung nunmehr sowohl von NAD (NADH) als

auch vom Kofaktor NADP (NADPH) abhängig sein oder die ursprüngliche Abhängigkeit von NAD (NADH) kann vollständig in eine Abhängigkeit von NADP (NADPH) überführt werden und umgekehrt.

Die Begriffe "$NAD^+$/NADH-Abhängigkeit" bzw. "$NADP^+$/NADPH-Abhängigkeit" sind erfindungsgemäß, wenn nicht anders definiert, weit auszulegen. Diese Begriffe umfassen sowohl "spezifische" Abhängigkeiten, d.h. ausschließlich Abhängigkeit von $NAD^+$/NADH bzw. $NADP^+$/NADPH, als auch die Abhängigkeit der erfindungsgemäß verwendeten Enzyme von beiden Kofaktoren, d.h. Abhängigkeit von $NAD^+$/NADH und $NADP^+$/NADPH.

Entsprechendes gilt für die verwendeten Begriffe "$NAD^+$/NADH-akzeptierend" bzw. "$NADP^+$/NADPH-akzeptierend".

Die Begriffe "$NAD^+$/NADH-regenerierend" bzw. "$NADP^+$/NADPH-regenerierend" sind erfindungsgemäß, wenn nicht anders definiert, weit auszulegen. Diese Begriffe umfassen sowohl "spezifische", d.h. ausschließliche Befähigung verbrauchten Kofaktor $NAD^+$/NADH bzw. $NADP^+$/NADPH zu regenerieren, als auch die Befähigung beide Kofaktoren, d.h. $NAD^+$/NADH und $NADP^+$/NADPH, zu regenerieren.

"Proteinogene" Aminosäuren umfassen insbesondere (Einbuchstabencode): G, A, V, L, I, F, P, M, W, S, T, C, Y, N, Q, D, E, K, R und H.

Unter einer "Immobilisierung" versteht man die kovalente oder nicht-kovalente Bindung eines erfindungsgemäß verwendeten Biokatalysators, wie z.B. einer 7ß-HSDH an einem festen, d.h. in dem umgebenden flüssigen Medium im Wesentlichen unlöslichen, Trägermaterial. Es können entsprechend auch ganze Zellen, wie die erfindungsgemäß verwendeten, rekombinanten Mikroorganismen mit Hilfe derartiger Träger immobilisiert sein.

Eine "im Vergleich zum nicht-mutierten Enzym verringerte Substratinhibition" bedeutet, dass die beim nicht-mutierten Enzym für ein bestimmtes Substrat beobachtete Substratinhibition nicht mehr zu beobachten ist, d.h. im Wesentlichen nicht mehr messbar ist, oder erst bei höherer Substratkonzentration einsetzt, d.h. der $K_i$-Wert erhöht ist.

Unter einer "Cholsäure-Verbindung" versteht man erfindungsgemäß Verbindungen mit dem Kohlenstoffgrundgerüst, insbesondere der Steroidstruktur der Cholsäure und dem Vorhandensein von Keto- und/oder Hydroxy- oder Acyloxy-Gruppen in der Ringposition 7 und gegebenenfalls den Ringpositionen 3 und/oder 12.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das $NH_4^+$-Salz bzw. solche Ammoniumsalze umfasst, worin wenigstens ein Wasserstoffatom durch einen $C_1C_6$-Alkylrest ersetzt sein kann. Typische Alkylreste sind insbesondere $C_1C_4$-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon.

"Alkylester" erfindungsgemäß eingesetzter Verbindungen sind insbesondere Niedrigalkyl-Ester, wie z.B. $C_1C_6$-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäß eingesetzter Säuren mit Ammoniak oder primären oder sekundären Monoaminen. Derartige Amine sind beispielsweise Mono- oder Di-$C_1$-$C_6$-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

"Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und $C_1$-$C_6$-Alkylgruppen, wie z.B. Benzoyl oder Toluoyl.

Die erfindungsgemäß eingesetzten bzw. hergestellten Hydroxysteroidverbindungen, wie z.B. Cholsäure, Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

In folgender Tabelle sind die Strukturformeln, deren chemischen Namen und die verwendeten Abkürzungen wesentlicher chemischer Verbindungen tabellarisch zusammengefasst:

| Formel | Abkürzung | Chemischer Name |
|---|---|---|
| 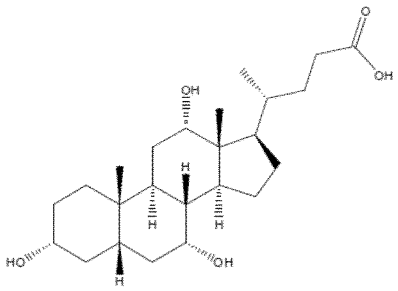<br>Cholsäure | CS | Cholsäure |
| 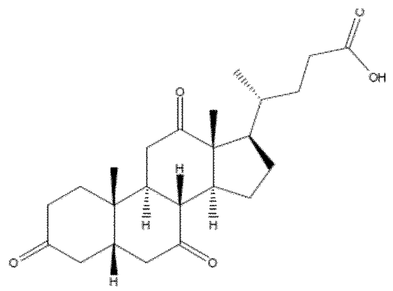<br>Dehydrocholsäure | DHCS | Dehydrocholsäure |
| 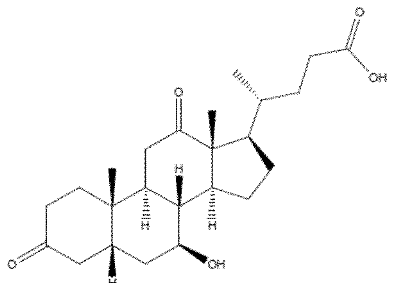<br>3,12-Diketo-7ß-Hydroxycholansäure | 3,12-Diketo-7β-CS | 3,12-Diketo-7β-Hydroxycholansäure |
| 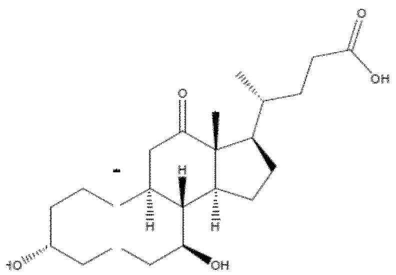<br>12keto-Ursodeoxycholsäure | 12Keto-UDCS | 12Keto-Ursodeoxycholsäure |
| 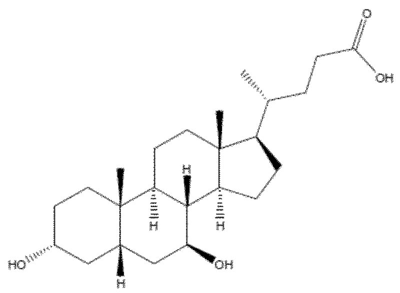<br>Ursodeoxycholsäure | UDCS | Ursodeoxycholsäure |

(fortgesetzt)

| Formel | Abkürzung | Chemischer Name |
|---|---|---|
| 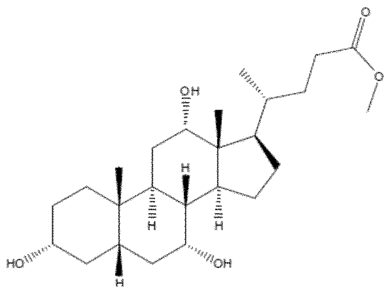 Cholsäure-methylester | CS-Methylester | Cholsäure-methylester |
| 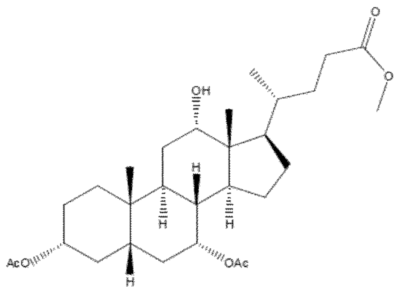 3,7-Di-O-Acetylcholsäuremethylester | 3,7 -Diacetyl-CS-methylester | 3,7-Di-O-Acetylcholsäuremethylester* |
| 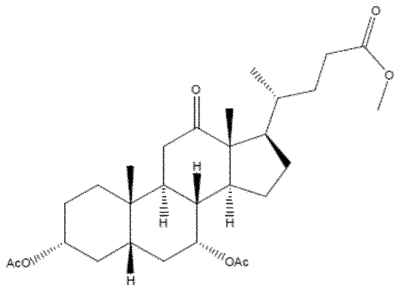 12-Keto-3,7-Di-O-Acetylcholsäuremethylester | 12 -Keto-3,7 -Diacetyl-CS-methylester | 12-Keto-3,7-Di-O-Acetylcholsäuremethylester* |
| 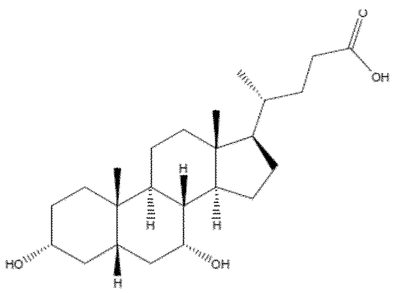 Chenodeoxycholsäure | CDCS | Chenodeoxycholsäure |

(fortgesetzt)

| Formel | Abkürzung | Chemischer Name |
|---|---|---|
| 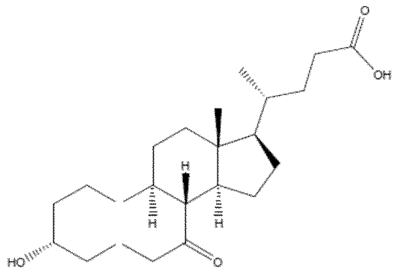 7-Keto-Lithocholsäure | 7-Keto-LCS | 7-Keto-Lithocholsäure |
| 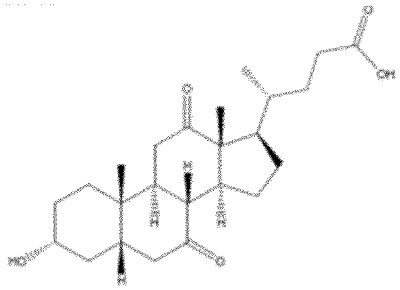 7,12-Diketo-Lithocholsäure | 7,12-Diketo-LCS | 7,12-Diketo-Lithocholsäure |
| 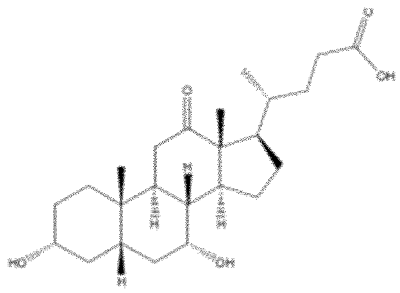 12-Keto-Chenodcoxycholsäure | 12-Keto-CDCS | 12-Keto-Chenodeoxycholsäure |

2. Proteine

Die vorliegende Erfindung ist nicht auf die konkret offenbarten Proteine bzw. Enzyme mit 7ß-HSDH-, FDH-, GDH- oder 3$\alpha$-HSDH Aktivität bzw. deren Mutanten beschränkt, sondern erstreckt sich vielmehr auch auf funktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. 7ß HSDH-Aktivität, besitzen.

So versteht man beispielsweise unter "funktionalen Äquivalenten" Enzyme, die im verwendeten Test auf 7ß-HSDH-, FDH-, GDH- oder 3$\alpha$-HSDH -Aktivität eine um mindestens 1 % , wie z.B. mindestens 10% oder 20 %, wie z.B. mindestens 50 % oder 75 % oder 90 % höhere oder niedrigere Aktivität eines Ausgangs-Enzyms, umfassend eine hierin definierte Aminosäuresequenz aufweisen.

Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C.

Die 7ß-HSDH-Aktivität kann mit Hilfe verschiedener bekannter Tests nachgewiesen werden. Ohne darauf beschränkt zu sein, sei ein Test unter Verwendung eines Referenzsubstrates, wie z. B. CS oder DHCS, unter standardisierten Bedingungen wie im experimentellen Teil definiert, zu nennen.

Tests zur Bestimmung der FDH-, GDH- oder 3$\alpha$-HSDH -Aktivität sind ebenfalls an sich bekannt.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte

Aminosäure aufweisen, aber trotzdem eine der oben genannten biologischen Aktivitäten besitzen. "Funktionale Äquivalente" umfassen somit die durch eine oder mehrere, wie z.B. 1, 2, 3, 4, 5, 6, 7, 8, 9, 10, 11, 12, 13, 14 oder 15 Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| **Ursprünglicher Rest** | **Beispiele der Substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder anderen N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxylgruppen, hergestellt durch Umsetzung mit Acylgruppen.

"Funktionale Äquivalente" umfassen natürlich auch Polypeptide, welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen

sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 % beziehungsweise wenigsten 80 %, wie z.B. 90, 91, 92, 93, 94, 95, 96, 97,98 oder 99 %, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Die Erfindung umfasst weiterhin die Anwendung des 7ß-HSDH-Wildtyps aus *Collinsella aerofaciens* ATCC 25986, wie er in der älteren internationalen Patentanmeldung PCT/EP2010/068576 der Anmelderin beschrieben ist.

Dieser aus *Collinsella aerofaciens* DSM 3979 erhältliche 7β-HSDH ist insbesondere durch wenigstens eine weitere der folgenden Eigenschaften, wie z.B. 2, 3, 4, 5, 6 oder 7 oder aller solcher Eigenschaften, gekennzeichnet:

a) Molekulargewicht (SDS-Gelelektrophorese): etwa 28-32 kDa, insbesondere etwa 29 bis 31 kDa oder etwa 30 kDa;

b) Molekulargewicht (Gelfiltration, unter nicht denaturierenden Bedingungen, wie insbesondere ohne SDS): etwa 53 bis 60 kDa, insbesondere etwa 55 bis 57 kDa , wie 56.1 kDa. Dies belegt die dimere Beschaffenheit der 7β-HSDH aus *Collinsella aerofaciens* DSM 3979;

c) Stereoselektiven Reduktion der 7-Carbonyl-Gruppe von 7-Keto-LCS in eine 7β-Hydroxy-Gruppe;

d) pH Optimum für die Oxidation von UDCS im Bereich von pH 8,5 bis 10,5, insbesondere 9 bis 10;

e) pH Optimum für die Reduktion von DHCS und 7-Keto-LCS im Bereich von pH 3,5 bis 6,5, insbesondere bei pH 4 bis 6;

f) wenigstens einen kinetischen Parameter aus folgender Tabelle für wenigstens eines der dort genannten Substrate/Kofaktoren; im Bereich von ±20%, insbesondere ±10%, ±5%, ±3% ±2% oder ±1% um den in der folgenden Tabelle jeweils konkret genannten Wert.

| | $K_M$ ($\mu$M) | $V_{max}$ (U/mg Protein)$^{b)}$ | $k_{cat}$ (1 $\mu$mol/($\mu$mol$\times$min)) |
|---|---|---|---|
| $NADP^+$ | 5.32 | 30.58 | 944.95 |
| NADPH | 4.50 | 33.44 | 1033.44 |
| UDCS | 6.23 | 38.17 | 1179.39 |

(fortgesetzt)

| | $K_M$ (μM) | $V_{max}$ (U/mg Protein)[b)] | $k_{cat}$ (1 μmol/(μmol×min)) |
|---|---|---|---|
| 7-Keto-LCS | 5.20 | 30.77 | 950.77 |
| DHCS | 9.23 | 28.33 | 875.35 |
| $NAD^+$ | -[a)] | - | Spuren |
| NADH | - | - | Spuren |
| a) konnten aufgrund der sehr geringen Aktivität nicht bestimmt werden<br>b) 1 U = 1 μmol/min | | | |

g) Phylogenetische Sequenzverwandtschaft der prokaryotischen 7β-HSDH aus *Collinsella aerofaciens* DSM 3979 mit der tierischen 11β-HSDH-Untergruppe, umfassend *Cavia porcellus, Homo sapiens* und *Mus musulus,* verwandt.

Beispielsweise zeigt diese 7β-HSDH folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f).

Eine derartige 7ß-HSDH oder davon abgeleitetes funktionales Äquivalent ist zudem gekennzeichnet durch

a) die stereospezifische Reduktion eines 7-Ketosteroids zum korrespondierenden 7ß-Hydroxysteroid, und/oder
b) die regiospezifische Hydroxylierung eines Ketosteroids umfassend eine Ketogruppe in 7-Position und wenigstens eine weitere Ketogruppe am Steroidgerüst zum korrespondierenden 7ß-Hydroxysteroid, wie insbesondere von Dehydrocholsäure (DHCS) in 7-Position zur korrespondierenden 3,12-Diketo-7ß-Cholansäure, und die z.B. NADPH abhängig ist.

Eine derartige 7ß-HSDH hat insbesondere eine Aminosäuresequenz gemäß SEQ ID NO:2 (Accession NO: ZP_01773061) oder eine davon abgeleiteten Sequenz mit einem Identitätsgrad von wenigstens 60%, wie z.B. wenigstens 65, 70, 75, 80, 85, oder 90, wie z.B. wenigstens 91, 92, 93, 94, 95, 96, 97, 98, 99 oder 99,5% zu dieser Sequenz; gegebenenfalls zusätzlich gekennzeichnet durch eine der folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f) gemäß obiger Definition.

3. Nukleinsäuren und Konstrukte

3. 1 Nukleinsäuren

Gegenstand der Erfindung sind auch Nukleinsäuresequenzen, die für ein Enzym mit 7ß-HSDH-, FDH-, GDH- und/oder 3α-HSDH Aktivität und deren Mutanten kodieren.

Die vorliegende Erfindung betrifft auch Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameters:

| | |
|---|---|
| Gap opening penalty | 10 |
| Gap extension penalty | 10 |
| Gap separation penalty range | 8 |

| | |
|---|---|
| Gap separation penalty | off |
| % identity for alignment delay | 40 |
| Residue specific gaps | off |
| Hydrophilic residue gap | off |

(fortgesetzt)

| | |
|---|---|
| Transition weighing | 0 |

Pairwise alignment parameter:

| | |
|---|---|
| FAST algorithm | on |
| K-tuplesize | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten.

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der

erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100 % komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10°C niedriger als die von DNA:RNA-Hybriden gleicher Länge.

Unter Standardbedingungen sind beispielsweise je nach Nukleinsäure Temperaturen zwischen 42 und 58°C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20°C bis 45°C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30°C bis 55°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10 % Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1, 5, 7, 14, 19, 34 oder 47 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO:1, 5, 7, 14, 19, 34 oder 47sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen). Über Teilbereiche der Sequenzen können die Homologien vorteilhaft höher liegen.

Weiterhin sind unter Derivaten auch Homologe der erfindungsgemäßen Nukleinsäuresequenzen, insbesondere der SEQ ID NO:1, 5, 7, 14, 19, 34 oder 47beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO:1, 5, 7, 14, 19, 34 oder 47 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO:1, 5, 7, 14, 19, 34 oder 47 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des Weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Proteine sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcärel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique u-sing an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden.

Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen, wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

3.2 Konstrukte

Gegenstand der Erfindung sind außerdem Expressionskonstrukte, enthaltend unter der genetischen Kontrolle regulativer Nukleinsäuresequenzen eine für wenigstens ein erfindungsgemäßes Polypeptid kodierende Nukleinsäuresequenz; sowie Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierin definiert, umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO:1, 5, 7, 14, 19, 34 oder 47 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN\text{-}III^{113}$-B1, Agt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

4. Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben. Einen Überblick über bakterielle Expressionssysteme für die heterologe Expression von Proteinen liefertz.B. auch Terpe, K. Appl. Microbiol. Biotechnol. (2006) 72: 211-222.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gramnegative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen *Escherichia, Pseudomonas, Streptomyces, Nocardia, Burkholderia, Salmonella, Agrobacterium, Clostridium* oder *Rhodococcus* verwendet. Ganz besonders bevorzugt ist die Gattung und Art *Escherichia coli.* Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit 7ß-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

5. Herstellung der UDCS

*1. Schritt: Chemische Umsetzung von CS zu DHCS*

Die Hydroxygruppen von CS werden mit Chromsäure bzw. Chromaten in saurer Lösung (z.B. $H_2SO_4$) zu Carbonylgruppe in an sich bekannter Weise auf klassischchemischem Weg oxidiert. Dadurch entsteht DHCS.

*2. Schritt: Enzymatische oder mikrobielle Umsetzung von DHCS zu 12-Keto-UDCS*

In wässriger Lösung wird DHCS durch 3$\alpha$-HSDH und 7$\beta$-HSDH bzw. Mutanten davon spezifisch zu 12-Keto-UDCS in Anwesenheit von NADPH bzw. NADH reduziert. Der Kofaktor NADPH bzw. NADH kann durch eine ADH bzw. FDH bzw. GDH bzw. Mutanten davon von Isopropanol bzw. Natium-formiat bzw. Glucose regeneriert werden. Die Reaktion läuft unter milder Bedingung. Beispielsweise kann die Reaktion bei pH = 6 bis 9, insbesondere etwa pH = 8 und bei etwa 10 bis 30, 15 bis 25 oder etwa 23°C durchgeführt werden. Im falle eines mikrobiellen Umsetzungsschrittes können rekombinante Mikroorganismen, welche die erforderliche(n) Enzymaktivität(en) exprimieren, in Gegenwart des umzusetzenden Substrates (DHCS) anaerob oder aerob in geeigneten Flüssigmedien kultiviert werden. Geeignete Kultivierungsbedingungen sind dem Fachmann an sich bekannt. Sie umfassen Umsetzungen im pH Bereich von beispielsweise 5 bis 10 oder 6 bis 9, bei Temperaturen im Bereich von 10 bis 60 oder 15 bis 45 oder 25 bis 40 oder 37°C. Geeignete Medien umfassen z.B. die unten beschriebenen LB und TB Medien. Die Umsetzungsdauer kann dabei z.B. batchweise oder kontinuierlich oder in sonstigen üblichen Verfahrensvarianten erfolgen (wie oben beschrieben). Die Umsetzungsdauer kann dabei z.B. im Bereich von Minuten bis mehreren Stunden oder Tagen liegen, und z.B. 1 h bis 48 h betragen. Gegebenenfalls kann, wenn Enzymaktivität nicht kontinuierlich exprimiert wird, diese durch Zugabe eines geeigneten Induktors, nach Erreichen einer Zielzelldichte, z.B. von etwa $OD_{600}$ = 0,5 bis 1,0, eingeleitet werden.

Weitere mögliche geeignete Abwandlungen des mikrobiellen Herstellungsverfahrens hinsichtlich Fahrweise der Fermentation, Zusätze zum Medium, Enzymimmobilisierung und Isolierung der Wertstoffe sind auch dem folgenden Abschnitt betreffend "Herstellung der Enzyme bzw. Mutanten" zu entnehmen

*3. Schritt Chemische Umsetzung von 12-Keto-UDCS zu UDCS*

Die 12-Carbonylgruppe von 12-Keto-UDCS wird mittels Wolff-Kishner-Reduktion in an sich bekannter Weise entfernt, dadurch entsteht UDCS aus 12-Keto-UDCS. In der Reaktion wird zuerst die Carbonylgruppe mit Hydrazin zum Hydrazon umgesetzt. Anschließend wird das Hydrazon in Gegenwart einer Base (z.B. KOH) auf 200°C erhitzt, hierbei wird Stickstoff abgespaltet und UDCS entsteht.

6. Rekombinante Herstellung der Enzyme und Mutanten

Gegenstand der Erfindung sind weiterhin Verfahren zur rekombinanten Herstellung erfindungsgemäßer Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl, Sonnenblumenöl, Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen werden. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

7. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

## Experimenteller Teil:

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wie bei Sambrook et al. (1989) a.a.O. beschrieben durchgeführt werden.

### A. Allgemeine Angaben

### Materialien:

Die genomische DNA von *Collinsella aerofaciens* DSM 3979 (ATCC 25986, frühere Bezeichnung *Eubacterium aerofaciens*) wurde von der Deutschen Sammlung für Mikroorganismen und Zellkulturen (DSMZ) bezogen. UDCS und 7-Keto-LCS sind an sich bekannte und in der Literatur beschriebene Ausgangsverbindungen. Alle übrigen Chemikalien wurden von Sigma-Aldrich und Fluka (Deutschland) bezogen. Sämtliche Restriktionsendonukleasen, die T4 DNA Ligase, die *Taq* DNA Polymerase, die *Phusion* DNA Polymerase und Isopropyl-β-D-1-thiogalactopyranosid (IPTG) wurden von Fermentas (Deutschland) bezogen.

### Medien:

LB-Medium, enthaltend Trypton 10 g, Hefeextrakt 5 g, NaCl 10 g pro Liter Medium

TB-Medium, enthaltend Trypton 12 g, Hefeextrakt 24 g, 4 mL Glycerin, 10 % TB.-Puffer (11,55 g $KH_2PO_4$, 62,7 g $K_2HPO_4$, ad 500 mL $H_2O$) pro Liter Medium

Minimalmedium, modifiziert nach Wilms et al., BIOTECHNOLOGY AND BIOENGI-NEERING, 2001 VOL. 73, NO. 2, 95-103

### Expressionsvektoren und Vektorkonstrukte

Zur Expression rekombinanter Proteine wurden folgende an sich bekannte Expressionsvektoren verwendet:

**pET21a(+),** (vgl. Fig. 3a)
**pET22b(+)** (vgl. Fig. 3b)
**pET28a(+)** (vgl. Fig. 3d)und
**pCOLADuet-1**

(jeweils Novagen, Madison, Wisconsin, USA).

Die Vektoren **pET21a(+)** und **pET22b(+)** besitzen jeweils eine *Multiple cloning site* (MCS), die jeweils unter der Kontrolle eines T7-Promotors mit nachgeschaltetem *lac*-Operator und der darauf folgenden Ribosomalen Bindestelle (rbs) stehen. Im C-terminalen Bereich der Expressionsregion befindet sich jeweils ein T7-Terminator. Beide Plasmide verfügen über ein ColE1-Replicon (pBR322-Replicon), ein Ampicillin-Resistenzgen (*bla*), einen f1-Ursprung und ein Gen, welches den *lac*-Inhibitor codiert (lacI).

Zusätzlich verfügt das pET21a(+)-Plasmid über einen T7·Tag im *N*-terminalen Bereich der MCS und einen optionalen His·Tag C-terminal der MCS. Das pET22b(+)-Plasmid verfügt im *N*-terminalen Bereich der MCS über eine pelB-Signalsequenz und einen His·Tag C-terminal von der MCS.

Der **pCOLADuet-1-**Vektor verfügt über zwei MCS, die jeweils unter der Kontrolle eines T7-Promotors mit nachgeschaltetem *lac*-Operator und nachfolgender Ribosomalen Bindestelle (rbs) stehen. C-terminal der beiden MCS befindet sich ein T7-Terminator. Des Weiteren verfügt dieser Vektor über ein Gen, welches den *lac*-Inhibitor codiert sowie über ein COLA-Replicon (ColA-Replicon).

In der vorliegenden Arbeit wurde eine modifizierte Variante des kommerziell erhältlichen pCOLADuet-1-Vektors verwendet. Bei dieser modifizierten Plasmidvariante (bezeichnet als **pCOLA(mod); vgl. Fig. 3c)**) wurde das Kanamycin-Resistenzgen des ursprünglichen Vektors durch ein Chloramphenicol-Resistenzgen ersetzt. Zudem wurde aus dem Chloramphenicol-Resistenzgen eine *Nco*I-Restriktionsschnittstelle mittels positionsgerichtete Punktmutagenese entfernt. Im *N*-terminalen Bereich der ersten MCS befindet sich ein His·Tag, während sich C-terminal der zweiten MCS ein S·Tag befindet.

Die ColE1-Replicons der pET-Plasmide und das COLA-Replicon des pCOLA-Plasmids sind miteinander kompatibel. Das ermöglicht das gleichzeitige stabile Einbringen eines pET-Plasmids und eines pCOLA-Plasmids in *Escherichia coli.* Somit können Kombinationen verschiedener Gene in *Escherichia coli* einkloniert werden, ohne dass diese

auf demselben Operon platziert sind. Bedingt durch unterschiedliche Kopienzahlen von pET-Vektoren (~40) und pCOLA-Vektoren (20-40) lassen sich zudem die Expressionslevel kotransformierter Gene beeinflussen.

Folgende Vektorkonstrukte wurden verwendet

**pET22b(+) 7β-HSDH:** ein pET22b(+)-Vektor in das die 7β-HSDH aus *Collinsella aerofaciens* ATCC 25986 über die Schnittstellen *Nde* I und *Hind* III in üblicher Weise einkloniert wurde.

**pET22b(+) 3α-HSDH:** ein pET22b(+)-Vektor in das die 3α-HSDH aus *Comamonas testosteroni* über die Schnittstellen *Nde* I und *EcoR* I in üblicher Weise einkloniert wurde (Oppermann et al.,J Biochem, 1996, 241(3):744-749).

**pET21a(+) FDH D221G** (vgl. Fig. 4a): ein pET21a(+)-Vektor in das die Formiatdehydrogenase aus *Mycobacterium vaccae* N10 über die Schnittstellen *Nde* I und *Eco*R I einkloniert wurde. Durch ortsgerichtete Mutagenese wurde der Aspartatrest (D) an Position 221 (ohne Berücksichtigung von Methionin in Position 1) bzw. Position 222 (gerechnet ab Metionin in Position 1; vgl SEQ ID NO: 15, 19, 35) der Formiatdehydrogenase durch einen Glycinrest ersetzt (vgl. Herstellungsbeispiel 4, unten). Die Formiatdehydrogenase trägt an der Position 1202 der Nukleotidsequenz eine einzelne Basendeletion, was zum Austausch der letzten Aminosäure Valin gegen ein Alanin führt. Gleichzeitig führt diese Basendeletion zum Ausschalten des Stoppcodons und zur Aktivierung des ursprünglich außerhalb des Leserahmens liegenden His·Tags (vgl. SEQ ID NO: 34 und 35).

**pET21a(+) 7β-HSDH:** ein pET21a(+)-Vektor, in den die 7β-HSDH aus *Collinsella aerofaciens* ATCC 25986 über die Schnittstellen *Nde* I und *Xho* I in üblicher Weise einkloniert wurde.
**pET21a(+) FDH D221G 7β-HSDH** (vgl. Fig. 4b)
**pET21a(+) FDH 7β-HSDH(G39A) 3α-HSDH** (vgl. Fig. 8)
**pCOLA(mod) 3α-HSDH** (vgl. Fig. 5)

**Mikroorganismen**

| Stamm | Genotyp |
|---|---|
| Escherichia coli DH5α | $F^-$endA1 glnV44 thi-1 recA1 relA1 gyrA96 deoR nupG Φ80dlacZΔM15 Δ(lacZYA-argF)U169, hsdR17($r_K^-$ $m_K$+),λ- |
| *Escherichia coli* BL21 (DE3) | $F^-$ ompT gal dcm Ion hsd$S_B$($r_B^-m_B^-$) λ(DE3 [lacl lacUV5-T7 gene 1 ind1 sam7 nin5]) |
| Escherichia coli BL21 (DE3) hdhA$^-$ KanR$^+$ (7α-HSDH Knock-out Stamm) (vgl. Herstellungsbeispiel 5) | $F^-$ ompT gal dcm Ion hsd$S_B$($r_B^-m_B^-$) λ(DE3 [lacl lacUV5-T7 gene 1 ind1 sam7 nin5]) hdhA$^-$KanR$^+$ |

Der *Escherichia coli* Stamm DH5α (Novagen, Madison, Wisconsin, USA) wurde bei 37°C in LB-Medium, enthaltend geeignete Antibiotika, vermehrt.

Der *Escherichia coli* Stamm BL21 (DE3) (Novagen, Madison, Wisconsin, USA) wurde bei 37°C in LB-Medium, enthaltend geeignete Antibiotika, vermehrt und wurde nach Induktion bei $OD_{600}$ = 0.8 mit 0.5 mM IPTG bei 25°C und 140 Upm gehalten.

**Methoden**

1. Standardbedingungen für 7ß-HSDH Aktivitätsbestimmung

Die Reaktionsmischung enthält ein Gesamtvolumen von 1 ml:

| | |
|---|---|
| 870 µl | 50 mM Kaliumphosphat Puffer, pH 8.0 |
| 10 µl | 10 mM UDCS (gelöst in Wasser, pH 8) |
| 10 µl | Enzymlösung (in Puffer wie oben, im Bereich von 1 zu 10 U/ml) |
| 100 µl | 1 mM NADP+ (in Puffer wie oben) |

Die Zunahme der Extinktion bei 340 nm wird gemessen und die Aktivität wird als Enzym-Unit (U, i.e. µmol/min) unter Verwendung des molaren Extinktionskoeffizienten von 6.22 $mM^{-1} \times cm^{-1}$, berechnet.

Standardbedingungen für 7ß-HSDH Aktivitätsbestimmung gemäß Herstellungsbeispiel 7

Die Reaktionsmischung enthält ein Gesamtvolumen von 1 ml

| | |
|---|---|
| 870 µl | 50 mM Kaliumphosphat (KPi) Puffer, pH 8.0 |
| 100 µl | 100 mM DHCA (gelöst in 50 mM KPi, pH 8) |
| 10 µl | Enzymlösung (in Puffer wie oben, im Bereich von 2 zu 6 U/ml) |
| 20 µl | 12,5 mM NADPH (gelöst in $ddH_2O$) |

Die Zunahme der Extinktion bei 340 nm wird gemessen und die Aktivität wird als Enzym-Unit (U, i.e. µmol/min) unter Verwendung des molaren Extinktionskoeffizienten von 6.22 $mM^{-1} \times cm^{-1}$, berechnet

2. Proteinbestimmung mittels BCA-Assay

Die Proben wurden mit BCA-Reagenz (von Interchim) gemischt und bei 37°C für 45 min inkubiert. Der Proteingehalt wurde bei 562 nm gegen einen Kalibrationskurve (BSA) im Konzentrationsbereich des verwendeten Assay bestimmt.

3. Dünnschichtchromatographie

5 bis 10 µg Probe wurden auf eine DC-Folie Kieselgel 60 (Merck) aufgetragen. Authentische Substanzen wurden als Referenz aufgetragen. Ein Ende der DC-Folie wurde in Laufmittel eingetaucht bis die Mobilphase oben erreicht. Die DC-Folie wurde getrocknet und mittels Molybdatophosphorsäure entwickelt.

4. Molekularbiologische Arbeitsmethoden:

Molekularbiologische Arbeiten erfolgen, soweit keine anderen Angaben gemacht werden, in Anlehnung an etablierte Methoden, z.B. beschrieben in:

Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989; Ausubel et al. (eds.), Current Protocols in Molecular Biology, John Wiley &Sons, NY (1993); Kriegler, Gene Transfer and Expression, A Laboratory Manual, Stockton Press, NY (1990).

## B. Beispiele

### Herstellungsbeispiel 1: Identifizierung der 7ß-HSDH Aktivität

Die genomische DNA-Sequenz von *Collinsella aerofaciens* ATCC 25986 wurde im Jahr 2007 von "Washington University Genome Sequencing Center" für das "human gut microbiome project" bei GenBank veröffentlicht. HSDHs gehören zu den "Short-Chain Dehydrogenasen". Da die biochemische Funktion der "Short-Chain Dehydrogenasen" aus *Collinsella aerofaciens* ATCC 25986 nicht in GenBank annotiert wurde, wurden 9 Kandidaten in Vektor pET22b+ einkloniert, und anschließend in *E. coli* BL21 (DE3) exprimiert.

Dazu wurden 7ß-HSDH kodierende Sequenzen PCR-amplifiziert. Die PCR-Produkte erhielt man unter Verwendung der genomischen DNA von *Collinsella aerofaciens* ATCC 25986 (DSM 3979) als Template und den Primer 5'-gggaattcCATATGAACCTGAGGGAGAAGTA-3' (SEQ ID NO:3) und 5'- cccAAGCTTCTAGTCGCGGTAGAACGA-3' (SEQ ID NO:4). Die *Nde*I und *Hin*dIII Schnittstellen in den Primer-Sequenzen sind unterstrichen. Das PCR-Produkt wurde mittels PCR-Purification-Kit (Qiagen) aufgereinigt und dann mit den Enzymen *Nde*I und *Hin*dIII geschnitten. Der entsprechende Vektor wurde auch mit den *Nde*I und *Hin*dIII geschnitten. Die Produkte wurden auf ein Agarose-Gel aufgetragen, aufgetrennt, aus diesem ausgeschnitten und aufgereinigt. Das geschnittene PCR-Produkt und der geschnittene Vektor wurden mittels T4-Ligase ligiert. Das Ligant wurde in *E. coli* DH5α transformiert. Der entstehende Vektor (enthält das Gen von 7ß-HSDH) wurde durch Sequenzierung bestätigt und in *E. coli* BL21 (DE3) transformiert und mit IPTG induziert und exprimiert.

Die Expression wurde in 50 ml LB-Medium durchgeführt. Zur Vorbereitung von Vorkultur wurde eine Kolonie auf LB-Agar-Platte in 5 ml LB-Medium (enthält entsprechende Antibiotika) gepickt und bei 37°C und 160 Upm über Nacht inkubiert. Das 50 ml LB-Medium (enthält entsprechende Antibiotika) wurde mit 500 µl Vorkultur angeimpft. Die Kultur wurde bei 37°C und 160 Upm inkubiert. Bis OD600 ca. 0,8 wurde die Expression durch Zugabe von 0,5 mM IPTG

induziert. Nach 6 h oder über Nacht wurden die Zellen abzentrifugiert. Die Pellets wurden in 6 ml Kaliumphosphat-Puffer (50 mM, pH 8, enthält 0,1 mM PMSF) resuspendiert und mittels Ultraschall aufgeschlossen. Die Zelltrümmer wurden durch Zentrifugation entfernt.

Zur Identifizierung 7β-HSDH-Aktivität wurde die Aktivität mittels photometrischer Methode untersucht. In einer 1 ml Küvette wurde hierzu Enzym und 0,1 mM Testsubstanz (UDCS) in Kaliumphosphat Puffer (50 mM, pH 8) gemischt. Nach Zugabe von NADPH(NADH) bzw. $NADP^+$($NAD^+$) wurde die Abbau oder Bildung von NAD(P)H gemessen. Ein Enzym von den 9 Kandidaten zeigt Aktivität (60 U/ml) gegen UDCS in Anwesenheit von $NADP^+$, aber keine Aktivität gegen CS. Die NADPH-abhängige 7β-HSDH-Aktivität von diesem Enzym wurde identifiziert.

In der photometrischen Untersuchung zeigte die 7β-HSDH die Aktivität 60 U/ml gegen UDCS, Aktivität 35 U/ml gegen 7-Keto-LCS und Aktivität 119 U/ml gegen DHCS in Anwesenheit von $NADP^+$ bzw. NADPH. Die Aktivität gegen CS ist nicht nachweisbar.

Das Gen, das die 7β-HSDH codiert, wurde in pET28a+ mit His-Tag umkloniert, um eine schnelle Reinigung zu ermöglichen. Diese 7β-HSDH mit His-Tag wurde aktiv in *E. coli* BL21(DE3) exprimiert wie oben beschrieben wurde. Die Aufreinigung wurde mit einer Talon-Säule durchgeführt. Die Säule wurde zuerst mit Kaliumphosphat-Puffer (50 mM, pH 8, mit 300 mM NaCl) equilibriert. Nachdem das Zelllysat geladen worden ist, wurde die Säule mit Kaliumphosphat-Puffer (50 mM, pH 8, mit 300 mM NaCl) gewaschen. Die 7β-HSDH wurde mit Kaliumphosphat-Puffer (50 mM, pH 8, mit 300 mM NaCl und 200 mM Imidazol) eluiert. Das Imidazol im Eluat wurde mittels Dialyse entfernt. Die Ausbeute der Aufreinigung betrug 76 % mit Reinheit ca. 90 %.

**Herstellungsbeispiel 2: Klonierung, Expression und Reinigung 7β-HSDH aus *Collinsella aerofaciens* ATCC 25986 in präparativem Maßstab sowie weitere Charakterisierung des Enzyms**

**2.1 Klonierung und Herstellung eines Expressionskontruktes**

Das für 7β-HSDH kodierende Gen wurde wiederum aus der genomischen DNA durch PCR und unter Verwendung von Primern, wie oben für Herstellungsbeispiel 1 beschrieben, amplifiziert:
Das PCR-Produkt wurde wiederum wie oben beschrieben gereinigt und mit den Restriktionsendonukleasen Ndel und Hindlll verdaut. Das verdaute PCR-Produkt wurde wiederum gereinigt und in den pET-28a(+)-Vektor unter Verwendung der T4-Ligase kloniert, um einen Expressionsvektor zu erzeugen. Das resultierende Expressionskonstrukt wurde anschließend in *E. coli* DH5α-Zellen transformiert. Das zu erwartende Protein sollte 20 Aminosäurereste umfassendes ein Signalpeptid und einen N-terminalen 6xHis-Tag sowie eine Thrombin-Schnittstelle aufweisen. Die Sequenz der insertierten DNA wurde durch Sequenzierung überprüft.

**2.2 Überexpression und Reinigung von 7ß-HSDH**

*E. coli* BL21(DE3) wurde mit dem Expressionskonstrukt transformiert. Dazu wurde der das Expressionskonstrukt enthaltende *E. coli* BL21 (DE3)-Stamm in LB-Medium (2 × 400 ml in 2 Liter-Schüttelflaschen) enthaltend 30 μg/ml Kanamycin vermehrt. Die Zellen wurden durch Zentrifugation (10,000×g, 15 min, 4°C) geerntet. Das Pellet wurde in 20 ml Phosphatpuffer (50 mM, pH 8, enthaltend 0.1 mM PMSF) resuspendiert. Die Zellen wurden unter konstanter Kühlung durch einminütige Ultraschallbehandlung (40 W Leistung, 40% Arbeitsintervall und 1 min Pause) unter Verwendung eines Ultraschallgeräts Sonifier 250 (Branson, Deutschland) aufgeschlossen. Der Aufschluss wurde dreimal wiederholt. Das Zellextrakt wurde zentrifugiert (22,000×g, 20 min, 4°C). Der Überstand wurde auf eine Talon-Säule (Clontech, USA), äquilibriert mit Beladungspuffer (50 mM Kaliumphosphat, 300 mM NaCl, pH 8) beladen. Das Verfahren wurde bei 24°C durchgeführt. Nicht gebundenes Material wurde durch Waschen der Säule mit Beladungspuffer (3 Säulenvolumina) ausgewaschen. Schwach bindendes Protein wurde durch Waschen mit Waschpuffer (20 mM Imidazol im Beladungspuffer; 3 Säulenvolumina) entfernt. Das His-Tag-7β-HSDH-Protein wurde mit Elutionspuffer (200 mM Imidazol im Beladungspuffer) eluiert. Das Eluat wurde in einem Dialyseschlauch mit einer molekularen Ausschlussgrenze von 5 kDa (Sigma, USA) in 2 Liter Kaliumphosphatpuffer (50 mM, pH 8) bei 4°C über Nacht dialysiert. Schließlich wurde die Probe in einen neuen Schlauch überführt und bei -20°C zur weiteren Analyse gelagert. Die Proteinkonzentration wurde unter Verwendung eines BCA Testkits (Thermo, USA) nach Herstellerangaben bestimmt. Außerdem wurde die Probe durch 12.5%ige SDS-PAGE und Färben mit Coomassie Brilliant Blue analysiert. Die Reinheit des Proteins wurde densitometrisch mit Hilfe von Scion Image Beta 4.0.2 (Scion, USA) bestimmt.

**2.3 Gelfiltration**

Gelfiltration wurde auf einem Pharmacia ÄKTA-Protein-Reinigungssystem durchgeführt, um das Molekulargewicht von 7β-HSDH zu bestimmen. Das gereinigte Enzym wurde auf eine Sephadex G-200-Säule aufgetragen, welche zuvor mit 50 mM Tris-HCl (pH 8), enthaltend 200 mM Natriumchlorid, äquilibriert worden war. Das Protein wurde mit

dem gleichen Puffer bei einer Flussrate von 1 ml/min eluiert. Das Molekulargewicht von 7β-HSDH wurde durch Vergleich seines Elutionsvolumens mit demjenigen von Proteinstandards (Serum Albumin (66 kDa), α-Amylase aus *Aspergillus oryzae* (52 kDa), Trypsin aus Schweinepankreas (24 kDa) und Lysozym aus Hühnerei (14.4 kDa)) bestimmt.

### 2.4 Enzymtest und kinetische Analyse

Das Reaktionsgemisch für den Enzymtest enthielt in einem Gesamtvolumen von 1 ml 50 μmol Kaliumphosphat (pH 8), 0.1 μmol NAD(P)H oder $NAD(P)^+$, Substrate und Protein. Das Reaktionsgemisch war in Küvetten mit einer Lichtpfadlänge von 1 cm enthalten. Die 7ß-HSDH-Aktivität wurde durch Aufzeichnung der Veränderung in der NAD(P)H-Konzentration über die Extinktion bei 340 nm mit Hilfe eines Spektrophotometers (Ultraspec 3000, Pharmacia Biotech, Großbritannien) bestimmt. Die Enzymaktivitäten wurden als Enzymeinheiten (U, d.h. μmol/min) unter Verwendung des molaren Extinktionskoeffizienten von 6,22 $mM^{-1} \times cm^{-1}$ bei 25°C bestimmt. Mehrere unterschiedliche Messungen mit den Variablen Substrat, Koenzym, Konzentration, pH-Wert, Puffer und Inkubationstemperatur wurden durchgeführt. Die kinetischen Konstanten wurden unter Verwendung von Standardmethoden bestimmt.

### 2.5 Biotransformation von 7-keto-Lithocholsäure durch die 7β-HSDH

Die Umwandlung von 7-Keto-LCS durch 7β-HSDH wurde durchgeführt, um die biochemische Funktion von 7β-HSDH zu verifizieren. 0.4 g 7-Keto-LCS wurden in 10 ml Kaliumphosphatpuffer (50 mM, pH 8) suspendiert und der pH-Wert wurde auf pH 8 durch Zugabe von 2 M Natriumhydroxid eingestellt. 0.2 ml Isopropanol, 100 U 7β-HSDH und 80 U Alkoholdehydrogenase (ADH-TE) aus *Thermoanaerobacter ethanolicus* (freundliche Zuwendung von Frau Dr. K. Momoi, ITB Universität, Stuttgart) und 1 μmol $NADP^+$wurden zugesetzt. Der gleiche Puffer wurde zugesetzt, um ein gesamtes Reaktionsvolumen von 20 ml zu erhalten. Das Reaktionsgemisch wurde bei 24°C inkubiert und 24 Stunden gerührt. Dabei wurde NADPH mit Hilfe der ADH über die Oxidation von 2-Propanol regeneriert. Das Produkt wurde mit 1 ml 2 M Salzsäure angesäuert und 5 × mit 5 ml Ethylacetat extrahiert. Die organische Lösung wurde anschließend destilliert.

### 2.6 Chromatographische Produktbestimmung

Eine HPLC-Analyse wurde auf einer Säule vom Typ Purospher® STAR RP-18 (Hitbar® RT 125-4 Pre-Packed Column, Purospher® STAR RP-18 endcapped, Merck, Deutschland), versehen mit einer Vorsäule des Typs LiChro-CART® STAR RP18 (endcapped, Merck, Deutschland) auf einem HPLC-System LC20AD (Shimadzu, Japan) bei einer Flussrate von 1 ml/min durchgeführt. Die mobile Phase bestand aus zwei Eluenten. Eluent A enthielt Acetonitril und Eluent B destilliertes Wasser (pH 2.6, eingestellt mit Orthophosphorsäure, 85%). Folgender Gradient wurde verwendet: A 35% (8 min) - 35%-43% (1% $min^{-1}$) - 43%-70% (1% $min^{-1}$) - 70% (5 min) - 70%-35% (17.5% $min^{-1}$) - 35% (5 min); eluent A 65% (8 min) - 65%-57% (1% $min^{-1}$) - 57%-30% (1% $min^{-1}$) - 30% (5 min) - 30%-65% (17.5% $min^{-1}$) - 65% (5 min). 20 μl Probe (1 mg/ml) wurden analysiert. Authentische UDCS, 7-Keto-LCS und CDCS wurden bei gleicher Konzentration als Standards verwendet. Die Aufzeichnung erfolgte durch UV-Detektion bei 200 nm.

### 2.7 Sequenzalignment und phylogenetische Analyse

Multiple Sequenz-Alignments wurden unter Verwendung der Clustal X-Software (Thompson et al., 1997, Nucleic Acid Research 25: 4876-82) erzeugt und modifiziert unter Verwendung der Jalview-Software (Clamp et al., 2004, Bioinformatics 20:426-7). Der phylogenetische Stammbaum wurde unter Verwendung des Programms TreeView 1.6.6 (Roderic 2001, http://taxonomy.zoology.gla.ac.uk/rod/rod.html) erzeugt.

### 2.8 Versuchsergebnisse:

#### 2.8.1 Identifizierung der 7ß-HSDH-Aktivität in einer präparativen Biotransformation

Um die Funktion des Enzyms zu bestätigen, wurde eine Biotransformation von 7-Keto-LCS im 10 ml-Maßstab durchgeführt, wobei das isolierte Enzym in Kombination mit ADH zur Regeneration von NADPH unter Verwendung von 2-Propanol, wie bereits beschrieben, eingesetzt wurde. Die HPLC-Analyse ergab, dass UDCS das einzige von dem Enzym produzierte Reaktionsprodukt (90% Umsatz) war. CDCS (Retentionszeit 19,4 min) wurde im Reaktionsgemisch nicht nachgewiesen. Das Ergebnis zeigt, dass das Enzym eine NADPH-abhängige 7β-HSDH darstellt und zur selektiven Reduktion der 7-Carbonyl-Gruppe von 7-Keto-LCS in eine 7β-Hydroxy-Gruppe befähigt ist.

Retentionszeit UDCS: 15,5 min

Retentionszeit 7-keto-LCS: 18,3 min

#### 2.8.2. Reinigung und Gelfiltration

Nach Klonierung des 7β-HSDH-Gens aus *Collinsella aerofaciens* DSM 3979 in den Expressionsvektor pET28a(+) und anschließende Überexpression erhielt man ein am N-terminus mit einem His-Tag versehenes Fusionsprotein mit einer 7β-HSDH-Ausbeute von 332.5 mg (5828 U) pro Literkultur. Das mit dem His-Tag versehene 7β-HSDH wurde in einer Stufe mit Hilfe einer Immobilisierte-Metallionen-Affinitätschromatographie gereinigt (Reinheit >90%, Ausbeute 76%, vgl. Fig. 2.). Die Hauptbanden der Bahnen 1 und 2 repräsentieren das erwartete Expressionsprodukt bei 30 kDa, welches dem aus der Aminosäuresequenz des Gens abgeleiteten vorhergesagten Molekulargewicht entspricht. Durch Gelfiltration ermittelt man jedoch für die 7β-HSDH ein Molekulargewicht von 56.1 kDa. Dies belegt die dimere Beschaffenheit der 7β-HSDH aus *Collinsella aerofaciens* DSM 3979.

#### 2.8.3. Sequenzalignments

Die Aminosäuresequenz der erfindungsgemäß verwendeten 7β-HSDH wurde mit bekannten HSDH-Sequenzen verglichen (Alignment nicht gezeigt). Die beobachtete Sequenzähnlichkeit weist darauf hin, dass das erfindungsgemäße Enzym zur Familie der kurzkettigen Dehydrogenasen (SDR) gehört. Es ist bekannt, dass SDRs sehr niedrige Homologie und Sequenzidentität aufweisen (Jornvall, H., B. Persson, M. Krook, S. Atrian, R. Gonzalez-Duarte, J. Jeffery, and D. Ghosh. 1995. Short-chain dehydrogenases/reductases (SDR). Biochemistry 34:6003-13 und Persson, B., M. Krook, and H. Jornvall. 1991. Characteristics of short-chain alcohol dehydrogenases and related enzymes. Eur J Biochem 200:537-43). Das Sequenzalignment ergibt jedoch deutlich die konservierten Domänen in der SDR Primärstruktur. Das N-terminale Motiv Gly-X-X-X-Gly-X-Gly (entsprechend Gly-41, Gly-45 and Gly-47, Nummerierung entsprechend dem Alignment) entspricht dem charakteristischen Dinukleotid-Bindungsmotiv der SDR-Superfamilie. Darüber hinaus sind drei stark konservierte Reste Ser-177, Tri-190 und Lys-194 zu beobachten, die der katalytischen Triade der SDR-Enzyme entsprechen.

#### 2.8.4. Phylogenetische Analyse

7α-HSDH aus *Clostridium sordellii, Brucella melitensis* und *Escherichia coli* gehören zur gleichen Untergruppe. Die beiden 3α-HSDHs zeigen eine ausgeprägtere Verwandtschaft als andere HSDHs. Interessanterweise ist die prokaryotische 7β-HSDH mit der tierischen 11β-HSDH-Untergruppe, umfassend *Cavia porcellus, Homo sapiens* und *Mus musulus*, verwandt.

#### 2.8.5. Kinetische Konstanten

Kinetische Gleichgewichtsanalysen wurden durchgeführt, um die absoluten Werte für $V_{max}$ und $K_M$ für UDCS, 7-Keto-LCS, DHCS, $NADP^+$ und NADPH mit Hilfe von Lineweaver-Burk-Plots zu bestimmen. In folgender Tabelle sind sämtliche kinetische Daten für die getesteten Substrate und Coenzyme, die aus Substratsättigungskurven und reziproke Auftragungen erhalten wurden, zusammengefasst. Die $V_{max}$, $K_M$ and $K_{cat}$-Werte für sämtliche Substrate und Coenzyme liegen im gleichen Bereich, wohingegen der $K_M$-Wert für DHCS signifikant höher lag als bei den anderen Substraten, eventuell bedingt durch die geringe Löslichkeit in Wasser. Das Enzym ist NADPH-abhängig und kinetische Konstanten für $NAD^+$ und NADH konnten aufgrund der sehr geringen Aktivität nicht bestimmt werden.

Zusammenfassung von kinetischen Konstanten für 7β-HSDH aus *Collinsella aerofaciens* DSM 3979.

| | $K_M$ (μM) | $V_{max}$ (U/mg Protein)[b] | $k_{cat}$ (1 μmol/(μmol×min)) |
|---|---|---|---|
| $NADP^+$ | 5.32 | 30.58 | 944.95 |
| NADPH | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| $NAD^+$ | -[a] | - | Spuren |
| NADH | - | - | Spuren |

a) konnten aufgrund der sehr geringen Aktivität nicht bestimmt werden
b) 1 U = 1 μmol/min

### 2.8.6. pH-Optimum

Weiterhin wurde die 7β-HSDH-Aktivität für verschiedene Substrate in Abhängigkeit vom pH-Wert mit gereinigtem Enzym bestimmt. Für die Oxidation von UDCS mit 7β-HSDH wurde eine optimale Aktivität im Bereich von pH 9 bis 10 bei allmählichem Abfall auf der sauren Seite beobachtet. Im Gegensatz dazu ergab sich für die Reduktion von DHCS und 7-keto-LCS durch die 7β-HSDH eine optimale Aktivität im Bereich von pH 4 bis 6 mit einem scharfen Abfall auf der sauren Seite und einem allmählichen Abfall auf der alkalischen Seite. Unterschiedliche Puffer haben nur einen geringen Einfluss auf die Aktivität der 7β-HSDH bei gleichem pH-Wert.

### 2.8.7. Thermische Stabilität

Die erfindungsgemäß verwendete, NADP-abhängige 7β-HSDH zeigt folgendes Stabilitätsverhalten: Nach 400 min war die Aktivität bei 30°C etwa 30 % niedriger als bei 23°C. Das Enzym war bei 30°C nach 1500 min vollständig inaktiviert, während bei 23°C und 1500 min die verbleibende Aktivität 20% betrug. Kein signifikanter Aktivitätsverlust wurde während der Lagerung bei -20°C in Kaliumphosphatpuffer (50 mM, pH 8) über einen Zeitraum von einigen Monaten nach mehrmaligem Einfrieren und Auftauen beobachtet.

### Herstellungsbeispiel 3: Herstellung von 7ß-HSDH Mutanten und deren Charakterisierung

Es wurde die Position 39 der Aminosäuresequenz (beinhaltend Start-Methionin) (vgl. SEQ ID NO: 2) mutiert.

### 3.1 Primer

Für die positionsgerichtete Mutagenese der 7ß-HSDH wurden die unten angegeben Mutageneseprimer verwendet. Die Primer wurden auf Grundlage der 7ß-HSDH-Gensequenz dahingehend ausgewählt, dass sie den gewünschten Aminosäurenaustausch bewirken. Es wurde darauf geachtet, dass die zu mutierende Base im Primer mittig lokalisiert ist, und dass die Schmelztemperaturen der Primerpaare sich im gleichen Bereich befinden.

Zur Erzeugung der G39A-Mutante wurde das Primerpaar 7beta_mut_G39A_fwd und 7beta *mut_G39A_*rev verwendet. Zur Erzeugung der G39S-Mutante wurde das Primerpaar 7beta_mut_G39S_fwd und 7beta_mut_G39S_rev verwendet.

*Glycin → Alanin*

Forward: 7beta_mut_G39A_fwd: CGTCGTCATGGTCGCCCGTCGCGAGG. SEQ ID NO: 9)

Revers: 7beta_mut_G39A_rev: CCTCGCGACGGGCGACCATGACGACG. SEQ ID NO: 10)

*Glycin → Serin*

Forward: 7beta_mut_G39S_fwd: CGTCGTCATGGTCAGCCGTCGCGAGG. SEQ ID NO: 11)

Revers: 7beta_mut_G39S_rev: CCTCGCGACGGCTGACCATGACGACG. SEQ ID NO: 12)

### 3.2 PCR-Programm

Bei der Reaktion erfolgte zuerst ein 2 min langer initialer Denaturierungsschritt bei 98°C. Danach wurden 25 Zyklen von Denaturierung (30 s bei 98°C), Primerhybridisierung (2,5 min bei 58°C) und Elongation (6 min bei 72°C) durchgeführt. Als letzter Schritt erfolgte eine finale Elongation von 15 min bei 72°C bevor die Polymerasekettenreaktion durch Kühlen auf 4°C beendet wurde.

### 3.3 PCR-Ansatz

| | |
|---|---|
| HF-Puffer (5x) | 4 µl |
| dNTP-Mix (10 mM) | 0,4 µl |
| *Forward*-Primer (10 µM) | 2 µl |
| *Revers*-Primer (10 µM) | 2 µl |
| Templat | 1 µl |
| *Phusion* Polymerase (2 U $\mu L^{-1}$) | 0,2 µl |
| DMSO | 1 µl |
| $ddH_2O$ | 9,4 µl |
| | **20 µl** |

Als Templat wurde ein pET22b-Vektor mit der 7β-HSDH verwendet

### 3.4 Durchführung

Um Aminosäuren in Proteinsequenzen gezielt austauschen zu können wird die DNA-Sequenz des entsprechenden Gens positionsgerichtet mutiert. Hierzu werden zueinander komplementäre Primer genutzt, die die gewünschte Mutation in ihrer Sequenz tragen. Als Templat dient N6-adeninmethylierte, doppelsträngige Plasmid-DNA, die das zu mutierende Gen tragen. N6-adeninmethylierte Plasmid-DNA werden aus $dam^+$ *E. coli*-Stamm wie zum Beispiel *E. coli* DH5 isoliert.

Die Polymerasekettenreaktion wird wie oben beschrieben durchgeführt. Dabei werden die Primer komplementär zum Templat verlängert, wodurch Plasmide mit der gewünschten Mutation entstehen, die einen Strangbruch aufweisen. Anders als bei anderen PCR-Reaktionen steigt die DNA-Ausbeute hierbei nur linear, da neu gebildete DNA-Moleküle nicht als Templat für die PCR-Reaktion dienen können.

Nach Abschluss der PCR-Reaktion wird die parentale, N6-adeninmethylierte DNA mit Hilfe des Restriktionsenzyms Dpnl verdaut. Dieses Enzym hat die Besonderheit, dass es unspezifisch N6-adeninmethylierte DNA restringiert, jedoch nicht die neu gebildete, nichtmethylierte DNA. Die Restriktion erfolgte, indem 1 µL Dpnl zum PCR-Reaktionsansatz hinzu gegeben und 1 h bei 37°C inkubiert wurde.

10 µl dieses Ansatzes wurden zur Transformation von 200 µl chemisch-kompetenten DH5α-Zellen verwendet. Nach Plasmidisolation wurde die erfolgreiche Mutation mittels Sequenzierung bestätigt.

### 3.5 Charakterisierung

Für jede der Mutanten sowie für das Wildtyp-Enzym wurden Kinetikmessungen im Mikrotiterplattenphotometer durchgeführt, indem die Umsatzraten der Enzyme sowohl mit gleichbleibenden Substratkonzentrationen bei variierten Cofaktorkonzentrationen als auch *vice versa* aufgezeichnet wurden. Zur Bestimmung der spezifischen Enzymaktivität wurden die Enzymkonzentrationen im Zelllysat densitometrisch bestimmt.

Zur Ermittlung der Abhängigkeit der Substratumsatzgeschwindigkeit von der Substratkonzentration wurde eine Cofaktorkonzentration von 100 µM NADPH bezogen auf das Reaktionsvolumen eingesetzt, während die Substratkonzentration über einen Bereich von 7 µM bis 10 mM Dehydrocholsäure mit 31 verschiedenen Konzentrationen variiert wurde (jeweils bezogen auf den Reaktionsansatz).

Zur Ermittlung der Abhängigkeit der Substratumsatzgeschwindigkeit von der Cofaktorkonzentration wurde eine Substratkonzentration von 0,3 mM Dehydrocholsäure bezogen auf das Reaktionsvolumen eingesetzt, während die Cofaktorkonzentration über einen Bereich von 25 µM bis 100 µM NADPH mit 8 verschiedenen Konzentrationen beim Wildtypprotein bzw. über einen Bereich von 6 µM bis 100 µM NADPH mit 16 verschiedenen Konzentrationen bei den beiden Mutanten variiert wurde (jeweils bezogen auf den Reaktionsansatz). Die Reaktionsgeschwindigkeit wurde bei diesen Messreihen durch lineare Regression über die ersten 4 Messungen (0 s - 18 s) ermittelt.

Die erhaltenen Daten wurden mit IGOR Pro ausgewertet. Aus den Auftragungen der Substrat- bzw. Cofaktorkonzentration gegen die spezifische Enzymaktivität lässt sich für die Messreihen bei konstanten Substrat- und variierten Cofaktorkonzentrationen der typische Kurvenverlauf einer Michaelis-Menten-Kinetik erkennen. Aus den Auftragungen der Messreihen mit konstanten Cofaktor- und variierten Substratkonzentrationen können hingegen typische Kurvenverläufe einer Michaelis-Menten-Kinetik mit Substratinhibierung erkannt werden (vgl. Figur 6). Aus dem Grund wurde das klassische Michaelis-Menten-Modell für die Auswertung der Messreihen bei konstanten Substrat- und variierten Kosubstratkonzentrationen und das Michaelis-Menten-Modell mit Substratinhibierung für die Messreihen mit konstanten Kosubstrat- und variierten Substratkonzentrationen verwendet.
$V_{max}$, $K_m$ und $K_i$ wurden mittels nichtlinearer Regression ermittelt.

$$v = v_{max} \cdot \frac{c_s}{K_m + \left(1 + \frac{c_s}{K_i}\right) c_s}$$

$v$ spezifische Enzymaktivität, U $mg^{-1}$ = µmol $min^{-1}$ $mg^{-1}$
$v_{max}$ maximale spezifische Enzymaktivität, U $mg^{-1}$ = µmol $min^{-1}$ $mg^{-1}$
$c_s$ Substrat- bzw. Kofaktorkonzentration, mol $L^{-1}$
$K_m$ Halbsättigungskonzentration, mol $L^{-1}$
$K_i$ Inhibitionskonstante, mol $L^{-1}$

Die ermittelten Parameter sind in nachfolgender Tabelle aufgeführt.

Tabelle : Ermittelte Parameter der drei verschiedenen 7β-HSDH-Varianten.

| | $K_{m,DCS}$, µM | $K_{i,DCS}$, mM | $K_{m.NADPH}$, µM | $V_{max}$, U/mg |
|---|---|---|---|---|
| 7β-HSDH WT | 31 ± 5 | 8,6 ± 1,5 | 46 ± 7 | 14,6 ± 1,0 |
| 7β-HSDH G39A | 33 ± 6 | 17 ± 4 | 16,4 ± 1,9 | 21,0 ± 1,1 |
| 7β-HSDH G39S | 75 ± 9 | 80 ± 50 | 13,1 ± 2,9 | 20,7 ± 1,0 |

Bei Betrachtung der gemessenen Daten fällt auf, dass die 7β-HSDH eine Substratinhibierung durch Dehydrocholsäure zeigt, die bei dem Wildtypprotein am stärksten auftritt und bei der G39S-Mutante am schwächsten ist. Vor allem bei dem letztgenannten Protein lässt sich die Frage stellen, ob überhaupt eine Substratinhibierung vorliegt, wie der hohe $K_i$ und dessen großer Fehler zeigen. Die Halbsättigungskonzentrationen für Dehydrocholsäure liegen im zweistelligen mikromolaren Bereich. Während sich diese für das Wildtypprotein und die G39A-Mutante mit 31±5 µM bzw. 33±6 µM nicht signifikant unterscheiden, liegt dieser Wert bei der G39S-Mutante mit 75±9 µM in etwa bei dem doppelten Wert der anderen zwei Enzyme. Bei den Halbsättigungskonzentrationen für den NADPH zeigen sich bei den Mutantenenzymen niedrigere Werte als bei dem Wildtyp-Enzym (46±7 µM beim Wildtyp gegenüber 16,4±1,9 µM bei der G39A-Mutante bzw. 13,1±2,9 µM bei der G39S-Mutante).

Die Werte für $v_{max}$ liegen bei beiden Mutanten in etwa bei dem 1,5-fachen Wert des Wildtypproteins (21,0±1,1 U/mg bei der G39A-Mutante bzw. 20,1±1,0 U/mg bei der G38S-Mutante gegenüber 14,6±1,0 U/mg bei dem Wildtypprotein). Anders als bei dem klassischen Michaelis-Menten-Modell sind bei dem Michaelis-Menten-Modell mit Substratinhibierung die $v_{max}$ Werte jedoch nicht als maximal erreichbare spezifische Enzymaktivitäten zu sehen, da sich die spezifischen Enzymaktivitäten mit zunehmender Substratkonzentration nicht asymptotisch an $v_{max}$ annähern, sondern wieder abnehmen. Die maximal erreichbaren Enzymaktivitäten, d.h. die spezifischen Enzymaktivitäten bei den Maxima der Kinetikkurven, liegen bei dem Wildtyp-Protein bei ~13 U/mg und bei den Mutantenproteinen bei ~19 U/mg (G39A) bzw. ~20 U/mg(G39S). Interessanter als die maximal erreichbaren Enzymaktivitäten sind die Enzymaktivitäten, die bei der für eine Ganzzellbiotransformation eingesetzten Substratkonzentrationen erreicht werden können. Diese sollen exemplarisch für die Substratkonzentration von 10 mM Dehydrocholsäure verglichen werden und betragen bei dem Wildtyp-Protein ~ 6,7 U/mg, für die G39A-Mutante ~13 U/mg und für die G39S-Mutante ~18 U/mg. Bei 10 mM Substratkonzentration wäre die G39A-Mutante demnach doppelt so aktiv wie das Wildtyp-Protein, die G39S-Mutante hätte sogar knapp dessen 3-fache Aktivität. Diese Unterschiede sollten bei höheren Substratkonzentrationen noch stärker ausgeprägt sein, da das Wildtyp-Protein die stärkere Substratinhibierung als die G39A-Mutante zeigt, und die G39A-Mutante wiederum stärker substratinhibiert ist als die G39S-Mutante.

**Herstellungsbeispiel 4: Herstellung und Charakterisierung der FDH D221G Mutante**

**4.1 Klonierung pET21a(+) FDH**

**4.1.1** PCR-Amplifizierung der *Mycobacterium vaccae* Formiatdehydrogenase.

Als Templat der Amplifizierung dient genomische DNA von *Mycobacterium vaccae*, welche von der Deutschen

Sammlung von Mikroorganismen und Zellkulturen GmbH (DMSZ), Braunschweig bezogen wurde. Primer für die Amplifizierung waren fdh_for (5'-CGATCATATGGCAAAGGTCCTGTGCGTTC-3') (SEQ ID NO:23) und fdh_rev (5'-GCTAGAATTCTCAGCCGCCTTCTTGAACT-3') (SEQ ID NO:24), bezogen von Eurofins MWG GmbH, Ebersberg. Die Erkennungsstellen für die Restriktionsenzyme sind unterstrichen. Der rev-Primer enthält die *EcoRI* Schnittstelle der for-Primer enthält die *NdeI*-Schnittstelle.
Die PCR-Ansätze und PCR-Programme sind in den Tabellen angegeben.

Tabelle: PCR-Ansatz für die Amplifizierung der Formiatdehydrogenase aus *Mycobacterium vaccae*

| Komponente | Volumen [μl] |
|---|---|
| 10x Taq Puffer (mit $Mg^{2+}$) | 5 |
| dNTPs (10 mM) | 1 |
| Fdh_for (100 μM) | 0,5 |
| Fdh_rev (100 μM) | 0,5 |
| Templat-DNA (>=100 ng/μL) | 1 |
| Taq DNA Polymerase (5 U/mL) | 0,5 |
| Destilliertes Wasser | 41,5 |

Tabelle: PCR-Programm für die Amplifizierung der Formiatdehydrogenase aus *Mycobacterium vaccae*

| Segment | Zyklenzahl | Denaturierung | Annealing | Elongation |
|---|---|---|---|---|
| 1 | 1 | 94°C, 2 min | | |
| 2 | 5 | 94°C, 30 sec | 55,6°C, 30 sec | 72°C, 75 sec |
| 3 | 25 | 94°C, 30 sec | 58,6 °C, 30 sec | 72°C, 75 sec |
| 4 | 1 | | | 72°C, 75 sec |

**4.1.2** Restriktionsverdau von pET21a(+) und FDH-PCR-Produkt

1-5 μg in Wasser gelöste DNA (pET21a(+) bzw. FDH-PCR-Produkt) werden mit 5μl 10x NEBuffer *EcoR*I*,* 2,5 μl NdeI (20 U/mL) und 2,5 μl EcoRI (20 U/mL) (jeweils New England Biolabs, Frankfurt) versetzt und mit destilliertem Wasser auf 50 μl Gesamtvolumen aufgefüllt. Die Ansätze wurden jeweils 1 h bei 37 °C inkubiert. Im Anschluss werden die geschnittenen DNA-Fragmente auf ein 1 %-iges Agarosegel (1 % (m/v) Agarose, 0,05 % (v/v) Ethidiumbromid) aufgetragen und die DNA-Fragmente 55 Minuten bei 120 V elektrophoretisch aufgetrennt. Anschließend wurden die Banden der richtigen Größe (1,2 kb bei dem FDH-Gen, 5,4 kb bei dem pET21a(+)-Plasmid) mit einem Skalpell aus dem Agarosegel ausgeschnitten und mit Hilfe des QIAQuick Gel Extraction Kit (QIAGEN, Hilden) gemäß Herstellerprotokoll isoliert.

**4.1.3** Ligation von geschnittenem pET21a(+) und FDH

100 ng geschnittene Vektor-DNA und 111 ng geschnittene FDH-DNA wurden mit 1 μl T4 Ligase (3 U/μL) und 1 μl 10x Ligase Puffer (jeweils New England Biolabs, Frankfurt) versetzt und mit destilliertem Wasser auf ein Gesamtvolumen von 10 μl aufgefüllt. Der Ligationsansatz wurde über Nacht bei 4°C inkubiert.

**4.1.4** Transformation des Ligationsansatzes in chemisch kompetente *E. coli* DH5α

Nach Beendigung des Ligationsschritts werden die 10 μL Ligationsansatz zu 200 μl nach Standardprotokoll hergestellte, chemisch kompetente *E. coli* DH5α gegeben. Danach erfolgt 30 Minuten lang ein Inkubationsschritt auf Eis, gefolgt von einem Hitzeschock bei 42°C (90 Sekunden). Anschließend werden 600 μl steriles LB-Medium zum Transformationsansatz zugegeben und die Zellen bei 200 rpm und 37°C in einem Schüttelinkubator 45 Minuten inkubiert. Im nächsten Schritt wird der Ansatz bei 3000 rpm 60 Sekunden in einer Tischzentrifuge abzentrifugiert, 700 μl vom Überstand werden verworfen, die Zellen im restlichen Überstand resuspendiert und auf einer LB-Agarplatte mit 100 mg/l

Ampicillin ausplattiert. Die Agarplatte wird im Anschluss bei 37°C über Nacht inkubiert.

### 4.2. Herstellung pET21a(+) FDH D221G

Die Herstellung einer FDH Mutante, welche nicht nur NADH sondern auch NADHP regenerieren kann, wird anhand der Mutante D221G näher erläutert.

Bei Aspartat (D) 221 handelt es sich um eine Aminosäure mit einer negativ geladenen, großen Seitenkette, die direkt neben dem Arginin (R)-Rest, durch welchen $NADP^+$ gebunden werden soll, liegt. Dies kann zur Abstoßung der Phosphatgruppe im $NADP^+$, die ebenfalls negativ geladen ist, führen. Das Aspartat wird daher durch den kleinen ungeladenen Aminosäurerest Glycin (G) ersetzt.

### 4.2.1 Verwendete Primer

| | |
|---|---|
| mt1: | 5'- C CTG CAC TAC ACC G**G**C CGT CAC CGC CTG C - 3' (SEQ ID NO: 30) |
| NI_fdh_R: | 5' -GCTCGAATTCTCAGACCGCCTTC--3'(SEQ ID NO:31) |

### 4.2.2 Durchführung

Zunächst wurde mit Hilfe des mt-Primers und des Primers NI_fdh_R ein Set von zwei komplementären Megaprimern erzeugt.

Als Template wurde Plasmid pET21a(+)FDH verwendet. Das verwendete PCR-Programm ist folgender Tabelle zu entnehmen:

Tabelle: PCR-Programm Megaprimer

| Segment | Zyklenzahl | Denaturierung | Annealing | Elongation |
|---|---|---|---|---|
| 1 | 1 | 94°C, 2 min | | |
| 2 | 30 | 94°C, 30 sec | 60°C, 30 sec | 72°C, 40 sec |
| 3 | 1 | 72°C, 5 min | | |

Durch Kombination von Primer mt1 mit dem Primer NI_fdh_R ergibt sich die Länge des Megaprimer zu 650 bp. Mit diesem PCR-Produkt der ersten PCR erfolgt eine Gelelektrophorese und Isolierung der gewünschten Bande aus dem Gel. Eine zweite PCR wird als Whole Plasmid PCR mit den Megaprimern als Primer und der Plasmid-DNA (pETfdh) als Template durchgeführt. Den Reaktionsansatz und das Temperaturschema für die Whole Plasmid PCR zeigen die folgenden Tabellen. Der 2x EZClone enzyme mix, die EZClone Solution 1, der 1,1 kb Marker und das *DpnI* stammten aus dem GeneMorph II EZClone Domain Mutagenesis Kit (Stratagene).

Tabelle: Ansatz für eine MEGA WHOP PCR (Gesamtvolumen 50 μL)

| Komponente | |
|---|---|
| Megaprimer | 250 ng (~ 2,5 μL aus einer Standard-PCR) |
| Template (pETfdh) | 50 ng |
| 2x EZClone enzyme mix | 25 μL |
| EZClone Solution 1 | 3 μL |
| dest. Wasser | ad 50 μL |

Der erste Schritt im PCR-Programm (68°C, 5 min) dient dazu, die durch die *Taq*-Polymerase unspezifisch angehängten Basen durch die 3'->5' Exonukleaseaktivität der in der MEGA WHOP PCR eingesetzten Polymerase zu entfernen.

Tabelle: PCR-Programm MEGA WHOP

| Segment | Zyklenzahl | Denaturierung | Annealing | Elongation |
|---|---|---|---|---|
| 1 | 1 | | | 68°C, 5 min |
| 2 | 1 | 95°C, 1 min | | |
| 3 | 25 | 95°C, 50 sec | 60°C, 50 sec | 68°C, 13 min |

Das PCR-Produkt ist ein doppelsträngiges Plasmid mit Einzelstrangbrüchen, die erst in *E. coli* geschlossen werden. Zu dem 50 $\mu$L PCR-Produkt wurden 10 U *DpnI* zugesetzt und der Ansatz bei 37°C für zwei Stunden inkubiert. *DpnI* baut nur methylierte DNA ab, d.h. die eingesetzte Template-DNA, nicht aber den Megaprimer oder das synthetisierte Plasmid. Das Template-Plasmid muss mit einem $dam^+$-Stamm (wie DH10B oder JM109) erzeugt werden, um methylierte Ausgangs-DNA zu erhalten.

**4.3 Expression und Isolierung der Mutante D221G**

Zunächst wurde eine LB-Vorkultur aus einer Gefrierkonserve oder mit einer Kolonie von einer Agarplatte inokuliert und die Vorkultur über Nacht inkubiert. Die Inkubation erfolgte bei 37°C und 250 Upm. Die OD der Vorkultur wurde bestimmt und die Kultur auf eine OD von 0,1 angeimpft. Beim Erreichen einer OD von 0,5-1 (nach etwa 2,5 h) wurde mit IPTG (Endkonzentration 1 mM) induziert. Die Zellen wurden drei Stunden nach der Induktion geerntet.

Zum Zellaufschluss wurde die mechanistische Methode mit Glasperlen eingesetzt. Dazu wurden zu 0,5 mL Zellprobe in einem 1,5 mL Reaktionsgefäß 0,5 mL Glasperlen zugegeben und das Reaktionsgefäß für 3 min bei maximaler Frequenz (30 $sec^{-1}$) in der Retsch-Schwingmühle geschüttelt. Nach dem Zellaufschluss wurden die Glasperlen abzentrifugiert (2 min, 13000 rpm). Die Kultur wurde vor und nach dem Aufschluss auf Eisgestellt, um die Proteindenaturierung durch die Erwärmung der Probe in der Schwingmühle so gering wie möglich zu halten. Dieses Aufschlussprotokoll ist für die Verwendung von bei -20°C eingefrorenen Proben optimiert.

**4.4 Charakterisierung der Mutante D221G**

Zusammenfassung kinetischer Konstanten der FDH bei pH 6, 7 und 8..

| Enzym | pH | Substrat | Cofaktor | Spezifische Aktivität (U $\times$ $mg^{-1}$) | $K_M$ (mM) Substrat | $K_M$ (mM) Cofaktor |
|---|---|---|---|---|---|---|
| FDH | 6 | Natriumformiat | $NAD^+$ | 4.6 | 46.86 | 0.83 |
| | 6 | Natriumformiat | $NADP^+$ | 1 | | 0.3 |
| | 7 | Natriumformiat | $NAD^+$ | 5.1 | 45.56 | 0.61 |
| | 7 | Natriumformiat | $NADP^+$ | 1.2 | | 0.51 |
| | 8 | Natriumformiat | $NAD^+$ | 4.7 | 59.26 | 0.52 |
| | 8 | Natriumformiat | $NADP^+$ | 1 | | 1.01 |

Die Daten belegen, dass die hergestellte Mutante sowohl $NAD^+$ als auch $NADP^+$ als Cofakor verwenden kann.

**Herstellungsbeispiel 5**: **Herstellung einer *E. coli* 7$\alpha$-HSDH Knockout-Mutante (*E. coli* BL21 (DE3) $hdhA^-$ $KanR^+$)**

Ziel ist die Deletion der störenden 7$\alpha$-HSDH Aktivität im Expressionsstamm *E. coli* BL21 (DE3).

Mit der im Folgenden beschriebenen Methode wird in das Zielgen der 7$\alpha$-HSDH ein Antibiotika-Resistenzgen insertiert, wodurch das Zielgen ausgeschalten wird.

**5.1 Sequenzinformation zur 7$\alpha$-HSDH aus *E. coli* BL21(DE3)**

Aminosäuresequenz: (SEQ ID NO:26)
Nukleotidsequenz (SEQ ID NO:25)
Accession: NC_012971 REGION: 1642470..1643237

### 5.2 Verwendete Primer

Folgende Primer für das Ausschalten der 7α-HSDH aus *E. coli* BL21 (DE3) wurden hergestellt:
Primer für das Retargeting des LI.LtrB Introns:

467|468a-IBS (SEQ ID NO:27)
AAAAAAGCTTATAATTATCCTTATAGGACGTCATGGTGCGCCCAGATAGGGTG

467|468a-EBS1d (SEQ ID NO:28)
CAGATTGTACAAATGTGGTGATAACAGATAAGTCGTCATGTTTAACTTACCTTTCTTTGT

467|468a-EBS2 (SEQ ID NO:29)
TGAACGCAAGTTTCTAATTTCGGTTTCCTATCGATAGAGGAAAGTGTCT

Insertion

Location

467|468a GCAGCTTTAGATGATGCATAGGAAGTCATG - intron - TTTATATTTTTATTT

### 5.3 Herstellung der Knockout-Mutante

Die Herstellung der Knockout-Mutante wurde mit Hilfe des Kits TargeTron™ Gene Knockout System von Sigma Aldrich nach Herstellerangaben durchgeführt. Es wurde zur Aufreinigung des PCR-Produkts gemäß Schritt B.6. des TargeTron™ Gene Knockout System der QIAquick PCR Purification Kit von Qiagen genutzt.
Die Ligation des HindIII/BsrGI-verdauten Intron-PCR-Produkts in den linearisierten pACD4K-C Vektor wurde wie folgt durchgeführt: Die Reaktion erfolgte über Nacht bei 16°C.

20μl-Ansatz:

| | |
|---|---|
| 2 μl | pACD4K-C linear vector (40 ng) |
| 6 μl | HindIII/BsrGI-digested intron PCR product |
| 2 μl | ATP (10 mM) |
| 2 μl | Ligase-Puffer (10 x) (Fermentas) |
| 2 μl | T4-Ligase (Fermentas) |
| 6 μl | $H_2O$ |

5 μl Ligationsreaktionslösung wurden zu 200 μl chemisch-kompetenten Zellen *E. coli* BL21 (DE3) gegeben und für 20 min auf Eis inkubiert. Die weitere Transformation erfolgte wie von Herstellerseite beschrieben.
Die Transformations-Ansätzewurden auf LB-Agar-Platten, enthaltend 33 pg/mL Kanamycin, ausplattiert. Es wurden Kanamycin-resistente Zellen gepickt und diese jeweils über mehrere Nächte in 5 ml LB-Übernacht-Kulturen überimpft (jeweils mit 5 μl einer Kanamycin-Lösung (33 mg/ml)). Abschließend wurde eine 200 ml LB-Kultur (mit 200 μl Kanamycin-Lösung (33 mg/mL)) mit einer Übernachtkultur angeimpft und für 5 h bei 37°C und 180 rpm im Schüttelinkubator inkubiert. Anschließend wurde die Temperatur auf 42°C für 1 Stunde erhöht. Mit dieser Kultur wurde eine 5 mL LB-Übernacht-Kulturen angeimpft (jeweils mit 5 μl einer Kanamycin-Lösung (33 mg/mL)). Nach Inkubation über Nacht bei 37°C und 180 rpm wurde die Kultur auf einer LB-Agar-Platte mit 33 μg/mL Kanamycin ausgestrichen. Nach Übernacht-Inkubation bei 37°C wurden Kolonien gepickt und auf LB-Agar-Platte mit 33 μg/mL Kanamycin und 34 μg/mL Chloramphenicol ausgestrichen.
Nach Übernacht-Inkubation bei 37°C wurden Chloramphenicol-sensitive Mutanten gefunden. Dies ist erforderlich, um den Verlust des Plasmids, welches das induzierbare Knockout-System trägt und nach erfolgreichem Knockout nicht mehr benötigt wird, zu bestätigen.

### 5.4. Nachweis des Knockouts

Das 7α-HSDH-Gen wurde über Kolonie-PCR mit den Primern

7alpha-ko-check_fwd (5' - TTAATTGAGCTCCTGTACCCCACCACC - 3') SEQ ID NO:32 und
7alpha-ko-check_rev (5' - GTGTTTAATTCTGACAACCTGAGACTCGAC - 3') SEQ ID NO:33

amplifiziert. Das entstandene Fragment hatte eine Länge von ca. 2,5-3 kb und wurde mit dem Primer 7alpha-ko-check_fwd sequenziert. Anhand der Sequenzierung konnte nachgewiesen werden, dass die DNA-Sequenz der 7α-HSDH durch ein Insert aus dem pACD4K-Vektor unterbrochen ist, was den Knockout der 7α-HSDH zur Folge hat. (Sequenzierdaten nicht gezeigt)

**Umsetzungsbeispiel 1: Enzymatische Umsetzung von 7-Keto-LCS durch die 7β-HSDH**

Zur Überprüfung der biochemischen Funktion der 7β-HSDH, wurde eine Umsetzung von 7-Keto-LCS durch die 7β-HSDH durchgeführt. Die 20 ml Umsetzung enthält 50 mM 7-Keto-LCS (ca. 0,4 g), 5 U/ml 7β-HSDH und 0,05 mM $NADP^+$. Zur Regenerierung von NADPH wurde 4 U/ml ADH und 1% Isopropanol eingesetzt (siehe Schema 1). Die Reaktion wurde bei pH8 und 24°C unter Rühren im Abzug durchgeführt. Da Aceton schneller als Isopropanol verdunstet, wird die Reaktion in Bildung von UDCS geschoben. 1% Isopropanol wurde nach 24 h, 48 h und 72 h nachgegeben. Das Produkt wurde mittels DC analysiert (Kieselgel 60, Merck, Laufmittel Petrolether und Ethylacetat 1:10, vol:vol). Auf DC wurde das Produkt mit authentischen Referenzen 7-Keto-LCS, UDCS und CDCS verglichen. Die DC-Analyse zeigt, dass UDCS aus 7-Keto-LCS von der 7β-HSDH gebildet wurde. Das Enantiomer CDCS ist auf DC nicht nachweisbar.

Schema 1: Schematische Darstellung von Reduktion von 7-Keto-LCS durch die 7β-HSDH. Die ADH regeneriert den Cofaktor NADPH.

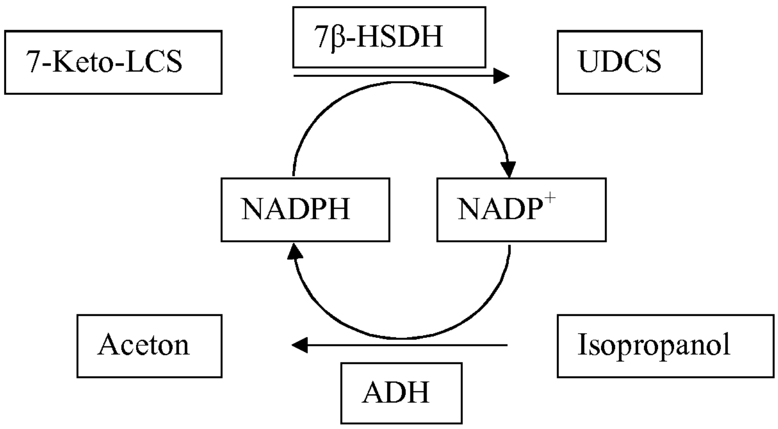


**Umsetzungsbeispiel 2: Enzymatische Herstellung von 3,12-Diketo-7β-CS aus DHCS durch 7β-HSDH**

Zur Überprüfung der Einsetzbarkeit der 7β-HSDH zu Herstellung von 12-Keto-UDCS aus DHCS, wurde eine Umsetzung von DHCS durch die 7β-HSDH durchgeführt. Die 50 ml Umsetzung enthält 50 mM DHCS (1 g), 5 U/ml 7β-HSDH und 0,05 mM $NADP^+$. Zur Regenerierung von NADPH wurde 4 U/ml ADH und 1% Isopropanol eingesetzt (siehe Schema 2). Die Reaktion wurde bei pH8 und 24°C unter Rühren im Abzug durchgeführt. Da Aceton schneller als Isopropanol verdunstet, wird die Reaktion in Bildung von 3,12-Diketo-7β-CS geschoben. Um vollständige Umsetzung zu erreichen, wurde 1% Isopropanol nach 24 h, 48 h und 72 h nachgegeben. Das Zwischenprodukt 3,12-Diketo-7β-CSwurde mittels DC analysiert. Das Edukt DHCS war nicht mehr nachweisbar auf DC (Kieselgel 60, Merck; Laufmittel Chlorform:Methanol:Essigsäure 10:1:0,08 vol:vol:vol).

Schema 2: Schematische Darstellung von Reduktion von DHCS durch die 7β-HSDH. Die ADH regeneriert den Cofaktor NADPH.

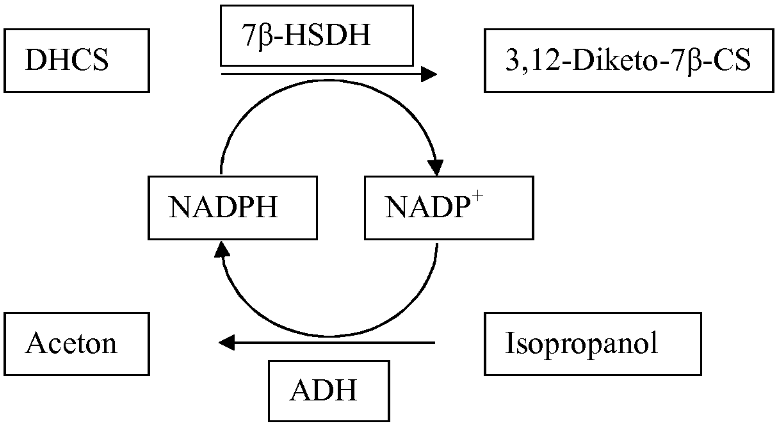


**Umsetzungsbeispiel 3: Enzymatische Umsetzung von 3,12-Diketo-7ß-CS zu 12-Keto-UDCS**

Das Zwischenprodukt 3,12-Diketo-7β-CS(hergestellt gemäß Umsetzungsbeispiel 2) wurde von einer 3α-HSDH (SEQ ID NO:5 und 6) aus *Comamonas testosteroni* (Mobus, E. and E. Maser, Molecular cloning, overexpression, and characterization of steroid-inducible 3alpha-hydroxysteroid dehydrogenase/carbonyl reductase from Comamonas testosteroni. A novel member of the short-chain dehydrogenase/reductase superfamily. J Biol Chem, 1998. 273(47): p. 30888-96) weiter zu 12-Keto-UDCS umgesetzt. Diese 3α-HSDH benötigt Cofaktor NADH, der von der FDH regeneriert wurde (siehe Abb. 3). Zu der Reaktion wurden 4 U/ml 3α-HSDH, 1 U/ml FDH (NADH-abhängig, Codexis), 200 mM Natiumformiat und 0,05 mM $NAD^+$ zugegeben. Nach 40 h wurde das Produkt mit 2 M HCl bis pH2 angesäuert und mit 6 mal 10 ml Ethylacetat extrahiert. Nach Evaporation wurden 1,07 g Produkt erhalten. Das Produkt 12-Keto-UDCS wurde mittels DC und NMR analysiert und bestätigt. Die 3alpha-HSDH wurde analog zur Herstellung von 7ß-HSDH, jedoch mit dem Plasmid pET22b+ hergestellt und ohne weitere Aufreinigung verwendet.

Schema 3: Schematische Darstellung von Reduktion von 3,12-Diketo-7β-CS durch die 3α-HSDH. Die FDH regeneriert den Cofaktor NADH.

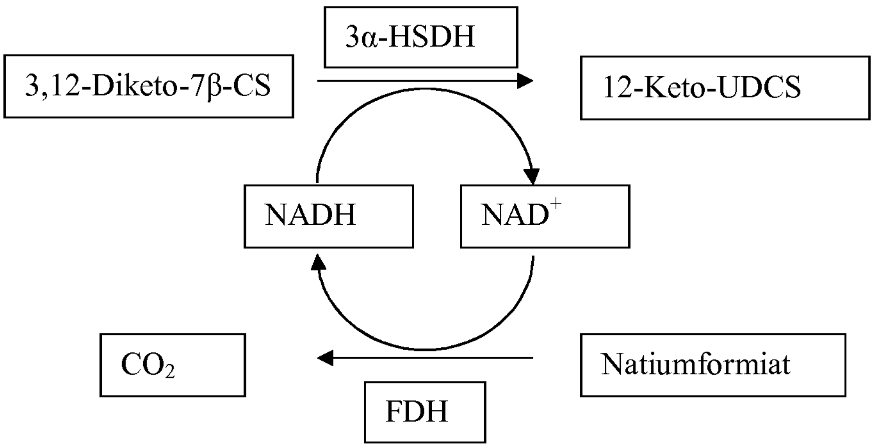


**Umsetzungsbeispiel 4: Chemische Umsetzung von CS zu DHCS**

In einem 2000L Rührwerksbehälter werden 1320L Eisessig vorgelegt und darin 110 kg (260mol) Cholsäure (CS) gelöst. Zu dieser Lösung werden 422 L Natriumhypochlorid Lösung (2,3 molar) bei 20 bis 40 °C zudosiert und die Reaktionslösung anschließend noch mindestens 1 Stunde zur Vervollständigung der Reaktion nachgerührt. Die Dehydocholsäure (DHCS)wird in einer Ausbeute von 100 kg (90%) durch Zentrifugieren isoliert.

**Umsetzungsbeispiel 5: Chemische Umsetzung von 12-Keto-UDCS zu UDCS**

105g (0,258mol) 12-Keto-UDCS werden in 384ml Triethylenglycol, 52,2g (1,304 mol) Natriumhydroxid und 75,95ml (1,563 mol) Hydrazinhydrat gelöst und langsam auf 180°C erwärmt. Dabei bildet sich das Hydrazon, das ab 160°C unter Abspaltung von Stickstoff in die UDCS umwandelt. Die Reaktion wird zur Vervollständigung der Umsetzung 8 Stunden bei 180°C gehalten. Die Reaktionsmischung wird unter 100°C gekühlt, mit 1500 ml Wasser versetzt. Anschließend durch Ansäuern mit Salzsäure die UDCS ausgefällt. Das Produkt wird in einer Ausbeute von 96,2 g bis 99,2 g (bis 95% - 98%) erhalten.

**Umsetzungsbeispiel 6: Enzymatische Umsetzung von DHCS zu 12-Keto-UDCS durch die 7β-HSDH, FHD D221G und 3α-HSDH**

In diesem Beispiel soll untersucht werden, ob eine zweistufige enzymatische Umsetzung von DHCS zu 12-Keto-UDCS bei gleichzeitiger Cofaktorregenerierung mit einer erfindungsgemäß verwendeten FDH-Mutante möglich ist. Da die verwendete FDH Mutante D221G sowohl $NADP^+$ als auch $NAD^+$ als Cofaktor akzeptiert, muss für die NADH-abhängige 3α-HSDH kein zusätzliches Cofaktorregenerierungssystem in den Reaktionsanssatz eingebracht werden. Beispiele für zwei Teilreaktionen sind im Folgenden grafisch veranschaulicht. FDH* bezeichnet die Mutante FDH D221G.

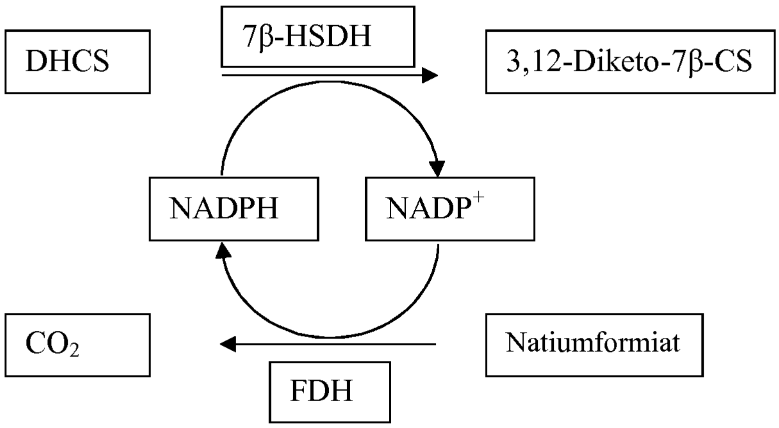


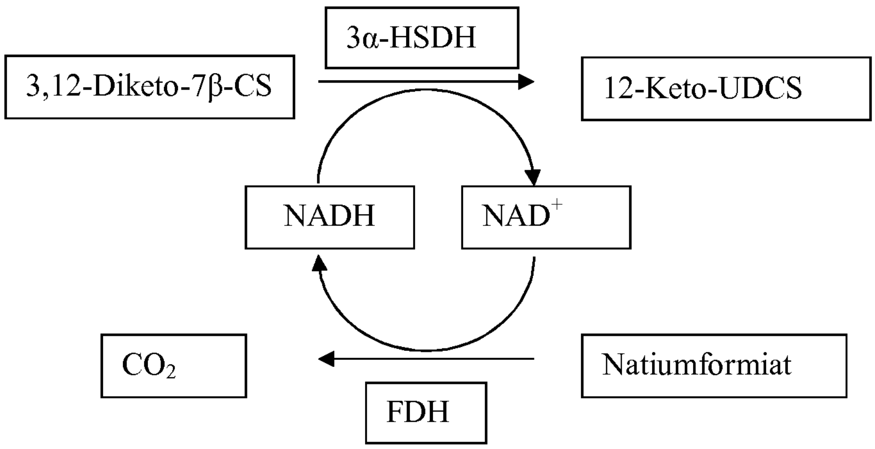


Hierzu wurden die Enzyme 7β-HSDH aus *Collinsella aerofaciens,* 3α-HSDH aus *Comamonas testosteroni* und die FDH-Mutante D221G abgeleitet von FDH aus *Mycobacterium vaccae* getrennt voneinander in einem modifizierten *E. coli* Expressionsstamm exprimiert und zur Umsetzung verwendet. Der dabei für die Expression verwendete *E. coli* Stamm wurde so modifiziert, dass er keine 7α-HSDH-Enzymaktivität exprimiert. Diese in E. coli verbreitete Nebenaktivität kann bei Umsetzungen des vorliegenden Typs (stereospezifische Umsetzung der 7-Keto-Gruppe) zu unerwünschten Nebenreaktionen und damit zu Verunreinigungen des herzustellenden Umsetzungsproduktes führen.

In dem folgenden Experiment wurde der Knock-out Stamm *E. coli* BL21(DE3)Δ7α-HSDH verwendet, der in der älteren Europäischen Patentanmeldung EP 10164003.5 der Anmelderin beschrieben wird.

### 6.1 Verwendete Plasmide,

Plasmide für die Expression von 7β-HSDH, 3α-HSDH und FHD D221G:

pET28a(+)-7β-HSDH,
pET22b(+)-3α-HSDH und
pET21a(+)-FDH-D221G.

### 6.2 Bakterienstämme und Kulturbedingungen:

Der oben bezeichnete Knock-out Stamm *E. coli* BL21(DE3)Δ7α-HSDH wurde bei 37°C in LB-Medium, enthaltend die erforderlichen Antibiotika, gezüchtet. Nach Induktion mit 0,5 mM IPTG nach Erreichen von $OD_{600}$ = 0,8 wurde die Inkubation bei 140 Upm über einen Zeitraum von 12 Stunden bei 25°C fortgeführt.

### 6.3 Enzymüberexpression und Reinigung

Überexpression von 7β-HSDH, 3α-HSDH und der FDH-Mutante D221G in *E. coli* BL21(DE3)Δ7α-HSDH und Enzym-Reinigung erfolgten unter den gleichen Bedingungen wie oben in Herstellungsbeispiel 2 für die Überexpression und Reinigung von 7β-HSDH in *E. coli* BL21(DE3) beschrieben. Die Ausbeuten je Liter Kulturmedium (Schüttelkolben bei $OD_{600}$ ~ 6) waren wie folgt:

7β-HSDH: 3883U (für DHCS und NADPH)
3α-HSDH: 6853 U (für DHCS und NADH)
FDH-Mutante: 47 U (für Natriumformiat und $NAD^+$).

Gehalt und Reinheit der Proteine wurde mittels SDS-PAGE und Densitometerscanning mittels Scieon Image Beta 4.0.2 (Scieon, USA) bestimmt.

### 6.4 Enzymatische Synthese von 12-Keto-UDCS im präparativen Maßstab

Ein 800 ml Reaktionsansatz, enthaltend 7β-HSDH (2,4 U x $ml^{-1}$), 3α-HSDH (2,4 U x $ml^{-1}$), FDH D221G (0,325 U x $ml^{-1}$), $NADP^+$ (10 μM), $NAD^+$ (10 μM), Natriumformiat (250 mM), DHCS (10 mM, 3,2 g) und Kaliumphosphat-Puffer (50 mM, pH 6), wurde bei 24°C gerührt. Sämtliche drei Enzyme, die in diesem Experiment verwendet wurden, wurden als Zellrohextrakte ohne zusätzlichen Reinigungsschritt eingesetzt. Nach 12 Stunden wurde die Reaktion durch Entfernung der Enzyme mittels Ultrafiltration unter Verwendung einer Membran mit einer Porengröße von 10 kDa (Millipore, USA) gestoppt. Das Produkt im Filtrat wurde durch Ansäuern mit Salzsäure auf pH 2 und anschließende Papierfiltration gereinigt. Nach Trocknen des Produktes bei 60°C über Nacht erhielt man 2,9 g des gewünschten Produktes.
Das Produkt wurde mittels HPLC und NMR analysiert.
Analysendaten (partiell):

$^1H$ NMR (deuteriertes DMSO, 500 MHz) δ = 3,92 (2H, m, H-3α und H-7β) und
$^1H$- NMR (deuteriertes DMSO, 125 MHz) δ =69,38 (CH, 3-C); δ = 69,09 (CH, 7-C); δ = = 213,86 (C, 12-C)
Ausbeute: 90,6 %
Reinheit: 99 %

### Umsetzungsbeispiel 7: Ganzzellbiotransformation von DHCS zu 12-Keto-UDCS durch Coexpression von FDH D221G, 7ß-HSDH und 3α-HSDH im Zweiplasmid-System

Es soll untersucht werden, ob eine zweistufige Ganzzellreduktion von Dehydrocholsäure (DHCS) zu 12-keto-Ursodesoxycholsäure (12-Keto-UDCS) (vgl. Schema gemäß Umsetzungsbeispiel 6, oben) möglich ist.
Hierzu wurde der oben hergestellte Knockout-Stamm *E. coli* BL21 (DE3) $hdhA^-$ $KanR^+$ pET21a(+) FDH 7β-HSDH pCOLA(mod) 3α-HSDH verwendet, in dem zusätzlich zur 7β-HSDH und der Mutante FDH D221G eine 3α-HSDH aus *Comamonas testosteroni* rekombinant exprimiert wird. Da die verwendete FDH Mutante sowohl $NADP^+$ als auch $NAD^+$ als Cofaktor akzeptiert musste für die NADH-abhängige 3α-HSDH kein zusätzliches Cofaktorregenerierungssystem in den Biotransformationsstamm eingebracht werden.

**7.1 Verwendeter Stamm:**

Escherichia coli BL21 (DE3) hdhA$^-$ KanR$^+$

**7.2 Verwendete Molekularbiologische Arbeitsmethoden**

7.2.1 Polymerasekettenreaktion

Zur Klonierung der 7β-HSDH wurde die Polymerasekettenreaktion (PCR) eingesetzt. Als Templat zur Amplifikation der 7β-HSDH diente das Plasmid pET22b(+) 7β-HSDH

PCR-Reaktionen wurden in 500 μL PCR-Tubes mit 20 μL Reaktionsvolumen durchgeführt. Die Reaktionen wurden in einem Thermocycler der Firma Eppendorf durchgeführt. Bei der Amplifikation von 7β-HSDH wurden jeweils 4 μL HF-Buffer, 1 μL Templat DNA, jeweils 1 μL Vorwärts- und Revers-Primer (10 μM), 0,4 μL Desoxynukleotidtriphosphat-Lösung (10 mM) sowie 0,2 μL Phusion DNA-Polymerase (2 U/μL) zugegeben. Das Ansatzvolumen wurde mit RNAse-freiem Wasser auf 20 μL eingestellt.

Bei der Reaktion erfolgte zuerst ein 2 min langer initialer Denaturierungsschritt bei 98°C. Danach wurden 34 Zyklen von Denaturierung (30 s bei 98°C), Primerhybridisierung (2 min bei 48°C) und Elongation (2 min bei 72°C) durchgeführt. Als letzter Schritt erfolgte eine finale Elongation von 10 min bei 72°C bevor die Polymerasekettenreaktion durch Kühlen auf 4°C beendet wurde.

7.2.2 Aufreinigung von DNA-Fragmenten mittels Gelextraktion

Um DNA-Fragmente aufzureinigen wurden diese zunächst mittels Agarose-Gelelektrophorese aufgetrennt. Die entsprechenden Banden wurden mittels UV-Licht sichtbar gemacht, anhand ihrer Größe identifiziert und mit einem Skalpell aus dem Gel geschnitten. Die Extraktion erfolgte mit dem QIAquick Gel Extraction Kit nach Protokoll des Herstellers. Zur Elution der aufgereinigten DNA wurden 30-50 μL H2O verwendet.

7.2.3 Restriktion mit Endonukleasen

Die Restriktionsreaktionen wurden in einem Gesamtvolumen von 20-50 μL durchgeführt. Dabei wurde die zu schneidende DNA mit 10-20 U der jeweiligen Restriktionsenzyme versetzt. Zusätzlich wurde der laut Hersteller empfohlene Reaktionspuffer verwendet und je nach Herstellerempfehlung gegebenenfalls 0,5 μL Bovines Serumalbumin (BSA, 10 mg/mL) zugegeben. Der Restriktionsverdau erfolgte für 2 h bei 37°C. Anschließend wurden die restringierten Fragmente entweder über Gelextraktion (Vektorverdau-Produkte) oder mit Hilfe des QIAquick PCR Purification Kits (verdaute PCR-Produkte) aufgereinigt.

7.2.4 Aufreinigung von DNA-Fragmenten unter Verwendung des QIAquick PCR Purification Kits

Um restringierte PCR-Fragmente aufzureinigen wurde DNA mit Hilfe des QIAquick PCR Purification Kits aufgereinigt. Die Aufreinigung erfolgte nach Herstellerangaben, wobei zur Elution der aufgereinigten DNA 30-50 μL $H_2O$verwendet wurde.

7.2.5 Ligation von DNA-Fragmenten

Für die Einklonierung restringierter DNA-Fragmente in Expressionsvektoren, wurden beide DNA-Moleküle mit denselben Restriktionsenzymen geschnitten. Durch die Verwendung zweier verschiedener Enzyme kann zum einen die Religation des Vektors verhindert werden und zum anderen das Insert in einer definierten Orientierung eingebaut werden. Als Enzym wurde die T4-DNA-Ligase verwendet, die die Bildung einer Phosphordiesterbindung zwischen einer freien 5'-Phosphatgruppe und einer freien 3'-OH-Ende einer Desoxyribonukleinsäure katalysiert.

Für die Klonierung der Expressionskonstrukte wurden jeweils 12 μL eines restringierten, aufgereinigten Vektors mit 4 μL eines gencodierenden, aufgereinigten DNA-Fragments mit restringierten Enden versetzt. Hinzu wurden 2 μL 10x Ligase-Puffer, 1 μL Adenosintriphosphat (ATP, 1 mM) und 1 μL T4-DNA-Ligase (400 U/μL) gegeben. Die Reaktionen verliefen über Nacht bei 16°C.

### 7.3 Verwendete Vektorkonstrukte

7.3.1 pET21a(+) FDH 7β-HSDH

Bei diesem Vektorkonstrukt wurde die 7ß-HSDH in pET21a(+) FDH einkloniert, so dass das 7beta-HSDH kodierende Gen *downstream* von der FDH gelegen ist. Zu diesem Zweck musste der FDH am 5’-Ende ein Stoppcodon eingefügt werden. Verglichen mit der Originalsequenz (Lys-Lys-Ala-Val-Stopp) wurde in diesem Konstrukt der C-terminale ValinRest durch einen Alanin-Rest ersetzt und weitere drei Aminosäuren angehängt, so dass folgende C-terminale Sequenz entsteht: Lys-Lys-Ala-Ala-Gly-Asn-Ser-Stopp. Des Weiteren wurde für eine gesteigerte Translation der 7ß-HSDH zwischen FDH und 7beta-HSDH eine zusätzliche Ribosomale Bindestelle eingefügt.
Zur PCR-Amplifikation der 7β-HSDH wurden die Primer 7beta_fwd_EcoRI und S_7beta_rev_HindIII verwendet.

7beta_fwd_EcoRI (SEQ ID NO: 13:
5’-G*C***GAATTCG**TGAAAGGAG*ATATAC*ATGAACCTGAGGGAGAAGTACGG-3’

S 7beta_rev_HindIII (SEQ ID NO: 3):

5’-CCC**AAGCTT**CTAGTCGCGGTAGAACGA-3’

Dargestellt sind die hybridisierende Sequenzbereiche (schwarz), Restriktionsschnittstellen (fett unterstrichen), Ribosomale Bindestellen (unterstrichen), Füllnukleotide (kursiv). Im Primer 7beta_fwd_EcoRI wurden zusätzlich ein Stoppcodon (doppelt unterstrichen) und ein Nukleotid zur Verschiebung des Leserahmens (fett) hinzugefügt.
Die Einklonierung erfolgte über die Schnittstellen *Eco*RI und *Hind*III.

7.3.2 pCOLA(mod) 3α-HSDH

Um Dehydrocholsäure zweistufig zu 12-keto-Ursodesoxycholsäure reduzieren zu können, müssen neben dem Cofaktorregenerierungssystem FDH auch die Enzyme 7β-HSDH und 3α-HSDH in einer Zelle vorliegen. Um das zu ermöglichen, wurde das Vektorkonstrukt pCOLA(mod) 3α-HSDH, ein modifiziertes Derivat des pCOLA-duet-Vektor, hergestellt, welches mit pET-Vektoren kotransformierbar ist.
Das 3α-HSDH codierende Gen wurde hierzu über die Schnittstellen *Nde*I und *Blp*I aus dem Vektor pET22b(+) 3α-HSDH ausgeschnitten und über dieselben Schnittstellen in MCS2 des pCOLA(mod)-Vektors einkloniert. Nach der Sequenzierung zeigte sich, dass an Position 45 der DNA-Sequenz ein Guanin durch ein Cytosin ersetzt wurde, woraus jedoch eine stille Mutation resultiert. Diese Mutation hat jedoch keine Auswirkung auf die Aminosäuresequenz, weshalb es sich hier um eine sogenannte Stille Mutation handelt.
Beide Vektoren wurden in den oben genannten Stamm wie unten beschrieben kotransformiert. Die Gene sind IPTG-induzierbar.

### 7.4 Hitzeschock-Transformation von *E. coli*-Zellen

Hierzu wurden 200 μL chemisch-kompetente *E. coli* BL21 (DE3) hdhA⁻ KanR⁺ oder DH5α aufgetaut und mit 1 μL DNA versetzt. Diese wurden zunächst 45 min auf Eis inkubiert. Danach wurden die Zellen 45 s bei 42°C einem Hitzeschock ausgesetzt. Anschließend wurde den Zellen 600 μL LB-Medium zugegeben und diese bei 37°C und 200 rpm geschüttelt, damit sie die gewünschte Antibiotikaresistenz ausbilden können. Daraufhin wurden die Zellen in einer Tischzentrifuge bei 3000 rpm 2 min zentrifugiert, in deren Anschluss 150 μL vom Überstand verworfen wurde. Die Zellen wurden mit dem restlichen Überstand resuspendiert und auf LB-Agarplatten mit dem entsprechenden Antibiotikum ausplattiert. Danach wurden die Platten bei 37°C über Nacht inkubiert.
Sollte eine Kotransformation von zwei verschiedenen Plasmiden erfolgen, wie dies zur Erstellung des Stammes notwendig ist, der die Plasmide pCOLA(mod) 3α-HSDH und pET21a(+) FDH 7β-HSDH enthält, wurden 50 μL chemisch-kompetenter *E. coli* BL21 (DE3) hdhA⁻ KanR⁺ mit jeweils 2 μL der einzubringenden Plasmide versetzt. Nach der Transformation wurden die Zellen in Vorkulturröhrchen mit 5 mL LB-Medium mit dem entsprechenden Antibiotikum gegeben und bei 37°C auf dem Vorkulturenschüttler 16-48 h bei 200 rpm inkubiert, bis sich ein Bakterienwachstum im Vorkulturröhrchen erkennen ließ. Daraufhin wurden 5 μL der Bakteriensuspension auf LB-Agarplatten mit dem entsprechenden Antibiotikum ausplattiert und bei 37°C über Nacht inkubiert.

### 7.5 Kultivierung des Stamms im Schüttelkolben:

Zur Expression rekombinanter Proteine wurde der entsprechende *E. coli* BL21 (DE3) hdhA⁻ KanR⁺ beinhaltend

die beiden Expressionsplasmide, in 5 mL LB-Medium mit Zugabe des entsprechenden Antibiotikums über Nacht bei 37°C und 200 rpm inkubiert. Anschließend wurden 200 mL TB-Medium mit Zugabe des entsprechenden Antibiotikums mit dieser Vorkultur inokuliert und bei 37°C, 250 rpm inkubiert. Bei Erreichen einer OD600 von 0,6-0,8 wurde die Expression des rekombinanten Proteins mit 1 mM Isopropyl-β-D-thiogalactopyranosid (IPTG) induziert und die Kultur bei 25°C, 160 rpm für weitere 21 h inkubiert.

**7.6 Durchführung der Ganzzellbiotransformation und Versuchsergebnis**

Die Reaktionen wurden in 2 mL Reaktionsvolumen bei pH 6,0 in Suspension durchgeführt, wobei eine Zelldichte von $OD_{600}$ = 20 eingesetzt wurde. Als Substratkonzentration wurden 25 mM bzw. 50 mM Dehydrocholsäure gewählt, die eingesetzte Kosubstratkonzentration betrug 400 mM. Die Versuche erfolgten unter Luftabschluss bei 25°C. Proben wurden nach 48 h genommen.

Bei dem Reaktionsansatz mit 25 mM ist kein Substrat mehr nachweisbar, während bei dem Reaktionsansatz mit 50 mM Dehydrocholsäure zwar ein Substrat-Peak detektierbar ist, dessen Peakfläche aber unterhalb der Grenze von 1 μg/mL Substrat in der Probe ist, für die die HPLC-Methode kalibriert ist. Gleiches gilt für die Peaks der 3,12-diketo-Ursodesoxycholsäure. Der größte Peak in den Chromatogrammen ist der des Produktes 12-keto- Ursodesoxycholsäure.

Es kann also überraschend gezeigt werden, dass eine zweistufige Ganzzellreduktion von Dehydrocholsäure zu 3-Keto-Ursodesoxycholsäure möglich ist.

Die Chromatogramme der HPLC-Messung sind in Figur 7 dargestellt.

Die HPLC-Analyse wurde wie folgt durchgeführt: Als Chromatographiesäule wurde die Umkehrphasen-Chromatographiesäule Hi-bar Purospher 125-4 RP-18e (5 μm) der Firma Merck, Darmstadt verwendet. Hierbei dient eine unpolare Phase als stationäre Phase, während eine polare Phase die mobile Phase bildet. Für die HPLC-Analytik wurde das Verfahren der Gradientenelution verwendet. Dabei wird dem Eluent phosphorsaures Wasser (pH 2,6) ein steigender Anteil an Acetonitril zugesetzt, dabei bedingt die Ansäuerung des Laufmittels einen einheitlichen Protonierungsgrad der zu untersuchenden Analyten. Nach Elution sämtlicher Komponenten wird die Chromatographiesäule mit dem Ausgangsverhältnis beider Lösungsmittel äquilibriert. Die Gradientenelution wurde durchgeführt, indem zunächst für 3 min ein Laufmittelgemisch der konstanten Zusammensetzung von 65 % (v/v) phosphorsaurem Wasser und 35 % (v/v) Acetonitril durch die HPLC-Säule gepumpt wurde. Von Minute 3 bis 7,5 wir der Anteil an Acetonitril linear auf 39 % (v/v) erhöht. Zwischen Minute 7,5 und 10 erfolgt eine weitere lineare Erhöhung des Acetonitrilanteils auf 40 % (v/v). Zur Elution aller Probenkomponenten wird der Acetonitrilanteil zwischen Minute 10 und 11 ebenfalls linear auf 70 % (v/v) erhöht und weitere zwei Minuten lang auf diesem Wert konstant gehalten. Danach wird der Acetonitrilanteil von Minute 13 bis 15 wieder auf den Ausgangswert von 35 % reduziert und die Säule mit dieser Laufmittelzusammensetzung 3 min lang äquilibriert. Der Fluss wird über die gesamte Dauer auf 1,000 mL/min eingestellt. Eluierte Analyten werden durch einen UV-Detektor bei einer Wellenlänge 200 nm detektiert.

**Umsetzungsbeispiel 8: Ganzzellbiotransformation von DHCS zu 12-Keto-UDCS durch Coexpression von FDH D221G, 7ß-HSDH und 3α-HSDH im Einplasmid-System**

Es soll untersucht werden, ob eine zweistufige Ganzzellreduktion von Dehydrocholsäure zu 12-keto-Ursodesoxycholsäure in einem zellulären Einplasmid-System möglich ist.

8.1 Verwendete Plasmide:

| Bezeichnung | Sequenz | SEQ ID NO |
|---|---|---|
| 3alpha_fwd_HindIII | 5'-CCCAAGCTTAAGGAGATATACATGTCCATCATCGTGATAAGCG-3' | 16 |
| 3alpha_rev_NotI | 5'-ATAAGAATGCGGCCGCTCAGAACTGTGTCGG GCG-3' | 17 |
| 7beta_mut_G39A_fwd | 5'-CGTCGTCATGGTCGCCCGTCGCGAGG-3' | 9 |
| 7beta_mut_G39A_rev | 5'-CCTCGCGACGGGCGACCATGACGACG-3' | 10 |

Restriktionsschnittstellen sind unterstrichen, ribosomale Bindestellen sind grau hinterlegt.

**8.2 Herstellung des Vektors:**

Die Herstellung des Plasmidkonstrukts pET21a(+) FDH 7β-HSDH(G39A) 3α-HSDH (Figur 8) erfolgte wie folgt: Ausgehend vom Plasmid pET21a(+) FDH 7β-HSDH wurde die Wildtyp-3α-HSDH (C. testosteroni; SEQ ID NO: 5,6) über die Schnittstellen HindIII und NotI hinter die 7β-HSDH einkloniert. Zur Amplifizierung der 3α-HSDH wurden die Primer 3alpha_fwd_HindIII und 3alpha_rev_NotI verwendet, als Templat diente hierzu das Plasmid pET22b(+) 3α-HSDH. Anschließend wurde die G39A-Muation über positionsgerichtete Mutagenese nach dem QuikChange-Protokoll in 7β-HSDH eingeführt. Dabei wurden die Mutageneseprimer 7beta_mut_G39A_fwd und 7beta_mut_G39A_rev verwendet.

**8.3 Umsetzung:**

Das erstellte Plasmid wurde in E. coli BL21 (DE3) hdhA$^-$ KanR$^+$ transformiert. Mit dem dadurch erzeugtem Stamm *E. coli* BL21 (DE3) hdhA$^-$ KanR$^+$ pET21a(+) FDH 7β-HSDH(G39A) 3α-HSDH wurden Ganzzellumsätze unter folgenden Bedingungen im 150 mL-Maßstab durchgeführt: Zelldichte OD 30, 50 mM DHCS, 750 mM Natriumformiat, suspendiert mit 50 mM Kaliumphosphatpuffer (pH 6,5) als Zell- und Substratsuspension. Die Reaktionen wurden 3,5 h bei 25°C durchgeführt. Die Ergebnisse der HPLC Analyse (Durchführung siehe Umsetzungsbeispiel 7, oben) sind in Figur 9 dargestellt.

**Herstellungsbeispiel 6: Herstellung weiterer 7β-HSDS Mutanten und deren Charakterisierung**

Eine Möglichkeit zur Generierung einer NADH-spezifischen 7β-HSDH besteht in der Modifikation des verfügbaren Enzyms durch verschiedene Mutageneseverfahren. Daher sollte mittels positionsgerichteter Mutagenese einzelne Aminosäuren der 7β-HSDH durch andere substituiert werden, welche die Änderung der Kofaktorspezifität der 7β-HSDH von NADPH zu NADH bedingen, um NADP vorteilhaft durch das kostengünstigere NAD ersetzen zu können.

**6.1 Primer**

Insgesamt wurden die 7β-HSDH Mutanten G39D, G39D/R40L, G39D/R40I und G39D/R40V erzeugt. Die G39D-Mutante wird durch Quickchange-Mutagenese erzeugt, die anderen Mutanten nach der PCR-Methode von Sanchis et al. (Sanchis J, Fernández L, Carballeira JD, Drone J, Gumulya Y, Höbenreich H, Kahakeaw D, Kille S, Lohmer R, Peyralans JJ, Podtetenieff J, Prasad S, Soni P, Taglieber A, Wu S, Zilly FE, Reetz MT. Improved PCR method for the creation of saturation mutagenesis libraries in directed evolution: application to difficult-to-amplify templates. Appl Microbiol Biotechnol. 2008 Nov;81(2):387-97). Ein Sequenzvergleich des Wildtyps und der erzeugten Mutanten ist in Figur 10 gezeigt. Folgende Primer wurden für die Mutagenesen verwendet, wobei die kursiv dargestellten Basen jeweils für die ausgetauschte Aminosäure kodieren:

**G39D**

7beta mut G39D fwd (SEQ ID NO:41):CGTCGTCATGGTC*GAC*CGTCGCGAGG

7beta mut G39D rev (SEQ ID NO:42): CCTCGCGACG*GTC*GACCATGACGACG

**G39D R40L**

7beta mut G39D R40L fwd (SEQ ID NO:43): CGTCGTCATGGTC*GACCTG*CGCGAGG

3alphamut_AntiMid_rev (SEQ ID NO:44): CCGCCGCATCCATACCGCCAGTTGTTTACCC

**G39D R40I**

7beta mut G39D R40I fwd (SEQ ID NO:45): CGTCGTCATGGTC*GACATT*CGCGAGG

3alphamut_AntiMid_rev (SEQ ID NO:44): CCGCCGCATCCATACCGCCAGTTGTTTACCC

**G39D R40V**

7beta mut G39D R40V fwd (SEQ ID NO:46): CGTCGTCATGGTC*GACGTT*CGCGAGG

3alphamut_AntiMid_rev (SEQ ID NO:44): CCGCCGCATCCATACCGCCAGTTGTTTACCC

### 6.2 Enzymkinetische Untersuchung der 7β-HSDH Mutanten

Die Bewertung der erstellten Mutanten erfolgt mittels enzymkinetischer Untersuchungen, deren Ergebnisse in Figur 11 grafisch dargestellt sind. Hierbei werden für die Untersuchung der unmodifizierten 7β-HSDH 0,1 mM NADPH als Kofaktor eingesetzt, für die Untersuchung der 7β-HSDH-Mutanten hingegen 0,5 mM NADH. Die Notwendigkeit zur Erhöhung der Kofaktorkonzentration bei der enzymkinetischen Untersuchung der 7β-HSDH Mutanten sind durch die bei den Mutanten erhöhten Halbsättigungskonzentrationen für den Kofaktor NADH bedingt. Aus den Auftragungen der Substratkonzentration gegen die spezifische Enzymaktivität lassen sich für die 7β-HSDH Mutanten die charakteristischen Kurvenverläufe der Michaelis- Menten-Kinetik erkennen, während sich aus der Auftragung für die unmodifiziert Wildtyp 7β-HSDH der Kurvenverlauf einer Michaelis-Menten-Kinetik mit Substratinhibierung erkennen lässt. Dementsprechend wurde das klassische Michaelis-Menten-Modell (Gleichung 1) für die Auswertung der Messreihen der 7β- HSDH Mutanten und das Michaelis-Menten-Modell mit Substratinhibierung für die Messreihe des unmodifizierten Wildtyp 7β-HSDH verwendet (Gleichung 2).

$$EA_X = v_{\max} \cdot \frac{c_s}{K_m + c_s}$$

$EA_x$: spezifische Enzymaktivität, U mg$^{-1}$= μmol min$^{-1}$ mg$^{-1}$
$V_{max}$: maximale spezifische Enzymaktivität, U mg$^{-1}$= μmol min$^{-1}$ mg$^{-1}$
$C_s$: Substrat- bzw. Kofaktorkonzentration, mol L$^{-1}$
$K_m$: Halbsättigungskonzentration, mol L$^{-1}$

$$EA_X = v_{\max} \cdot \frac{c_s}{K_m + (1 + \frac{c_s}{K_i})c_s}$$

$EA_x$: spezifische Enzymaktivität, U mg$^{-1}$= μmol min$^{-1}$ mg$^{-1}$

$v_{max}$: maximale spezifische Enzymaktivität, U mg$^{-1}$= μmol min$^{-1}$ mg$^{-1}$

$c_s$: Substrat- bzw. Kofaktorkonzentration, mol L$^{-1}$

$K_m$: Halbsättigungskonzentration, mol L$^{-1}$

$K_i$: Inhibitionskonstante, mol L$^{-1}$

In der nachfolgenden Tabelle sind enzymkinetische Parameter der unmodifizierten Wildtyp 7β-HSDH und deren Mutanten mit veränderter Kofaktorspezifität zusammengefasst. Für den Wildtyp wird NADPH als Kofaktor eingesetzt, während für die Mutanten NADH als Kofaktor eingesetzt wird.

| | $K_m$, DHCA, μM | $K_i$, DHCA, μM | $v_{max}$, U mg-1 |
|---|---|---|---|
| **Wildtyp** | 31 ± 5 | 8600 ± 1500 | 14,6±1,0 |
| **G39D** | 660 ±120 | n. b. | 2,90±0,16 |
| **G39D R40I** | 920 ±170 | n. b. | 4,64±0,27 |
| **G39D R40V** | 880 ±120 | n. b. | 2,69±0,12 |
| **G39D R40L** | 560 ± 80 | n. b. | 1,60±0,07 |

**Herstellungsbeispiel 7: Herstellung weiterer 7β-HSDS Mutanten und deren Charakterisierung**

In einem zum Herstellungsbeispiel 6 parallelen Ansatz wurden nochmals 7β-HSDS-Mutanten hergestellt.

### 7.1 Primer

Für die positionsgerichtete Mutagenese der 7β-HSDH wurden die unten angegeben Mutageneseprimer verwendet. Die Primer wurden auf Grundlage der 7ß-HSDH-Gensequenz dahingehend ausgewählt, dass sie den gewünschten Aminosäurenaustausch bewirken. Es wurde darauf geachtet, dass die zu mutierende Base im Primer mittig lokalisiert ist, und dass die Schmelztemperaturen der Primerpaare sich im gleichen Bereich befinden.
Zur Erzeugung der Mutanten wurden folgende Primerpaare verwendet:

**Tabelle:** Verwendete Primer für die positionsgerichtete Mutagenese der 7ß-HSDH

| ***Bezeichnung*** | ***Position*** | ***Primer*** | ***5'-> 3' Sequenz*** |
|---|---|---|---|
| G39D_for | G39D | forward | GTCGTCATGGTC*GAC*CGTCGCGAGGAG |
| G39D_rev | | reverse | CTCCTCGCGACG*GTC*GACCATGACGAC |
| G39D/R40I_for | G39D/R40I | forward | GTCATGGTC*GACATT*CGCGAGGAG |
| G39D/R40I_rev | | reverse | CTCCTCGCG*AATGTC*GACCATGAC |
| R40D_for | R40I | forward | GTCATGGTCGGC*GAT*CGCGAGGAGAAG |
| R40D_rev | | reverse | CTTCTCCTCGCG*ATC*GCCGACCATGAC |
| R40D/R41I_for | R40D/R41I | forward | GTCATGGTCGGC*GATATC*GAGGAGAAGCTG |
| R40D/R41I_rev | | reverse | CAGCTTCTCCTC*GATATC*GCCGACCATGAC |
| DIN_for | G39D/R40I/R41 N | forward | ATGGTC*GACATTAAC*GAGGAGAAGCTG |
| DIN_rev | | reverse | CAGCTT*CTCCTCGTT*AATGTCGACCAT |

### 7.2 PCR-Programm

Bei der Reaktion erfolgte zuerst ein 2 min langer initialer Denaturierungsschritt bei 98°C. Danach wurden 23 Zyklen von Denaturierung (30 s bei 98°C), Primerhybridisierung (1 min bei 60 - 68°C) und Elongation (3,5 min bei 72°C) durchgeführt. Als letzter Schritt erfolgte eine finale Elongation von 10 min bei 72°C bevor die Polymerasekettenreaktion durch Kühlen auf 4°C beendet wurde.

### 7.3 PCR-Ansatz

**Tabelle:** PCR-Ansatz für die Erzeugung der verschiedenen 7β-HSDH Varianten

| | |
|---|---|
| HF-Puffer (5x) | 10 μl |
| dNTP-Mix (10 mM) | 1,0 μl |
| Forward-Primer (10 pmol/μl) | 5 μl |
| *Revers*-Primer (10 pmol/μl) | 5 μl |
| Templat | 1,5 μl |
| *Phusion* Polymerase | 0,5 μl |
| DMSO | 2,5 μl |
| dd$H_2O$ | 24,5 μl |
| | **50 μl** |

Als Templat wurde ein pET21a-Vektor mit der 7β-HSDH(Wildtyp) verwendet.

### 7.4 Durchführung

Um Aminosäuren in Proteinsequenzen gezielt austauschen zu können, wird die DNA-Sequenz des entsprechenden Gens positionsgerichtet mutiert. Hierzu werden zueinander komplementäre Primer genutzt, die die gewünschte Mutation in ihrer Sequenz tragen. Als Templat dient N6-adeninmethylierte, doppelsträngige Plasmid-DNA, die das zu mutierende Gen tragen. N6-adeninmethylierte Plasmid-DNA werden aus $dam^+$ *E. coli*-Stamm wie zum Beispiel *E. coli* DH5 isoliert.

Die Polymerasekettenreaktion wird wie oben beschrieben durchgeführt. Dabei werden die Primer komplementär zum Templat verlängert, wodurch Plasmide mit der gewünschten Mutation entstehen, die einen Strangbruch aufweisen. Anders als bei anderen PCR-Reaktionen steigt die DNA-Ausbeute hierbei nur linear, da neu gebildete DNA-Moleküle nicht als Templat für die PCR-Reaktion dienen können.

Nach Abschluss der PCR-Reaktion wurde das PCR-Produkt mittels PCR-Purification-Kit (Analytik Jena) aufgereinigt und die parentale, N6-adeninmethylierte DNA mit Hilfe des Restriktionsenzyms *Dpn*I verdaut. Dieses Enzym hat die Besonderheit, dass es unspezifisch N6-adeninmethylierte DNA restringiert, jedoch nicht die neu gebildete, nichtmethylierte DNA. Die Restriktion erfolgte, indem 1 μL *Dpn*I zum PCR-Reaktionsansatz hinzu gegeben und für 2 h oder über Nacht bei 37°C inkubiert wurde.

5 μl dieses Ansatzes wurden zur Transformation von 100 μl chemisch-kompetenten DH5α-Zellen verwendet. Nach Plasmidisolation wurde die erfolgreiche Mutation mittels Sequenzierung bestätigt.

### 7.5 Charakterisierung

Die Mutation wurde über positionsgerichtete Mutagenese nach dem QuikChange-Protokoll in die 7β-HSDH eingeführt. Dabei wurden die Mutageneseprimer aus der im Kapitel 7.1 gezeigten Tabelle verwendet.

Mittels der im Kapitel "Methoden" genannten Assays für die photometrische Aktivitätmessung wurde die Aktivität der mutierten Enzyme in Gegewart von NADPH beziehungsweise NADH gemessen. Die ermittelten Aktivitäte sind in der nachfolgenden Tabelle zusammengefasst.

**Tabelle:** ermittelte Aktivität der verschiedenen 7β-HSDH-Varianten.

| **erzeugte Mutante** | *Volumetrische Aktivität* ***[U/ml]*** | | *Spezifische Aktivität* ***[U/mg]*** | |
|---|---|---|---|---|
| | NADPH | NADH | NADPH | NADH |
| G39D | 119 | 2 | 5,1 | 0,1 |
| G39D/R40I | 0 | 21 | 0 | 0,8 |
| R40D | 0 | 3 | 0 | 0,1 |
| R40D/R41I | 0 | 3 | 0 | 0,2 |
| G39D/R40/R41N | 0 | 6 | 0 | 0,3 |
| 7ß-HSDH (WT) | 134 | 0 | 5,6 | 0 |

### Umsetzungsbeispiel 9: Einsatz von NADH abhängiger 7β-HSDH in der Ganzzellreduktion von Dehydrocholsäure zu 3,12-diketo-UDCA

Nachfolgend soll der Einsatz NADH-abhängiger 7β-HSDH, hergestellt im Herstellungsbeispiel 6. in der ganzzellbiokatalytischen Umsetzung von DHCA zu 3,12-diketo-UDCA demonstriert werden. Zur besseren Verständlichkeit ist eine Übersicht möglicher Umsetzungswege und Reaktionsprodukte in Figur 12 gezeigt.

Im vorliegenden Fall wurden die 7β-HSDH-Mutanten zusammen mit einer NADH-abhängigen Formiatdehydrogenase aus *Mycobacterium vaccae* in einen Expressionsvektor eingefügt. Hierbei trägt der Vektor, in den 7β-HSDH (G39D) eingefügt ist, die Bezeichnung pFr7(D), entsprechend SEQ ID NO:49; der Vektor, in den 7β-HSDH (G39D R40L) eingefügt ist, die Bezeichnung pFr7(DL), entsprechend SEQ ID NO:50; der Vektor, in den 7β-HSDH (G39D R40I) eingefügt ist, die Bezeichnung pFr7(DI), entsprechend SEQ ID NO:51, und der Vektor, in den 7β-HSDH (G39D R40V) eingefügt ist, die Bezeichnung pFr7(DV), entsprechend SEQ ID NO:52. Eine allgemeine Plasmidkarte dieser Vektoren ist in Figur 13 gezeigt.

Diese Vektoren wurden in den Stamm *E. coli* BL49 (identisch mit E.coli BL21(DE3) $hdhA^-$ $KanR^+$ ) transformiert. Von diesen Stämmen wurden zunächst 5 mL Übernachtkulturen in LB-Medium mit 100 mg $L^{-1}$ Ampicillin angezüchtet. Jeweils 1 mL dieser Übernachtkulturen wurden am Folgetag in 200 mL TB-Medium in Schüttelkolben mit 100 mg $L^{-1}$ Ampicillin transferiert. Diese Kulturen wurden bei 37 °C und 250 rpm im Schüttelinkubator kultiviert, bis eine OD von 0,6 - 0,8 erreicht wurde. Anschließend erfolgte die Induktion durch 1 mM Isopropyl-β-D-thiogalactopyranosid (IPTG). Die anschließende Expressionsphase wurde 21 h lang bei 25 °C und 160 rpm durchgeführt. Die Zellernte erfolgte durch 10-minütige Zentrifugation bei 3220 *g*.

Mit den auf diese Weise hergestellten Zellen wurden Ganzzellbiotransformationsreaktionen angesetzt. Die Konzentrationen des Substrats DHCA, und des Produkts 3,12-diketo-UDCA im Reaktionsansatz zu verschiedenen Zeitpunkten wurde durch Hochleistungsflüssigkeitschromatographie (HPLC) ermittelt.

Die Reaktionsansätze für die Biotransformationen enthielten 17,7 g $L^{-1}$BTM Ganzzellbiokatalysatoren, 100 mM Substrat (DHCA), 500 mM Natriumformiat und sind in 50 mM Kaliumformiatpuffer (pH 6,5) suspendiert. Der Prozess lief im 20 mL Maßstab als Satzverfahren ohne pH-Kontrolle ab. Die Konzentrationen an Produkt und Substrat nach 24 h sind in Figur 14 dargestellt. Es ist zu entnehmen, dass sämtliche Mutanten für die ganzzellbiokatalytische Umsetzung von DHCA zu 3,12-diketo-UDCA geeignet sind.

**Umsetzungsbeispiel 10: Einsatz von NADH abhängiger 7β-HSDH in der zweistufigen Ganzzellreduktion von Dehydrocholsäure zu 12-keto-UDCA**

Nachfolgend soll der Einsatz NADH-abhängiger 7β-HSDH in der zweistufigen ganzzellbiokatalytischen Umsetzung von DHCA zu 12-diketo-UDCA demonstriert werden. Die Mutante 7β-HSDH (G39D) wurden hierbei zusammen mit einer NADH-abhängigen Formiatdehydrogenase aus *Mycobacterium vaccae* und einer NADH-abhängigen 3α-HSDH aus *Comamonas testosteroni* in einen Expressionsvektor eingefügt. Hierbei ist die Verwendung von 7β-HSDH (G39D) exemplarisch für sämtliche NADH-abhängigen 7β-HSDH gezeigt. Der Vektor trägt die Bezeichnung pFr3T7(D) und ist schematisch in Figur 15 dargestellt.

Diese Vektoren wurden in den Stamm *E. coli* BL49 (identisch mit *E.coli* BL21(DE3) $hdhA^-$ $KanR^+$) transformiert, der entstehende Stamm trägt die Bezeichnung *E. coli* BL49 pFr3T7(D). Dieser Stamm wurde im 7 L-Bioreaktor bei einem Arbeitsvolumen von 4 L im definierten Minimalmedium kultiviert. Nach einer kurzen Satzphase bei 37 °C erfolgte eine substratlimitierte exponentielle Wachstumsphase bei 30 °C. Bei Erreichen einer Optischen Dichte von 25 - 30 wurde die Proteinexpression der Zellen durch Zugabe von 0,5 mM IPTG induziert. Die Expression erfolgte danach für 24 h bei 20 °C. Die Zellen (32,0 g L-1BTM) wurden durch Zentrifugation geerntet, mit Kaliumphosphatpuffer (pH 6,5) resuspendiert und nach Standardprotokoll bei -20 °C gelagert.

Die Biotransformation wurde im 20 mL-Maßstab unter folgenden Reaktionsbedingungen angesetzt: 100 mM DHCA, 17,7 g $L\text{-}1_{BTM}$ des gelagerten Biokatalysators, 500 mM Ammoniumformiat, 26 % Glycerin, 50 mM $MgCl_2$, suspendiert in 50 mM KPi-Puffer (pH 6,5). Durch manuelle pH-Einstellung wurde der pH während der ersten 5 Stunden des Biotransformationsprozesses auf pH 6,5 gehalten. Der zeitliche Verlauf der Gallensalzkonzentrationen ist in Figur 16 dargestellt. Nach 24 h konnten 92 % des Produkts 12-keto-UDCA gebildet werden. Die Umsetzung von DHCA zu 3,12-diketo-UDCA und die Umsetzung von 7,12-diketo-UDCA zu 12-keto-UDCA zeigt, dass sämtliche in Figur 12 dargestellten Umsetzungen der 7β-HSDH tatsächlich durch 7β-HSDH (G39D) katalysiert werden.

**Umsetzungsbeispiel 11: Zweistufige Reduktion von Dehydrocholsäure in einem Einzell-System**

Ein Syntheseweg für Ursodesoycholsäure beinhaltet die chemische Oxidation von Cholsäure zu Dehydrocholsäure mit den anschließenden asymmetrischen enzymatischen Reduktionen der 3-Carbonylgruppe zur 3α-Hydroxygruppe und der 7-Carbonylgruppe zur 7β-Hydroxygruppe gefolgt von der chemischen Entfernung der 12-Carbonylgruppe.

Für diesen Syntheseweg werden zur Katalyse der enzymatischen Reduktionsreaktionen die Enzyme 7β-Hydroxysteroiddehydrogenase (7β-HSDH) und 3α-Hydroxysteroiddehydrogenase (3α-HSDH) benötigt. Bei der Umsetzung werden zudem Kofaktoren (NADH bzw. NADPH) stöchiometrisch verbraucht, die in einem wirtschaftlichen Prozess regeneriert werden müssen. Hierzu werden häufig enzymatische Regenerierungsmöglichkeiten bevorzugt. Geeignete Enzyme sind unter anderem Formiatdehydrogenasen (FDH), Glucosedehydrogenasen (GDH), Glucose-6-Phosphat-Dehydrogenasen (G6PDH), Alkoholdehydrogenasen (ADH) oder Phosphitdehydrogenasen (PtDH).

Anders als beim Einsatz von isolierten Enzymen ist es bei einer Ganzzellbiokatalyse, bei der erforderliche Enzyme innerhalb eines Wirtsorganismus, typischerweise einem einzelligen Mikroorganismus, nicht möglich, einzelne Enzyme in individuellen Mengen zum Reaktionsmedium zuzugeben. Hierbei muss die Ausbalancierung der Enzymaktivitäten durch Modifikation der Expressionsstärken aller beteiligten Enzyme entweder durch Kultivierungsmethoden oder durch gentechnische Modifikation des Ganzzellbiokatalysators erfolgen.

Im vorliegenden Beispiel wurden erfindungsgemäß Vektoren verwendet, die sämtliche drei Gene für 7β-HSDH, 3α-HSDH und FDH enthalten. Die Vektoren sind so konstruiert, dass die Expressionsstärke der drei Gene im designierten Wirtsstamm, insbesondere auf *Escherichia coli* BL21 (DE3) basierende Ganzzellbiokatalysatorstämmen, in der Form eingestellt sind, dass alle drei Enzyme möglichst ähnliche Enzymaktivitäten aufweisen.

Die drei an der Ganzzellreduktion von Dehydrocholsäure zu 12-keto-Ursodesoxycholsäure benötigten Gene liegen auf ein und demselben Vektor. Es handelt sich um Gene, die für folgende Enzyme kodieren: eine NADPH-abhängige 7β-HSDH aus *Collinella aerofaciens,* eine NADH-abhängige 3α-HSDH aus *Comamonas testosteroni,* eine sowohl NAD- als auch NADP-abhängige, mutierte FDH aus *Mycobacterium vaccae.* Die Expressionsstärken dieser drei

Gene ist durch das genetische Konstrukt dahingehend ausbalanciert, dass eine möglichst optimale Ganzzellbiokatalyse durchführbar ist. Im vorliegenden Beispiel wurden die 7β-HSDH-Mutanten G39A und G39S statt eines unmodifizierten Wildtyp-Enzyms in einen Vektor für die Ganzzellbiokatalyse eingesetzt. Die Vektoren tragen die Bezeichnungen pF(G)r7(A)r3 und pF(G)r7(S)r3 und sind in den Figuren 17a und b dargestellt. Figur 18 zeigt eine schematische Darstellung des möglicher Umsetzungswege und Reaktionsprodukte.

Diese erfindungsgemäßen Vektoren wurden in *Escherichia coli*-Produktionsstämme transformiert. Diese Stämme stellen den Ganzzellbiokatalysator dar. Bei dem verwendeten Wirtstamm handelt es sich um einen modifizierten *E. coli* BL21 (DE3) mit der Bezeichnung *E. coli* BL49. Allerdings ist der Wirtsorganismus nicht auf diesen Wirtsstamm beschränkt. Der mit pF(G)r7(A)r3 transformierte *E. coli* BL49 trägt die Bezeichnung *E. coli* BL49 pF(G)r7(A)r3, der mit pF(G)r7(S)r3 transformierte *E. coli* BL49 trägt die Bezeichnung *E. coli* BL49 pF(G)r7(S)r3.

### 11.1 Zweistufige Ganzzellreduktion von Dehydrocholsäure im 20 mL-Maßstab

Der Vergleich der Biokatalysatoren erfolgte durch Ganzzellbiotransformation im 20 mL-Maßstab. Hierzu wurde zunächst eine 5 mL Übernachtkultur in LB-Medium mit 100 mg L-1 Ampicillin angezüchtet. 1 mL dieser Übernachtkultur wurde am Folgetag in 200 mL TB-Medium in Schüttelkolben mit 100 mg L-1 Ampicillin transferiert. Diese Kulturen wurden bei 37 °C und 250 rpm im Schüttelinkubator kultiviert, bis eine OD von 0,6 - 0,8 erreicht wurde. Anschließend erfolgte die Induktion durch 1 mM Isopropyl-β-D-thiogalactopyranosid (IPTG). Die anschließende Expressionsphase wurde 21 h lang bei 25 °C und 160 rpm durchgeführt. Die Zellernte erfolgte durch 10-minütige Zentrifugation bei 3220 g.

Mit den auf diese Weise hergestellten Zellen wurden Ganzzellbiotransformationsreaktionen angesetzt. Maßgeblich für die Beurteilung der Zellen war die Menge des gebildeten Produktes (12-keto-UDCA) im Reaktionsansatz. Die Konzentrationen des Substrats DHCA, der Intermediate 3,12-diketo-UDCA und 7,12-diketo-UDCA sowie des Produkts 12-keto-UDCA im Reaktionsansatz wurden durch Hochleistungsflüssigkeitschromatographie (HPLC) ermittelt.

Die Reaktionsansätze für die Biotransformationen enthielten 11,8 g L-1BTM Ganzzellbiokatalysatoren, 100 mM Substrat (DHCA), 500 mM Natriumformiat und waren in 50 mM Kaliumformiatpuffer (pH 6,5) suspendiert. Der Prozess lief im 20 mL Maßstab als Satzverfahren ohne pH-Kontrolle ab. In der nachfolgenden Tabelle sind Ergebnisse der oben beschriebenen Ganzzellbiotransformation nach 5 h Prozess dargestellt. Hier sind beide neuen Stämme *E. coli* BL49 pF(G)r7(S)r3 und *E. coli* BL49 pF(G)r7(A)r3 einem Referenzstamm *E. coli* BL49 pF(G)r7 pC3 gegenübergestellt. Während beim Referenzstamm *E. coli* BL49 pF(G)r7 pC3 lediglich 5,7 ± 1,0 % Produkt (12-keto-UDCA) gebildet wurden, konnte mit den neuen Biokatalysatoren beim Stamm *E. coli* BL49 pF(G)r7(S)r3 38,4 ± 8,2 % 12-keto-UDCA und beim Stamm *E. coli* BL49 pF(G)r7(A)r3 47.7 ± 2,1 % 12-keto-UDCA gebildet werden. Damit wurde die Produktkonzentration im Reaktionsansatz gegenüber dem Referenzstamm auf das 6,7-fache (*E. coli* BL49 pF(G)r7(S)r3) beziehungsweise auf das 8,4-fache (*E. coli* BL49 pF(G)r7(A)r3) gesteigert.

Die folgende Tabelle zeigt dementsprechend Gallensalzanteile von Biotransformationsansätzen nach 5 h bei Einsatz von 100 mM Substrat (DHCA). Gezeigt sind die Ansätze mit den zwei neuen Biokatalysatorstämmen *E. coli* BL49 pF(G)r7(A)r3 und *E. coli* BL49 pF(G)r7(S)r3 im Vergleich mit dem Stamm nach dem bisherigen Stand der Technik *E. coli* BL49 pF(G)r7 pC3:

| | Gallensalzanteile, % | | | |
|---|---|---|---|---|
| | 12-keto-UDCA | 3,12-diketo-UDCA | 7,12-diketo-UDCA | DHCA |
| E. coli BL49 pF(G)r7 pC3 | 5,7 ± 1,0 % | 9,2 ± 0,2 % | 40,5 ± 1,9 % | 44,6 ± 3,1 % |
| E. coli BL49 pF(G)r7(A)r3 | 47.7 ± 2,1 % | 2,4 ± 0,5 % | 48,9 ± 1,6 % | 1,0 ± 0,0% |
| E. coli BL49 pF(G)r7(S)r3 | 38,4 ± 8,2 % | 2,7 ± 2,7 % | 57,4 ± 4,4 % | 1,5 ± 1,8 % |

### 11.2 Zweistufige Ganzzellreduktion von Dehydrocholsäure im 1 L-Maßstab

Durch Prozesskontrolle wurde die Produktbildung mit dem Biokatalysator *E. coli* BL49 pF(G)r7(A)r3 gegenüber dem Millilitermaßstab gesteigert. Hierzu wurde der Biokatalysator im 7 L-Bioreaktor bei einem Arbeitsvolumen von 4 L im definierten Minimalmedium kultiviert. Nach einer kurzen Satzphase bei 37 °C erfolgte eine substratlimitierte exponentielle Wachstumsphase bei 30 °C. Bei Erreichen einer optischen Dichte von 25 - 30 wurde die Proteinexpression der Zellen durch Zugabe von 0,5 mM IPTG induziert. Die Expression erfolgte danach für 10 h bei 25 °C, bevor die Zellen durch Zentrifugation geerntet und bei -20 °C gelagert wurden.

Die Biotransformation wurde in einem 1 L-Bioreaktor unter folgenden Reaktionsbedingungen angesetzt: 70 mM DHCA, 17,7 g L-1BTM des gelagerten Biokatalysators, 500 mM Natriumformiat, 26 % (v/v) Glycerin, 50 mM MgCl2, suspendiert in 50 mM KPi-Puffer (pH 6,5). Durch pH-Regelung wurde der pH während der gesamten Biotransformation

auf pH 6,5 gehalten. Die zeitlichen Verläufe der Gallensalzkonzentrationen sind in Figur 19 dargestellt. Nach 21 h konnten 99,4 % Produkt (12-keto-UDCA) gebildet werden und lediglich 0,6 % des Nebenprodukts 3,12-diketo-UDCA waren nachweisbar.

**Umsetzungsbeispiel 12: Zweistufige Reduktion von Dehydrocholsäure in einem Einzell-System unter Verwendung einer Glukose-Dehydrogenase zur Kofaktorregenerierung**

In diesem Beispiel wird in einem Ganzzellbiokatalysator (Einzell-System) für die zweistufige Reduktion von DHCA zu 12-keto-UDCA neben einer NADPH-abhängigen 7β-HSDH aus *Collinella aerofaciens* (Mutante G39A) und einer NADH-abhängigen 3α-HSDH aus *Comamonas testosteroni* eine sowohl NAD- als auch NADP-abhängige GDH aus *Bacillus subtilis* für die Kofaktorregenerierung exprimiert. Die Nukleinsäuresequenz und die zugehörige Aminosäuresequenz dieser GDH sind als SEQ ID NO:47 beziehungsweise SEQ ID NO:48 angegeben.

Die dafür verwendeten Vektoren tragen die Bezeichnungen p3T7(A)rG und p7(A)T3rG und sind in Figur 20 beziehungsweise Figur 21 dargestellt.

Die erfindungsgemäßen Vektoren wurden in *Escherichia coli*-Produktionsstämme transformiert. Diese Stämme stellen den Ganzzellbiokatalysator dar. Bei dem verwendeten Wirtstamm handelt es sich um einen modifizierten *E. coli* BL21 (DE3) mit der Bezeichnung *E. coli* BL49 (identisch mit E.coli BL21(DE3) $hdhA^-$ $KanR^+$). Allerdings ist der Wirtsorganismus nicht auf diesen Wirtsstamm beschränkt. Der mit p7(A)T3rG transformierte *E. coli* BL49 trägt die Bezeichnung *E. coli* BL49 p7(A)T3rG, der mit p3T7(A)rG transformierte *E. coli* BL49 trägt die Bezeichnung *E. coli* BL49 p3T7(A)rG.

Mit den neu hergestellten Biokatalysatoren bei kann Einsatz von insgesamt 17,7 g/L $_{BTM}$ Biokatalysatoren 100 mM DHCA zu 98 % zu 12-keto-UDCA umgesetzt werden.

**12.1 Zweistufige Ganzzellreduktion von Dehydrocholsäure im 20 mL-Maßstab**

Der Vergleich der Biokatalysatoren erfolgte durch Ganzzellbiotransformation im 20 mL-Maßstab. Die Kultivierung und die Konzentrationsermittlung des Substrats DHCA, der Intermediate 3,12-diketo-UDCA und 7,12-diketo-UDCA sowie des Produkts 12-keto-UDCA im Reaktionsansatz erfolgte wie im Umsetzungsbeispiel 11.1 beschrieben.

Die Reaktionsansätze für die Biotransformationen enthielten 11,8 g/L BTM Ganzzellbiokatalysatoren, 100 mM Substrat (DHCA), 500 mM Glucose und waren in 50 mM Kaliumformiatpuffer (pH 7,3) suspendiert. Der Prozess lief im 20 mL Maßstab als Satzverfahren ohne pH-Kontrolle ab. Eine Perforation der Biokatalysatoren war nicht notwendig. In der nachfolgenden Tabelle sind Ergebnisse der oben beschriebenen Ganzzellbiotransformation nach 24 h Prozess dargestellt, wobei die Ergebnisse der Ganzzellbiotransformation mit den beiden Stämmen *E. coli* BL49 p7(A)T3rG und *E. coli* BL49 p3T7(A)rG gezeigt sind. Mit den neuen Biokatalysatoren kann unter Einsatz von 11,8 g L-1BTM Biokatalysator innerhalb von 24 h 100 mM Dehydrocholsäure (DHCA) zu 46 % (*E. coli* BL49 p3T7(A)rG) beziehungsweise zu 68 % (*E. coli* BL49 p7(A)T3rG) zu 12-keto-Ursodesoxycholsäure (12-keto-UDCA) umgesetzt werden. In der nachfolgenden Tabelle sind Gallensalzanteile von Biotransformationsansätzen nach 24 h bei Einsatz von 100 mM Substrat (DHCA) gezeigt (Ansätze mit den zwei neuen Biokatalysatorstämmen *E. coli* BL49 p3T7(A)rG und *E. coli* BL49 p7(A)T3rG):

| | Gallensalzanteile, % | | | |
|---|---|---|---|---|
| | 12-keto-UDCA | 3,12-diketo-UDCA | 7,12-diketo-UDCA | DHCA |
| *E. coli* BL49 p3T7(A)rG | 46 ± 6 % | 7 ± 1 % | 7 ± 1 % | 40,0 ± 2% |
| *E. coli* BL49 p7(A)T3rG | 68 ± 19% | 15 ± 3 % | 1 ± 1 % | 12 ± 10 % |

**12.2 Zweistufige Ganzzellreduktion von Dehydrocholsäure mit im Bioreaktor kultivierten Zellen des Stammes E.coli BL49 p7(A)T3rG im 20 mL-Maßstab**

Der Stamm *E. coli* BL49 p7(A)T3rG wurde im 7 L-Bioreaktor bei einem Arbeitsvolumen von 4 L im definierten Minimalmedium kultiviert. Nach einer kurzen Satzphase bei 37 °C erfolgte eine substratlimitierte exponentielle Wachstumsphase bei 30 °C. Bei Erreichen einer optischen Dichte von 25 - 30 wurde die Proteinexpression der Zellen durch Zugabe von 0,5 mM IPTG induziert. Die Expression erfolgte danach für 24 h bei 20 °C. Die Zellen (46,7 g / L BTM) wurden durch Zentrifugation geerntet, mit Kaliumphosphatpuffer (pH 6,5) resuspendiert und nach Standardprotokoll bei -20 °C gelagert. Die Enzymaktivitäten betrugen zum Erntezeitpunkt: 16,4 U/mL 7β-HSDH, 3,6 U/mL 3α-HSDH, 44,9 U/mL GDH (NADP), 18,1 U/mL GDH (NAD).

Die Biotransformation wurde im 20 mL-Maßstab unter folgenden Reaktionsbedingungen angesetzt: 100 mM

DHCA, 17,7 g/L BTM des gelagerten Biokatalysators, 500 mM Glucose, 10 mM $MgCl_2$, suspendiert in 50 mM KPi-Puffer (pH 7). Durch manuelle pH-Einstellung wurde der pH während der gesamten Biotransformation auf pH 7 gehalten. Die Reaktionen wurden entweder ohne Kofaktorzugabe oder mit Zugabe von 0,1 mM NAD durchgeführt. Die zeitlichen Verläufe der Gallensalzkonzentrationen sind in Figur 22 dargestellt. Nach 2 h wurden mit und ohne NAD-Zugabe jeweils ≥98 % Produkt (12-keto-UDCA) gebildet.

Bei Einsatz von insgesamt 17,7 g/L BTM Biokatalysatoren wurde 100 mM DHCA zu 98 % zu 12-keto-UDCA umgesetzt.

**Umsetzungsbeispiel 13: Zweistufige Reduktion von Dehydrocholsäure unter paralleler Verwendung von zwei verschiedenen Ganzzellbiokatalysatoren**

Im vorliegenden Beispiel wurden zwei Ganzzellbiokatalysatoren anstelle von einem Ganzzellbiokatalysator verwendet. Für diesen Zweck wurden in einem dieser Biokatalysatoren eine NADP-spezifische FDH aus *Mycobacterium vaccae* und eine NADPH-spezifische 7β-HSDH aus *Collinsella aerofaciens* exprimiert, während im anderen Biokatalysator eine NAD-abhängige FDH aus *Mycobacterium vaccae* und eine NADH-abhängige 3α-HSDH aus *Comamonas testosteroni* exprimiert wurden

Im vorliegenden Beispiel wurden konkret der Vektor pF(G)r7(A), welcher Gene für eine NADP-spezifische FDH aus *Mycobacterium vaccae* und für eine NADPH-spezifische 7β-HSDH aus *Collinsella aerofaciens* beinhaltet, sowie der Vektor pFr3, welcher Gene für eine NAD-abhängige FDH aus *Mycobacterium vaccae* und für eine NADH-abhängige 3α-HSDH aus *Comamonas testosteroni* beinhaltet, verwendet. Die Vektoren pF(G)r7(A) und pFr3 sind in Figur 23 a und b dargestellt. Figur 24 zeigt eine Reaktionsschema mit möglichen Reaktionswegen und Produkten. Allerdings ist die Erfindung nicht auf diese zwei genannten Vektoren beschränkt, sondern kann sämtliche denkbaren Vektoren umfassen, die Gene für eine 7β-HSDH und ein geeignetes Kofaktorregenerationsenzym in Kombination mit Vektoren, die Gene für eine 3α-HSDH und ein geeignetes Kofaktorregenerationsenzym beinhalten.

**13.1 Ganzzellreduktion von DHCA unter Einsatz zweier Biokatalysatoren in unterschiedlichen Mischungsverhältnissen**

Im folgenden Beispiel ist die Ganzzellreduktion von DHCA zu 12-keto-UDCA mit unterschiedlichen Mischungsverhältnisse der Biokatalysatoren *E. coli* BL49 pF(G)r7(A) und *E. coli* BL49 pFr3 gezeigt. Hierbei wurden bei drei Ansätzen jeweils 17,7 g $L^{-1}_{BTM}$ Gesamtbiokatalysatormenge eingesetzt. Allerdings wurden in den Ansätzen unterschiedliche Anteile der zwei Biokatalysatoren eingesetzt. Diese sind

- 8,85 g L-1BTM *E. coli* BL49 pF(G)r7(A) und 8,85 g $L^{-1}_{BTM}$ *E. coli* BL49 pFr3
- 10,33 g L-1BTM *E. coli* BL49 pF(G)r7(A) und 7,38 g $L^{-1}_{BTM}$ *E. coli* BL49 pFr3
- 11,80 g L-1BTM *E. coli* BL49 pF(G)r7(A) und 5,90 g $L^{-1}_{BTM}$ *E. coli* BL49 pFr3

Diese Biokatalysereaktionen wurden bei einem Arbeitsvolumen von 20 mL durchgeführt. Weitere Bestandteile der Ansätze waren: 70 mM DHCA, 500 mL Natriumformiat, 26 % Glycerin, 50 mM MgCl2, suspendiert in 50 mL Kaliumphosphatpuffer (pH 6,5). Die Reaktionen wurden 24 h lang durchgeführt. Die Gallensalzanteile nach 24 h sind in Fig. 25 gezeigt.

Im Ansatz mit 8,85 g $L^{-1}_{BTM}$ *E. coli* BL49 pF(G)r7(A) und 8,85 g $L^{-1}_{BTM}$ *E. coli* BL49 pFr3 werden 79 % 12-keto-UDCA, 4 % 3,12-diketo-UDCA und 17 % 7,12-diketo-UDCA gebildet, im Ansatz mit 10,33 g $L^{-1}_{BTM}$ *E. coli* BL49 pF(G)r7(A) und 7,38 g $L^{-1}_{BTM}$ *E. coli* BL49 pFr3 werden 87 % 12-keto-UDCA, 9 % 3,12-diketo-UDCA und 4 % 7,12-diketo-UDCA gebildet und im Ansatz mit 11,80 g $L^{-1}_{BTM}$ *E. coli* BL49 pF(G)r7(A) und 5,90 g $L^{-1}_{BTM}$ *E. coli* BL49 pFr3 werden 71 % 12-keto-UDCA und 29 % 3,12-diketo-UDCA gebildet. An den Anteilen der gebildeten Intermediate ist zu erkennen, dass bei dem Ansatz mit 8,85 g $L^{-1}_{BTM}$ *E. coli* BL49 pF(G)r7(A) und 8,85 g $L^{-1}_{BTM}$ *E. coli* BL49 pFr3 die Reaktion der 3α-HSDH mit erhöhter Geschwindigkeit abläuft, da hier ein hoher Anteil am Intermediat 7,12-diketo-UDCA gebildet wird, während bei dem Ansatz 11,80 g $L^{-1}_{BTM}$ *E. coli* BL49 pF(G)r7(A) und 5,90 g $L^{-1}_{BTM}$ *E. coli* BL49 pFr3 die Reaktion der 7β-HSDH mit erhöhter Geschwindigkeit abläuft und dementsprechend ein hoher Anteil am Intermediat 3,12-diketo-UDCA gebildet wird. Beim Ansatz mit 10,33 g $L^{-1}_{BTM}$ *E. coli* BL49 pF(G)r7(A) und 7,38 g $L^{-1}_{BTM}$ *E. coli* BL49 pFr3 sind die Reaktionen der 7β-HSDH und der 3α-HSDH so abgestimmt, dass beide ungefähr gleich schnell ablaufen, was an den annähernd gleichmäßigen Intermediatbildungen zu sehen ist. Infolge dessen kann bei diesem Ansatz der höchste Anteil am Produkt 12-keto-UDCA gebildet werden (87 %).

Dieses Beispiel zeigt, wie sich durch Anpassung der eingesetzten Biokatalysatoranteile die Geschwindigkeit einzelner Reaktionsschritte beeinflussen lassen.

**13.1 Ganzzellreduktion von DHCA unter Einsatz zweier Biokatalysatoren im 1 L-Maßstab**

Durch Prozesskontrolle konnte die Produktbildung unter Einsatz der Biokatalysatoren *E. coli* BL49 pF(G)r7(A) und *E. coli* BL49 pFr3 gegenüber dem Millilitermaßstab gesteigert werden. Die Biotransformation wurde in einem 1 L-Bioreaktor unter folgenden Reaktionsbedingungen angesetzt: 90 mM DHCA, mit 8,85 g $L^{-1}_{BTM}$ *E. coli* BL49 pF(G)r7(A) und 8,85 g $L^{-1}_{BTM}$ *E. coli* BL49 pFr3, 500 mM Ammoniumformiat, 26 % (v/v) Glycerin, 50 mM MgCl2, suspendiert in 50 mM KPi-Puffer (pH 6,5). Durch pH-Regelung mit Ameisensäure wurde der pH während der gesamten Biotransformation auf pH 6,5 gehalten. Die zeitlichen Verläufe der Gallensalzkonzentrationen sind in Fig. 26 dargestellt. Nach 20 h konnten 99,4 % Produkt (12-keto-UDCA) gebildet werden und lediglich insgesamt 0,6 % der Intermediate 3,12-diketo-UDCA und 7,12-diketo-UDCA waren nachweisbar.

Mit der Verwendung der erfindungsgemäßen Biokatalysatoren konnten hier bei Einsatz von insgesamt 17,7 g $L^{-1}_{BTM}$ Biokatalysatoren 90 mM DHCA zu 99,5 % zu 12-keto-UDCA umgesetzt werden.

Zuordnung von SEQ ID NOs:

| SEQ ID NO: | Beschreibung | Typ |
|---|---|---|
| 1 | 7ß-HSDH | NS |
| 2 | 7ß-HSDH | AS |
| 3 | Primer S 7beta_rev_HindIII | NS |
| 4 | Primer | NS |
| 5 | 3α-HSDH (C. testosteroni) | NS |
| 6 | 3α-HSDH (C. testosteroni) | AS |
| 7 | 3α-HSDH (R. norvegicus) | NS |
| 8 | 3α-HSDH (R. norvegicus) | AS |
| 9 | Primer 7beta_mut_G39A_fwd | NS |
| 10 | Primer 7beta_mut_G39A_rev | NS |
| 11 | Primer 7beta_mut_G39S_fwd | NS |
| 12 | Primer 7beta_mut_G39S_rev | NS |
| 13 | Primer 7beta_fwd_EcoRI | NS |
| 14 | FDH D221G | NS |
| 15 | FDH D221G | AS |
| 16 | Primer 3alpha_fwd_Hindlll | NS |
| 17 | Primer 3alpha_rev_Notl | NS |
| 18 | pET22a FDH D221G 7ß-HSDH | NS |
| 19 | FDH D221G | AS |
| 20 | 7beta-HSDH | AS |
| 21 | pCOLA(mod) 3α-HSDH | NS |
| 22 | 3α-HSDH | AS |
| 23 | Primer fdh_for | NS |
| 24 | Primer fdh_rev | NS |
| 25 | 7α-HSDH | NS |
| 26 | 7α-HSDH | AS |
| 27 | Primer 467\|468a-IBS | NS |

(fortgesetzt)

| SEQ ID NO: | Beschreibung | Typ |
|---|---|---|
| 28 | Primer 467\|468a-EBS1d | NS |
| 29 | Primer 467\|468a-EBS2 | NS |
| 30 | Primer mt1 | NS |
| 31 | Primer NI_fdh_R | NS |
| 32 | Primer 7alpha-ko-check_fwd | NS |
| 33 | Primer 7alpha-ko-check_rev | NS |
| 34 | FDH D221G mit Deletion und His-Tag | NS |
| 35 | FDH D221G mit Deletion und His-Tag | AS |
| 36 | FDH Wildtyp, M. vaccae | AS |
| 37 | 7β-HSDH G39D | AS |
| 38 | 7β-HSDH G39D/R40L | AS |
| 39 | 7β-HSDH G39D/R40I | AS |
| 40 | 7β-HSDH G39D/R40V | AS |
| 41 | Primer für G39D (7beta mut G39D fwd) | NS |
| 42 | Primer für G39D (7beta mut G39D rev) | NS |
| 43 | Primer für G39D R40L (7beta mut G39D R40L fwd) | NS |
| 44 | Primer 3alphamut_AntiMid_rev | NS |
| 45 | Primer für G39D R40I (7beta mut G39D | NS |
| | R40I fwd) | |
| 46 | Primer 7beta mut G39D R40V fwd | NS |
| 47 | GDH, B. subtilis | NS |
| 48 | GDH B. subtilis | AS |
| 49 | Vektor pFr7(D) | NS |
| 50 | Vektor pFr7(DL) | NS |
| 51 | Vektor pFr7(DI) | NS |
| 52 | Vektor pFr7(DV) | NS |
| 53 | PCR Primer "G39D for" | NS |
| 54 | PCR Primer "G39D_rev" | NS |
| 55 | PCR Primer "G39D/R40I_for" | NS |
| 56 | PCR Primer "G39D/R40I_rev" | NS |
| 57 | PCR Primer "R40D_for" | NS |
| 58 | PCR Primer "R40D_rev" | NS |
| 59 | PCR Primer "R40D/R41I_for" | NS |
| 60 | PCR Primer "R40D/R41I_rev" | NS |
| 61 | PCR Primer "DIN_for" | NS |
| 62 | PCR Primer "DIN_rev" | NS |
| 63 | Plasmid pFr3T7(D), Fig. 15 | NS |
| 64 | Plasmid pF(G)r7(A)r3, Fig. 17a | NS |

(fortgesetzt)

| SEQ ID NO: | Beschreibung | Typ |
|---|---|---|
| 65 | Plasmid pF(G)r7(S)r3, Fig. 17b | NS |
| 66 | Plasmid p3T7(A)rG, Fig. 20 | NS |
| 67 | Plasmid p7(A)T3rG, Fig. 21 | NS |
| 68 | Plasmid pF(G)r7(A), Fig. 23a | NS |
| 69 | Plasmid pFr3, Fig. 23b | NS |
| AS = Aminosäuresequenz<br>NS = Nukleinsäuresequenz | | |

**Patentansprüche**

1. 7ß-HSDH, welche wenigstens die stereospezifische enzymatische Reduktion eines 7-Ketosteroids zum korrespondierenden 7-Hydroxysteroid katalysiert, wobei das Enzym wenigstens eine Mutation im Sequenzmotiv VMVGRRE gemäß Position 36 bis 42 von SEQ ID NO:2 und wenigstens 80 % Sequenzidentität zu SEQ ID NO:2 aufweist.

2. 7ß-HSDH, welche wenigstens die stereospezifische enzymatische Reduktion eines 7-Ketosteroids zum korrespondierenden 7-Hydroxysteroid katalysiert, wobei das Enzym wenigstens eine Mutation im Sequenzmotiv VMVGRRE gemäß Position 36 bis 42 von SEQ ID NO:2 und wenigstens 60 % Sequenzidentität zu SEQ ID NO:2 aufweist, ausgewählt unter

    a) den Einfachmutanten $G39X_1$ und $R40X_2$
    b) den Zweifach-Mutanten $G39X_1$ $R40X_2$; $R40X_2$ $R41X_3$ und $G39X_1$ $R41X_3$ oder
    c) der Dreifach-Mutante $G39X_1$ $R40X_2$ $R41X_3$,

    wobei $X_1$, $X_2$ und $X_3$ jeweils unabhängig voneinander für einen mutierten Aminosäurerest stehen.

3. Rekombinanter Mikroorganismus, der wenigstens eine Nukleinsäuresequenz kodierend für ein Enzym nach einem der vorhergehenden Ansprüche oder wenigstens eine diese kodierende Nukleinsäuresequenz enthaltende Expressionskassette oder wenigstens einen diese kodierende Nukleinsäuresequenz enthaltenden Vektor trägt und zusätzlich gegebenenfalls die kodierende Sequenz für ein weiteres Enzym, ausgewählt unter Hydroxysteroiddehydrogenasen, wie eine 3α-Hydroxysteroiddehydrogenase (3α-HSDH), und zur Kofaktorregenerierung geeigneten Dehydrogenasen, trägt.

4. Verfahren zur enzymatischen oder mikrobiellen Synthese von 7ß-Hydroxysteroiden, wobei man das korrespondierende 7-Ketosteroid in Gegenwart einer 7ß-HSDH gemäß der Definition in einem der Ansprüche 1 und 2 oder in Gegenwart eines dieses Enzym exprimierenden rekombinanten Mikroorganismus umsetzt, und wenigstens ein gebildetes Reduktionsprodukt aus dem Reaktionsansatz gegebenenfalls isoliert.

5. Verfahren nach Anspruch 4, wobei die Reduktion in Gegenwart und insbesondere unter Verbrauch von NADPH und/oder NADH erfolgt.

6. Verfahren nach Anspruch 5, wobei verbrauchtes NADPH durch Kopplung mit einem NADPH-regenerierenden Enzym regeneriert wird, wobei dieses insbesondere ausgewählt ist unter NADPH Dehydrogenasen, Alkoholdehydrogenasen (ADH), und NADPH regenerierenden Formiatdehydrogenasen (FDH) und einer NADPH-regenerierenden Glucosedehydrogenase (GDH), Glucose-6-Phosphat-Dehydrogenase (G-6-PDH), oder Phosphit-Dehydrogenasen (PtDH), wobei das NADPH-regenerierende Enzym gegebenenfalls von einem rekombinanten Mikroorganismus exprimiertwird; und/oderwobei verbrauchtes NADH durch Kopplung mit einem NADH-regenerierenden Enzym regeneriert wird, wobei dieses insbesondere ausgewählt ist unter NADH-Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH), wobei das NADH-regenerierende Enzym ggf. in einem rekombinanten Mikroorganismus exprimiert wird.

7. Verfahren nach Anspruch 6, wobei das NADPH-regenerierende Enzym ausgewählt ist unter einer $NAD^+$-abhängigen Formiatdehydrogenase (FDH), welche wenigstens die enzymatische Oxidation von Ameisensäure zu $CO_2$ katalysiert, und zusätzlich $NADP^+$ als Kofaktor akzeptiert.

8. Verfahren nach Anspruch 7, wobei die $NADP^+$-akzeptierende FDH wenigstens eine Mutation in der Aminosäure-Sequenz einer FDH aus *Mycobacterium vaccae* N10 gemäß SEQ ID NO: 36 oder einer davon abgeleiteten Aminosäuresequenz mit wenigstens 60 % Sequenzidentität zu SEQ ID NO: 36 aufweist.

**9.** Verfahren nach einem der Ansprüche 7 oder 8, wobei die NADP$^+$-akzeptierende FDH wenigstens eine Mutation im Sequenzmotiv TDRHRL gemäß Position 221 bis 226 von SEQ ID NO:36 und wenigstens 60 % Sequenzidentität zu SEQ ID NO: 36 aufweist.

**10.** Nukleinsäure, ausgewählt unter solchen,

a) gleichzeitig kodierend für eine FDH nach einem der Ansprüche 7 bis 9 und eine 7ß-HSDH nach einem der Ansprüche 1 und 2 und gegebenenfalls eine 3α-HSDH; oder
b) kodierend für ein Fusionsprotein umfassend eine FDH nach einem der Ansprüche 7 bis 9 und eine 7ß-HSDH nach einem der Ansprüche 1 und 2 und gegebenenfalls eine 3α-HSDH; oder
c) gleichzeitig kodierend für FDH und eine 7β-HSDH nach einem der Ansprüche 1 und 2 und ggf. eine 3α-HSDH; oder
d) kodierend für ein Fusionsprotein, umfassend die FDH, eine 7β-HSDH nach einem der Ansprüche 1 bis 2 und ggf. eine 3α-HSDH; oder
e) gleichzeitig kodierend für eine GDH, eine 7β-HSDH nach einem der Ansprüche 1 und 2 und ggf. eine 3α-HSDH; oder
f) kodierend für ein Fusionsprotein, umfassend eine GDH, eine 7β-HSDH nach einem der Ansprüche 1 und 2 und ggf. eine 3α-HSDH.

**11.** Rekombinanter Mikroorganismus, der wenigstens eine Nukleinsäuresequenz gemäß Anspruch 10 trägt.

**12.** Rekombinanter Mikroorganismus, welcher zur gleichzeitigen Expression einer 7ß-HSDH nach einem der Ansprüche 1 oder2, einer NADP$^+$-akzeptierenden FDH nach einem der Ansprüche 7 bis 9 und/ oder den entsprechenden NAD$^+$-abhängigen FDH und gegebenenfalls von 3α-HSDH befähigt ist; oder welcher zur gleichzeitigen Expression einer 7β-HSDH nach einem der Ansprüche 1 oder 2, einer GDH und ggf. von 3α-HSDH befähigt ist.

**13.** Rekombinanter Mikroorganismus nach Anspruch 12, wobei die FDH ein Enzym gemäß der Definition in einem der Ansprüche 7 bis 9 ist; und wobei die NAD$^+$-abhängige FDH eine FDH aus *Mycobacterium vaccae* N10 gemäß SEQ ID NO: 36 oder eine davon abgeleitete FDH mit wenigstens 60 % Sequenzidentität zu SEQ ID NO: 36 ist.

**14.** Verfahren zur Herstellung von Ursodesoxycholsäure (UDCS) der Formel (1)

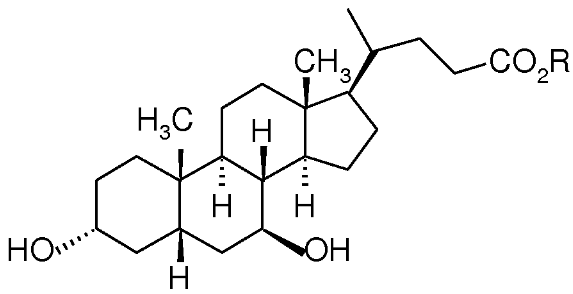

(1)

worin
R für Alkyl, H, ein Alkalimetallion oder $N(R^3)_4^+$ steht, worin die Reste $R^3$ gleich oder verschieden sind und für H oder Alkyl stehen,
wobei man

a) gegebenenfalls eine Cholsäure (CS) der Formel (2)

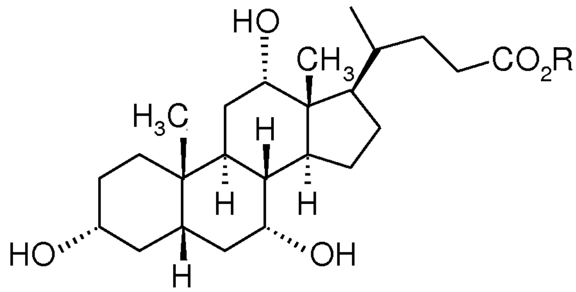

(2)

worin R die oben angegebenen Bedeutungen besitzt, zur Dehydrocholsäure (DHCS) der Formel (3)

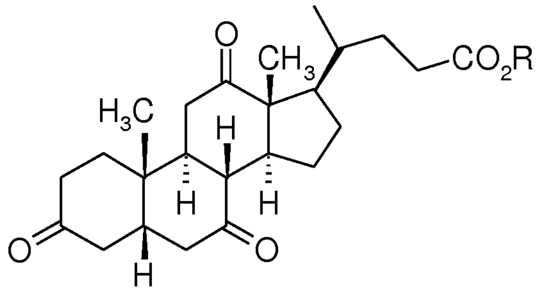

(3)

worin R die oben angegebenen Bedeutungen besitzt, chemisch oxidiert;

b) DHCS in Gegenwart wenigstens einer 7ß-HSDH gemäß der Definition in einem der Ansprüche 1 und 2 und in Gegenwart wenigstens einer 3α-HSDH zur korrespondierenden 12-Ketoursodesoxychiolsäure (12-Keto UDCS) der Formel (5)

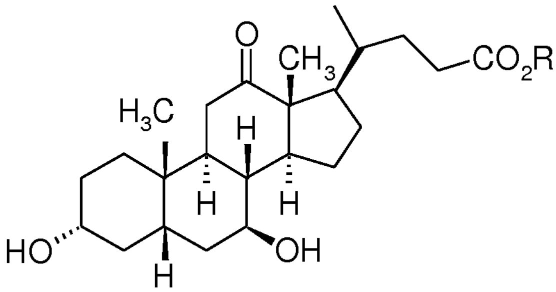

(5)

worin R die oben angegebenen Bedeutungen besitzt, insbesondere in Gegenwart und unter Verbrauch von NADH und /oder NADPH

reduziert und anschließend

c) 12-Keto-UDCS der Formel (5) chemisch zu UDCS reduziert; und

d) das Reaktionsprodukt gegebenenfalls weiter aufreinigt.

**15.** Verfahren nach Anspruch 14, wobei zumindest Schritt b) in Gegenwart eines rekombinanten Mikroorganismus nach Anspruch 3 durchgeführt wird.

**16.** Verfahren nach Anspruch 14 oder 15, wobei Schritt b) mit gleichen oder verschiedenen Kofaktorregenerierungssystemen gekoppelt ist.

**17.** Verfahren nach Anspruch 16, wobei Schritt b), 7ß-HSDH Teilschritt, mit einem Kofaktorregenerierungssystem gekoppelt ist, bei welchem verbrauchtes NADPH durch eine $NADP^+$-akzeptierenden FDH gemäß der Definition in einem der Ansprüche 7 bis 9 unter Verbrauch von Ameisensäure oder eines Salzes davon regeneriert wird; oder mit einem Kofaktorregenerierungssystem gekoppelt ist, bei welchem verbrauchtes NADPH durch eine ADH unter Verbrauch von Isopropanol regeneriert wird; oder mit einem Kofaktorregenerierungssystem gekoppelt ist, bei welchem verbrauchtes NADPH durch eine GDH unter Verbrauch von Glucose regeneriert wird; oder mit einem Kofaktorregenerierungssystem gekoppelt ist, bei welchem verbrauchtes NADH durch eine NADH regenerierende GDH, ADH oder FDH regeneriert wird.

**18.** Verfahren nach Anspruch 16 oder 17, wobei Schritt b), 3α-HSDH Teilschritt, mit einem Kofaktorregenerierungsschritt gekoppelt ist, bei welchem NADPH durch eine $NADP^+$-akzeptierenden FDH gemäß der Definition in einem der Ansprüche 7 bis 9 unter Verbrauch von Ameisensäure oder eines Salzes davon regeneriert wird; oder wobei Schritt b) mit einem Kofaktorregenerierungsschritt gekoppelt ist, bei welchem NADH durch eine $NAD^+$ -und $NADP^+$-akzeptierende FDH gemäß der Definition in einem der Ansprüche 7 bis 9 oder durch die nichtmutierte FDH jeweils unter Verbrauch von Ameisensäure oder eines Salzes davon, oder durch eine $NAD^+$-akzepierende GDH unter Verbrauch von Glucose regeneriert wird.

**19.** Verfahren zur Herstellung von UDCS der Formel (1)

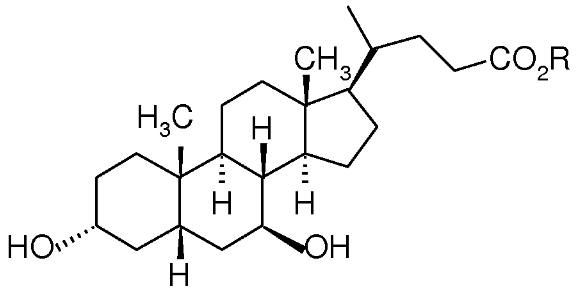

(1)

worin
R für Alkyl, H, ein Alkalimetallion oder $N(R^3)_4{}^+$steht, worin die Reste $R^3$ gleich oder verschieden sind und für H oder Alkyl stehen,
wobei man

a) gegebenenfalls eine CS der Formel (2)

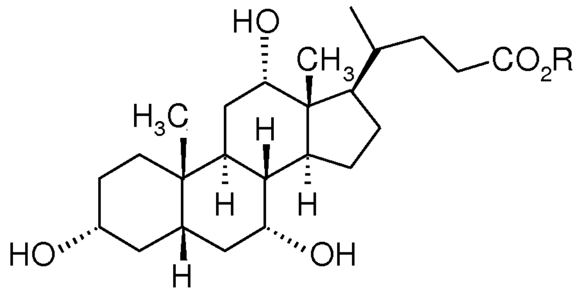

(2)

worin R die oben angegebenen Bedeutungen besitzt, zur DHCS der Formel (3)

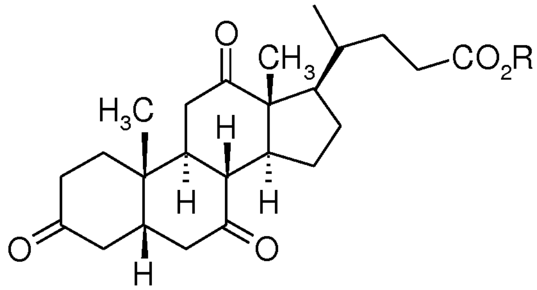

(3)

worin R die oben angegebenen Bedeutungen besitzt, chemisch oxidiert;
b) DHCS in Gegenwart wenigstens einer 7ß-HSDH und in Gegenwart wenigstens einer 3α-HSDH zur korrespondierenden 12-Keto UDCS der Formel (5)

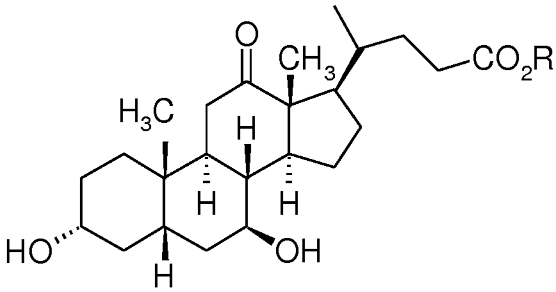

(5)

worin R die oben angegebenen Bedeutungen besitzt, insbesondere in Gegenwart und unter Verbrauch von NADH und/oder NADPH, reduziert und anschließend

c) 12-Keto-UDCS der Formel (5) chemisch zu UDCS reduziert; und

d) das Reaktionsprodukt gegebenenfalls weiter aufreinigt;

wobei die Umsetzungen des Schritts b) in Gegenwart eines rekombinanten Mikroorganismus nach einem der Ansprüche 11 bis 13, oder unter Verwendung wenigstens einer Nukleinsäuresequenz nach Anspruch 10 erfolgen.

**20.** Verfahren nach Anspruch 19, wobei man einen rekombinanten Mikroorganismus nach Anspruch 12 einsetzt, der wenigstens eine 7ß-HSDH nach einem der Ansprüche 1 und 2, wenigstens eine $NADP^+$-akzeptierende FDH nach einem der Ansprüche 7 bis 9 und wenigstens eine 3α-HSDH gleichzeitig exprimiert; oder der wenigstens eine 7β-HSDH nach einem der Ansprüche 1 und 2, wenigstens eine GDH und wenigstens eine 3α-HSDH gleichzeitig exprimiert.

**21.** Nukleinsäuremolekül, umfassend eine für eine 7ß-HSDH nach Anspruch 1 oder 2 kodierende Nukleinsäuresequenz.

**22.** Expressionskassette, umfassend eine Nukleinsäuresequenz nach Anspruch 21.

**23.** Vektor, umfassend wenigstens eine Expressionskassette nach Anspruch 22.

## Claims

**1.** 7β-HSDH which catalyzes at least the stereospecific enzymatic reduction of a 7-ketosteroid to the corresponding 7-hydroxysteroid, wherein the enzyme has at least one mutation in the sequence motif VMVGRRE according to position 36 to 42 of SEQ ID NO:2 and at least 80% sequence identity to SEQ ID NO:2.

**2.** 7β-HSDH which catalyzes at least the stereospecific enzymatic reduction of a 7-ketosteroid to the corresponding 7-hydroxysteroid, wherein the enzyme has at least one mutation in the sequence motif VMVGRRE according to position 36 to 42 of SEQ ID NO:2 and at least 60% sequence identity to SEQ ID NO:2, selected from

a) the single mutants $G39X_1$ and $R40X_2$

b) the double mutants $G39X_1$ $R40X_2$, $R40X_2$ $R41X_3$ and $G39X_1$ $R41X_3$ or

c) the triple mutant $G39X_1$ $R40X_2$ $R41X_3$,

wherein $X_1$, $X_2$ and $X_3$ stand, in each case independently of one another, for a mutated amino acid residue.

**3.** Recombinant microorganism which carries at least one nucleic acid sequence coding for an enzyme according to one of the preceding claims or at least one expression cassette containing this coding nucleic acid sequence or at least one vector containing this coding nucleic acid sequence and in addition optionally carries the coding sequence for another enzyme, selected from hydroxysteroid dehydrogenases, such as a 3α-hydroxysteroid dehydrogenase (3α-HSDH), and dehydrogenases suitable for cofactor regeneration.

**4.** Process for enzymatic or microbial synthesis of 7β-hydroxysteroids, wherein the corresponding 7-ketosteroid is reacted in the presence of a 7β-HSDH according to the definition in one of Claims 1 and 2 or in the presence of a recombinant microorganism expressing this enzyme, and at least one reduction product formed is optionally isolated from the reaction mixture.

5. Process according to Claim 4, wherein the reduction takes place in the presence of and especially with consumption of NADPH and/or NADH.

6. Process according to Claim 5, wherein spent NADPH is regenerated by coupling with an NADPH-regenerating enzyme, wherein this is selected in particular from NADPH dehydrogenases, alcohol dehydrogenases (ADH), and NADPH-regenerating formate dehydrogenases (FDH) and an NADPH-regenerating glucose dehydrogenase (GDH), glucose-6-phosphate-dehydrogenase (G-6-PDH), or phosphite dehydrogenases (PtDH), wherein the NADPH-regenerating enzyme optionally is expressed by a recombinant microorganism; and/or wherein spent NADH is regenerated by coupling with an NADH-regenerating enzyme, wherein this is selected in particular from NADH-dehydrogenases, NADH-regenerating formate dehydrogenases (FDH), NADH-regenerating alcohol dehydrogenases (ADH), NADH-regenerating glucose-6-phosphate-dehydrogenases (G6PDH), NADH-regenerating phosphite dehydrogenases (PtDH) and NADH-regenerating glucose dehydrogenases (GDH), wherein the NADH-regenerating enzyme optionally is expressed in a recombinant microorganism.

7. Process according to Claim 6, wherein the NADPH-regenerating enzyme is selected from an $NAD^+$-dependent formate dehydrogenase (FDH), which at least catalyzes the enzymatic oxidation of formic acid to $CO_2$, and additionally accepts $NADP^+$ as cofactor.

8. Process according to Claim 7, wherein the $NADP^+$-accepting FDH has at least one mutation in the amino acid sequence of an FDH from *Mycobacterium vaccae* N10 according to SEQ ID NO:36 or an amino acid sequence derived therefrom with at least 60% sequence identity to SEQ ID NO:36.

9. Process according to one of Claims 7 and 8, wherein the $NADP^+$-accepting FDH has at least one mutation in the sequence motif TDRHRL according to position 221 to 226 of SEQ ID NO:36 and at least 60% sequence identity to SEQ ID NO:36.

10. Nucleic acid selected from those

a) simultaneously coding for an FDH according to one of Claims 7 to 9 and a 7β-HSDH according to one of Claims 1 and 2 and optionally a 3α-HSDH; or
b) coding for a fusion protein comprising an FDH according to one of Claims 7 to 9 and a 7β-HSDH according to one of Claims 1 and 2 and optionally a 3α-HSDH; or
c) simultaneously coding for FDH and a 7β-HSDH according to one of Claims 1 and 2 and optionally a 3α-HSDH; or
d) coding for a fusion protein, comprising the FDH, a 7β-HSDH according to one of Claims 1 to 2 and optionally a 3α-HSDH; or
e) simultaneously coding for a GDH, a 7β-HSDH according to one of Claims 1 and 2 and optionally a 3α-HSDH; or
f) coding for a fusion protein, comprising a GDH, a 7β-HSDH according to one of Claims 1 and 2 and optionally a 3α-HSDH.

11. Recombinant microorganism which carries at least one nucleic acid sequence according to Claim 10.

12. Recombinant microorganism which is capable of simultaneous expression of a 7β-HSDH according to one of Claims 1 and 2, an $NADP^+$-accepting FDH according to one of Claims 7 to 9 and/or the corresponding $NAD^+$-dependent FDH and optionally of 3α-HSDH; or which is capable of simultaneous expression of a 7β-HSDH according to one of Claims 1 and 2, a GDH and optionally of 3α-HSDH.

13. Recombinant microorganism according to Claim 12, wherein the FDH is an enzyme according to the definition in one of Claims 7 to 9; and wherein the $NAD^+$-dependent FDH is an FDH from *Mycobacterium vaccae* N10 according to SEQ ID NO:36 or an FDH derived therefrom with at least 60% sequence identity to SEQ ID NO:36.

14. Process for preparing ursodeoxycholic acid (UDCA) of formula (1)

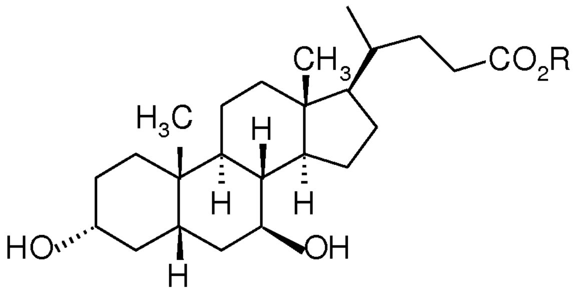

(1)

in which
R stands for alkyl, H, an alkali metal ion or $N(R^3)_4^+$, in which the residues $R^3$ may be identical or different and stand for H or alkyl,
wherein

a) optionally a cholic acid (CA) of formula (2)

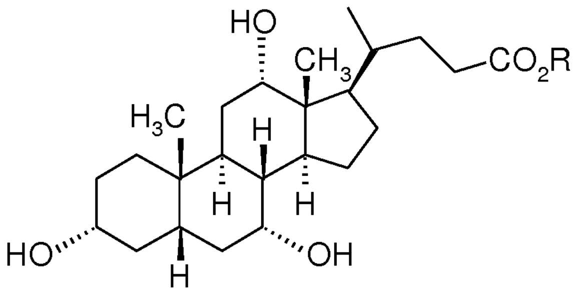

(2)

in which R has the meanings given above, is oxidized chemically to dehydrocholic acid (DHCA) of formula (3)

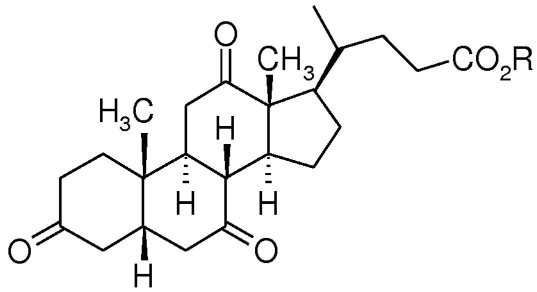

(3)

in which R has the meanings given above;
b) DHCA is reduced in the presence of at least one 7β-HSDH according to the definition in one of Claims 1 and 2 and in the presence of at least one 3α-HSDH to the corresponding 12-keto-ursodeoxycholic acid (12-keto UDCA) of formula (5)

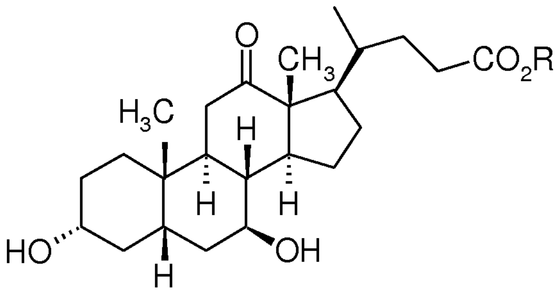

(5)

in which R has the meanings given above, especially in the presence of and with consumption of NADH and/or NADPH, and then

c) 12-keto-UDCA of formula (5) is reduced chemically to UDCA; and

d) the reaction product optionally is further purified.

**15.** Process according to Claim 14, wherein at least step b) is carried out in the presence of a recombinant microorganism according to Claim 3.

**16.** Process according to Claim 14 or 15, wherein step b) is coupled with identical or different cofactor regeneration systems.

**17.** Process according to Claim 16, wherein step b), 7β-HSDH partial step, is coupled to a cofactor regeneration system, in which spent NADPH is regenerated by an $NADP^+$-accepting FDH according to the definition in one of Claims 7 to 9 with consumption of formic acid or a salt thereof; or is coupled to a cofactor regeneration system in which spent NADPH is regenerated by an ADH with consumption of isopropanol; or is coupled to a cofactor regeneration system in which spent NADPH is regenerated by a GDH with consumption of glucose; or is coupled to a cofactor regeneration system in which spent NADH is regenerated by an NADH-regenerating GDH, ADH or FDH.

**18.** Process according to Claim 16 or 17, wherein step b), 3α-HSDH partial step, is coupled to a cofactor regeneration step, in which NADPH is regenerated by an $NADP^+$-accepting FDH according to the definition in one of Claims 7 to 9 with consumption of formic acid or a salt thereof; or wherein step b) is coupled to a cofactor regeneration step, in which NADH is regenerated by an $NAD^+$- and $NADP^+$-accepting FDH according to the definition in one of Claims 7 to 9 or by the nonmutated FDH in each case with consumption of formic acid or a salt thereof, or by an $NAD^+$-accepting GDH with consumption of glucose.

**19.** Process for preparing UDCA of formula (1)

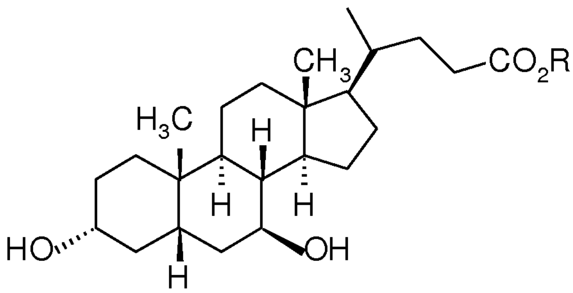

(1)

in which

R stands for alkyl, H, an alkali metal ion or $N(R^3)_4^+$, in which the residues $R^3$ may be identical or different and stand for H or alkyl,

wherein

a) optionally a CA of formula (2)

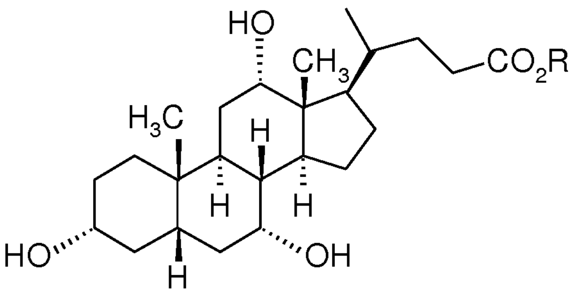

(2)

in which R has the meanings given above, is oxidized chemically to the DHCA of formula (3)

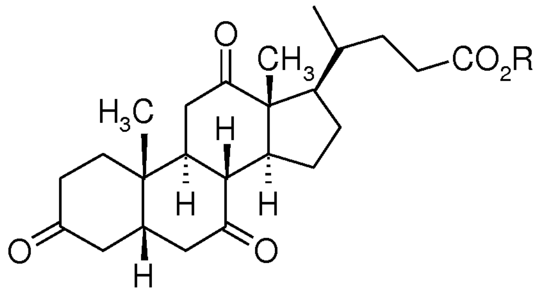

(3)

in which R has the meanings given above;

b) DHCA is reduced in the presence of at least one 7β-HSDH and in the presence of at least one 3α-HSDH to the corresponding 12-keto UDCA of formula (5)

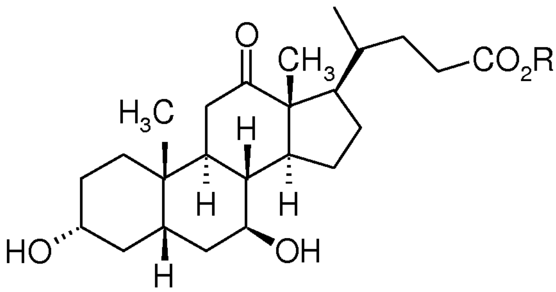

(5)

in which R has the meanings given above, especially in the presence of and with consumption of NADH and/or NADPH, and then

c) 12-keto-UDCA of formula (5) is reduced chemically to UDCA; and

d) the reaction product optionally is further purified;

wherein the reactions of step b) take place in the presence of a recombinant microorganism according to one of Claims 11 to 13, or using at least one nucleic acid sequence according to Claim 10.

**20.** Process according to Claim 19, wherein a recombinant microorganism according to Claim 12 is used, which simultaneously expresses at least one 7β-HSDH according to one of Claims 1 and 2, at least one $NADP^+$-accepting FDH according to one of Claims 7 to 9 and at least one 3α-HSDH; or simultaneously expresses at least one 7β-HSDH according to one of Claims 1 and 2, at least one GDH and at least one 3α-HSDH.

**21.** Nucleic acid molecule, comprising a nucleic acid sequence coding for a 7β-HSDH according to Claim 1 or 2.

**22.** Expression cassette, comprising a nucleic acid sequence according to Claim 21.

**23.** Vector, comprising at least one expression cassette according to Claim 22.

**Revendications**

**1.** 7β-HSDH, qui catalyse au moins la réduction enzymatique stéréospécifique d'un 7-cétostéroïde en le 7-hydroxystéroïde correspondant, l'enzyme présentant au moins une mutation dans le motif séquentiel VMVGRRE selon les positions 36 à 42 de SEQ ID NO : 2 et au moins 80 % d'identité de séquence avec SEQ ID NO : 2.

**2.** 7β-HSDH, qui catalyse au moins la réduction enzymatique stéréospécifique d'un 7-cétostéroïde en le 7-hydroxystéroïde correspondant, l'enzyme présentant au moins une mutation dans le motif séquentiel VMVGRRE selon les positions 36 à 42 de SEQ ID NO : 2 et au moins 60 % d'identité de séquence avec SEQ ID NO : 2, choisie parmi :

a) les mutants simples $G39X_1$ et $R40X_2$,

b) les mutants doubles $G39X_1$ $R40X_2$, $R40X_2$ $R41X_3$ et $G39X_1$ $R41X_3$ ou

c) les mutants triples G39$X_1$ R40$X_2$ R41$X_3$,

$X_1$, $X_2$ et $X_3$ représentant chacun indépendamment les uns des autres un radical acide aminé muté.

**3.** Microorganisme recombinant, qui porte au moins une séquence d'acides nucléiques codant pour une enzyme selon l'une quelconque des revendications précédentes ou au moins une cassette d'expression contenant cette séquence d'acides nucléiques codante ou au moins un vecteur contenant cette séquence d'acides nucléiques codante, et qui porte en outre éventuellement la séquence codante pour une autre enzyme, choisie parmi les hydroxystéroïde-déshydrogénases, telles qu'une 3α-hydroxystéroïde-déshydrogénase (3α-HSDH), et les déshydrogénases appropriées pour la régénération du cofacteur.

**4.** Procédé de synthèse enzymatique ou microbienne de 7β-hydroxystéroïdes, selon lequel le 7-cétostéroïde correspondant est mis en réaction en présence d'une 7β-HSDH selon la définition dans l'une quelconque des revendications 1 et 2 ou en présence d'un microorganisme recombinant exprimant cette enzyme, et au moins un produit de réduction formé est éventuellement isolé à partir de la préparation de réaction.

**5.** Procédé selon la revendication 4, selon lequel la réduction a lieu en présence et notamment avec consommation de NADPH et/ou de NADH.

**6.** Procédé selon la revendication 5, selon lequel le NADPH consommé est régénéré par couplage avec une enzyme de régénération de NADPH, celle-ci étant notamment choisie parmi les NADPH-déshydrogénases, les alcool-déshydrogénases (ADH) et les formiate-déshydrogénases de régénération de NADPH (FDH) et une glucose-déshydrogénase de régénération de NADPH (GDH), la glucose-6-phosphate-déshydrogénase (G-6-PDH), ou les phosphite-déshydrogénases (PtDH), l'enzyme de régénération de NADPH étant éventuellement exprimée par un microorganisme recombinant ; et/ou selon lequel le NADH consommé est régénéré par couplage avec une enzyme de régénération de NADH, celle-ci étant notamment choisie parmi les NADH-déshydrogénases, les formiate-déshydrogénases de régénération de NADH (FDH), les alcool-déshydrogénases de régénération de NADH (ADH), les glucose-6-phosphate-désydrogénases de régénération de NADH (G6PDH), les phosphite-déshydrogénases de régénération de NADH (PtDH), ainsi que les glucose-déshydrogénases de régénération de NADH (GDH), l'enzyme de régénération de NADH étant éventuellement exprimée dans un microorganisme recombinant.

**7.** Procédé selon la revendication 6, selon lequel l'enzyme de régénération de NADPH est choisie parmi une formiate-déshydrogénase dépendante de $NAD^+$ (FDH), qui catalyse au moins l'oxydation enzymatique d'acide formique en $CO_2$, et accepte en outre $NADP^+$ en tant que cofacteur.

**8.** Procédé selon la revendication 7, dans lequel la FDH acceptant $NADP^+$ présente au moins une mutation dans la séquence d'acides aminés d'une FDH issue de *Mycobacterium vaccae* N10 selon SEQ ID NO : 36 ou d'une séquence d'acides aminés dérivée de celle-ci présentant au moins 60 % d'identité de séquence avec SEQ ID NO : 36.

**9.** Procédé selon l'une quelconque des revendications 7 ou 8, dans lequel la FDH acceptant $NADP^+$ présente au moins une mutation dans le motif séquentiel TDRHRL selon les positions 221 à 226 de SEQ ID NO : 36 et au moins 60 % d'identité de séquence avec SEQ ID NO : 36.

**10.** Acide nucléique, choisi parmi ceux

a) codant simultanément pour une FDH selon l'une quelconque des revendications 7 à 9 et une 7β-HSDH selon l'une quelconque des revendications 1 et 2, et éventuellement une 3α-HSDH ; ou
b) codant pour une protéine de fusion comprenant une FDH selon l'une quelconque des revendications 7 à 9 et une 7β-HSDH selon l'une quelconque des revendications 1 et 2, et éventuellement une 3α-HSDH ; ou
c) codant simultanément pour une FDH et une 7β-HSDH selon l'une quelconque des revendications 1 et 2, et éventuellement une 3α-HSDH ; ou
d) codant pour une protéine de fusion, comprenant la FDH, une 7β-HSDH selon l'une quelconque des revendications 1 et 2, et éventuellement une 3α-HSDH ; ou
e) codant simultanément pour une GDH, une 7β-HSDH selon l'une quelconque des revendications 1 et 2, et éventuellement une 3α-HSDH ; ou
f) codant pour une protéine de fusion, comprenant une GDH, une 7β-HSDH selon l'une quelconque des revendications 1 et 2, et éventuellement une 3α-HSDH.

**11.** Microorganisme recombinant, qui porte au moins une séquence d'acides nucléiques selon la revendication 10.

**12.** Microorganisme recombinant, qui est apte à l'expression simultanée d'une 7β-HSDH selon l'une quelconque des revendications 1 ou 2, d'une FDH acceptant $NADP^+$ selon l'une quelconque des revendications 7 à 9 et/ou de la FDH dépendante de $NAD^+$ correspondante et éventuellement de 3α-HSDH ; ou qui est apte à l'expression simultanée d'une 7β-HSDH selon l'une quelconque des revendications 1 ou 2, d'une GDH et éventuellement de 3α-HSDH.

**13.** Microorganisme recombinant selon la revendication 12, dans lequel la FDH est une enzyme selon la définition dans l'une quelconque des revendications 7 à 9 ; et dans lequel la FDH dépendante de $NAD^+$ est une FDH issue de *Mycobacterium vaccae* N10 selon SEQ ID NO : 36 ou une FDH dérivée de celle-ci présentant au moins 60 % d'identité de séquence avec SEQ ID NO : 36.

**14.** Procédé de fabrication d'acide ursodésoxycholique (UDCS) de formule (1)

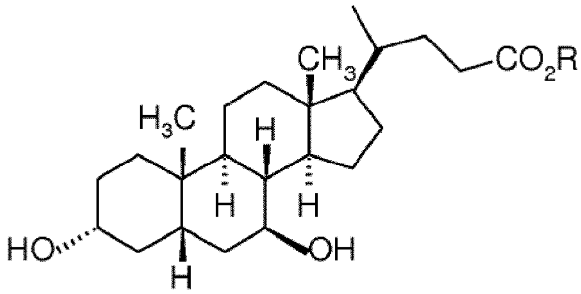

(1)

dans laquelle
R représente alkyle, H, un ion de métal alcalin ou $N(R^3)_4{}^+$, les radicaux $R^3$ étant identiques ou différents, et représentant H ou alkyle,
selon lequel

a) éventuellement un acide cholique (CS) de formule (2)

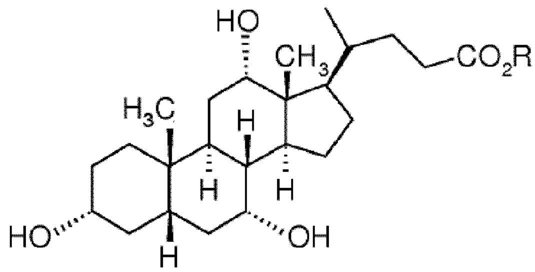

(2)

dans laquelle R a les significations indiquées précédemment, est oxydé chimiquement pour former l'acide déshydrocholique (DHCS) de formule (3)

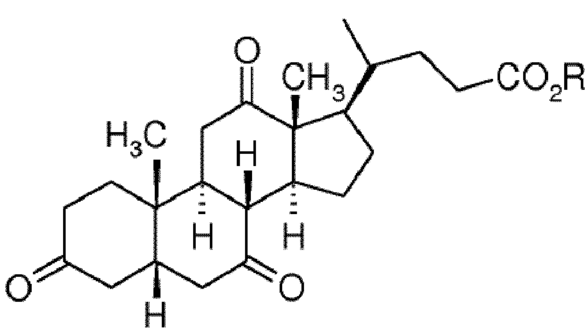

(3)

dans laquelle R a les significations indiquées précédemment ;

b) le DHCS est réduit en présence d'au moins une 7β-HSDH selon la définition dans l'une quelconque des revendications 1 et 2 et en présence d'au moins une 3α-HSDH pour former l'acide 12-céto-ursodésoxychiolique (12-céto-UDCS) correspondant de formule (5)

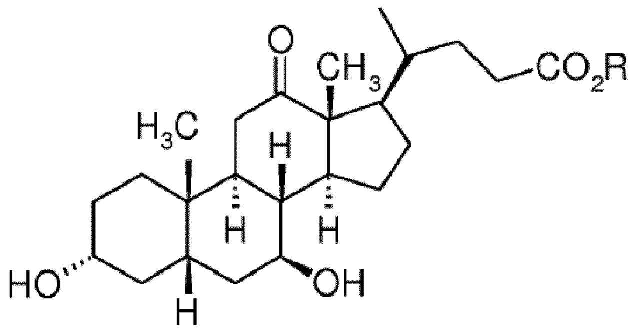

(5)

dans laquelle R a les significations indiquées précédemment, notamment en présence et avec consommation de NADH et/ou de NADPH,
puis
c) le 12-céto-UDCS de formule (5) est réduit chimiquement en UDCS ; et
d) le produit de réaction est éventuellement davantage purifié.

**15.** Procédé selon la revendication 14, selon lequel au moins l'étape b) est réalisée en présence d'un microorganisme recombinant selon la revendication 3.

**16.** Procédé selon la revendication 14 ou 15, selon lequel l'étape b) est couplée avec des systèmes de régénération du cofacteur identiques ou différents.

**17.** Procédé selon la revendication 16, selon lequel l'étape b), étape partielle 7β-HSDH, est couplée avec un système de régénération du cofacteur, dans lequel le NADPH consommé est régénéré par une FDH acceptant $NADP^+$ selon la définition dans l'une quelconque des revendications 7 à 9 avec consommation d'acide formique ou d'un sel de celui-ci ; ou est couplée avec un système de régénération du cofacteur, dans lequel le NADPH consommé est régénéré par une ADH avec consommation d'isopropanol ; ou est couplée avec un système de régénération du cofacteur dans lequel le NADPH consommé est régénéré par une GDH avec consommation de glucose ; ou est couplée avec un système de régénération du cofacteur dans lequel le NADH consommé est régénéré par une GDH, une ADH ou une FDH de régénération du NADH.

**18.** Procédé selon la revendication 16 ou 17, dans lequel l'étape b), étape partielle 3α-HSDH, est couplée avec une étape de régénération du cofacteur dans laquelle le NADPH est régénéré par une FDH acceptant $NADP^+$ selon la définition dans l'une quelconque des revendications 7 à 9 avec consommation d'acide formique ou d'un sel de celui-ci ; ou dans lequel l'étape b) est couplée avec une étape de régénération du cofacteur dans laquelle le NADH est régénéré par une FDH acceptant $NAD^+$ et $NADP^+$ selon la définition dans l'une quelconque des revendications 7 à 9, ou par la FDH non mutée, à chaque fois avec consommation d'acide formique ou d'un sel de celui-ci, ou par une GDH acceptant $NAD^+$ avec consommation de glucose.

**19.** Procédé de fabrication d'UDCS de formule (1)

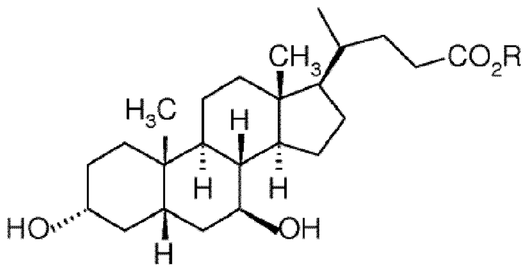

(1)

dans laquelle

R représente alkyle, H, un ion de métal alcalin ou $N(R^3)_4^+$, les radicaux $R^3$ étant identiques ou différents, et représentant H ou alkyle,
selon lequel

a) éventuellement un CS de formule (2)

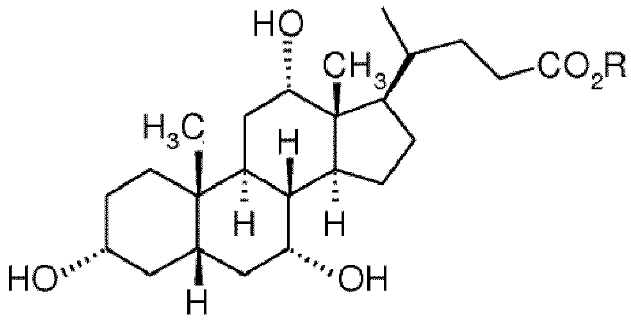

(2)

dans laquelle R a les significations indiquées précédemment, est oxydé chimiquement en DHCS de formule (3)

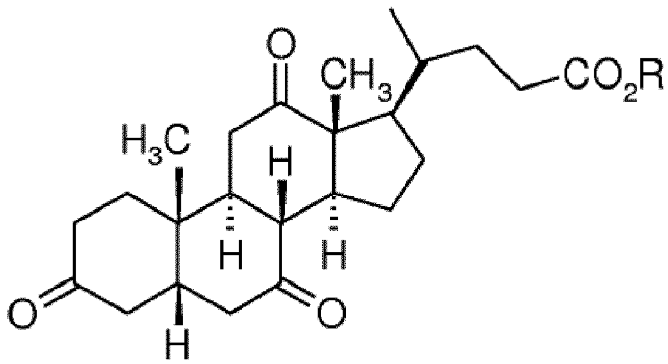

(3)

dans laquelle R a les significations indiquées précédemment ;
b) le DHCS est réduit en présence d'au moins une 7β-HSDH et en présence d'au moins une 3α-HSDH pour former le 12-céto-UDCS correspondant de formule (5)

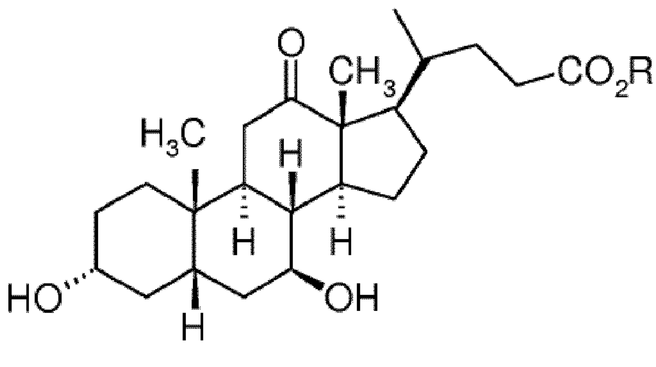

(5)

dans laquelle R a les significations indiquées précédemment, notamment en présence et avec consommation de NADH et/ou de NADPH, puis
c) le 12-céto-UDCS de formule (5) est réduit chimiquement en UDCS ; et
d) le produit de réaction est éventuellement davantage purifié ;

les réactions de l'étape b) ayant lieu en présence d'un microorganisme recombinant selon l'une quelconque des revendications 11 à 13, ou en utilisant au moins une séquence d'acides nucléiques selon la revendication 10.

**20.** Procédé selon la revendication 19, selon lequel un microorganisme recombinant selon la revendication 12 est utilisé,

qui exprime simultanément au moins une 7β-HSDH selon l'une quelconque des revendications 1 et 2, au moins une FDH acceptant $NADP^+$ selon l'une quelconque des revendications 7 à 9 et au moins une 3α-HSDH ; ou qui exprime simultanément au moins une 7β-HSDH selon l'une quelconque des revendications 1 et 2, au moins une GDH et au moins une 3α-HSDH.

**21.** Molécule d'acide nucléique, comprenant une séquence d'acides nucléiques codant pour une 7β-HSDH selon la revendication 1 ou 2.

**22.** Cassette d'expression, comprenant une séquence d'acides nucléiques selon la revendication 21.

**23.** Vecteur, comprenant au moins une cassette d'expression selon la revendication 22.

Zugang: ZP_01773061

```
  1 mnlrekygew glilgategv gkafcekiaa ggmnvvmvgr reeklnvlag eiretygvet
 61 kvvradfsqp gaaetvfaat egldmgfmsy vaclhsfgki qdtpwekhea minvnvvtfl
121 kcfhhymrif aaqdrgavin vssmtgisss pwngqygagk afilkmteav acecegtgvd
181 vevitlgttl tpsllsnlpg gpqgeavmki altpeecvde afeklgkels viagqrnkds
241 vhdwkanhte deyirymgsf yrd
```

**Fig. 1a**

Zugangs-Nr.: NZ_AAVN02000010, Region: 52005..52796

```
  1 atgaacctga gggagaagta cggtgagtgg ggcctgatcc tgggcgcgac cgagggcgtc
 61 ggcaaggcgt tctgcgagaa gatcgccgcc ggcggcatga acgtcgtcat ggtcggccgt
121 cgcgaggaga agctgaacgt gctcgcaggc gagatccgcg agacctacgg cgtggagacc
181 aaggtcgtgc gcgccgactt tagccagccc ggcgctgccg agaccgtctt cgccgcgacc
241 gagggcctgg acatgggctt catgagctac gtggcctgcc tgcacagctt cggtaagatc
301 caggacaccc cctgggagaa gcacgaggcc atgatcaacg tcaacgtcgt gaccttcctc
361 aagtgcttcc accactacat gcggatcttt gccgcccagg accgcggcgc cgtgatcaac
421 gtctcgtcga tgaccggcat cagctccagc ccctggaacg gccagtacgg cgcgggcaag
481 gccttcatcc tcaagatgac cgaggccgtg gcctgcgagt gcgagggcac cggcgtcgac
541 gtcgaggtca tcaccctcgg caccaccctg accccagcc tgctgtccaa cctccccggc
601 ggcccgcagg gcgaggccgt catgaagatc gccctcaccc ccgaggagtg cgttgacgag
661 gcctttgaga agctgggtaa ggagctctcc gtcatcgccg gccagcgcaa caaggactcc
721 gtccacgact ggaaggcaaa ccacaccgag gacgagtaca tccgctacat ggggtcgttc
781 taccgcgact ag
```

**Fig.1b**

3α-HSDH

Quelle: *Comamonas testosteroni* ATCC 11996

1 msiivisgca tgigaatrkv leaaghqivg idirdaevia dlstaegrkq aiadvlakcs
61 kgmdglvlca glgpqtkvlg nvvsvnyfga telmdaflpa lkkghqpaav vissvasahl
121 afdknplala leageeakar aivehageqg gnlayagskn altvavrkra aawgeagvrl
181 ntiapgatet pllqaglqdp rygesiakfv ppmgrraeps emasviaflm spaasyvhga
241 qividggida vmrptqf

**Fig.1c**

Nukleinsäuresequenz codierend für 3α-HSDH

Quelle: *Comamonas testosteroni* ATCC 11996

Accession: AF092031, Region: 158..931

1 atgtccatca tcgtgataag cggctgcgcc accggcattg gtgcggctac gcgcaaggtc
61 ctggaggcgg ccggtcacca gatcgtaggc atcgatatac gcgatgcgga agtgattgcc
121 gatctctcga cggccgaagg tcgaaagcag gcgattgccg atgtactggc gaagtgcagc
181 aagggcatgg acggcctggt gctgtgcgcc ggcctgggac cgcagaccaa ggtgcttggc
241 aatgtggttt cggtcaatta ttttggcgcg accgagctga tggatgcctt tttgccagcg
301 ctgaaaaaag gccatcagcc cgcagccgtc gtcatctcgt ccgtggcttc cgcgcatctg
361 gcttttgaca agaacccact ggcgctggca ctggaagccg gcgaggaagc caaggcccgc
421 gccattgtcg aacatgcggg agagcagggc ggaaatctgg cctatgcggg cagcaagaat
481 gctttgacgg tggctgtgcg caaacgcgcc gccgcctggg gcgaggctgg cgtgcgcctg
541 aacaccatcg cccccggtgc aaccgagact cccttgctgc aggcgggcct gcaggacccg
601 cgctatggcg aatccattgc caagttcgtt cctcccatgg gccgccgtgc cgagccgtcc
661 gagatggcgt cggtcatcgc ctttttgatg agcccggccg caagctatgt gcatggcgcg
721 cagatcgtca ttgatggcgg cattgatgcg gtgatgcgcc cgacacagtt ctga

**Fig.1d**

3α-HSDH

Quelle: Rattus norvegicus (Norway rat)
Genbank ACCESSION M61937

MDSISLRVALNDGNFIPVLGFGTTVPEKVAKDEVIKATKIAIDN
GFRHFDSAYLYEVEEEVGQAIRSKIEDGTVKREDIFYTSKLWSTFHRPELVRTCLEKT
LKSTQLDYVDLYIIHFPMALQPGDIFFPRDEHGKLLFETVDICDTWEAMEKCKDAGLA
KSIGVSNFNCRQLERILNKPGLKYKPVCNQVECHLYLNQSKMLDYCKSKDIILVSYCT
LGSSRDKTWVDQKSPVLLDDPVLCAIAKKYKQTPALVALRYQLQRGVVPLIRSFNAKR
IKELTQVFEFQLASEDMKALDGLNRNFRYNNAKYFDDHPNHPFTDE

Fig.1e

atggattcca tatctctgcg tgtagcacta aatgatggta acttcattcc tgtactgggg
tttggaacca ctgtgcctga gaaggttgct aaggatgaag ttatcaaggc tactaaaata
gctatagata atggattccg ccattttgac tctgcttatt tgtacgaagt agaagaggaa
gtgggccaag ccattagaag caagattgaa gacggcactg tgaagagaga agatatattc
tatacttcaa agctttggag cactttccat agaccagagc tggtccgaac ttgcttggaa
aagacactga aaagcactca actggactat gtggatcttt atattattca tttcccaatg
gctttgcagc ctggagatat atttttccca cgagatgagc atggaaaact attgtttgaa
acagtggata tctgtgacac atgggaggcc atggaaaagt gtaaggatgc aggattggcc
aagtctattg gggtgtccaa ctttaactgc aggcagctgg agaggattct gaataagcca
gggctcaaat acaagcctgt gtgcaaccag gtggaatgtc acctttatct caaccagagc
aaaatgctgg actattgtaa gtcaaaagac atcattctgg tttcctactg cacgctggga
agttcacgag acaaaacatg ggtggatcag aaaagtccag ttctcctaga tgatccagtt
ctttgtgcca tagcaaagaa gtacaagcaa accccagccc tagttgccct tcgctaccag
ctgcagcgtg gggttgtgcc cctgatcagg agtttcaacg cgaagcggat caaagagcta
acacaggttt ttgaattcca gttggcttca gaggacatga aagccctgga tggcttgaac
agaaatttca gatacaacaa tgcaaaatat tttgatgacc atcccaatca tccatttact
gatgaatag

Fig.1f

FDH-Sequenz, D221G-Mutante:

atggcaaaggtcctgtgcgttctttacgatgatccggtcgacggctacccgaagacctatgcccgcgacgatcttccgaa
gatcgaccactatccgggcggccagatcttgccgacgccgaaggccatcgacttcacgcccgggcagttgctcggctccg
tctccggcgagctcggcctgcgcgaatatctcgaatccaacggccacaccctggtcgtgacctccgacaaggacggcccc
gactcggtgttcgagcgcgagctggtcgatgcggatgtcgtcatctcccagcccttctggccggcctatctgacgcccga
gcgcatcgccaaggccaagaacctgaagctcgcgctcaccgccggcatcggttccgaccacgtcgatcttcagtcggcta
tcgaccgcaacgtcaccgtggcggaagtcacctactgcaactcgatcagcgtcgccgagcatgtggtgatgatgatcctg
tcgctggtgcgcaactatctgccctcgcacgaatgggcgcggaagggcggctggaacatcgccgactgcgtctcccacgc
ctacgacctcgaggcgatgcatgtcggcaccgtggccgccggccgcatcggtctcgcggtgctgcgccgtctggcgccgt
tcgacgtgcacctgcactacaccggccgtcaccgcctgccggaatcggtcgagaaggagctcaacctcacctggcacgcg
acccgcgaggacatgtatccggtttgcgacgtggtgacgctgaactgcccgctgcaccccgaaaccgagcacatgatcaa
tgacgagacgctgaagctgttcaagcgtggcgcctacatcgtcaacaccgcccgcggcaagctgtgcgaccgcgatgccg
tggcacgtgcgctcgaatccggccggctggccggctatgccggcgacgtgtggttcccgcagccggcgccgaaggaccac
ccctggcggacgatgccctataacggcatgaccccgcacatctccggcaccacgctgaccgcgcaggcgcgttatgcggc
gggcacccgcgagatcctggagtgcttcttcgagggccgtccgatccgcgacgaatacctcatcgtgcagggcggcgctc
ttgccggcaccggcgcgcattcctactcgaagggcaatgccaccggcggttcggaagaggccgccaagttcaagaaggcg
gtctga

**Fig.1g**

Aminosäure-Sequenz der D221G-Mutante

MAKVLCVLYDDPVDGYPKTYARDDLPKIDHYPGGQILPTPKAIDFTPGQLLGSVSGELGLREYLESNGHTLVVTSDKDGPDSV
FERELVDADVVISQPFWPAYLTPERIAKAKNLKLALTAGIGSDHVDLQSAIDRNVTVAEVTYCNSISVAEHVVMMILSLVRNY
LPSHEWARKGGWNIADCVSHAYDLEAMHVGTVAAGRIGLAVLRRLAPFDVHLHYT**G**RHRLPESVEKELNLTWHATREDMYPVC
DVVTLNCPLHPETEHMINDETLKLFKRGAYIVNTARGKLCDRDAVARALESGRLAGYAGDVWFPQPAPKDHPWRTMPYNGMTP
HISGTTLTAQARYAAGTREILECFFEGRPIRDEYLIVQGGALAGTGAHSYSKGNATGGSEEAAKFKKAV*

**Fig.1h**

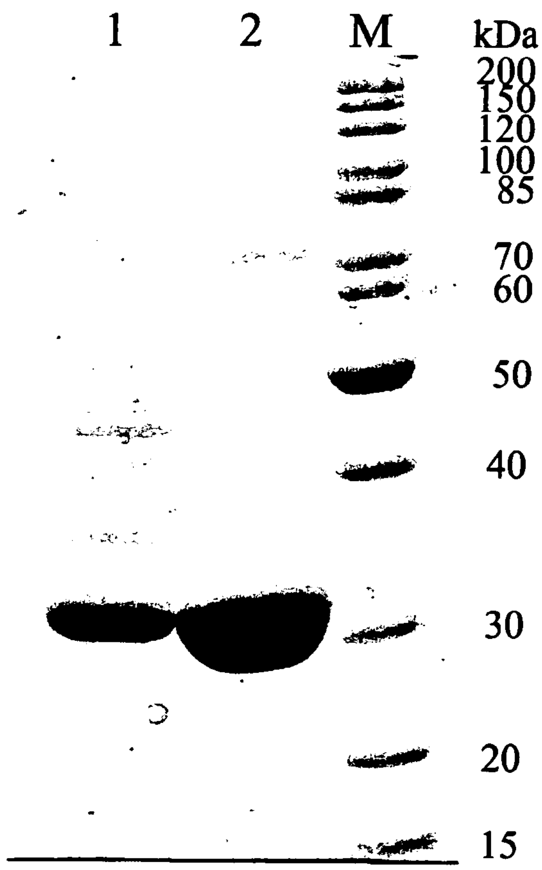


Fig. 2

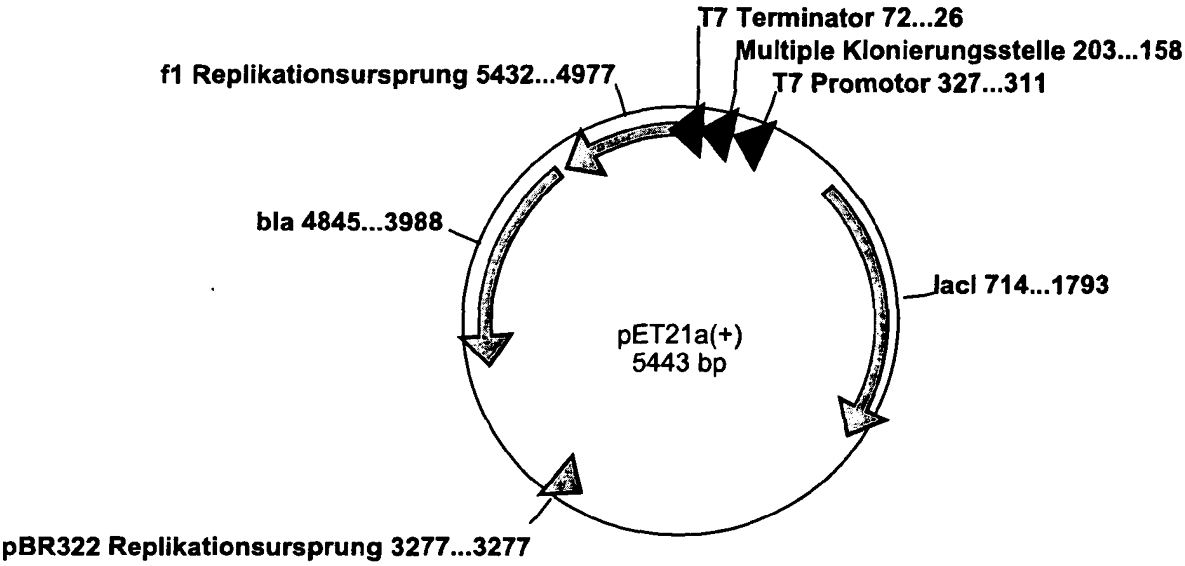
T7 Terminator 72...26
Multiple Klonierungsstelle 203...158
T7 Promotor 327...311
f1 Replikationsursprung 5432...4977
bla 4845...3988
lacI 714...1793
pET21a(+)
5443 bp
pBR322 Replikationsursprung 3277...3277

Fig. 3a

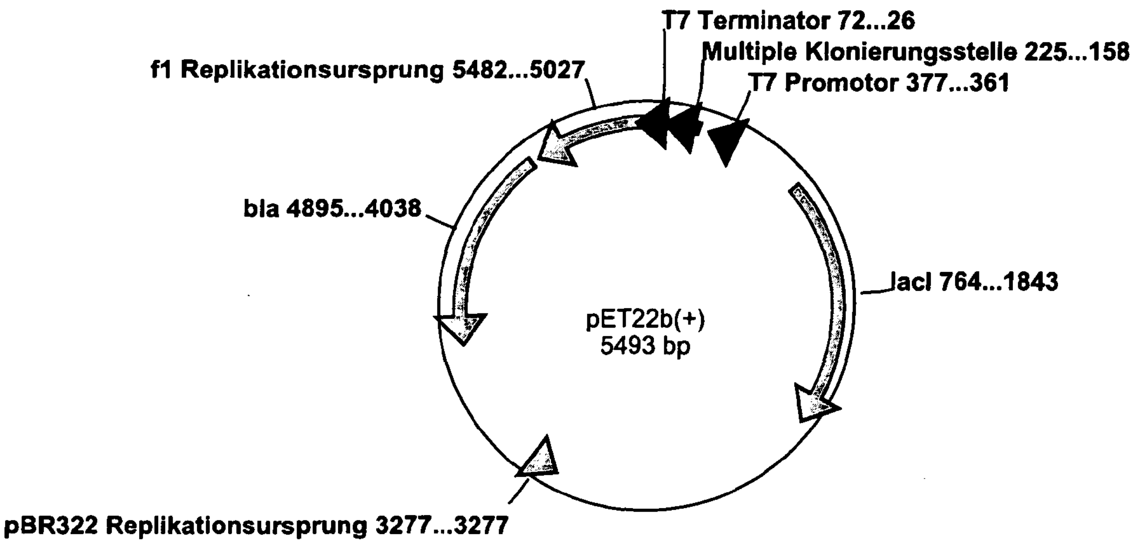
T7 Terminator 72...26
Multiple Klonierungsstelle 225...158
T7 Promotor 377...361
f1 Replikationsursprung 5482...5027
bla 4895...4038
lacI 764...1843
pET22b(+)
5493 bp
pBR322 Replikationsursprung 3277...3277

Fig. 3b

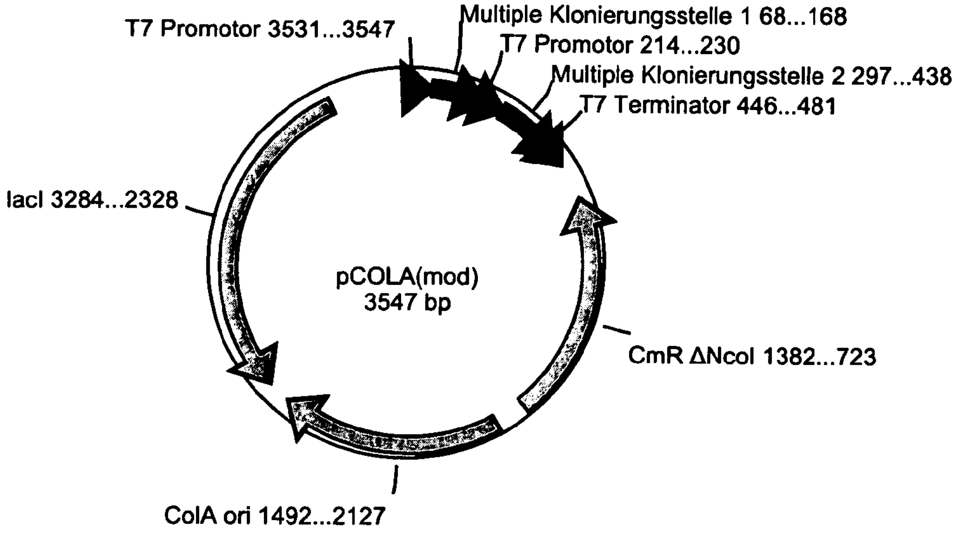
T7 Promotor 3531...3547
Multiple Klonierungsstelle 1 68...168
T7 Promotor 214...230
Multiple Klonierungsstelle 2 297...438
T7 Terminator 446...481
lacI 3284...2328
pCOLA(mod)
3547 bp
CmR ΔNcoI 1382...723
ColA ori 1492...2127

Fig. 3c

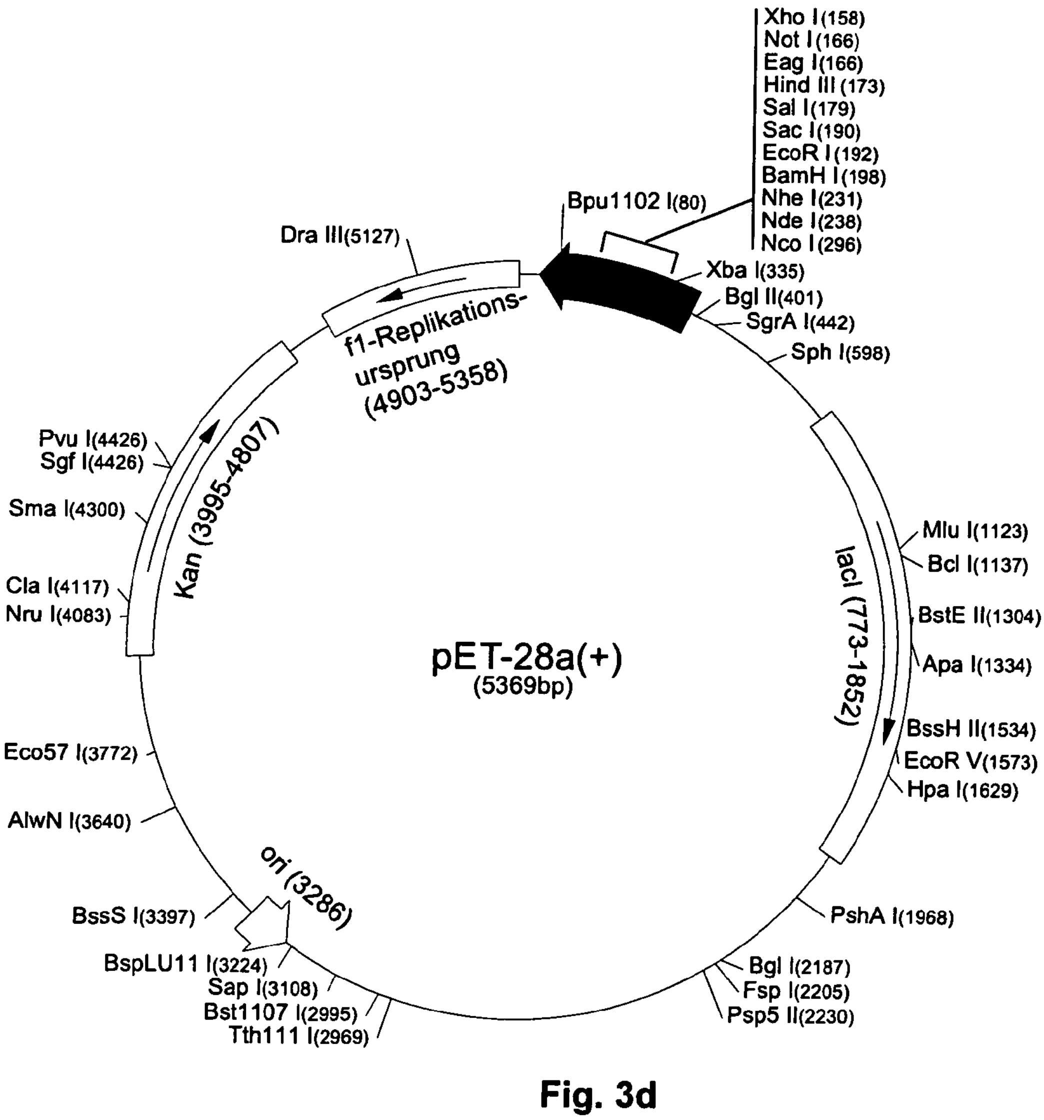
Xho I(158)
Not I(166)
Eag I(166)
Hind III (173)
Sal I(179)
Sac I(190)
EcoR I(192)
BamH I(198)
Nhe I(231)
Nde I(238)
Nco I(296)
Bpu1102 I(80)
Dra III(5127)
Xba I(335)
Bgl II(401)
SgrA I(442)
Sph I(598)
f1-Replikations-ursprung (4903-5358)
Pvu I(4426)
Sgf I(4426)
Sma I(4300)
Kan (3995-4807)
Cla I(4117)
Nru I(4083)
Mlu I(1123)
Bcl I(1137)
lacI (773-1852)
BstE II(1304)
Apa I(1334)
pET-28a(+)
(5369bp)
BssH II(1534)
EcoR V(1573)
Hpa I(1629)
Eco57 I(3772)
AlwN I(3640)
ori (3286)
BssS I(3397)
PshA I(1968)
BspLU11 I(3224)
Sap I(3108)
Bst1107 I(2995)
Tth111 I(2969)
Bgl I(2187)
Fsp I(2205)
Psp5 II(2230)

Fig. 3d

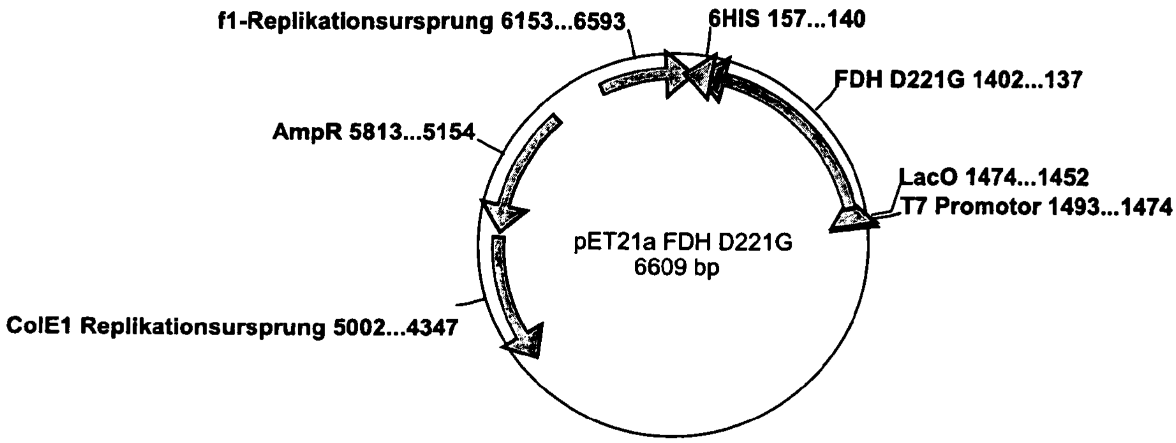
f1-Replikationsursprung 6153...6593
6HIS 157...140
FDH D221G 1402...137
AmpR 5813...5154
LacO 1474...1452
T7 Promotor 1493...1474
pET21a FDH D221G
6609 bp
ColE1 Replikationsursprung 5002...4347

**Fig. 4a**

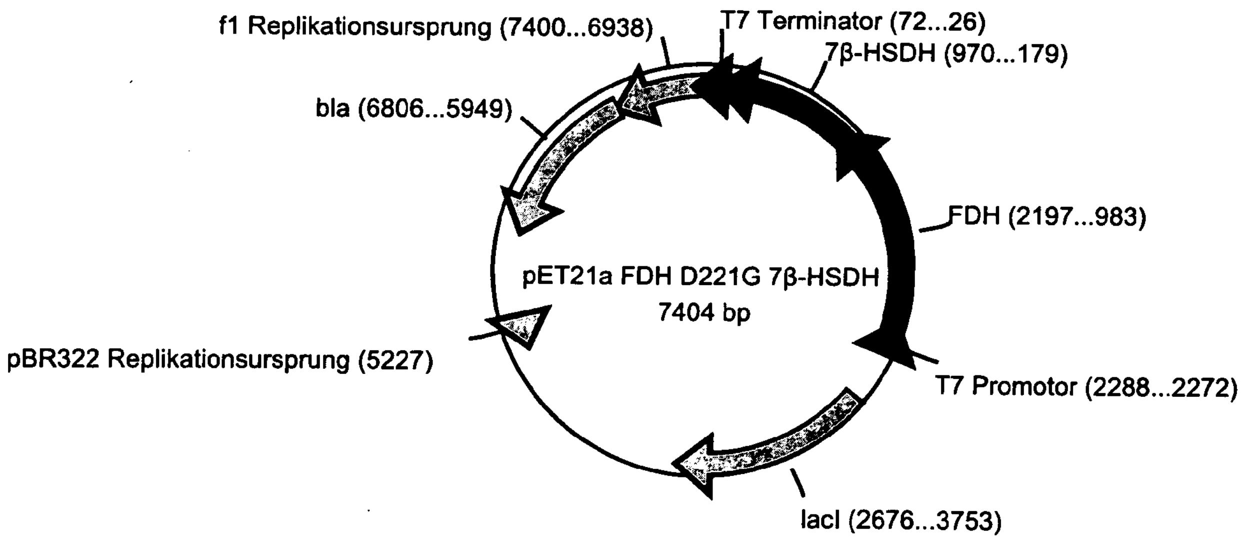
f1 Replikationsursprung (7400...6938)
T7 Terminator (72...26)
7β-HSDH (970...179)
bla (6806...5949)
FDH (2197...983)
pET21a FDH D221G 7β-HSDH
7404 bp
pBR322 Replikationsursprung (5227)
T7 Promotor (2288...2272)
lacI (2676...3753)

**Fig. 4b**

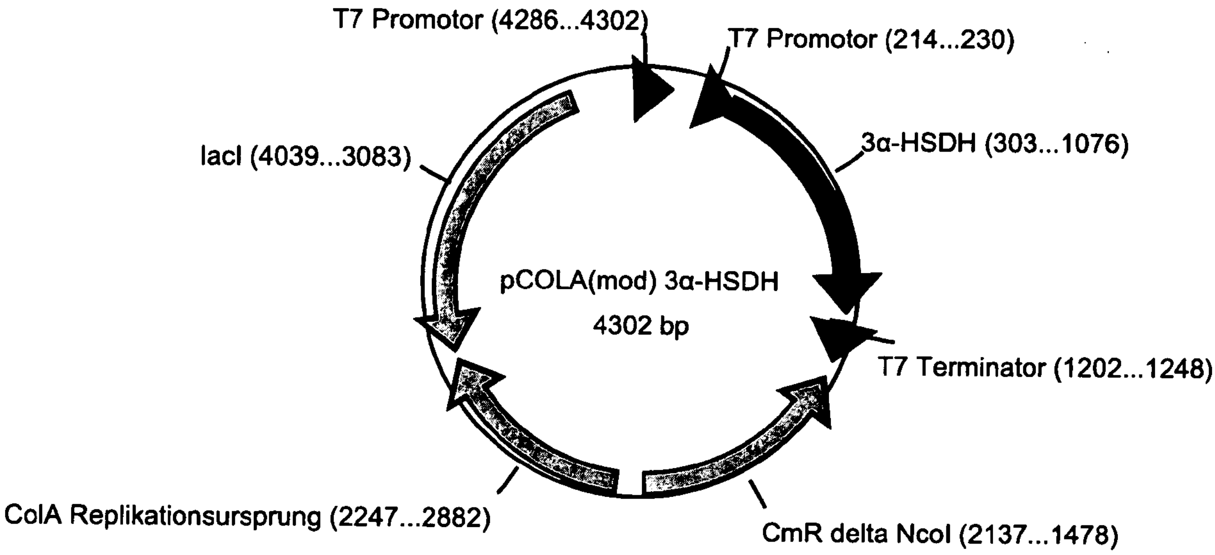
T7 Promotor (4286...4302)
T7 Promotor (214...230)
3α-HSDH (303...1076)
lacI (4039...3083)
pCOLA(mod) 3α-HSDH
4302 bp
T7 Terminator (1202...1248)
ColA Replikationsursprung (2247...2882)
CmR delta NcoI (2137...1478)

**Fig. 5**

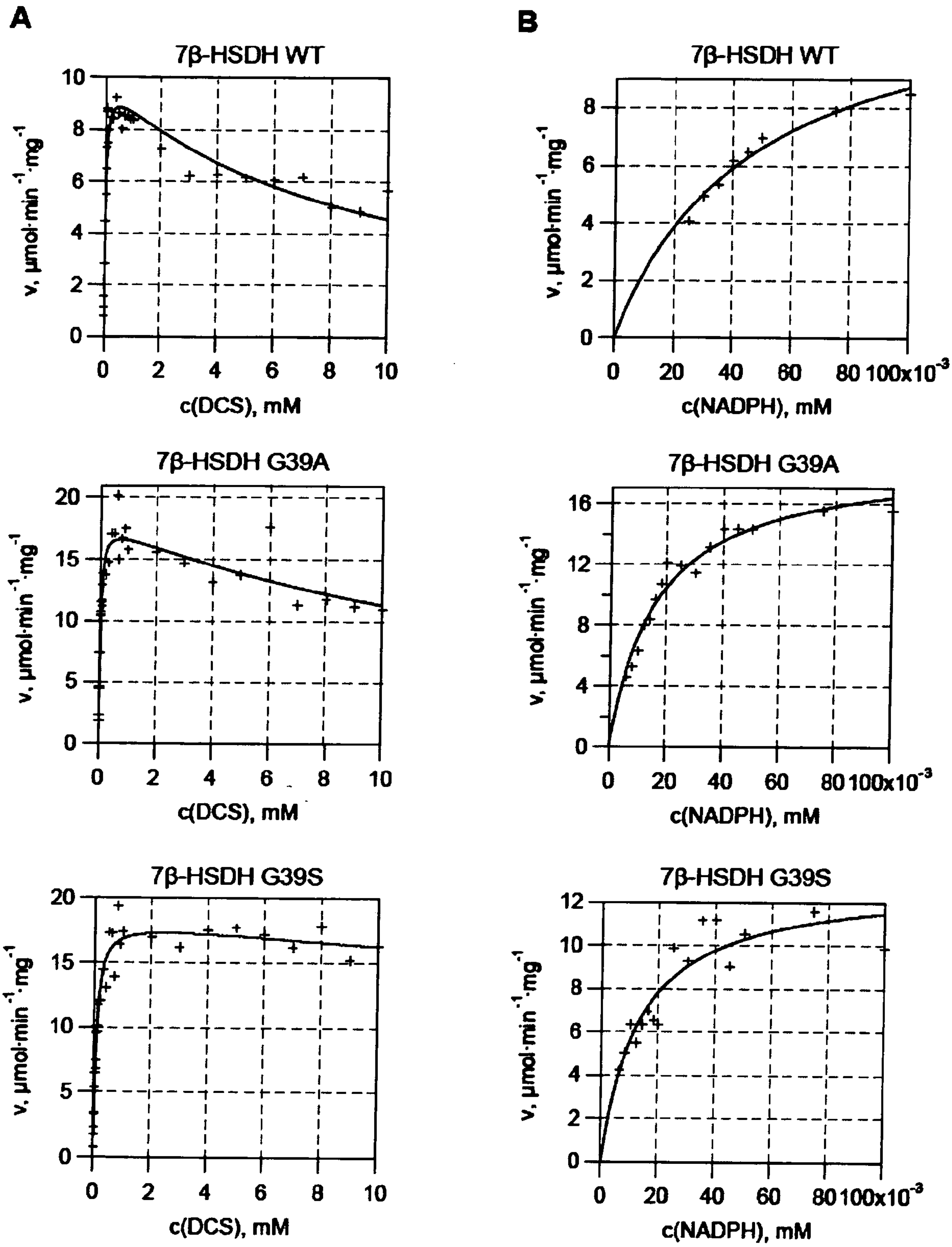
A
B
7β-HSDH WT
v, µmol·min⁻¹·mg⁻¹
c(DCS), mM
7β-HSDH WT
c(NADPH), mM
80 100x10⁻³
7β-HSDH G39A
c(DCS), mM
7β-HSDH G39A
c(NADPH), mM
7β-HSDH G39S
c(DCS), mM
7β-HSDH G39S
c(NADPH), mM

Fig. 6

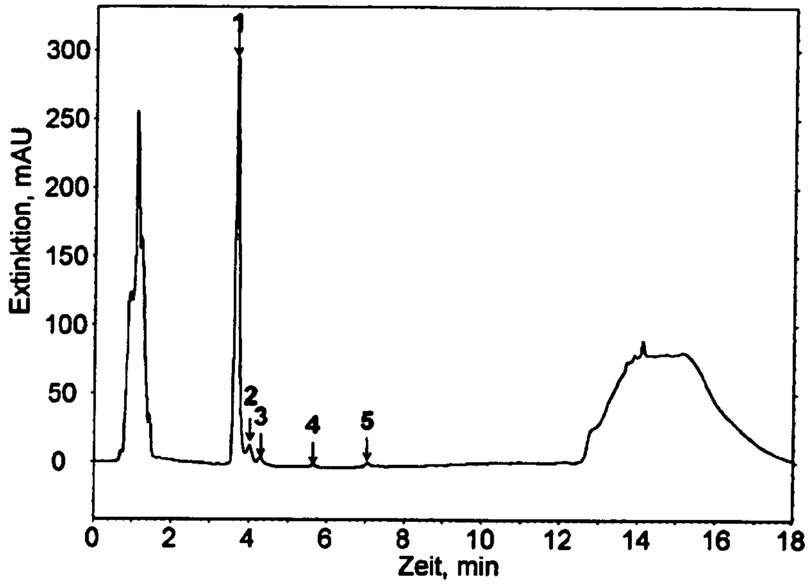
Extinktion, mAU
300
250
200
150
100
50
0
1
2
3
4
5
0
2
4
6
8
10
12
14
16
18
Zeit, min

Fig. 7

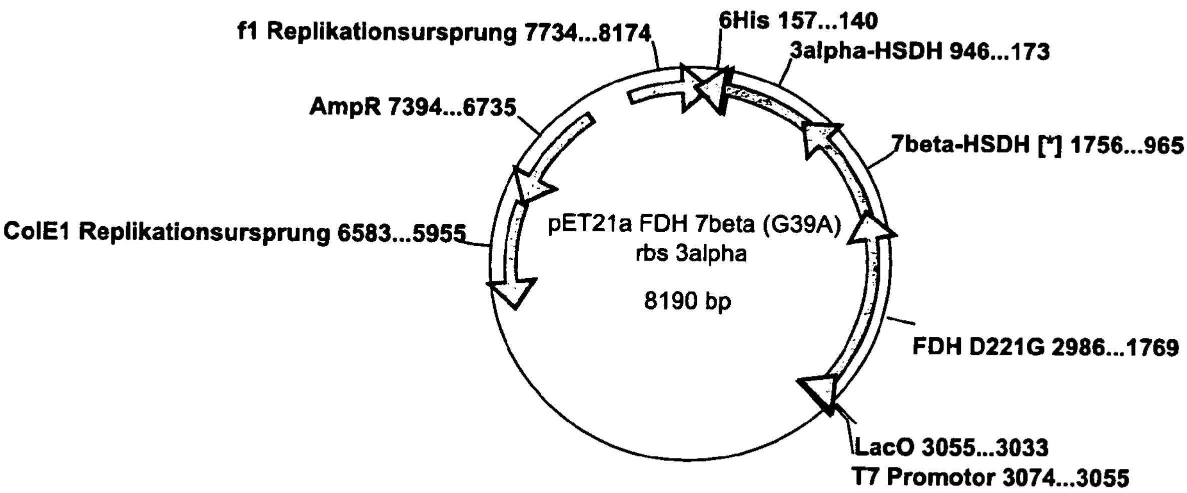
f1 Replikationsursprung 7734...8174
6His 157...140
3alpha-HSDH 946...173
AmpR 7394...6735
7beta-HSDH [*] 1756...965
ColE1 Replikationsursprung 6583...5955
pET21a FDH 7beta (G39A)
rbs 3alpha
8190 bp
FDH D221G 2986...1769
LacO 3055...3033
T7 Promotor 3074...3055

Fig. 8

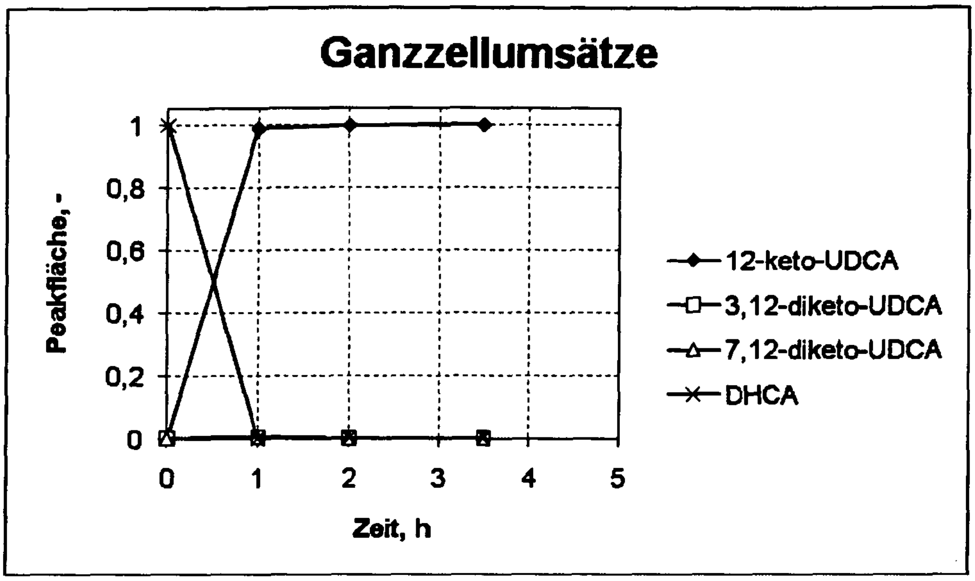


Fig. 9

```
7beta-HSDH Wildtyp      MNLREKYGEW GLILGATEGV GKAFCEKIAA GGMNVVMVGR REEKLNVLAG
7beta-HSDH G39D         MNLREKYGEW GLILGATEGV GKAFCEKIAA GGMNVVMV[illegible]R REEKLNVLAG
7beta-HSDH G39D R40L    MNLREKYGEW GLILGATEGV GKAFCEKIAA GGMNVVMV[illegible] REEKLNVLAG
7beta-HSDH G39D R40I    MNLREKYGEW GLILGATEGV GKAFCEKIAA GGMNVVMV[illegible] REEKLNVLAG
7beta-HSDH G39D R40V    MNLREKYGEW GLILGATEGV GKAFCEKIAA GGMNVVMV[illegible] REEKLNVLAG

7beta-HSDH Wildtyp      EIRETYGVET KVVRADFSQP GAAETVFAAT EGLDMGFMSY VACLHSFGKI
7beta-HSDH G39D         EIRETYGVET KVVRADFSQP GAAETVFAAT EGLDMGFMSY VACLHSFGKI
7beta-HSDH G39D R40L    EIRETYGVET KVVRADFSQP GAAETVFAAT EGLDMGFMSY VACLHSFGKI
7beta-HSDH G39D R40I    EIRETYGVET KVVRADFSQP GAAETVFAAT EGLDMGFMSY VACLHSFGKI
7beta-HSDH G39D R40V    EIRETYGVET KVVRADFSQP GAAETVFAAT EGLDMGFMSY VACLHSFGKI

7beta-HSDH Wildtyp      QDTPWEKHEA MINVNVVTFL KCFHHYMRIF AAQDRGAVIN VSSMTGISSS
7beta-HSDH G39D         QDTPWEKHEA MINVNVVTFL KCFHHYMRIF AAQDRGAVIN VSSMTGISSS
7beta-HSDH G39D R40L    QDTPWEKHEA MINVNVVTFL KCFHHYMRIF AAQDRGAVIN VSSMTGISSS
7beta-HSDH G39D R40I    QDTPWEKHEA MINVNVVTFL KCFHHYMRIF AAQDRGAVIN VSSMTGISSS
7beta-HSDH G39D R40V    QDTPWEKHEA MINVNVVTFL KCFHHYMRIF AAQDRGAVIN VSSMTGISSS

7beta-HSDH Wildtyp      PWNGQYGAGK AFILKMTEAV ACECEGTGVD VEVITLGTTL TPSLLSNLPG
7beta-HSDH G39D         PWNGQYGAGK AFILKMTEAV ACECEGTGVD VEVITLGTTL TPSLLSNLPG
7beta-HSDH G39D R40L    PWNGQYGAGK AFILKMTEAV ACECEGTGVD VEVITLGTTL TPSLLSNLPG
7beta-HSDH G39D R40I    PWNGQYGAGK AFILKMTEAV ACECEGTGVD VEVITLGTTL TPSLLSNLPG
7beta-HSDH G39D R40V    PWNGQYGAGK AFILKMTEAV ACECEGTGVD VEVITLGTTL TPSLLSNLPG

7beta-HSDH Wildtyp      GPQGEAVMKI ALTPEECVDE AFEKLGKELS VIAGQRNKDS VHDWKANHTE
7beta-HSDH G39D         GPQGEAVMKI ALTPEECVDE AFEKLGKELS VIAGQRNKDS VHDWKANHTE
7beta-HSDH G39D R40L    GPQGEAVMKI ALTPEECVDE AFEKLGKELS VIAGQRNKDS VHDWKANHTE
7beta-HSDH G39D R40I    GPQGEAVMKI ALTPEECVDE AFEKLGKELS VIAGQRNKDS VHDWKANHTE
7beta-HSDH G39D R40V    GPQGEAVMKI ALTPEECVDE AFEKLGKELS VIAGQRNKDS VHDWKANHTE

7beta-HSDH Wildtyp      DEYIRYMGSF YRD
7beta-HSDH G39D         DEYIRYMGSF YRD
7beta-HSDH G39D R40L    DEYIRYMGSF YRD
7beta-HSDH G39D R40I    DEYIRYMGSF YRD
7beta-HSDH G39D R40V    DEYIRYMGSF YRD
```

Fig. 10

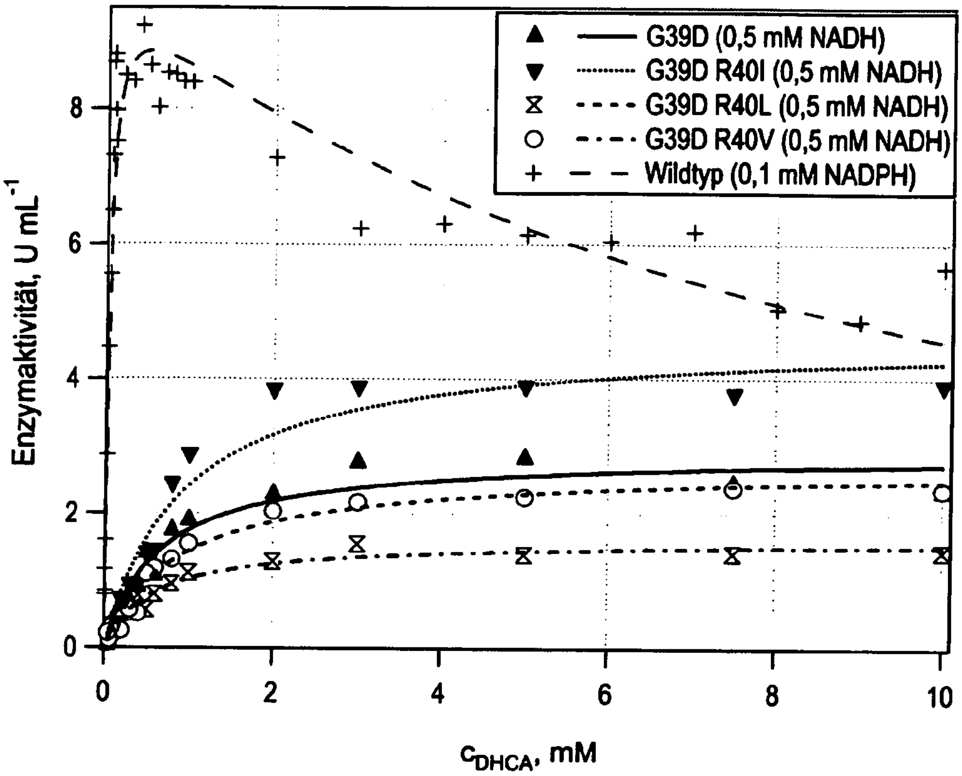


**Fig. 11**

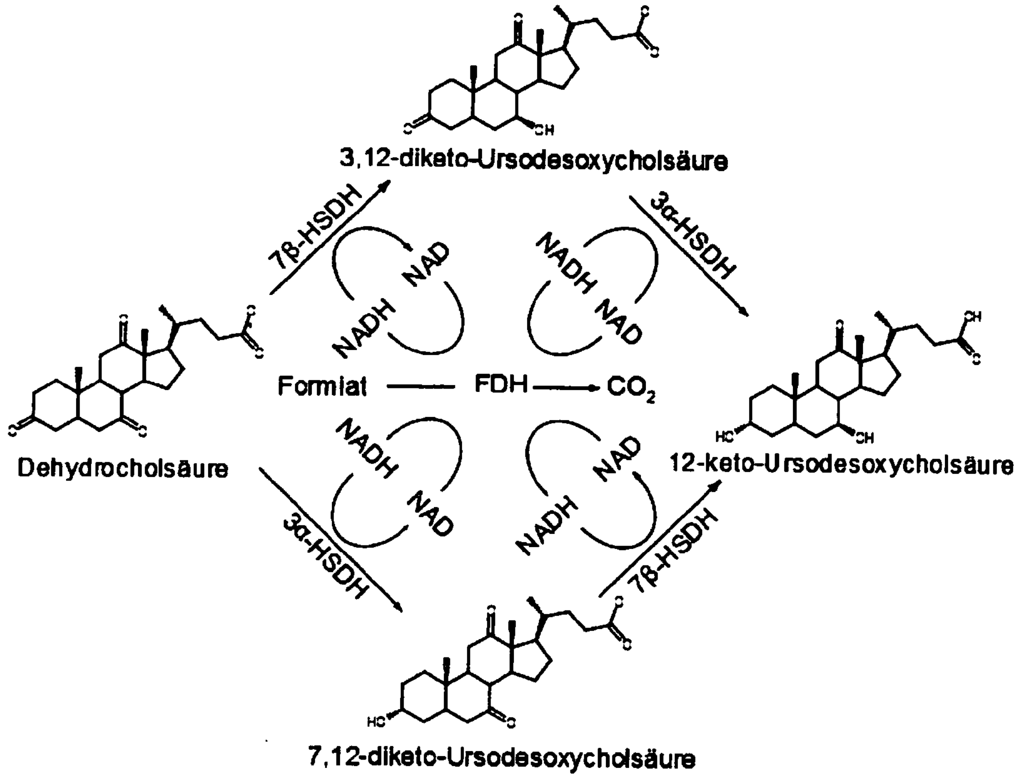


3,12-diketo-Ursodesoxycholsäure

Dehydrocholsäure

12-keto-Ursodesoxycholsäure

7,12-diketo-Ursodesoxycholsäure

**Fig.12**

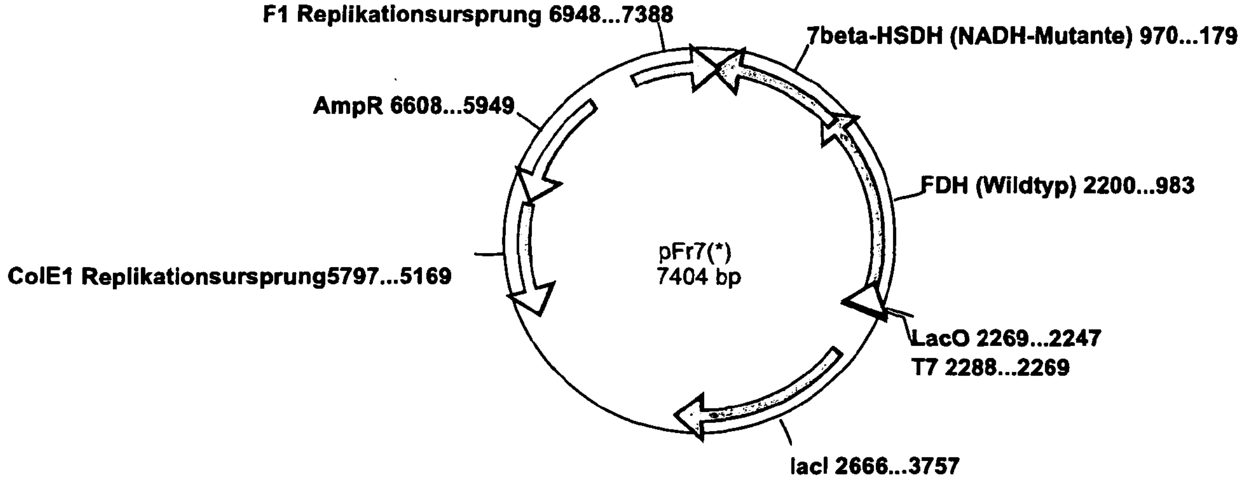
F1 Replikationsursprung 6948...7388
7beta-HSDH (NADH-Mutante) 970...179
AmpR 6608...5949
FDH (Wildtyp) 2200...983
ColE1 Replikationsursprung5797...5169
pFr7(*)
7404 bp
LacO 2269...2247
T7 2288...2269
lacI 2666...3757

Fig. 13

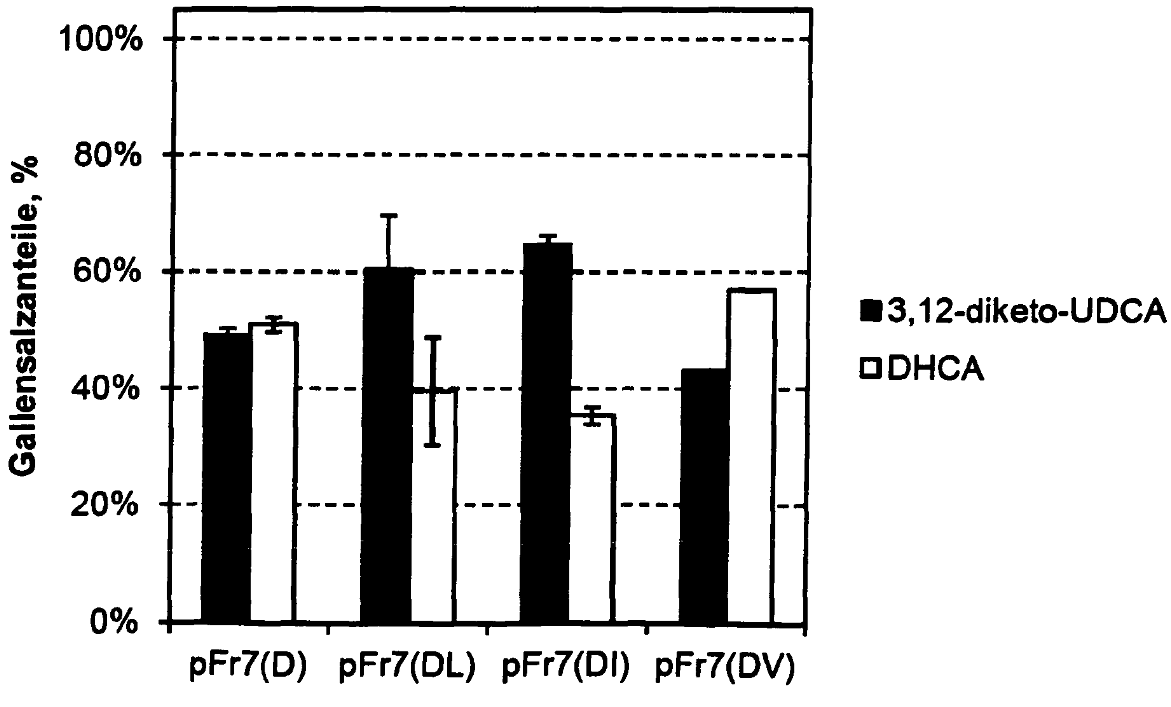
100%
80%
60%
40%
20%
0%
Gallensalzanteile, %
pFr7(D)
pFr7(DL)
pFr7(DI)
pFr7(DV)
3,12-diketo-UDCA
DHCA

Fig. 14

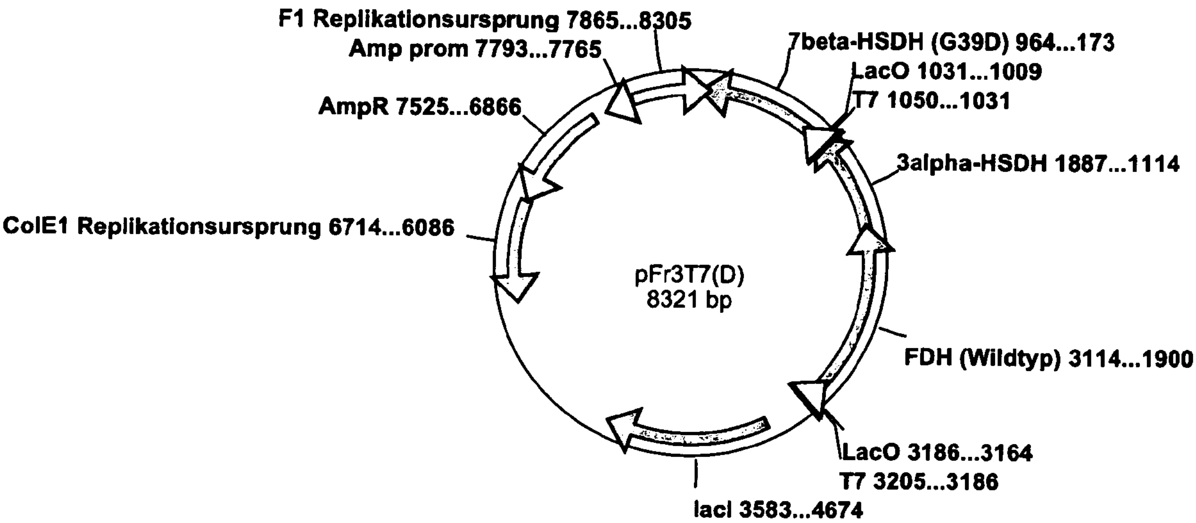
F1 Replikationsursprung 7865...8305
Amp prom 7793...7765
AmpR 7525...6866
ColE1 Replikationsursprung 6714...6086
7beta-HSDH (G39D) 964...173
LacO 1031...1009
T7 1050...1031
3alpha-HSDH 1887...1114
pFr3T7(D)
8321 bp
FDH (Wildtyp) 3114...1900
LacO 3186...3164
T7 3205...3186
lacI 3583...4674

Fig. 15

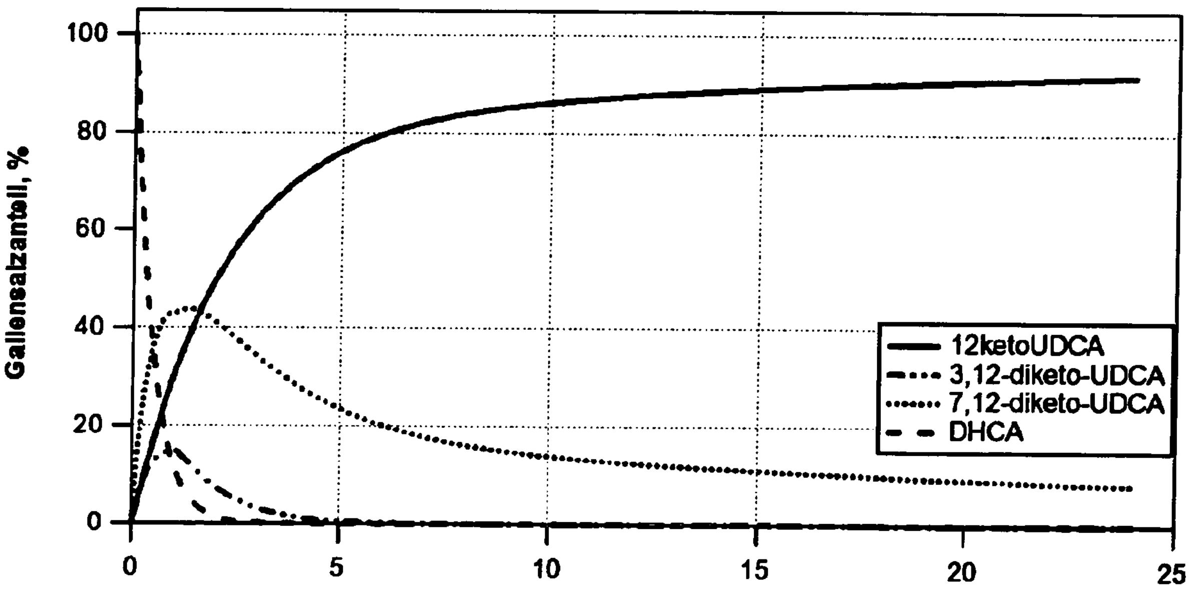
Gallensalzanteil, %
100
80
60
40
20
0
0
5
10
15
20
25
12ketoUDCA
3,12-diketo-UDCA
7,12-diketo-UDCA
DHCA

Fig. 16

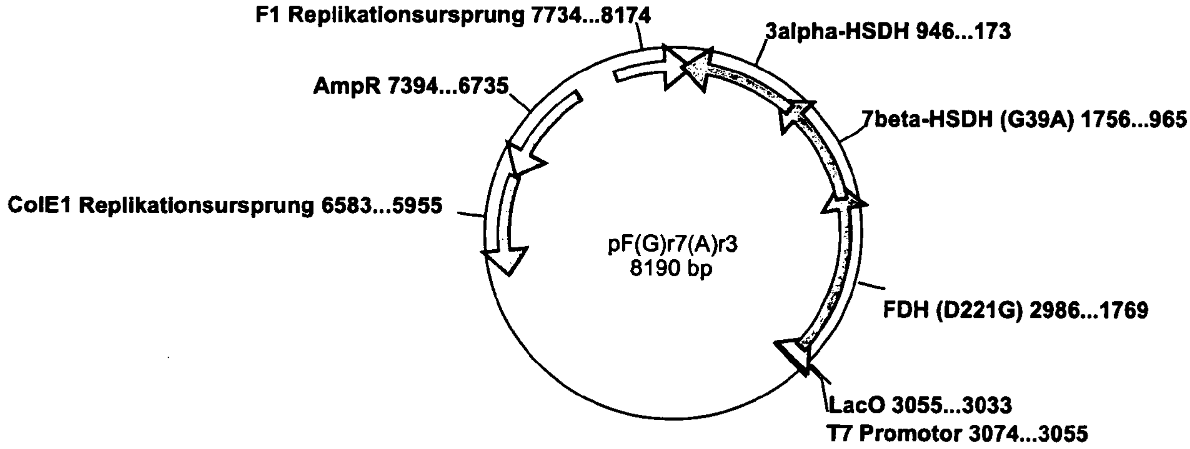


Fig. 17a

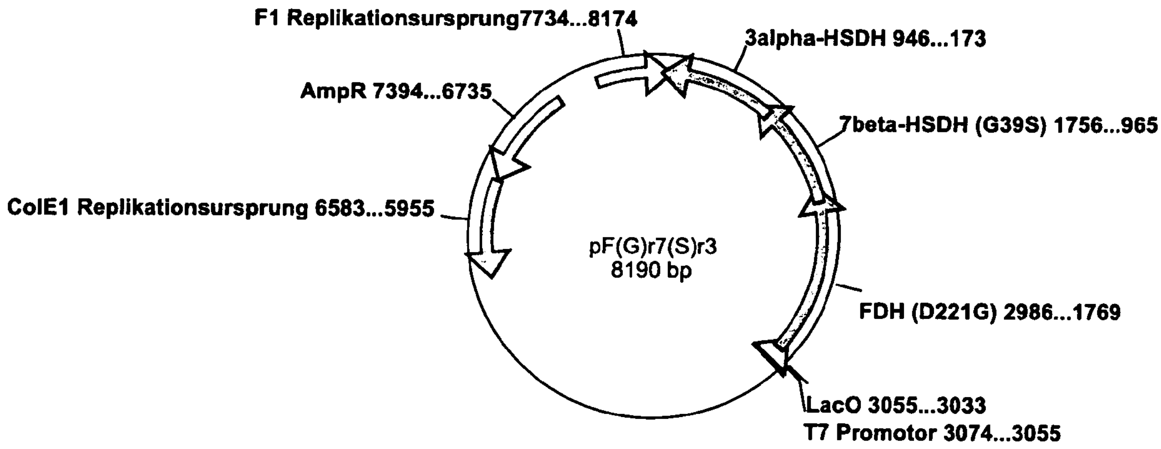


Fig. 17b

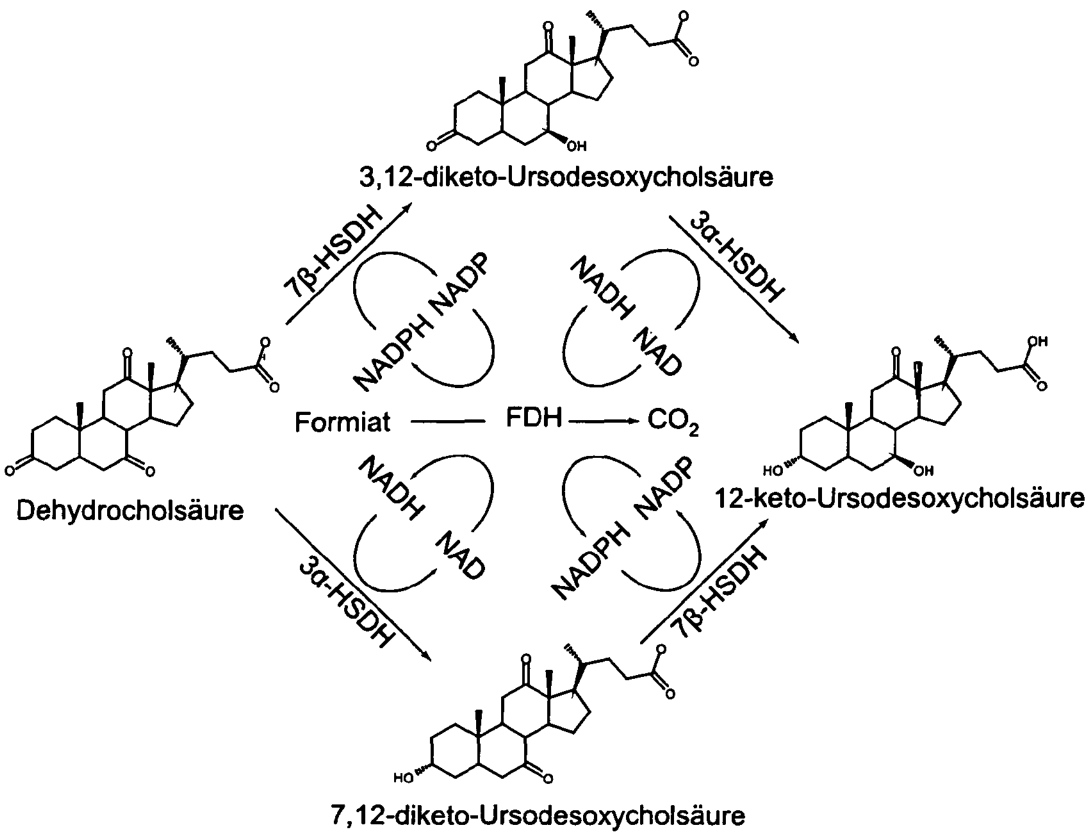
3,12-diketo-Ursodesoxycholsäure
Dehydrocholsäure
12-keto-Ursodesoxycholsäure
7,12-diketo-Ursodesoxycholsäure
7β-HSDH
3α-HSDH
3α-HSDH
7β-HSDH
NADPH
NADP
NADH
NAD
NADH
NAD
NADPH
NADP
Formiat
FDH
$CO_2$

Fig. 18

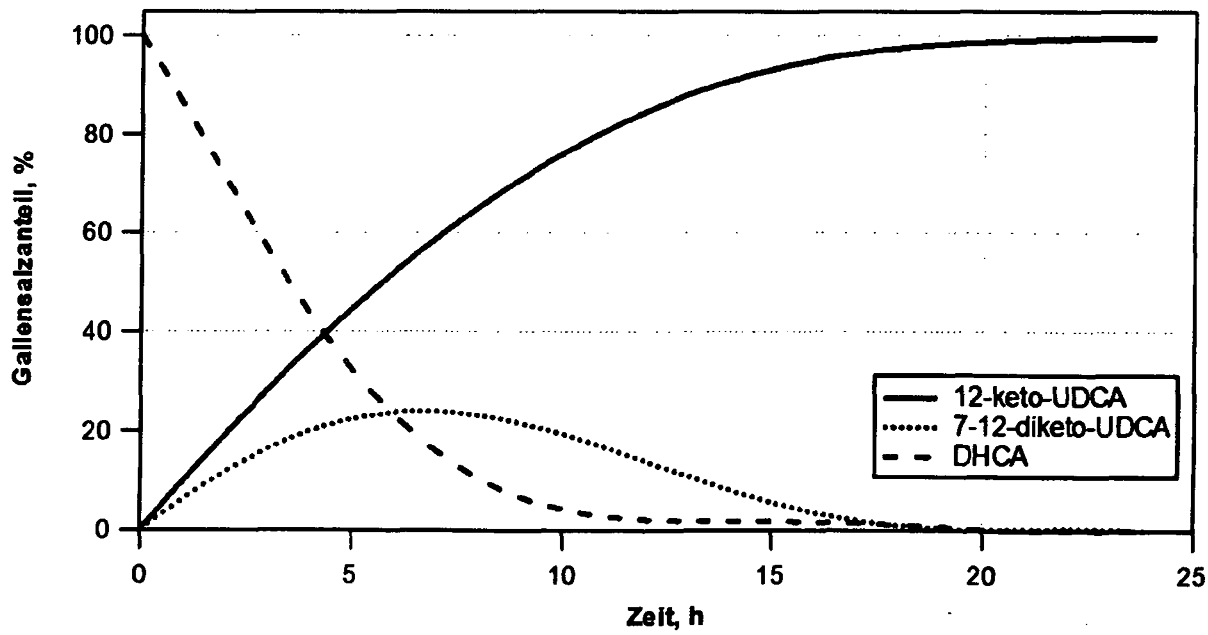
Gallensalzanteil, %
Zeit, h
0
20
40
60
80
100
0
5
10
15
20
25
12-keto-UDCA
7-12-diketo-UDCA
DHCA

Fig. 19

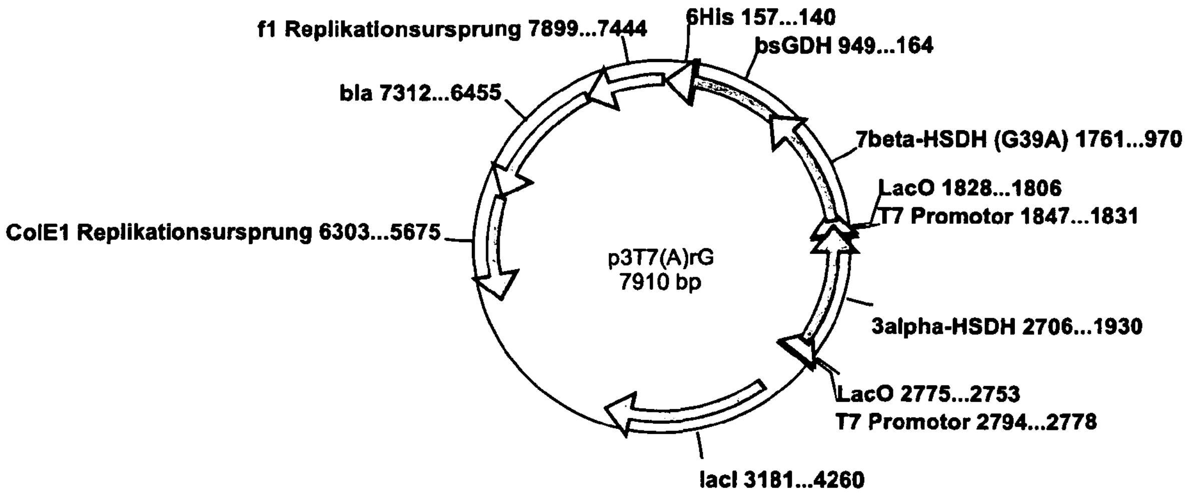

f1 Replikationsursprung 7899...7444
6His 157...140
bsGDH 949...164
bla 7312...6455
7beta-HSDH (G39A) 1761...970
LacO 1828...1806
T7 Promotor 1847...1831
ColE1 Replikationsursprung 6303...5675
p3T7(A)rG
7910 bp
3alpha-HSDH 2706...1930
LacO 2775...2753
T7 Promotor 2794...2778
lacI 3181...4260


Fig. 20

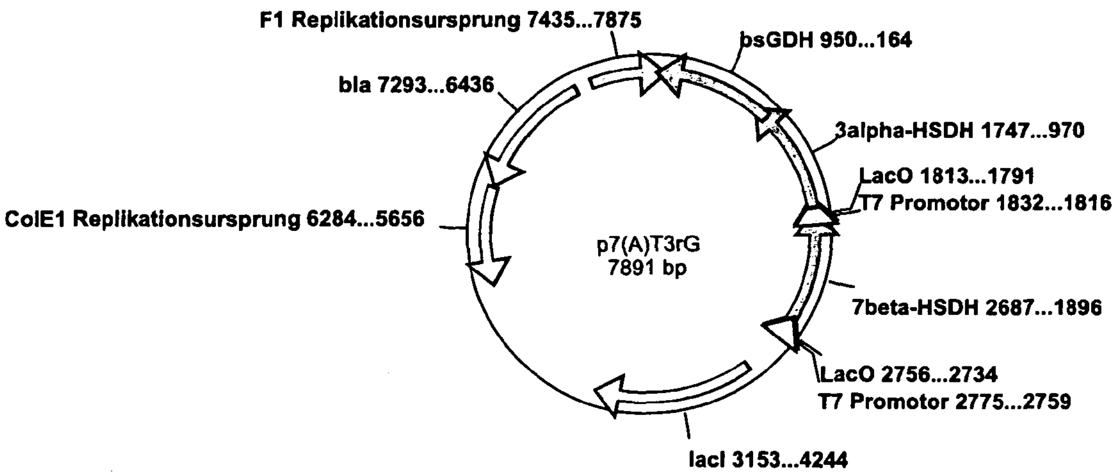

F1 Replikationsursprung 7435...7875
bsGDH 950...164
bla 7293...6436
3alpha-HSDH 1747...970
LacO 1813...1791
T7 Promotor 1832...1816
ColE1 Replikationsursprung 6284...5656
p7(A)T3rG
7891 bp
7beta-HSDH 2687...1896
LacO 2756...2734
T7 Promotor 2775...2759
lacI 3153...4244


Fig. 21

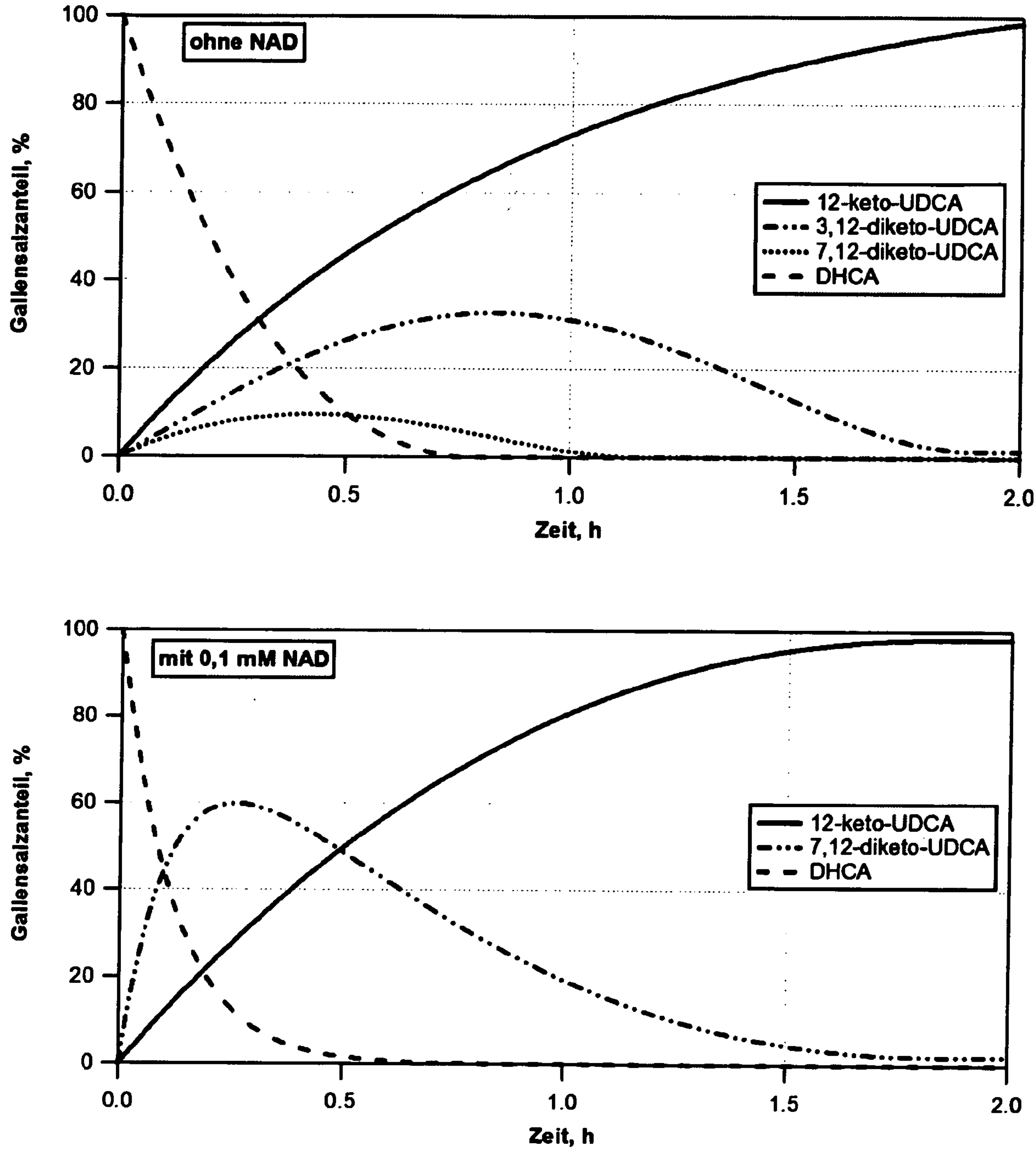
ohne NAD
Gallensalzanteil, %
100
80
60
40
20
0
0.0
0.5
1.0
1.5
2.0
Zeit, h
12-keto-UDCA
3,12-diketo-UDCA
7,12-diketo-UDCA
DHCA
mit 0,1 mM NAD
Gallensalzanteil, %
100
80
60
40
20
0
0.0
0.5
1.0
1.5
2.0
Zeit, h
12-keto-UDCA
7,12-diketo-UDCA
DHCA

**Fig. 22**

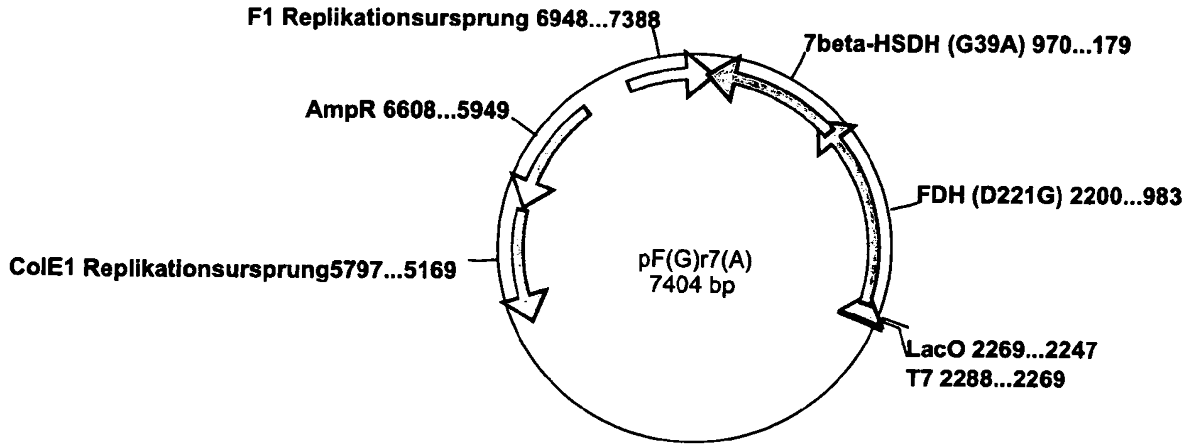


Fig. 23a

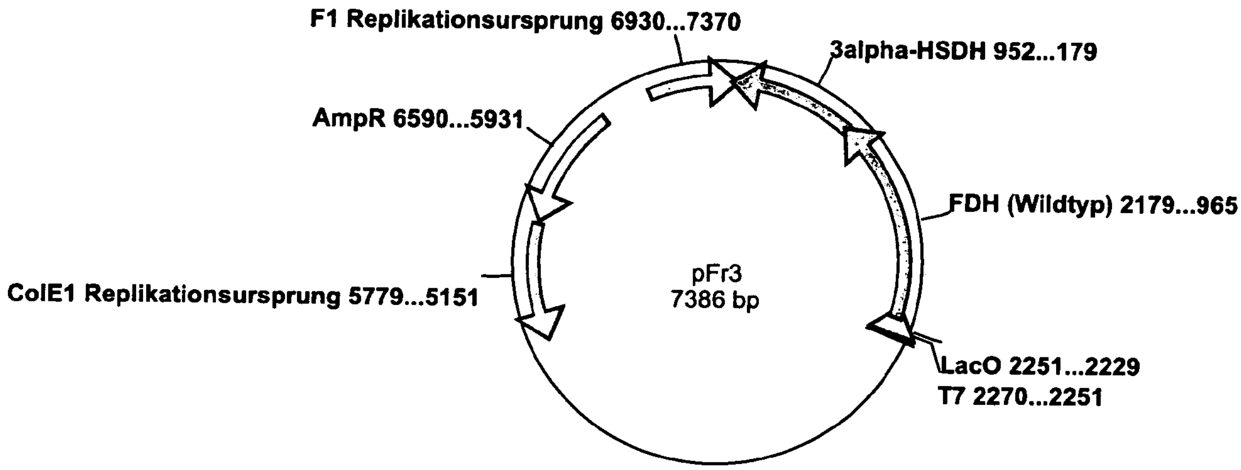


Fig. 23b

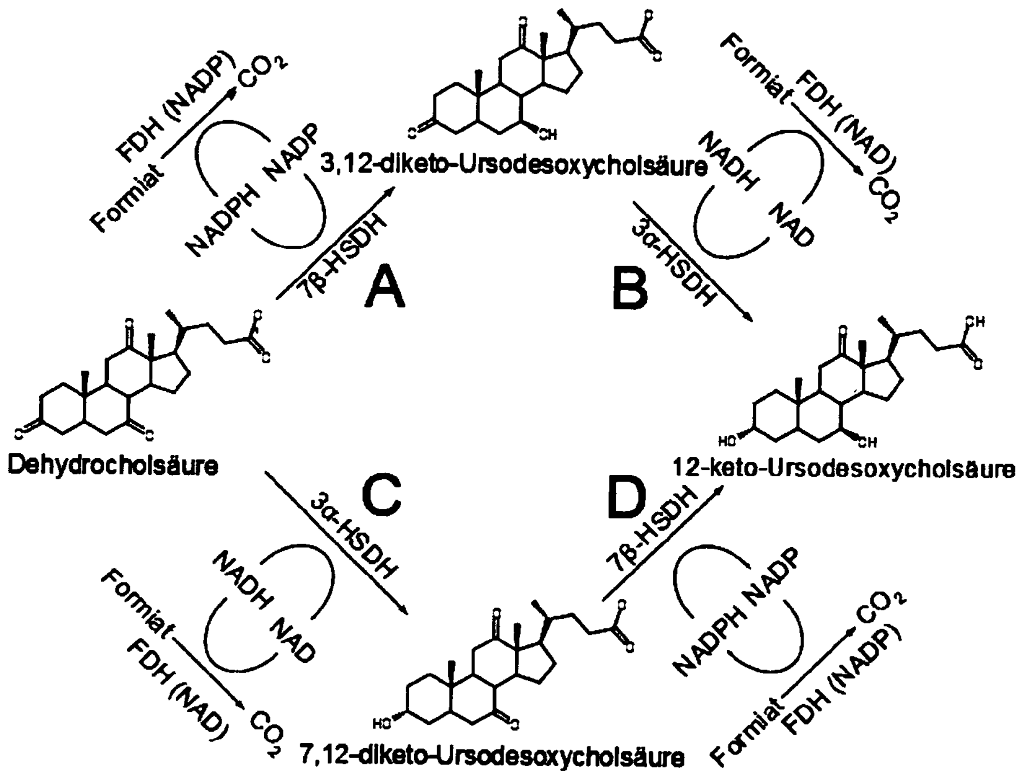


3,12-diketo-Ursodesoxycholsäure

Dehydrocholsäure

12-keto-Ursodesoxycholsäure

7,12-diketo-Ursodesoxycholsäure

Fig. 24

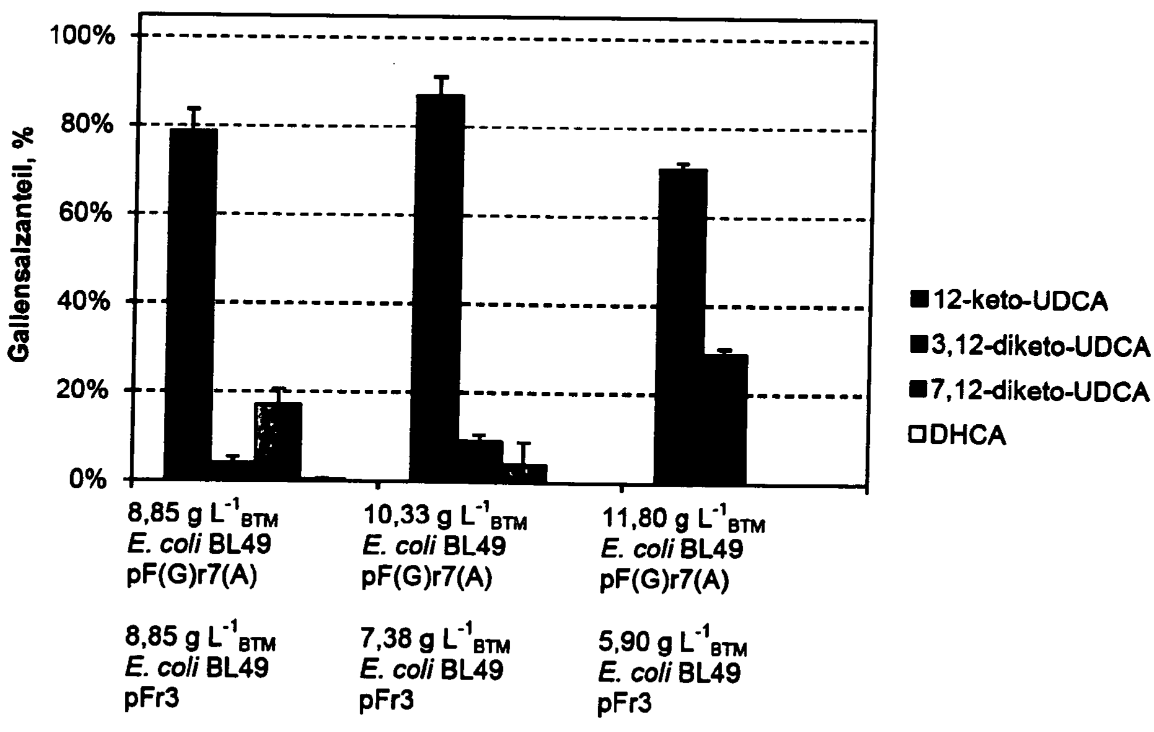


Fig. 25

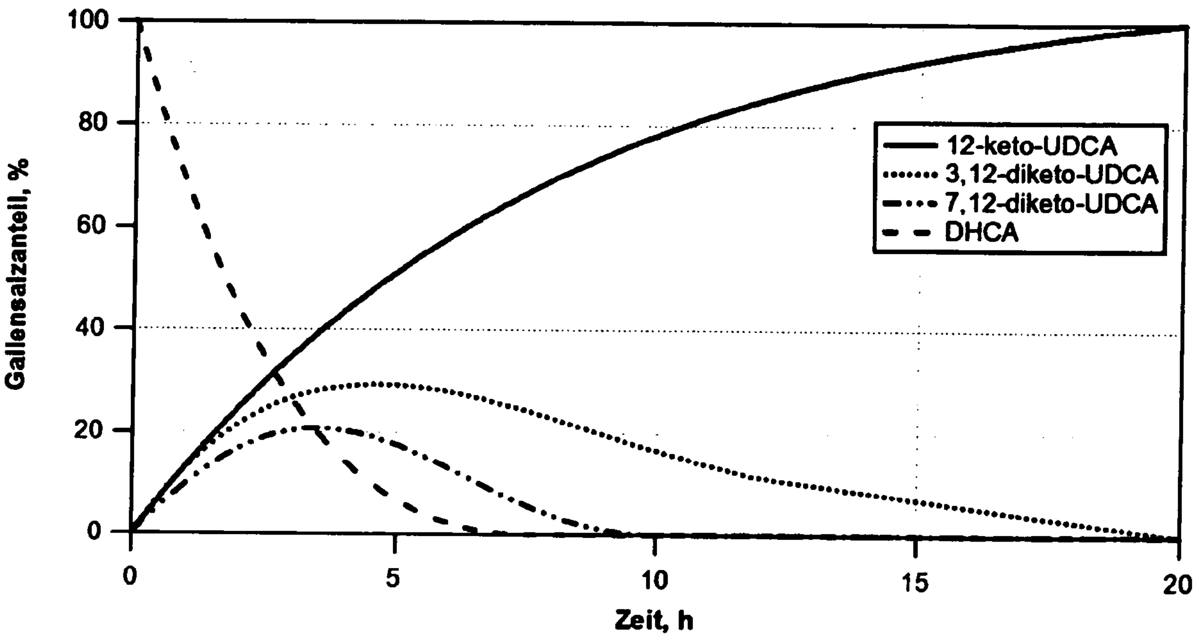
100
80
60
40
20
0
Gallensalzanteil, %
0
5
10
15
20
Zeit, h
12-keto-UDCA
3,12-diketo-UDCA
7,12-diketo-UDCA
DHCA

Fig. 26

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2009002190 W
- EP 2010068576 W
- EP 1149849 A
- EP 1069183 A
- DE OS100193773 A
- EP 10164003 A


### In der Beschreibung aufgeführte Nicht-Patentliteratur


- **MONTI, D. et al.** One-Pot Multienzymatic Synthesis of 12-Ketoursodeoxycholic Acid: Subtle Cofactor Specificities Rule the Reaction Equilibria of Five Biocatalysts Working in a Row. *Advanced Synthesis & Catalysis,* 2009
- **ZHU, D. et al.** Enzymatic enantioselective reduction of -ketoesters by a thermostable 7 -hydroxysteroid dehydrogenase from Bacteroides fragilis. *Tetrahedron,* 2006, vol. 62 (18), 4535-4539
- **MACDONALD, I.A. ; P.D. ROACH.** Bile induction of 7 alpha- and 7 beta-hydroxysteroid dehydrogenases in Clostridium absonum. *Biochim Biophys Acta,* 1981, vol. 665 (2), 262-9
- **HIRANO, S. ; N. MASUDA.** Characterization of NADP-dependent 7 beta-hydroxysteroid dehydrogenases from Peptostreptococcus productus and Eubacterium aerofaciens. *Appl Environ Microbiol,* 1982, vol. 43 (5), 1057-63
- **TISHKOV et al.** *Biomolecular Engineering,* 2006, vol. 23, 89-110
- **S HIRANO ; N MASUDA.** Characterization of NADP-dependent 7 beta-hydroxysteroid dehydrogenases from Peptostreptococcus productus and Eubacterium aerofaciens. *Appl Environ Microbiol.,* 1982
- **J E PAWLOWSKI ; M HUIZINGA ; T M PENNING.** Cloning and sequencing of the cDNA for rat liver 3 alphahydroxysteroid/dihydrodiol dehydrogenase. *The Journal of Biological Chemistry,* 15. Mai 1991, vol. 266, 8820-8825
- **R.K. SCOPES.** Protein Purification. Springer Verlag, 1982
- **PEARSON ; LIPMAN.** *Proc. Natl. Acad, Sci. (USA),* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **LKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500, http://www.ebi.ac.uk/Tools/clustalw/index.html#
- Phosphoamiditmethode. Voet, Voet. Wiley Press, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **SAMBROOK, J. ; FRITSCH, E.F. ; MANIATIS, T.** Molecular Cloning (A Laboratory Manual). Cold Spring Harbor Laboratory Press, 1989, 9.31-9.57
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- In vitro mutagenesis protocols. Humana Press, 1996
- **KEGLER-EBO DM ; DOCKTOR CM ; DIMAIO D.** *Nucleic Acids Res,* 1994, vol. 22, 1593

- **BARETTINO D ; FEIGENBUTZ M ; VALCÄREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- An efficient random mutagenesis technique u-sing an E.coli mutator strain. **GREENER A ; CALLAHAN M ; JERPSETH B.** In vitro mutagenesis protocols. Humana Press, 1996
- **STEMMER WPC.** *Nature,* 1994, vol. 370, 389
- **STEMMER WPC.** *Proc Natl Acad Sci USA,* 1994, vol. 91, 10747
- **REETZ MT ; JAEGER K-E.** *Topics Curr Chem,* 1999, vol. 200, 31
- Methods for optimizing industrial enzymes by directed evolution. **ZHAO H ; MOORE JC ; VOLKOV AA ; ARNOLD FH.** Manual of industrial microbiology and biotechnology. American Society for Microbiology, 1999
- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- Cloning Vectors. Elsevier, 1985
- Current Protocols in Molecular Biology. Wiley Interscience, 1997
- **SAMBROOK et al.** Molecular Cloning: A Laboratory Manual. Cold Spring Harbor Laboratory Press, 1989
- **TERPE, K.** *Appl. Microbiol. Biotechnol.,* 2006, vol. 72, 211-222
- Einführung in die Bioverfahrenstechnik. **CHMIEL.** Bioprozeßtechnik. Gustav Fischer Verlag, 1991, vol. 1
- **STORHAS.** Bioreaktoren und periphere Einrichtungen. Vieweg Verlag, 1994
- Manual of Methods für General Bacteriology. American Society für Bacteriology, 1981
- Applied Microbiol. Physiology, A Practical Approach. IRL Press, 1997, 53-73
- **COOPER, F. G.** Biochemische Arbeitsmethoden. Verlag Walter de Gruyter
- **SCOPES, R.** Protein Purification. Springer Verlag
- **HARLOW, E. ; LANE, D.** Antibodies: A Laboratory Manual. Cold Spring Harbor (N.Y.) Press, 1988
- Immobilization of Enzymes. **J. LALONDE ; A. MARGOLIN.** Enzyme Catalysis in Organic Synthesis. Wiley-VCH, 2002, vol. III, 991-1032
- **WILMS et al.** *BIOTECHNOLOGY AND BIOENGI-NEERING,* 2001, vol. 73 (2), 95-103
- **OPPERMANN et al.** *J Biochem,* 1996, vol. 241 (3), 744-749
- Current Protocols in Molecular Biology. John Wiley &Sons, 1993
- **KRIEGLER.** Gene Transfer and Expression, A Laboratory Manual. Stockton Press, 1990
- **THOMPSON et al.** *Nucleic Acid Research,* 1997, vol. 25, 4876-82
- **CLAMP et al.** *Bioinformatics,* 2004, vol. 20, 426-7
- **JORNVALL, H. ; B. PERSSON ; M. KROOK ; S. ATRIAN ; R. GONZALEZ-DUARTE ; J. JEFFERY ; D. GHOSH.** Short-chain dehydrogenases/reductases (SDR). *Biochemistry,* 1995, vol. 34, 6003-13
- **PERSSON, B. ; M. KROOK ; H. JORNVALL.** Characteristics of short-chain alcohol dehydrogenases and related enzymes. *Eur J Biochem,* 1991, vol. 200, 537-43
- **MOBUS, E. ; E. MASER.** Molecular cloning, overexpression, and characterization of steroid-inducible 3alpha-hydroxysteroid dehydrogenase/carbonyl reductase from Comamonas testosteroni. A novel member of the short-chain dehydrogenase/reductase superfamily. *J Biol Chem,* 1998, vol. 273 (47), 30888-96
- **SANCHIS J ; FERNÁNDEZ L ; CARBALLEIRA JD ; DRONE J ; GUMULYA Y ; HÖBENREICH H ; KAHAKEAW D ; KILLE S ; LOHMER R ; PEYRALANS JJ.** Improved PCR method for the creation of saturation mutagenesis libraries in directed evolution: application to difficult-to-amplify templates. *Appl Microbiol Biotechnol.,* November 2008, vol. 81 (2), 387-97